# EUROPEAN PATENT APPLICATION

(11) **EP 3 770 255 A1**
(43) Date of publication of application: **27.01.2021**
(21) Application number: 20187954.1
(22) Date of filing: 16.09.2009
(51) Int. Cl.: C12N 15/10, C12N 15/11, C12Q 1/68, C12Q 1/6804, C12Q 1/6806, C12Q 1/6809, C12Q 1/6888, C12Q 1/6883

(54) **PROCESSES AND COMPOSITIONS FOR METHYLATION-BASED ENRICHMENT OF FETAL NUCLEIC ACID FROM A MATERNAL SAMPLE USEFUL FOR NON-INVASIVE PRENATAL DIAGNOSES**

(30) Priority: 16.09.2008 US 192264 P
(62) Divisional of application: 16173137.7
(71) Applicant: Sequenom, Inc., San Diego, CA 92121 (US); Sequenom Center For Molecular Medicine, Grand Rapids, MI 49503 (US)
(72) Inventor: EHRICH, Mathias, San Diego, CA 92109 (US); NYGREN, Anders, Olof, Herman, San Diego, CA 92122 (US)
(74) Representative: Vossius & Partner Patentanwälte Rechtsanwälte mbB

(57) **Abstract**

The present invention relates to a method for determining the presence or absence of a fetal aneuploidy using fetal DNA from a maternal sample, wherein the maternal sample comprises differentially methylated maternal and fetal DNA, comprising a) selectively digesting the maternal DNA in a maternal sample using one or more methylation sensitive restriction enzymes thereby enriching the fetal DNA; b) determining the amount of fetal DNA from a target chromosome; c) determining the amount of fetal DNA from a reference chromosome; and d) comparing the amount of fetal DNA from step b) to step c), wherein a biologically or statistically significant difference between the amount of target and reference fetal DNA is indicative of the presence of a fetal aneuploidy.

SEQUENCE LISTING

<110> SEQUENOM, INC.
      SEQUENOM CENTER FOR MOLECULAR MEDICINE

<120> PROCESSES AND COMPOSITIONS FOR METHYLATION-BASED ENRICHMENT OF
      FETAL NUCLEIC ACID FROM A MATERNAL SAMPLE USEFUL FOR NON-
      INVASIVE PRENATAL DIAGNOSES

<130> T1450 EP/2

<150> US 61/192,264
<151> 2008-09-16

<160> 179

<170> PatentIn version 3.5

<210> 1
<211> 305
<212> DNA
<213> Homo sapiens

<400> 1
cagcaggcgc gctcccggcg aatctgcctg aatcgccgtg aatgcggtgg ggtgcagggc      60

aggggctggt tttctcagcc ggtcttggct tttctctttc tctcctgctc caccagcagc     120

ccctccgcgg gtcccatggg ctccgcgctc agaacagccc ggaaccaggc gccgctcgcc     180

gctcgctggg ggccacccgc ctctccccgg aacagcctcc cgcgggcctc ttggcctcgc     240

actggcgccc tcacccacac atcgtccctt tatccgctca gacgctgcaa agggccttct     300

gtctc                                                                 305


<210> 2
<211> 336
<212> DNA
<213> Homo sapiens

<400> 2
gctttggatt tatcctcatt ggctaaatcc ctcctgaaac atgaaactga aacaaagccc      60

tgaaccccct caggctgaaa agacaaaccc cgcctgaggc cgggtcccgc tccccacctg     120

gagggaccca attctgggcg ccttctggcg acggtccctg ctagggacgc tgcgctctcc     180

gagtgcgagt tttcgccaaa ctgataaagc acgcagaacc gcaatcccca aactaacact     240

gaacccggac ccgcgatccc caaactgaca agggacccgg aacagcgacc cccaaaccga     300

cacgggactc gggaaccgct atctccaaag ggcagc                               336


<210> 3
<211> 139
<212> DNA
<213> Homo sapiens

<400> 3
tttccacaac agggagccag cattgaggcg cccagatggc atctgctgga aatcacgggc      60

cgctggtgaa gcaccacgcc ttacccgacg tggggaggtg atcccccacc tcatcccacc 120

cccttctgtc tgtctcctt 139

<210> 4
<211> 292
<212> DNA
<213> Homo sapiens

<400> 4
gctggacaag gagcgctcac tgtagctctg ctgtggattg tgttggggcg aagagatggg 60

taagaggtca aagtcgtagg attctggcga ccgcctacca agggattggg tccacagcac 120

agaggtctga tcgcttcctt ctctgctctg ccacctccag acagcagctc taaccagctg 180

cccagcagca agaggatgcg cacggctttc accagcacgc agctgctaga gctggagcgc 240

gagttcgctt ctaatatgta cctgtcccgc ctacgtcgca tcgagatcgc ga 292

<210> 5
<211> 190
<212> DNA
<213> Homo sapiens

<400> 5
tgcctgacac tgaccccagg cgcagccagg aggggctttg tgcgggagag ggagggggac 60

cccagcttgc ctggggtcca cgggactctc ttcttcctag ttcactttct tgctaaggcg 120

aaggtcctga ggcaggacga gggctgaact gcgctgcaat cgtccccacc tccagcgaaa 180

cccagttgac 190

<210> 6
<211> 706
<212> DNA
<213> Homo sapiens

<400> 6
tcggcggaga gacctcgagg agagtatggg gaaaggaatg aatgctgcgg agcgcccctc 60

tgggctccac ccaagcctcg gaggcgggac ggtgggctcc gtcccgaccc cttaggcagc 120

tggaccgata cctcctggat cagaccccac aggaagactc gcgtggggcc cgatatgtgt 180

acttcaaact ctgagcggcc accctcagcc aactggccag tggatgcgaa tcgtgggccc 240

tgaggggcga gggcgctcgg aactgcatgc ctgtgcacgg tgccgggctc tccagagtga 300

gggggccgta aggagatctc caaggaagcc gaaaaaagca gccagttggg cttcgggaaa 360

gacttttctg caaaggaagt gatctggtcc cagaactcca gggttgaccc cagtacctga 420

cttctccggg agctgtcagc tctcctctgt tcttcgggct tggcgcgctc ctttcataat 480

ggacagacac cagtggcctt caaaaggtct ggggtggggg aacggaggaa gtggccttgg 540

gtgcagagga agagcagagc tcctgccaaa gctgaacgca gttagcccta cccaagtgcg 600

cgctggctcg gcatatgcgc tccagagccg gcaggacagc ccggccctgc tcaccccgag 660

gagaaatcca acagcgcagc ctcctgcacc tccttgcccc agagac 706

<210> 7
<211> 325
<212> DNA
<213> Homo sapiens

<400> 7
agatcccggt gcatttaaag gccggcgtga tctgcaccac gtacctatct cggattctca 60

gtttcacttc gctggtgtct gccaccatct ttaccacatc ccggtagcta catttgtcta 120

ccgcttgagc caccagcgtc tgaaacctgg accggatttt gcgcgccgag aggtagccgg 180

aggcggtaat gaattccacc cagagggaca tgctcctctt gcgcccgtcg ctcaacttca 240

gcaccgcgca gccgggcagt gagccatcgt ccacgaagtt gaacaccccc atttggttga 300

gataaagcac cacttcaaat tcggt 325

<210> 8
<211> 663
<212> DNA
<213> Homo sapiens

<400> 8
actatgcctt gagggtcaaa acgtctggat ttcctgatcg atgctgtcgt cgctgtccac 60

ggagctactg tcgccgtcag agcgggaagg cacgttcagg gagtagaagc gtgggcttgc 120

agaaagggac ctgttgctgc cttacatggg ggccggcagg gtagtcttgg aaatgcccaa 180

gattgcttcc gcgcgcgtca gttcagcgga cgtgtctgcc tggcacgagg accgttctac 240

aaactcgttc ctggaagccg ggctcgctgg aggcggagct ttggtttcct tcgggagctt 300

gtggggaatg gtcagcgtct aggcaccccg ggcaagggtc tgtggccttg gtggccactg 360

gcttcctcta gctgggtgtt ttcctgtggg tctcgcgcaa ggcacttttt tgtggcgctg 420

cttgtgctgt gtgcggggtc aggcgtcctc tctcctcccg gcgctgggcc ctctggggca 480

ggtccccgtt ggcctccttg cgtgtttgcc gcagctagta cacctggatg gcctcctcag 540

tgccgtcgtt gctgctggag tctgacgcct cgggcgcctg cgccgcactt gtgacttgct 600

ttccccttct cagggcgcca gcgctcctct tgaccccgct tttattctgt ggtgcttctg 660

aag 663

<210> 9
<211> 1985
<212> DNA
<213> Homo sapiens

<400> 9
gcaagtcggg tagctaccgg gtgctggaga actccgcacc gcacctgctg gacgtggacg 60

cagacagcgg gctcctctac accaagcagc gcatcgaccg cgagtccctg tgccgccaca 120

atgccaagtg ccagctgtcc ctcgaggtgt tcgccaacga caaggagatc tgcatgatca 180

aggtagagat ccaggacatc aacgacaacg cgccctcctt ctcctcggac cagatcgaaa 240

tggacatctc ggagaacgct gctccgggca cccgcttccc cctcaccagc gcacatgacc 300

ccgacgccgg cgagaatggg ctccgcacct acctgctcac gcgcgacgat cacggcctct 360

ttggactgga cgttaagtcc cgcggcgacg gcaccaagtt cccagaactg gtcatccaga 420

aggctctgga ccgcgagcaa cagaatcacc atacgctcgt gctgactgcc ctggacggtg 480

gcgagcctcc acgttccgcc accgtacaga tcaacgtgaa ggtgattgac tccaacgaca 540

acagcccggt cttcgaggcg ccatcctact tggtggaact gcccgagaac gctccgctgg 600

gtacagtggt catcgatctg aacgccaccg acgccgatga aggtcccaat ggtgaagtgc 660

tctactcttt cagcagctac gtgcctgacc gcgtgcggga gctcttctcc atcgacccca 720

agaccggcct aatccgtgtg aagggcaatc tggactatga ggaaaacggg atgctggaga 780

ttgacgtgca ggcccgagac ctggggccta accctatccc agcccactgc aaagtcacgg 840

tcaagctcat cgaccgcaac gacaatgcgc cgtccatcgg tttcgtctcc gtgcgccagg 900

gggcgctgag cgaggccgcc cctcccggca ccgtcatcgc cctggtgcgg gtcactgacc 960

gggactctgg caagaacgga cagctgcagt gtcgggtcct aggcggagga gggacggcgg 1020

gcggcggggg cctgggcggg cccgggggtt ccgtcccctt caagcttgag gagaactacg 1080

acaacttcta cacggtggtg actgaccgcc cgctggaccg cgagacacaa gacgagtaca 1140

acgtgaccat cgtggcgcgg gacgggggct ctcctcccct caactccacc aagtcgttcg 1200

cgatcaagat tctagacgag aacgacaacc cgcctcggtt caccaaaggg ctctacgtgc 1260

ttcaggtgca cgagaacaac atcccgggag agtacctggg ctctgtgctc gcccaggatc 1320

ccgacctggg ccagaacggc accgtatcct actctatcct gccctcgcac atcggcgacg 1380

tgtctatcta cacctatgtg tctgtgaatc ccacgaacgg ggccatctac gccctgcgct 1440

cctttaactt cgagcagacc aaggcttttg agttcaaggt gcttgctaag gactcggggg 1500

cgcccgcgca cttggagagc aacgccacgg tgagggtgac agtgctagac gtgaatgaca 1560

acgcgccagt gatcgtgctc cccacgctgc agaacgacac cgcggagctg caggtgccgc 1620

gcaacgctgg cctgggctat ctggtgagca ctgtgcgcgc cctagacagc gacttcggcg 1680

agagcgggcg tctcacctac gagatcgtgg acggcaacga cgaccacctg tttgagatcg 1740

acccgtccag cggcgagatc cgcacgctgc accctttctg ggaggacgtg acgcccgtgg 1800

tggagctggt ggtgaaggtg accgaccacg gcaagcctac cctgtccgca gtggccaagc 1860

tcatcatccg ctcggtgagc ggatcccttc ccgagggggt accacgggtg aatggcgagc 1920

agcaccactg ggacatgtcg ctgccgctca tcgtgactct gagcactatc tccatcatcc 1980

tccta 1985

<210> 10
<211> 213
<212> DNA
<213> Homo sapiens

<400> 10
atgcgccctc tgcaccccta gagccagaag acgctaggtg ggctgcgcgc tctgccaggc 60

gaaggctgga gcgcagacgg caaagccgcg cgtttcagcc gtggtcgggt ccgcaggacc 120

tgggcgtggg gacaccacca ggcaggagca gaggcaggac tgggacgcca aaagctgaga 180

atcctcgatg cccgcgcgag agccccgtgt tat 213

<210> 11
<211> 1558
<212> DNA
<213> Homo sapiens

<400> 11
ttctggaaac cgggccccac ttgcaggccc ggccaccttg ggttctggtg gccgaagccg 60

gagctgtgtt tctcgcagac tcggggagct acattgtgcg taggcaattg tttagtttga 120

aaggaggcac atttcaccac gcagccagcg ccctgcatgc aggagaagcc cccagggccc 180

agggtcggct ggctttagag gccacttagg ttgttttaag cacatgtgaa agggcagaca 240

gcaggggagc aggatatggg taagatcttc gggtctcaga acaggggctg cccttgggct 300

gtcccggcgc cctgggctct gacactgaag ggtggaatgg aggaaggaat ggagaaagga 360

cggtggaact ttcgcttccc ctctgggccg ccttcccagg gtcatgcctg agctgctttg 420

atcccagtgt cgcgcatctt ggtccgctac ctcccaggcg atagctactg ggctcctcgc 480

tggcctcact gggggccatc ccgggcagtg gcctgccctc cgaggcccgc gggacccagc 540

ccagagctga ggttggagtt ctccgggcca cgttccgggt cgcttaggct cggagatttc 600

ccggagaccg tcgtcctccc tttctgcttg gcactgcgga gctccctcgg cctctctcct 660

cctctggtcc ctaaggcccg gagtggttgg cggtactggg gcccgtcgtc atctctgctt 720

ctaaggcatt cagactgggc tccagctggg accggcagag gaggttctca aggaaactgg 780

tgggaaatat agttttcttt cgtctggtcg tttaatttaa atgcaacttc ccttggggac 840

attttcctgg acgttaacca gaccaccttg agatgtcgtt gatgacctag agacccagat 900

gatgcgtccc aggaaagttc actgctgact attgtcactc ttggcgttat atctatagat 960

atagacctat gtacatatct ccaccctgat ctctccgtgg acatgaaacc cacctacctt 1020

gtgaaagccc tacgggtgac acatgactac tacgtctctg tcccaacagg ggctgggcct 1080

cccctgccta atagttgcca ggagtttcgc agcccaagtg aataatgtct tatggctgaa 1140

cgtggccaag gactcctgtg atttaggtcc caggaggagc agagacgtcc ccgccccgcc 1200

tgggccctgc cgcattcaaa gctggaagaa ggcgctgatc agagaagggg cttccaggtc 1260

ctgggttaga acaacaacaa acaaacgaaa ctccacaaca gacacgcctg cccatgaccc 1320

cacgcaagga cataggaagt tctgtcgcct tcctgctccg cggatagccg cctgccgtct 1380

gctgccacca gaacgcacgg acgctcgggg tggaggtagt caatgggcag caggggaccc 1440

ccagccccca caagcgcggc tccgaggacc tggaagcggg tgcctgtcgc tctccgcagg 1500

ctccgctctg cctccaggag caagatcccc aaaagggtct ggaagctgtg gagaaaac 1558

<210> 12
<211> 1264
<212> DNA
<213> Homo sapiens

<400> 12
ttttttaaac acttcttttc cttctcttcc tcgttttgat tgcaccgttt ccatctgggg 60

gctagaggag caaggcagca gccttcccag ccagcccttg ttggcttgcc atcgtccatc 120

tggcttataa aagtttgctg agcgcagtcc agagggctgc gctgctcgtc ccctcggctg 180

gcagaagggg gtgacgctgg gcagcggcga ggagcgcgcc gctgcctctg gcgggctttc 240

ggcttgaggg gcaaggtgaa gagcgcaccg gccgtggggt ttaccgagct ggatttgtat 300

gttgcaccat gccttcttgg atcggggctg tgattcttcc cctcttgggg ctgctgctct 360

ccctccccgc cggggcggat gtgaaggctc ggagctgcgg agaggtccgc caggcgtacg 420

gtgccaaggg attcagcctg gcggacatcc cctaccagga gatcgcaggt aagcgcgggc 480

gcgctgcagg ggcaggctgc agccctcggc tgccgcacgt cccactggcc gcccggcgtc 540

cccttccttc cccctgttgc tgagttggtg ctcactttct gccaccgcta tgggactccg 600

cgtctccgtg ttgggcggcg gatgctcctg cggcttcttc ggcgggggaa ggtgtgcgtc 660

tccgccgcct cattgtgtgc acacgcggga gcaccctggc tcccgcctcc cgctgctctc 720

gcgcccttct accccttagt tgatggctca ggcccggctg gccagggagc ccgggtcact 780

ccggggcggc tgcaaggcgc agacggagag ccgagccggg cgctcactcc gcgttctggt 840

tcgggcaaac ttggaagaac tgcgaccgca gtttgcccag cgccacagtc tgagtggcgc 900

cttctccact cccgcccttg cgccggcagg ggcggtggag agacgcggag ggctccccca 960

gcccctctct cccctatccg tccttcgggc gacagagcgc ccggcgctcg ggccgggggc 1020

gggcaaggct gggagggacc ctcgccgggg acctggcctc tggacgccgg cgtttcaagg 1080

ctggtttggg gacttcacgg gctgcctgtt tcagatgtgg ggcgggcttt cccgttaggg 1140

ttcctcagtg cttccccagt tgctgttggc cactcagggc ccggggacac cctgccaccc 1200

ggtctggagc cggcctcgtc tgccagcgaa cagccaactt tagcgggtgg ctcagctggg 1260

gatt                                                                1264

<210> 13
<211> 761
<212> DNA
<213> Homo sapiens

<400> 13
cactcagtgt gtgcatatga gagcggagag acagcgacct ggaggccatg ggtgggggcg      60

ggtggtgaag ctgccgaagc ctacacatac acttagcttt gacacttctc gtaggttcca     120

aagacgaaga cacggtggct tcagggagac aagtcgcaag ggcgactttt ccaagcggga     180

gatggtgaag tctttggacg tgtagtgggt aggtgatgat ccccgcagcc gcctgtaggc     240

ccgcagactt cagaaaacaa gggccttctg tgagcgctgt gtcctccccg gaatccgcgg     300

cttaacacat tctttccagc tgcggggcca ggatctccac cccgcgcatc cgtggacaca     360

cttagggtcg cctttgtttt gcgcagtgat tcaagttggg taacccttgc tcaacacttg     420

ggaaatgggg agaatctccc ccacccgcaa cctcccgcac cccaggttcc caaaatctga     480

atctgtatcc tagagtggag gcagcgtcta gaaagcaaag aaacggtgtc caaagacccc     540

ggagagttga gtgagcgcag atccgtgacg cctgcggtac gctagggcat ccaggctagg     600

gtgtgtgtgt gcgggtcggg gggcgcacag agaccgcgct ggtttaggtg gacccgcagt     660

cccgcccgca tctggaacga gctgcttcgc agttccggct cccggcgccc cagagaagtt     720

cggggagcgg tgagcctagc cgccgcgcgc tcatgtttat t                         761

<210> 14
<211> 1198
<212> DNA
<213> Homo sapiens

<400> 14
agtcactcca ggatcagagg ccgcgtcggt tctgcttggg gcatgggcag agggaggctg      60

ctggggccaa gccccggctg gacgcgaggg aagaaactcg tcccaggacc cgcacgccca     120

tacctggctg tcccagagct cttccctagg ccggcacctt cgctcttcct cttccccacc     180

ccctagccct tttgtctctt tttcagacgg atgttttcag tctcaagtgg ttttattttc     240

cgcacaaaac cctgagatca agggcagatc acagactgta ccggaggctc gggtttccct     300

ggactctgtg ctgttctgcg tcccagggtt ggctaggaag gaaggcctgg gccggcgagg     360

tgacgggtct cccgcccagg tcggcaggac ggggggaggt gtgtcccggt aggtccctgg     420

tgagctcacc cgtggcatcg gggacccgcg ggaacccacc gggcgcccac tagagactcg     480

ggtcctaccc tcccccacac tactccaccg aaatgatcgg aagggcgcgc taggcctgct     540

tccaagggct cagtgataaa ggcctcaaaa tcacactcca tcaagacttg gttgaagctt     600

tgggtaggtt tgttgttgtt gttgttgttg tttgtttgtt tgttttagca gacacgtcct 660

ggaaagaggt cctcagaacc caaaggttca ataatgattt gtggatggat tgattatagt 720

ctgatatcgc tctggttcca cagaaacccg gagctccttg gcccactgtt accccagcag 780

acctaaatgg acggtttctg tttttcactg gcagctcaga actggaccgg aagaagttcc 840

cctccacttc ccccctcccg acaccagatc attgctgggt ttttattttc gggggaaaaa 900

caacaacaac aacaacaaaa aaaacactag gtccttccag actggatcag gtgatcgggc 960

aaaaaccctc aggctagtcc ggctgggtgc ccgagcatga aaaggcctcc gtggccgttt 1020

gaacagggtg ttgcaaatga gaacttttgt aagccataac cagggcatcc tgagggtctg 1080

agttcacggt caaggctgtg ggctactagg tccagcgagt ccaggcctcg ccccgccccc 1140

gagctgccac agccaagatc ttcggcaggg aattcgagac cagggtcctc ccactcct 1198

<210> 15
<211> 377
<212> DNA
<213> Homo sapiens

<400> 15
tttcgtgccg ctgttttcaa tgcgctaacg aggcacgtta ttcttagccg cgtccgggag 60

gggatcacat tcctgcgcag ttgcgctgct ggcggaagtg acttgttttc taacgaccct 120

cgtgacagcc agagaatgtc cgtttctcgg agcgcagcac agcctgtccc atcgagaagc 180

ctcgggtgag gggcccggtg ggcgcccgga ggccgctgga gggctgtggg agggacggtg 240

gctccccact cccgtggcga agggcaggca aaccagaagc ctcttttgag agccgtttgg 300

gattgagacg agtaagccac agcgagtggt tagaagtagg ttaggaagaa ggggaggtaa 360

gaaagccgag tagggtt 377

<210> 16
<211> 256
<212> DNA
<213> Homo sapiens

<400> 16
gttcggtgga caagggggca gcgcccacag caagccggaa agagggaggc gcggggccgc 60

gcttggggcc tgccgctgca cgccagcctg ggcaaagagc tgccaccttc tgcgggcgaa 120

gcgggtcggg acgcaggacg gcagcggggc tggaggcagc tacgtgggtc cacaccccca 180

tgccctgcaa ggctccttgg ccctgcttct cctctgtctc ggcgggagag gagcagcctc 240

ggttttacag aatttc 256

<210> 17
<211> 189
<212> DNA
<213> Homo sapiens

<400> 17
tgtgccattt agtgagaggt gttttgggca aagaatcaat ttaactgtga ctgaccgacg 60

ggcttgactg tattaattct gctaccgaaa aaaaaaaaaa aaaaaaagca atgagccgca 120

agccttggac tcgcagagct gccggtgccc gtccgagagc cccaccagcg cggctcacgc 180

ctcagtctc 189


<210> 18
<211> 707
<212> DNA
<213> Homo sapiens

<400> 18
agagtcccag ttctgcaggc cgctccaggg ctaggggtag agatggtggc aggtggtgcg 60

tcaactctct agggaagagg aacttgcatt acaaagactt gtctttctga gctgaagtca 120

aaacgggggc gtcaagcgcg ctccgtttgg cggcggtgga ggggccgcgc gcccgcgctg 180

tcccagccgg agctgccctg gctggtgatt ggaggtttaa cgtccggaat tcaggcgctt 240

ctgcagctca gatttgccgg ccaaggggcc tcagttgcaa cttttcaaaa tggtgtttct 300

ggaaaataac aaattcagac tcaactggtg acagcttttg gctatagaga atgaaactgc 360

ttccctttgg cggtggaact cttaaacttc gaagagtgaa agaatacaat gaaataaaat 420

gccataagat cactggattt ttcagaaaaa ggaagacccc aaattactcc caaaatgagg 480

ctttgtaaat tcttgttaaa aatctttaaa tctcgaattt ccccctacaa catctgatga 540

gtgctttaag agcaaacgag caaatcccac ctcgagaatc aacaaaccca agctctggcc 600

aaggctctcc ccgcgttttc ttctcgtgac ctggggaatg tcccgcccca tcgctcacct 660

ggctcttgtc atctcgctca tcttgaagtg acccgtggac aatgctg 707


<210> 19
<211> 182
<212> DNA
<213> Homo sapiens

<400> 19
agctgccctc tgtggccatg agcgggtgtc cagccccttc caaggctgca ccggggagac 60

gctggttttc tgctcgctgt gaccgaacaa agcccctaag agtcagtgcg cggaacagaa 120

gagccggacc ccgacgggcc gagtcccaac gtgaggcacc cggcagagaa aacacgttca 180

cg 182


<210> 20
<211> 179
<212> DNA
<213> Homo sapiens

<400> 20

cctcggcagc accggcatgg ctggaggcca gtacggccag gtgtggcggg agggagcgcc 60

gtctggcttg ggtcgtccat cctgacagga cgctgcaagg gcaggagccc cgcgccccgt 120

gtcctgcgcc cccgctcgag gacaagcccc agccgccggt ctccgctggg ttccgacag 179

<210> 21
<211> 369
<212> DNA
<213> Homo sapiens

<400> 21
ctttaagagg ctgtgcaggc agacagacct ccaggcccgc taggggatcc gcgccatgga 60

ggccgcccgg gactatgcag gagccctcat caggcgagtg ccccgcgtcc ccctgattgc 120

cgtgcgcttc caatcgcctt gcgttcggtg gcctcatatt cccctgtgcg cctctagtac 180

cgtaccccgc tcccttcagc cccctgctcc ccgcattctc ttgcgctccg cgaccccgcg 240

cacacaccca tccgccccac tggtgcccaa gccgtccagc cgcgcccgcg ggcagagccc 300

aatcccgtcc cgcgcctcct caccctcttg cagctgggca caggtaccag gtgtggctct 360

tgcgaggtg 369

<210> 22
<211> 176
<212> DNA
<213> Homo sapiens

<400> 22
agacttgcag aactcggccc ccctggagga gacctaaccg ccacggtctt ggggaggttc 60

cggagggcct cggttgtctg cactcccaac accaagaaac ccctgagacg cgaagctgcc 120

agcgtgctgc cctcagagca gggcgacgca aagccagcgg accccggggt ggcggg 176

<210> 23
<211> 167
<212> DNA
<213> Homo sapiens

<400> 23
tgctcggctg gggggctcgc tccgcacttt cggtgccaga aaatgcccag aggagcgggg 60

cggccccaga gcctcctttc ggggcgcgag gcccggcgcg tgtgtacgga gtccagtccc 120

cccagggagt ggggtgcccg caccttcccc tccgcgctcg gagccac 167

<210> 24
<211> 1205
<212> DNA
<213> Homo sapiens

<400> 24
tcttgcacac ctgcttgtag ttctgcaccg agatctggtc gttgaggaac tgcacgcaga 60

gcttggtgac ctgggggatg tgcaggatct tgctgaccga cagcacctcc tccaccgtgt 120

ccagggacag ggtcacgttg gccgtgtaga ggtactcgag caccaggcgc agcccgatgg 180

acgagcagcc ctgcagcacc aggttgttga tggcccgggg gctggtcagc agcttgtcgt 240

cgggggagga agaaggagtc ccgggctcct cctgcggcgg cggctgctgc tgctgtgacg 300

gctgctgctg cggcggctgc tgctggtcct tgggggcccc caggccgtcc tggccgccga 360

cccctccccc gagagggggg tggctggaga agagcgatcg gaagtactgc gagcaggagg 420

ccagcacggc cttgtggcaa tggaactgct ggccctgggc cgtcagggtc acgtcgcaaa 480

acagctgctt cctccacagc aggttgaggc cgtgcagcag gttgtcgctg tggctggggt 540

cgaaggtgga ggtcctgtcc ccggatctgg acatggcgag ctgactcggt gcacctggct 600

ttaaaccctc ctccaacctg gcagacaggg gtgggggatg ggagggaggg gagcagggtg 660

gtggagcggg tggggtgtgg tcggggtggg gaagggtgtg gaggggaggg gagggcgaag 720

aacaagaatc aaggctcagc ttgactccct cctggcgcgc tccggacccc gaccctagga 780

ggaaagtccg aagacgctgg atccgtgagc gccaccagaa gggccctgtc tggggtcccg 840

gcgccggttc tgcgccctgc ggctcctctc gccacctccc acacacttcg tccctcactt 900

tcctaaaacc aaccacctca gctcggctgt tggcagcaac agcagtggca gcagcgacgg 960

caaagtggcg gctgaggccg aggcacctcg tgggctcgtg tccatgccgg gccagatgaa 1020

gggaaaggcc gggaagtggg gagccggggg tgccctgaaa gctcagaggc gaccgacggc 1080

gaaggttcca ggtcaacttg tgcccgaagc tttgcttttc gcagttggcc cagtttgggg 1140

gagggggtag gaacaggggc ccgaccagcg tgcggggtgt gcgaatctta gctctccaaa 1200

agctg 1205

<210> 25
<211> 44
<212> DNA
<213> Homo sapiens

<400> 25
cctctgtgtt agtgccctcg ggaatttggt tgatggggtg tttg 44

<210> 26
<211> 5002
<212> DNA
<213> Homo sapiens

<400> 26
tgatgtcgca cctgaacggc ctgcaccacc cgggccacac tcagtctcac gggccggtgc 60

tggcacccag tcgcgagcgg ccaccctcgt cctcatcggg ctcgcaggtg gccacgtcgg 120

gccagctgga agaaatcaac accaaagagg tggcccagcg catcacagcg gagctgaagc 180

gctacagtat cccccaggcg atctttgcgc agagggtgct gtgccggtct caggggactc 240

tctccgacct gctccggaat ccaaaaccgt ggagtaaact caaatctggc agggagacct 300

tccgcaggat gtggaagtgg cttcaggagc ccgagttcca gcgcatgtcc gccttacgcc 360

tggcaggtaa ggccggggct agccaggggc caggctgctg ggaagagggc tccgggtccg 420

gtgcttgtgg cccaagtctg cgcgccgagt cacttctctt gattctttcc ttctctttcc 480

tatacacgtc ctctttcttc tcgtttttat ttcttcttcc atttctcttt tctcttccgc 540

tcttccccta ctttcccttc tcccttttct ttttctttct tactctctcc ttgtccctga 600

gctttcattg accgaccccc ccccatttca ttcgccctcc cctcaatgtg ccaacctttg 660

ccctatttcc gatcttccca ggtactggga ggcgggatgg gggtgtgcgt tttcctctag 720

gagccctgtc tttccaagac ccacagaaac caggacctgc ccttattcaa aaccccatgc 780

acttcaagtc tcttttagac aacacatttc aattttccgg gctgactagt ctccctgtgc 840

agaggcagtt gagaggcttt gctctgcaga gggaaaagag ctctctactc tcccacccac 900

catataggca aacttatttg gtcattggct gaaggcacag ccttgccccc gcggggaacc 960

ggcggccagg atacaacagc gctcctggag cccatctctg gccttggcgt tggcgcaggg 1020

actttctgac cgggcttgag gggctcgggc cagctccaat gtcactacct acagcgaggg 1080

cagggtgtaa ggttgagaag gtcacattca ccgctttggg aggacgtggg agaagagact 1140

gaggtggaaa gcgctttgcc ttgctcaccg gccgtccttg ccccggtccc agcgtttgct 1200

gggatttgcc aggatttgcc ggggctccgg gagaccctga gcactcgcag gaagaggtgc 1260

tgagaaatta aaaattcagg ttagttaatg catccctgcc gccggctgca ggctccgcct 1320

ttgcattaag cgggcgctga ttgtgcgcgc ctggcgaccg cggggaggac tggcggcccg 1380

cgggagggga cgggtagagg cgcgggttac attgttctgg agccggctcg gctctttgtg 1440

cctcctctag cggccaagct gcgaggtaca gccctctatt gttctaggag cacagaaacc 1500

tcctgtgtgg gcggcgggtg cgcgagctag agggaaagat gcagtagtta ctgcgactgg 1560

cacgcagttg cgcgcttttg tgcgcacgga ccccgcgcgg tgtgcgtggc gactgcgctg 1620

cccctaggag caagccacgg gcccagaggg gcaaaatgtc caggtccccc gctgggaagg 1680

acacactata ccctatggca agccagggtg ggcgacttcc catggatcgg gtggaggggg 1740

gtatctttca ggatcggcgg gcggtctagg ggaacaattc gtggtggcga tgatttgcat 1800

agcgcgggtc ttgggatgcg cgcggttccg agccagcctc gcacagctcg cttccggagc 1860

tgcgagctca ggtttccacc cccgatcccc cgggctttcc tcgcaccgct gagcccagct 1920

tgtggggtgc actcgaccaa cgcccgacag ggctggggaa tgtgacaggc agcaggttca 1980

cccgggcttg ggagggggga gtttccgctt tgacagcatt ttcctttgcc gtctgctggt 2040

ggattcctat tcccagtcgg taatcgcccc gcagtgttga tctaagaagg taaagaaaac 2100

taggtttccc tgcaaagagc ctcccccaaa tcggcggact ccggatactt tgagtggatt 2160

tagaaattta tgtaatcttt ctcctttagt ttatttttca tcctctccta cagttttctc 2220

tgatttgctg ttggttcggg gcaagataaa gcagccagta gagagcgata ataatagcgg 2280

cgggaaatga actggagact ggctgacagt tcttaacatt ttgtcataga tccccccgaa 2340

tgtcccaggc tgtctctggt gggttttagt acccgccggc ttcttgggca ccggggacca 2400

gaaggaactt ggcagctggt cttaggggta cagttaaagg caggatgaca gctattctcc 2460

tgctcatctc agagcgctgc cgccccctca tgccggtcgc gcaaagaaca cagcttttaa 2520

aaaacacgtg ccttctgccc atataggtct gaaagtgatg aggaaagtaa tgcttcgcct 2580

attagcgagt ttcagctttt aaaatgatcc caagcgttgc tgagatgaga aagcgtggca 2640

tcccgggggt cctcagcccc acccgcgccc atggtgcaag tctgcaggga caggcccggg 2700

acagcactgc ccacgctgct agattttccg cagaggatcg ctgaagctgc cttcgtggga 2760

gacagaatgc ctcctccagc gagtggaaaa ggcctgctga ggaccccgct ttgctcgagc 2820

attcaaatgt gtgtctgttt tattaccctg ggttgaaaag ggacaagagc tttagccttt 2880

ttatctggcc attttatcag caactacaag tgtgttgagt ggttattatt acataggagg 2940

cttttcagtt tggggtcagt agatcagtct cttcagacac tgatgcagaa gctgggactg 3000

gtaagtaggt attatgtgct cggagcgcta ggggacagga gcaaatggag aagaaaagcg 3060

gaggctttct ccgcccggag tatcgatcgg aatccccgcc ggtacgccgc agagggccct 3120

cgccgttggg ccccgggggt ttaacaagcc cagccgctcc gcaggcggct cggccggact 3180

ctcagaccgg tgcctggaag acaccgtccc tgcccccctc ccgccaaacc tgcctcttct 3240

ctttctctca taggttatag gttccctttc tctctcattt tggccccgcc cccgggtcct 3300

gccaaacagc caagcaggcc ggggtttagg gggctcagaa tgaagaggtc tgatttggcc 3360

agcgccggca aagctcaccc ttaggcgagg tcacaacaga ggcaggtcct tcctgcccag 3420

cctgccggtg tagtcacagc caagggtggc acttgaaagg aaaagggaga aaacttcgga 3480

gaaatttaga ttgccccaac gttagatttc agagaaattg actccaaatg cacggattcg 3540

ttcggaaagg gcggctaagt ggcaggtggt tgcaaccccg cccggtcggg ccttcgcaga 3600

ggttccccaa gaccagccct tgcagggcgg ttttcagcaa cctgacaaga ggcggccaag 3660

acaaatttct gcgggttcga gcacacactc tcgggcgttg ggccccagag acctctaaac 3720

caagcacaaa caagaaggga gtgagagaac ccaggctaga acttgcacgg gcatcccact 3780

gaggaaaagc gaggcctcgg tggcaggcat gttttcttcc gacgcccgaa aatcgagccg 3840

agcgcccgac tacatttact gcagaggttt ccgcctccag tgagcccgga tcccccagcg 3900

gcctgcccgg agctggtctc cagtccccgc cgtagtccga cgcacggccc tctcctggca 3960

gcaagctccc agcggccagt ctgaagccaa ttctgttcag gcggccgagg gcccttagcc 4020

aacccaccat gatgtcgcct gggccacctg atgcccgcag cggcgggaca cggcccgggc 4080

agtgcgcagt ggctcctgct aggggcaccg cgtgcgtgct tgtctcccgc tgcgccgggg 4140

acgtccttgg gtgacacggg ccgctgggca cctcccaagc cgaggaaacg gacccccttc 4200

gcagagtctc gcgcccaccc cccaacctcc cacctcgttt ctcgctgcta gggctcccga 4260

ctcagcccac ctctcctggc ggtttagtta gggatcagag ctggagaggc tgaacgcaac 4320

ccgtgccagt acggaacaga cgatatgttt gcctgctagc tgcttggatg aataattgaa 4380

aagttcgctg cagtctgtgc ttcgtcaagt cccgggtgcc gggagaacac cttcccaaca 4440

cgcatcaggg tgggcgggag cgggcagagg aggcgggacc cgagggagga gagtgaaccc 4500

gagcaggaga agcagcccag gcagccaggc gccctcgatg cgagaggctg ggcatttatt 4560

tttattccag gctttccact gtgtggttat gtcactttct caaacaaatg tgtatatgga 4620

gggagatcga tgctgataat gtttagaaga ttaaaagagc attaatgctg gcaacaataa 4680

cgtaaacgtg tggacccaga tttcattgat ctggaacttg atccggcgcg tttccagtaa 4740

gcccgacggc gcgctcttcc cagcagagcg ctcaccagcg ccacggcccc gcggttttcc 4800

agcggtgccg cttcgccagc tctgcgcggg ttctcccgtc tgaccgcagc tcctcccccg 4860

cgaggcccca gcccgcctta cttccccgag gttttctcct cctctcgcgg ggctctctgc 4920

cctctgcacc ccctcccccg acctctgcac cacccgcccc tgtgcgcaca caccgctact 4980

tgcgcttccg gcgatccgcc tg 5002

<210> 27
<211> 150
<212> DNA
<213> Homo sapiens

<400> 27
aaccggagat ctgcttggtg aactgagagg agtccttagg agagcgggga cgccaggggc 60

cgggggacac ttcgctctcg ccctagggaa ggtggtcttg acgctttcta ttgaagtcaa 120

acttgaaaat atcagctgcc gctggactat 150

<210> 28
<211> 339
<212> DNA
<213> Homo sapiens

<400> 28
cgtgagcaga acgcccgccc tggagcagtt aggaccgaag gtctccggag agtcgccggc 60

ggtgccaggt aacgcagagg gctcgggtcg ggccccgctt ctggggcttg ggactccggg 120

cgcgcggagc cagccctctg gggcgaaatc cccgggcggc gtgcgcggtc cctctccgcg 180

ctgtgctctc ccagcaactc cctgccacct cgacgagcct accggccgct ccgagttcga 240

cttcctcgga cttagtggga gaaggggttg gaaatgggct gccgggactg ggggagctgc 300

tctctggaag cagggaagct ggggcgcacc ggggcaggt 339

<210> 29
<211> 1961
<212> DNA
<213> Homo sapiens

<400> 29
tagaagagga agactcctct ggccccacta ggtatcatcc gcgctctccc gctttccacc 60

tgcgccctcg cttgggccaa tctctgccgc acgtgtccat ccctgaactg cacgctatcc 120

tccacccccg gggggttcct gcgcactgaa agaccgttct ccggcaggtt ttgggatccg 180

gcgacggctg accgcgcgcc gcccccacgc ccggttccac gatgctgcaa tacagaaagt 240

ttacgtcggc cccgacccgc gcgggactgc agggtccgcc ggagcgcggc gcagaggctt 300

ttcctgcgcg ttcggccccg ggaaaggggc gggagggctg gctccgggag cgcacgggcg 360

cggcggggag ggtactcact gtgaagcacg ctgcgcccat ggatcatgtc tgtgcgttac 420

accagaggct ccgggctcca ctaattccat ttagagacgg gaagacttcc agtggcgggg 480

ggaggacagg gtcgagaggt gttaaagacg caaagcaaga aggaaataaa ggggggccga 540

gagggagacc gagaggaagg gggagctccg agcccacgct gcagccagat ccggatgagt 600

ccgtcctccg ccccgggcgg gctctcgctc tcgctggccc tcagcgccgc gcagccagca 660

gcatccccac cgtgacgctc gcatcacacc cgggcgccgg ccgccaccat ccgcgccgcc 720

gccgtcagga ccctcctccc gggcatcgtc gccgccgcgg ggtcgggagg acgcggcgcg 780

cgggaggcgg cggtcgcagg gcgagccccg ggacgccccg agccggggcc ggggccgggg 840

agagggcgca gcgaggtggg ggccagtcca gaccgacggc agcgacggag cgggcggcgg 900

cggcggcgcc ggcggcggcg gggtggctca gtccccagtc tcagacgcgc cgcgcagcag 960

gtcggagcag cctccccggg aggatgtcca gcggcagcgc tcctcgctcc agcccttggg 1020

gatcttccgc tgaggcattg aaggcaggaa gaaggggtcc gtcatcggct cgccgggctg 1080

cgcgccacct ctgctatctt gcggaaagag gagcgggtgg gtgggcgtct gggaggcggg 1140

ctggagggcg gtgcagggga gcggggcggc cggggggggg gccggggggc ggggaaggga 1200

gggaggagaa aggagccgga agagggcaga gttaccaaat gggctcctta gtcatggctt 1260

ggggctccac gaccctcctg gaagcccgga gcctgggtgg gatagcgagg ctgcgcgcgg 1320

ccggcgcccc ggggctggtg cgcggcagaa tggggccgcg gcggcggcag caaggacatc 1380

ccagccgcgc ggatctgggg gaggggcggg gagggggtga ggacccggct gggatccgcg 1440

gctcggcccg ccaggcgcag gagagaggat gcagccgcaa atcccgagcc ggatcctcgt 1500

gccggacgga aggcgtggaa gcgggagggg ccttcgtgtg aaaatccctt gtggggtttg 1560

gtgtttcact ttttaaaggt tagaccttgc gggctctctg cctcccaccc cttcttttcc 1620

atccgcgtaa aggaactggg cgccccctct ccctccctcc ctggggcgca ggtttcgccg 1680

cggactccgc gctcagcttg ggagacacgg caggggcgcg ccccagggaa aggcggccgt 1740

aaaagtttcg cggttgagca ctgggcctga tgtccagtcc ccccaccaaa ttactcctgc 1800

aaagacgcgg gcttcttgca attgagcccc ccacctcgag gtatttaaaa ccaccccaag 1860

gcacacacgg acccccgttc ccccgcgcca cttcctccta caggctcgcg cggcgcgtta 1920

aagtctggga gacacgagtt gcggggaaac agcaccggaa g 1961

<210> 30
<211> 314
<212> DNA
<213> Homo sapiens

<400> 30
aagaaacagc tcatttcgga gctgaggaca aggcgtggga agaagacgcg tttggtttca 60

cccaggcggg tggcggcaaa gctgtgggat gcgcgctgca cactccttcc gtcatcccgt 120

tcccaccttc cacacacacc tgcgggaggt cggacatgtc ctgattgcgt gttcatcacg 180

atggcaaacc gaacatgagg agaacgccac tgacgctggg tgcgccggct ttcccagccc 240

tcgtgcataa cggggaggga gatgcagaag ttttttccaa catcggtgca aaggggaagc 300

tgaggttttc ctat 314

<210> 31
<211> 584
<212> DNA
<213> Homo sapiens

<400> 31
tctgtcagct gctgccatgg ggcagcggga aggccctgga gggtgcctgg gctgtgtctg 60

gtcccggcca cgcgtccctg cagcgtctga gaccttgtgg aacacacttg acccggcgct 120

gggacggggt cggcccacac gcaccgccag cccgcaggag tgaggtgcag gctgccgctg 180

gctccttagg cctcgacagc tctcttgagg tcggccctcc tcccctcccg agagctcagc 240

agccgcagac ccaggcagag agagcaaagg aggctgtggt ggcccccgac gggaacctgg 300

gtggccgggg gacacaccga ggaactttcc gccccccgac gggctctccc accgaggctc 360

aggtgctcgt gggcagcaag gggaagcccc atggccatgc cgcttccctt tcaccctcag 420

cgacgcgccc tcctgtgccc gcggggaaca agacggctct cggcggccat gcaggcggcc 480

tgtcccacga acacgatgga gacctcagac gccgtcccca ccctgtcact gtcaccatca 540

cccatcctgt cccctcacgc ctccccacat cccatcatta ctac 584

<210> 32
<211> 349
<212> DNA

<213> Homo sapiens

<400> 32
gaagtagaat cacagtaaat gaggagttag ggaatttagg gtagagatta aagtaatgaa 60

cagaggagga ggcctgagac agctgcagag agaccctgtg ttccctgtga ggtgaagcgt 120

ctgctgtcaa agccggttgg cgctgagaag aggtaccggg ggcagcaccc gcctcctggg 180

agagggatgg gcctgcgggc acctggggga accgcacgga cacagacgac actataaacg 240

cgggcgagac atcaggaccc gggaaacaga aggacgcgcg tttcgagcag ctgcccagtg 300

ggccacaagc cccgccacgc cacagcctct tcccctcagc acgcagaga 349


<210> 33
<211> 3510
<212> DNA
<213> Homo sapiens

<400> 33
   tactccggcg acgggaggat gttgagggaa gcctgccagg tgaagaaggg gccagcagca 60
   gcacagagct tccgactttg ccttccaggc tctagactcg cgccatgcca agacgggccc 120
   ctcgactttc acccctgact cccaactcca gccactggac cgagcgcgca aagaacctga 180
   gaccgcttgc tctcaccgcc gcaagtcggt cgcaggacag acaccagtgg gcagcaacaa 240
   aaaaagaaac cgggttccgg gacacgtgcc ggcggctgga ctaacctcag cggctgcaac 300
   caaggagcgc gcacgttgcg cctgctggtg tttattagct acactggcag gcgcacaact 360
   ccgcgccccg actggtggcc ccacagcgcg caccacacat ggcctcgctg ctgttggcgg 420
   ggtaggcccg aaggaggcat ctacaaatgc ccgagccctt tctgatcccc acccccccgc 480
   tccctgcgtc gtccgagtga cagattctac taattgaacg gttatgggtc atccttgtaa 540
   ccgttggacg acataacacc acgcttcagt tcttcatgtt ttaaatacat atttaacgga 600
   tggctgcaga gccagctggg aaacacgcgg attgaaaaat aatgctccag aaggcacgag 660
   actggggcga aggcgagagc gggctgggct tctagcggag accgcagagg gagacatatc 720
   tcagaactag gggcaataac gtgggtttct ctttgtattt gtttattttg taactttgct 780
   acttgaagac caattattta ctatgctaat ttgtttgctt gtttttaaaa ccgtacttgc 840
   acagtaaaag ttccccaaca acggaagtaa cccgacgttc ctcacactcc ctaggagact 900
   gtgtgcgtgt gtgcccgcgc gtgcgctcac agtgtcaagt gctagcatcc gagatctgca 960
   gaaacaaatg tctgaattcg aaatgtatgg gtgtgagaaa ttcagctcgg ggaagagatt 1020
   agggactggg ggagacaggt ggctgcctgt actataagga accgccaacg ccagcatctg 1080
   tagtccaagc agggctgctc tgtaaaggct tagcaatttt ttctgtaggc ttgctgcaca 1140
   cggtctctgg cttttcccat ctgtaaaatg ggtgaatgca tccgtacctc agctacctcc 1200
   gtgaggtgct tctccagttc gggcttaatt cctcatcgtc aagagttttc aggtttcaga 1260
   gccagcctgc aatcggtaaa acatgtccca acgcggtcgc gagtggttcc atctcgctgt 1320
   ctggcccaca gcgtggagaa gccttgccca ggcctgaaac ttctctttgc agttccagaa 1380
   agcaggcgac tgggacggaa ggctctttgc taacctttta cagcggagcc ctgcttggac 1440
   tacagatgcc agcgttgccc ctgccccaag gcgtgtggtg atcacaaaga cgacactgaa 1500
   aatacttact atcatccggc tcccctgcta ataaatggag gggtgtttaa ctacaggcac 1560
   gaccctgccc ttgtgctagc gcggttaccg tgcggaaata actcgtccct gtacccacac 1620
   catcctcaac ctaaaggaga gttgtgaatt ctttcaaaac actcttctgg agtccgtccc 1680
   ctccctcctt gcccgccctc tacccctcaa gtccctgccc ccagctgggg gcgctaccgg 1740
   ctgccgtcgg agctgcagcc acggccatct cctagacgcg cgagtagagc accaagatag 1800
   tggggacttt gtgcctgggc atcgtttaca tttgggcgcc caaatgccca cgtgttgatg 1860
   aaaccagtga gatgggaaca ggcggcggga aaccagacag aggaagagct agggaggaga 1920
   ccccagcccc ggatcctggg tcgccagggt tttccgcgcg catcccaaaa ggtgcggctg 1980
   cgtggggcat caggttagtt tgttagactc tgcagagtct ccaaaccatc ccatccccca 2040
   acctgactct gtggtggccg tattttttac agaaatttga ccacgttccc tttctccctt 2100
   ggtcccaagc gcgctcagcc ctccctccat cccccttgag ccgcccttct cctccccctc 2160
   gcctcctcgg gtccctcctc cagtccctcc ccaagaatct cccggccacg ggcgcccatt 2220
   ggttgtgcgc agggaggagg cgtgtgcccg gcctggcgag tttcattgag cggaattagc 2280
   ccggatgaca tcagcttccc agccccccgg cgggcccagc tcattggcga ggcagcccct 2340
   ccaggacacg cacattgttc cccgcccccg cccccgccac cgctgccgcc gtcgccgctg 2400
   ccaccgggct ataaaaaccg gccgagcccc taaaggtgcg gatgcttatt atagatcgac 2460
   gcgacaccag cgcccggtgc caggttctcc cctgaggctt ttcggagcga gctcctcaaa 2520
   tcgcatccag agtaagtgtc cccgccccac agcagccgca gcctagatcc cagggacaga 2580
   ctctcctcaa ctcggctgtg acccagaatg ctccgataca gggggtctgg atccctactc 2640
   tgcgggccat ttctccagag cgactttgct cttctgtcct ccccacactc accgctgcat 2700
   ctccctcacc aaaagcgaga agtcggagcg acaacagctc tttctgccca agccccagtc 2760
   agctggtgag ctccccgtgg tctccagatg cagcacatgg actctgggcc ccgcgccggc 2820
   tctgggtgca tgtgcgtgtg cgtgtgtttg ctgcgtggtg tcgatggaga taaggtggat 2880
   ccgtttgagg aaccaaatca ttagttctct atctagatct ccattctccc caaagaaagg 2940
   ccctcacttc ccactcgttt attccagccc gggggctcag ttttcccaca cctaactgaa 3000
   agcccgaagc ctctagaatg ccacccgcac cccgagggtc accaacgctc cctgaaataa 3060
   cctgttgcat gagagcagag gggagataga gagagcttaa ttataggtac ccgcgtgcag 3120
   ctaaaaggag ggccagagat agtagcgagg gggacgagga gccacgggcc acctgtgccg 3180
   ggaccccgcg ctgtggtact gcggtgcagg cgggagcagc ttttctgtct ctcactgact 3240
   cactctctct ctctctccct ctctctctct ctcattctct ctcttttctc ctcctctcct 3300
   ggaagttttc gggtccgagg gaaggaggac cctgcgaaag ctgcgacgac tatcttcccc 3360
   tggggccatg gactcggacg ccagcctggt gtccagccgc ccgtcgtcgc cagagcccga 3420
   tgaccttttt ctgccggccc ggagtaaggg cagcagcggc agcgccttca ctgggggcac 3480
   cgtgtcctcg tccaccccga gtgactgccc 3510

<210> 34
<211> 333
<212> DNA
<213> Homo sapiens

<400> 34
ttaattcgaa aatggcagac agagctgagc gctgccgttc ttttcaggat tgaaaatgtg 60

ccagtgggcc aggggcgctg ggacccgcgg tgcggaagac tcggaacagg aagaaatagt 120

ggcgcgctgg gtgggctgcc ccgccgccca cgccggttgc cgctggtgac agtggctgcc 180

cggccaggca cctccgagca gcaggtctga gcgtttttgg cgtcccaagc gttccgggcc 240

gcgtcttcca gagcctctgc tcccagcggg gtcgctgcgg cctggcccga aggatttgac 300

tctttgctgg gaggcgcgct gctcagggtt ctg 333

<210> 35
<211> 385
<212> DNA
<213> Homo sapiens

<400> 35
ccggtcccca gtttggaaaa aggcgcaaga agcgggcttt tcagggaccc cggggagaac 60

acgagggctc cgacgcggga gaaggattga agcgtgcaga ggcgccccaa attgcgacaa 120

tttactggga tccttttgtg gggaaaggag gcttagaggc tcaagctata ggctgtccta 180

gagcaactag gcgagaacct ggccccaaac tccctcctta cgccctggca caggttcccg 240

gcgactggtg ttcccaaggg agccccctga gcctaccgcc cttgcagggg gtcgtgctgc 300

ggcttctggg tcataaacgc cgaggtcggg ggtggcggag ctgtagaggc tgcccgcgca 360

gaaagctcca ggatcccaat atgtg 385

<210> 36
<211> 105
<212> DNA
<213> Homo sapiens

<400> 36
gcgcaggtcc ccccagtccc cgagggagtg cgcccgacgg aaacgcccct agcccgcggg 60

cctcgctttc ctctcccggg ttcctgggtc acttcccgct gtctc 105

<210> 37
<211> 147
<212> DNA
<213> Homo sapiens

<400> 37
ttccctcgcg gctttggaaa gggggtgcaa atgcaccctt ctgcgggccc gctacccgct 60

gcaacacctg tgtttccttt ctgggcacct tctaggtttc tagatattgc tgtgaatacg 120

gtcctccgct gtacagttga aaacaaa 147

<210> 38
<211> 365
<212> DNA
<213> Homo sapiens

<400> 38
tgggaattta ggtcgggcac tgccgatatg tcgccttcca caaggcgggc ccgggcctct 60

gctgaccgtg caccggtcct ggggctgggt aattctgcag cagcagcgca gcccatgccg 120

gggaatttgc gggcagagga gacagtgagg cccgcgttct gtgcgggaac tcccgagctc 180

acagagccca agaccacacg gctgcatctg cttggctgac tgggccaggc ccacgcgtag 240

taacccggac gtctctctct cacagtcccc ttgcgtctgg ccagggagct gccaggctgc 300

accccgcggt ggggatcggg agaggggcag tgtcgcccat ccccggaagg ctgagcctgg 360

tgcag 365

<210> 39
<211> 418
<212> DNA
<213> Homo sapiens

<400> 39
cggttttctc ctggaggact gtgttcagac agatactggt ttccttatcc gcaggtgtgc 60

gcggcgctcg caagtggtca gcataacgcc gggcgaattc ggaaagcccg tgcgtccgtg 120

gacgacccac ttggaaggag ttgggagaag tccttgttcc cacgcgcgga cgcttccctc 180

cgtgtgtcct tcgagccaca aaaagcccag accctaaccc gctcctttct cccgccgcgt 240

ccatgcagaa ctccgccgtt cctgggaggg gaagcccgcg aggcgtcggg agaggcacgt 300

cctccgtgag caaagagctc ctccgagcgc gcggcgggga cgctgggccg acaggggacc 360

gcgggggcag ggcggagagg acccgccctc gagtcggccc agccctaaca ctcaggac 418

<210> 40
<211> 906
<212> DNA
<213> Homo sapiens

<400> 40
agggaatcgg gctgaccagt cctaaggtcc cacgctcccc tgacctcagg gcccagagcc      60

tcgcattacc ccgagcagtg cgttggttac tctccctgga aagccgcccc cgccggggca     120

agtgggagtt gctgcactgc ggtctttgga ggcctaggtc gcccagagta ggcggagccc     180

tgtatccctc ctggagccgg cctgcggtga ggtcggtacc cagtacttag ggagggagga     240

cgcgcttggt gctcagggta ggctgggccg ctgctagctc ttgatttagt ctcatgtccg     300

cctttgtgcc ggcctctccg atttgtgggt ccttccaaga aagagtcctc tagggcagct     360

agggtcgtct cttgggtctg gcgaggcggc aggccttctt cggacctatc cccagaggtg     420

taacggagac tttctccact gcagggcggc ctggggcggg catctgccag gcgagggagc     480

tgccctgccg ccgagattgt ggggaaacgg cgtggaagac accccatcgg agggcaccca     540

atctgcctct gcactcgatt ccatcctgca acccaggaga aaccatttcc gagttccagc     600

cgcagaggca cccgcggagt tgccaaaaga gactcccgcg aggtcgctcg gaaccttgac     660

cctgacacct ggacgcgagg tctttcagga ccagtctcgg ctcggtagcc tggtccccga     720

ccaccgcgac caggagttcc ttcttccctt cctgctcacc agccggccgc cggcagcggc     780

tccaggaagg agcaccaacc cgcgctgggg gcggaggttc aggcggcagg aatggagagg     840

ctgatcctcc tctagccccg gcgcattcac ttaggtgcgg gagccctgag gttcagcctg     900

actttc                                                                906

<210> 41
<211> 860
<212> DNA
<213> Homo sapiens

<400> 41
cactacggat ctgcctggac tggttcagat gcgtcgttta aggggggggg ctggcactcc      60

agagaggagg gggcgctgca ggttaattga tagccacgga agcacctagg cgccccatgc     120

gcggagccgg agccgccagc tcagtctgac ccctgtcttt tctctcctct tccctctccc     180

acccctcact ccgggaaagc gagggccgag gtaggggcag atagatcacc agacaggcgg     240

agaaggacag gagtacagat ggagggacca ggacacagaa tgcaaaagac tggcaggtga     300

gaagaaggga gaaacagagg gagagagaaa gggagaaaca gagcagaggc ggccgccggc     360

ccggccgccc tgagtccgat ttccctcctt ccctgaccct tcagtttcac tgcaaatcca     420

cagaagcagg tttgcgagct cgaatacctt tgctccactg ccacacgcag caccgggact     480

gggcgtctgg agcttaagtc tgggggtctg agcctgggac cggcaaatcc gcgcagcgca     540

tcgcgcccag tctcggagac tgcaaccacc gccaaggagt acgcgcggca ggaaacttct     600

gcggcccaat ttcttcccca gctttggcat ctccgaaggc acgtacccgc cctcggcaca     660

agctctctcg tcttccactt cgacctcgag gtggagaaag aggctggcaa gggctgtgcg     720

cgtcgctggt gtggggaggg cagcaggctg cccctccccg cttctgcagc gagttttccc 780

agccaggaaa agggagggag ctgtttcagg aatttcagtg ccttcaccta gcgactgaca 840

caagtcgtgt gtataggaag 860

<210> 42
<211> 452
<212> DNA
<213> Homo sapiens

<400> 42
ggagcctgaa gtcagaaaag atggggcctc gttactcact ttctagccca gcccctggcc 60

ctgggtcccg cagagccgtc atcgcaggct cctgcccagc ctctggggtc gggtgagcaa 120

ggtgttctct tcggaagcgg gaagggctgc gggtcgggga cgtcccttgg ctgccacccc 180

tgattctgca tccttttcgc tcgaatccct gcgctaggca tcctccccga tcccccaaaa 240

gcccaagcac tgggtctggg ttgaggaagg gaacgggtgc ccaggccgga cagaggctga 300

aaggaggcct caaggttcct ctttgctaca aagtggagaa gttgctctac tctggagggc 360

agtggccttt tccaaacttt tccacttagg tccgtaagaa aagcaattca tacacgatca 420

gcgctttcgg tgcgaggatg gaaagaaact tc 452

<210> 43
<211> 1992
<212> DNA
<213> Homo sapiens

<400> 43
ttttcctgtt acagagctga gcccactcat gtggtgccaa gtagcgacta tctctcggcc 60

acctccaccc agagcaatgt gggcgccccc agcgggtggg agcgattgcc gagcggcgca 120

agggcgttta acgcctaacc ccctcctcct gggttgccaa gccgctaggt cgccgtttcc 180

aacgtggctg cgcgggactg aagtccgacg actcctcgtc ctcagtagga gacacacctc 240

ccactgcccc cagccacgcg agctatgggc agaatcgggg caacggtaat atctggatgg 300

ggcaggctcc cctgaggctg tgcttaagaa aaaaggaatc tggagtagcc tgaggggccc 360

cacgaggggg cctcctttgc gatcgtctcc cagccttagg ccaaggctac ggaggcaggc 420

ggccgagtgt tggcgcccag cccggccgag gactggatgg aggacgagaa gcagcctgcc 480

tctgggcgac agctgcggac gcagcctcgc cgcctcgccg cctcagcctc ggtcccagcg 540

tctctaaagc cgcgcccatt ttacagatgc agggcaggga gacaagaggc atctccgggg 600

gccgagtaga atgatggcgc gggttctccc ggcgccctga tttcgaggct gcgcccgggg 660

ccctacatgc aggcggggag gcctgggccg aaggcgtctg caaggagggg cgagtctgcc 720

cggtccgggc agggagtgag gccacagtca gttctcccta ggaggccgcg cagcgggtag 780

ggtatgggac tgggggacgc aacggggacc tggccgaatc agagccctca gcagagaacg 840

ccgaaaactc tggggccggc cgctcgcttc ccgctagtgg gaatggtttc cggtcatccg 900

ttcccagtcc agccccgggt agggagctct gatttgcaat gcacagcact tgcgaggttc 960

gaatgccccc gcaatttgca gatggaaata ctaagcctag gccgggcgtg gtggctcaag 1020

cctatcatct cagccctttg ggaggccaag ccgggaggat tgtttgagcc caagaattca 1080

aaaccagcct gagcaacata gcgaccccgt ctctacaaaa taaaataaaa taaattatcc 1140

gggcgtggtg gcacgcgcct gtggttccag ctactccgga ggctgaggtg ggaggatcgc 1200

ttgagtccgg gaggtcgagg ctacagtgag ccgtgatcgc accactgcac tccagcctgg 1260

gcgacagagt gagaccttgt ctcaaaaaag gaaaaaaaga aaaagaaagt aagcttcaaa 1320

gaagctctga taatagttct gggtcgtgca gcggtggcgg ccccgcgctc tcgcccctaa 1380

agcaagcgct ctttgtactg ggtggaggag ctttgagtag tgagggtgga gatgcagctt 1440

cggggtggcg cagccaccct gacactaggc ccggggtcgc agtgggacag aagagtctgc 1500

cgctctgact tgggctctga gttccaaggg cgcccggcac ttctagcctc ccaggcttgc 1560

gcgctggcgc ctttgccatc cgtgccgaag tggggagacc tagccgcgac caccacgagc 1620

gcagcggtga cacccagagg tcccaccggg cccctgggca gggtaacctt agcctgtccg 1680

cttcggcagc tttgcgaaga gtggcgcgca gctagggctg aggctcttgc ggacctgcgg 1740

tcgaagcagg cggctgagcc agttcgatcg ccaaggcctg ggctgccgac agtggtgcgc 1800

gctctgttcc gccgcggccg ggccaggcgc tctggaatag cgatgggggg acacggcctc 1860

caactttctg cagagaccat cgggcagctc cgggcctaag cagcgacctc accgaaggtt 1920

cctgggaacc tttgccaaaa tcccagcctc tgcctcggtc cagctaaacc gtgtgtaaac 1980

aagtgcacca ag 1992

<210> 44
<211> 448
<212> DNA
<213> Homo sapiens

<400> 44
ataaaggacc gggtaatttc gcggaatgcg gattttgaga caggcccaga cggcggcgga 60

ttccctgtgt cccccaactg gggcgatctc gtgaacacac ctgcgtccca ccccgatcct 120

aggttggggg gaaagggtat gggaaccctg agcccagagc gcgccccgct ctttcctttg 180

ctccccggct tccctggcca gccccctccc ggctggtttc ctcgctcact cggcgcctgg 240

cgtttcgggc gtctggagat caccgcgtgt ctggcacccc aacgtctagt ctccccgcag 300

gttgaccgcg gcgcctggag ccgggaatag gggtggggag tccggagaac caaacccgag 360

cctgaagttg ccattcgggt gactcccgag aaagcccggg agcattttgg ccaatgcggg 420

tttttacctg aacttcagca tcttcacc 448

<210> 45
<211> 395
<212> DNA
<213> Homo sapiens

<400> 45
aattggaaaa ccctggtatt gtgcctgttt gggggaagaa aacgtcaata aaaattaatt 60

gatgagttgg cagggcgggc ggtgcgggtt cgcggcgagg cgcagggtgt catggcaaat 120

gttacggctc agattaagcg attgttaatt aaaaagcgac ggtaattaat actcgctacg 180

ccatatgggc ccgtgaaaag gcacaaaagg tttctccgca tgtggggttc cccttctctt 240

ttctccttcc acaaaagcac cccagcccgt gggtcccccc tttggcccca aggtaggtgg 300

aactcgtcac ttccggccag ggaggggatg gggcggtctc cggcgagttc caagggcgtc 360

cctcgttgcg cactcgcccg cccaggttct ttgaa 395

<210> 46
<211> 491
<212> DNA
<213> Homo sapiens

<400> 46
gggaagcgat cgtctcctct gtcaactcgc gcctgggcac ttagcccctc ccgtttcagg 60

gcgccgcctc cccggatggc aaacactata aagtggcggc gaataaggtt cctcctgctg 120

ctctcggttt agtccaagat cagcgatatc acgcgtcccc cggagcatcg cgtgcaggag 180

ccatggcgcg ggagctatac cacgaagagt tcgcccgggc gggcaagcag gcggggctgc 240

aggtctggag gattgagaag ctggagctgg tgcccgtgcc ccagagcgct cacggcgact 300

tctacgtcgg ggatgcctac ctggtgctgc acacggccaa gacgagccga ggcttcacct 360

accacctgca cttctggctc ggtaagggac ggcgggcggc gggaccccga cgcaccaagg 420

ccggcgaggg gagggcgtag gggtctgaga tttgcaggcg tgggagtaaa ggggaccgca 480

aactgagcta g 491

<210> 47
<211> 1284
<212> DNA
<213> Homo sapiens

<400> 47
ctcaggggcg ggaagtggcg ggtgggagtc acccaagcgt gactgcccga ggcccctcct 60

gccgcggcga ggaagctcca taaaagccct gtcgcgaccc gctctctgca ccccatccgc 120

tggctctcac ccctcggaga cgctcgcccg acagcatagt acttgccgcc cagccacgcc 180

cgcgcgccag ccaccgtgag tgctacgacc cgtctgtcta ggggtgggag cgaacggggc 240

gcccgcgaac ttgctagaga cgcagcctcc cgctctgtgg agccctgggg ccctgggatg 300

atcgcgctcc actccccagc ggactatgcc ggctccgcgc cccgacgcgg accagccctc 360

ttggcggcta aattccactt gttcctctgc tcccctctga ttgtccacgg cccttctccc 420

gggcccttcc cgctgggcgg ttcttctgag ttacctttta gcagatatgg agggagaacc 480

cgggaccgct atcccaaggc agctggcggt ctccctgcgg gtcgccgcct tgaggcccag 540

gaagcggtgc gcggtaggaa ggtttccccg gcagcgccat cgagtgagga atccctggag 600

ctctagagcc ccgcgccctg ccacctccct ggattcttgg gctccaaatc tctttggagc 660

aattctggcc cagggagcaa ttctctttcc ccttccccac cgcagtcgtc accccgaggt 720

gatctctgct gtcagcgttg atcccctgaa gctaggcaga ccagaagtaa cagagaagaa 780

acttttcttc ccagacaaga gtttgggcaa gaagggagaa aagtgaccca gcaggaagaa 840

cttccaattc ggttttgaat gctaaactgg cggggccccc accttgcact ctcgccgcgc 900

gcttcttggt ccctgagact tcgaacgaag ttgcgcgaag ttttcaggtg gagcagaggg 960

gcaggtcccg accggacggc gcccggagcc cgcaaggtgg tgctagccac tcctgggttc 1020

tctctgcggg actgggacga gagcggattg ggggtcgcgt gtggtagcag gaggaggagc 1080

gcggggggca gaggagggag gtgctgcgcg tgggtgctct gaatccccaa gcccgtccgt 1140

tgagccttct gtgcctgcag atgctaggta acaagcgact ggggctgtcc ggactgaccc 1200

tcgccctgtc cctgctcgtg tgcctgggtg cgctggccga ggcgtacccc tccaagccgg 1260

acaacccggg cgaggacgca ccag 1284

<210> 48
<211> 554
<212> DNA
<213> Homo sapiens

<400> 48
tggagaacct tgggctctgt ggcctcaaag gtaggggtga tttcgagggg ccggcacctc 60

acagggcagg ttccaccgcg gaaacgcagt catcgcccag cgaccctgct cctggccctc 120

agcctccccc caggtttctt tttctcttga atcaagccga ggtgcgccaa tggccttcct 180

tgggtcggat ccggggggcc agggccagct tacctgcttt caccgagcag tggatatgtg 240

ccttggactc gtagtacacc cagtcgaagc cggcctccac cgccaggcgg gccagcatgc 300

cgtacttgct gcggtcgcgg tcagacgtgg tgatgtccac tgcgcggccc tcgtagtgca 360

gagactcctc tgagtggtgg ccatcttcgt cccagccctc ggtcacccgc agtttcactc 420

ctggccactg gttcatcacc gagatggcca aagcgttcaa cttgtcctta cacctctgcg 480

aagacaaggg gacccccacc gacggacacg ttagcctggg caaccgccac ccctcccggc 540

ccctccatca gcct 554

<210> 49
<211> 772
<212> DNA
<213> Homo sapiens

<400> 49
tctcacgacc catccgttaa cccaccgttc ccaggagctc cgaggcgcag cggcgacaga 60

ggttcgcccc ggcctgctag cattggcatt gcggttgact gagcttcgcc taacaggctt 120

ggggagggtg ggctgggctg ggctgggctg ggctgggtgc tgcccggctg tccgcctttc 180

gttttcctgg gaccgaggag tcttccgctc cgtatctgcc tagagtctga atccgacttt 240

ctttcctttg ggcacgcgct cgccagtgga gcacttcttg ttctggcccc gggctgatct 300

gcacgcggac ttgagcaggt gccaaggtgc cacgcagtcc cctcacggct ttcggggggt 360

cttggagtcg ggtggggagg gagacttagg tgtggtaacc tgcgcaggtg ccaaagggca 420

gaaggagcag ccttggatta tagtcacggt ctctccctct cttccctgcc atttttaggg 480

ctttctctac gtgctgttgt ctcactgggt ttttgtcgga gccccacgcc ctccggcctc 540

tgattcctgg aagaaagggt tggtcccctc agcaccccca gcatcccgga aaatggggag 600

caaggctctg ccagcgccca tcccgctcca cccgtcgctg cagctcacca attactcctt 660

cctgcaggcc gtgaacacct tcccggccac ggtggaccac ctgcagggcc tgtacggtct 720

cagcgcggta cagaccatgc acatgaacca ctggacgctg gggtatccca at 772

<210> 50
<211> 1362
<212> DNA
<213> Homo sapiens

<400> 50
tggtttcctt tcgcttctcg cctcccaaac acctccagca agtcggaggg cgcgaacgcg 60

gagccagaaa cccttcccca aagtttctcc cgccaggtac ctaattgaat catccatagg 120

atgacaaatc agccagggcc aagatttcca gacacttgag tgacttcccg gtccccgagg 180

tgacttgtca gctccagtga gtaacttgga actgtcgctc ggggcaaggt gtgtgtctag 240

gagagagccg gcggctcact cacgctttcc agagagcgac ccgggccgac ttcaaaatac 300

acacagggtc atttataggg actggagccg cgcgcaggac aacgtctccg agactgagac 360

attttccaaa cagtgctgac attttgtcgg gccccataaa aaatgtaaac gcgaggtgac 420

gaacccggcg gggagggttc gtgtctggct gtgtctgcgt cctggcggcg tgggaggtta 480

tagttccaga cctggcggct gcggatcgcc gggccggtac ccgcgaggag tgtaggtacc 540

ctcagcccga ccacctcccg caatcatggg gacaccggct tggatgagac acaggcgtgg 600

aaaacagcct tcgtgaaact ccacaaacac gtggaacttg aaaagacaac tacagccccg 660

cgtgtgcgcg agagacctca cgtcacccca tcagttccca cttcgccaaa gtttcccttc 720

agtggggact ccagagtggt gcgccccatg cccgtgcgtc ctgtaacgtg ccctgattgt 780

gtacccctct gcccgctcta cttgaaatga aaacacaaaa actgttccga attagcgcaa 840

ctttaaagcc ccgttatctg tcttctacac tgggcgctct taggccactg acagaaacat 900

ggtttgaacc ctaattgttg ctatcagtct cagtcagcgc aggtctctca gtgacctgtg 960

acgccgggag ttgaggtgcg cgtatcctta aacccgcgcg aacgccaccg gctcagcgta 1020

gaaaactatt tgtaatccct agtttgcgtc tctgagcttt aactccccca cactctcaag 1080

cgcccggttt ctcctcgtct ctcgcctgcg agcaaagttc ctatggcatc cacttaccag 1140

gtaaccggga tttccacaac aaagcccggc gtgcgggtcc cttcccccgg ccggccagcg 1200

cgagtgacag cgggcggccg gcgctggcga ggagtaactt ggggctccag cccttcagag 1260

cgctccgcgg gctgtgcctc cttcggaaat gaaaaccccc atccaaacgg ggggacggag 1320

cgcggaaacc cggcccaagt gccgtgtgtg cgcgcgcgtc tg 1362

<210> 51
<211> 476
<212> DNA
<213> Homo sapiens

<400> 51
gaaagccatc cttaccattc ccctcaccct ccgccctctg atcgcccacc cgccgaaagg 60

gtttctaaaa atagcccagg gcttcaaggc cgcgcttctg tgaagtgtgg agcgagcggg 120

cacgtagcgg tctctgccag gtggctggag ccctggaagc gagaaggcgc ttcctccctg 180

catttccacc tcaccccacc cccggctcat ttttctaaga aaaagttttt gcggttccct 240

ttgcctccta cccccgctgc cgcgcggggt ctgggtgcag acccctgcca ggttccgcag 300

tgtgcagcgg cggctgctgc gctctcccag cctcggcgag ggttaaaggc gtccggagca 360

ggcagagcgc cgcgcgccag tctattttta cttgcttccc ccgccgctcc gcgctccccc 420

ttctcagcag ttgcacatgc cagctctgct gaaggcatca atgaaaacag cagtag 476

<210> 52
<211> 300
<212> DNA
<213> Homo sapiens

<400> 52
atcgaaaatg tcgacatctt gctaatggtc tgcaaacttc cgccaattat gactgacctc 60

ccagactcgg ccccaggagg ctcgtattag gcagggaggc cgccgtaatt ctgggatcaa 120

aagcgggaag gtgcgaactc ctctttgtct ctgcgtgccc ggcgcgcccc cctcccggtg 180

ggtgataaac ccactctggc gccggccatg cgctgggtga ttaatttgcg aacaaacaaa 240

agcggcctgg tggccactgc attcgggtta aacattggcc agcgtgttcc gaaggcttgt 300

<210> 53
<211> 1246
<212> DNA
<213> Homo sapiens

<400> 53
   atcaacatcg tggctttggt cttttccatc atggtgagtg aatcacggcc agaggcagcc 60
   tgggaggaga gacccgggcg gctttgagcc cctgcagggg agtccgcgcg ctctctgcgg 120
   ctcccttcct cacggcccgg cccgcgctag gtgttctttg tcctcgcacc tcctcctcac 180
   ctttctcggg ctctcagagc tctccccgca atcatcagca cctcctctgc actcctcgtg 240
   gtactcagag ccctgatcaa gcttccccca ggctagcttt cctcttcttt ccagctccca 300
   gggtgcgttt cctctccaac ccggggaagt tcttccgtgg actttgctga ctcctctgac 360
   cttcctaggc acttgcccgg ggcttctcaa ccctcttttc tagagcccca gtgcgcgcca 420
   ccctagcgag cgcagtaagc tcataccccg agcatgcagg ctctacgttc ctttccctgc 480
   cgctccgggg gctcctgctc tccagcgccc aggactgtct ctatctcagc ctgtgctccc 540
   ttctctcttt gctgcgccca agggcaccgc ttccgccact ctccgggggg tccccaggcg 600
   attcctgatg ccccctcctt gatcccgttt ccgcgctttg gcacggcacg ctctgtccag 660
   gcaacagttt cctctcgctt cttcctacac ccaacttcct ctccttgcct ccctccggcg 720
   cccccttttt aacgcgcccg aggctggctc acacccacta cctctttagg cctttcttag 780
   gctccccgtg tgcccccctc accagcaaag tgggtgcgcc tctcttactc tttctaccca 840
   gcgcgtcgta gttcctcccc gtttgctgcg cactggccct aacctctctt ctcttggtgt 900
   cccccagagc tcccaggcgc ccctccaccg ctctgtcctg cgcccggggc tctcccggga 960
   atgaactagg ggattccacg caacgtgcgg ctccgcccgc cctctgcgct cagacctccc 1020
   gagctgcccg cctctctagg agtggccgct ggggcctcta gtccgccctt ccggagctca 1080
   gctccctagc cctcttcaac cctggtagga acacccgagc gaaccccacc aggagggcga 1140
   cgagcgcctg ctaggccctc gccttattga ctgcagcagc tggcccgggg gtggcggcgg 1200
   ggtgaggttc gtaccggcac tgtcccggga caacccttgc agttgc 1246

<210> 54
<211> 984
<212> DNA
<213> Homo sapiens

<400> 54
acaaataaaa caccctctag cttcccctag actttgttta actggccggg tctccagaag 60

gaacgctggg gatgggatgg gtggagagag ggagcggctc aaggacttta gtgaggagca 120

ggcgagaagg agcacgttca ggcgtcaaga ccgatttctc cccctgcttc gggagacttt 180

tgaacgctcg gagaggcccg gcatctcacc actttacttg gccgtagggg cctccggcac 240

ggcaggaatg agggaggggg tccgattgga cagtgacggt ttggggccgt tcggctatgt 300

tcagggacca tatggtttgg ggacagcccc agtagttagt aggggacggg tgcgttcgcc 360

cagtccccgg atgcgtaggg aggcccagtg gcaggcagct gtcccaagca gcgggtgcgc 420

gtccctgcgc gctgtgtgtt cattttgcag agccagcctt cggggaggtg aaccagctgg 480

gaggagtgtt cgtgaacggg aggccgctgc ccaacgccat ccggcttcgc atcgtggaac 540

tggcccaact gggcatccga ccgtgtgaca tcagccgcca gctacgggtc tcgcacggct 600

gcgtcagcaa gatcctggcg cgatacaacg agacgggctc gatcttgcca ggagccatcg 660

ggggcagcaa gccccgggtc actaccccca ccgtggtgaa acacatccgg acctacaagc 720

agagagaccc cggcatcttc gcctgggaga tccgggaccg cctgctggcg gacggcgtgt 780

gcgacaagta caatgtgccc tccgtgagct ccatcagccg cattctgcgc aacaagatcg 840

gcaacttggc ccagcagggt cattacgact catacaagca gcaccagccg acgccgcagc 900

cagcgctgcc ctacaaccac atctactcgt accccagccc tatcacggcg gcggccgcca 960

aggtgcccac gccacccggg gtgc 984

<210> 55
<211> 545
<212> DNA
<213> Homo sapiens

<400> 55
aggaggcgca acgcgctgcc agggcggctt tatcctgccg ccacagggcg gggaccagcc 60

cggcagccgg gtgtccagcg ccgctcacgt gcctcgcctg gagcttagct ctcagactcc 120

gaagagggcg actgagactt gggcctggga gttggcttcg gggtacccaa ggcgacgaca 180

gctgagttgt accacgaagc tcaggccgag gcctcctccc ttgtctggcc ttcgaatcca 240

tactggcagc ctctcctctc aggcactccg cgggccgggc cactaggccc cctgctcctg 300

gagctgcgct atgatccggg tcttgagatg cgcgcgattc tctctgaacc ggtggagagg 360

aggctctgcc ccgcgcggag cgaggacagc ggcgcccgag cttcccgcgc ctctccaggg 420

cccaatggca agaacagcct ccgaagtgcg cggatgacag gaaaagatct tcagttcttc 480

tgccgctaga gaagtgcggg atacaagcct ctattggatc cacaacctgg agtcctgcct 540

tcgga 545

<210> 56
<211> 1533
<212> DNA
<213> Homo sapiens

<400> 56
atctgcgtgc ccttttctgg gcgagccctg ggagatccag ggagaactgg gcgctccaga 60

tggtgtatgt ctgtaccttc acagcaaggc ttcccttgga tttgaggctt cctattttgt 120

ctgggatcgg ggtttctcct tgtcccagtg gcagccccgc gttgcgggtt ccgggcgctg 180

cgcggagccc aaggctgcat ggcagtgtgc agcgcccgcc agtcgggctg gtgggttgtg 240

cactccgtcg gcagctgcag aaaggtggga gtgcaggtct tgcctttcct caccgggcgg 300

ttggcttcca gcaccgaggc tgacctatcg tggcaagttt gcggcccccg cagatcccca 360

gtggagaaag agggctcttc cgatgcgatc gagtgtgcgc ctccccgcaa agcaatgcag 420

accctaaatc actcaaggcc tggagctcca gtctcaaagg tggcagaaaa ggccagacct 480

aactcgagca cctactgcct tctgcttgcc ccgcagagcc ttcagggact gactgggacg 540

cccctggtgg cgggcagtcc catccgccat gagaacgccg tgcagggcag cgcagtggag 600

gtgcagacgt accagccgcc gtggaaggcg ctcagcgagt ttgccctcca gagcgacctg 660

gaccaacccg ccttccaaca gctggtgagg ccctgcccta cccgccccga cctcgggact 720

ctgcgggttg gggatttagc cacttagcct ggcagagagg ggagggggtg gccttgggct 780

gaggggctgg gtacagccct aggcggtggg ggagggggaa cagtggcggg ctctgaaacc 840

tcacctcggc ccattacgcg ccctaaacca ggtctccctg gattaaagtg ctcacaagag 900

aggtcgcagg attaaccaac ccgctccccc gccctaatcc ccccctcgtg cgcctgggga 960

cctggcctcc ttctccgcag ggcttgctct cagctggcgg ccggtcccca agggacactt 1020

tccgactcgg agcacgcggc cctggagcac cagctcgcgt gcctcttcac ctgcctcttc 1080

ccggtgtttc cgccgcccca ggtctccttc tccgagtccg gctccctagg caactcctcc 1140

ggcagcgacg tgacctccct gtcctcgcag ctcccggaca cccccaacag tatggtgccg 1200

agtcccgtgg agacgtgagg gggacccctc cctgccagcc cgcggacctc gcatgctccc 1260

tgcatgagac tcacccatgc tcaggccatt ccagttccga aagctctctc gccttcgtaa 1320

ttattctatt gttatttatg agagagtacc gagagacacg gtctggacag cccaaggcgc 1380

caggatgcaa cctgctttca ccagactgca gacccctgct ccgaggactc ttagtttttc 1440

aaaaccagaa tctgggactt accaggttta gctctgccct ctcctctcct ctctacgtgg 1500

ccgccgctct gtctctccac gccccacctg tgt 1533

<210> 57
<211> 702
<212> DNA
<213> Homo sapiens

<400> 57
aggtctcttc agactgccca ttctccgggc ctcgctgaat gcgggggctc tatccacagc 60

gcgcggggcc gagctcaggc aggctggggc gaagatctga ttctttcctt cccgccgcca 120

aaccgaatta atcagtttct tcaacctgag ttactaagaa agaaaggtcc ttccaaataa 180

aactgaaaat cactgcgaat gacaatacta tactacaagt tcgttttggg gccggtgggt 240

gggatggagg agaaagggca cggataatcc cggagggccg cggagtgagg aggactatgg 300

tcgcggtgga atctctgttc cgctggcaca tccgcgcagg tgcggctctg agtgctggct 360

cggggttaca gacctcggca tccggctgca ggggcagaca gagacctcct ctgctagggc 420

gtgcggtagg catcgtatgg agcccagaga ctgccgagag cactgcgcac tcaccaagtg 480

ttaggggtgc ccgtgataga ccgccaggga aggggctggt tcggagggaa ttcccgctac 540

cgggaaggtc ggaactcggg gtgatcaaac aaggaatgca tctcacctcc gtgggtgctt 600

gtgctgcgca aggaattatt accggagcgg ttgcgatggc ctttgcccgg cgacccaaga 660

agagtaagca aactaccgtc cacccagcgg atcaggtcca at 702

<210> 58
<211> 3180
<212> DNA
<213> Homo sapiens

<400> 58
gatgtcctgt ttctagcagc ctccagagcc aagctaggcg agaggcgtag gaggcagaga 60

gagcgggcgc gggaggccag ggtccgcctg ggggcctgag gggacttcgt ggggtcccgg 120

gagtggccta gaaacaggga gctgggaggg ccgggaagag cttgaggctg agcgggggac 180

gaacgggcag cgcaaagggg agatgaacgg aatggccgag gagccacgca ttcgccttgt 240

gtccgcggac ccttgttccc gacaggcgac caagccaagg ccctccggac tgacgcggcc 300

tgagcagcag cgagtgtgaa gtttggcacc tccggcggcg agacggcgcg ttctggcgcg 360

cggctcctgc gtccggctgg tggagctgct gcgccctatg cggcctgccg agggcgccgc 420

cgagggcccg cgagctccgt ggggtcgggg tggggggacc cgggagcgga cagcgcggcc 480

cgaggggcag gggcaggggc gcgcctggcc tggggtgtgt ctgggccccg gctccgggct 540

cttgaaggac cgcgagcagg aggcttgcgc aatcccttgg ctgagcgtcc acggagaaag 600

aaaaagagca aaagcagagc gagagtggag cgagggatgg gggcgggcaa agagccatcc 660

gggtctccac caccgccctg acacgcgacc cggctgtctg ttggggaccg cacgggggct 720

cgggcgagca ggggagggag gagcctgcgc ggggctcgtg ttcgcccagg aatcccggag 780

aagctcgaag acggtctggt gttgaacgca cacgtggact ccatttcatt accaccttgc 840

agctcttgcg ccacggaggc tgctgctgcc cggcggctgc tacccaccga gacccacgtg 900

gcccctcccc aggggtgtag gggtgacggt tgtcttctgg tgacagcaga ggtgttgggt 960

ttgcgactga tctctaacga gcttgaggcg caaacctagg attccctgag tgttggggtg 1020

cggcgggggg gcaagcaagg tgggacgacg cctgcctggt ttccctgact agttgcgggg 1080

ggtgggggcc ggctctcagg ggccaccaga agctgggtgg gtgtacagga aaatattttt 1140

ctcctgccgt gtttggcttt ttcctggcat ttttgcccag ggcgaagaac tgtcgcgcgg 1200

ggcagctcca ccgcggaggg agaggggtcg cgaggctggc gcgggaagcg ctgtaggtgg 1260

cagtcatccg tccacgccgc acaggccgtc tgcgccgtcg gaccatcggg aggtctgcag 1320

caactttgtc ccggccagtc cccttgtccg ggaaggggct gagcttcccg acactctacc 1380

ctccccctct tgaaaatccc ctggaaaatc tgtttgcaat gggtgtttcc gcggcgtcca 1440

ggtctgggct gccgggggag gccgagcggc tgctgcagcc tccctgctgc caggggcgtc 1500

ggactccgct tcgctcacta cgcccaggcc cctcaggggc ccacgctcag gacttcgggg 1560

ccacacagca ggacccggtg ccccgacgac gagtttgcgc aggacccggg ctgggccagc 1620

cgcggagctg ggaggaaggg gcgggggtc ggtgcagcgg atcttttctg ttgctgcctg 1680

tgcggcggca ggaagcgtct tgaggctccc caagactacc tgaggggccg cccaagcact 1740

tcagaagccc aaggagcccc cggccacccc cgctcctggc ctttttgcca acgactttga 1800

aagtgaaatg cacaagcacc agcaattgac ttcccttccg tggttattta ttttgtcttt 1860

gtggatggtg ggcagatggg gagagaggcc cctacctaac ctcggtggct ggtccctaga 1920

ccacccctgc cagccggtgt ggggaggagc tcaggtccgc gggagagcga atgggcgcca 1980

ggaggtggga cagaatcctg ggaaggtaca gcggacgccc tggaagctcc cctgatgccc 2040

cagagggccc ttcctgggaa acctcccggg ggggtgcccc ataccatccc acccggctgt 2100

cttggcccct cccagggagc cgcaggagaa actagcccta cacctgggat tcccagagcc 2160

ttctgctggg gctcctgccc ccgacttcgg ataaccagct ccgcacaggt ccccgagaag 2220

ggccgctggc ctgcttattt gatactgccc cctcccagac aggggctggt cgagcccctg 2280

gttctgctgc cagactgaag ccttccagac gccacctcgg tttgggcccc cagggccctc 2340

aggggcccca ggagaggaga gctgctatct agctcagcca caggctcgct cctggtgggg 2400

gccaggctga aggagtggac cctggagagg tcgggaacct tttaacagcc gtgggctgga 2460

gggtggctac taagtgttcg gtctgggaag aggcatgacc cgcaccatcc cggggaaata 2520

aacgacttct taagggaatc ttctcgctga gcgggtgctc tgggccagga gattgccacc 2580

gccagcccac ggaacccaga tttgggctct gccttgagcg ggccgcctgt ggcttcccgg 2640

gtcgctcccc cgactcagaa agctctcaag ttggtatcgt tttcccggcc ctcggaggtg 2700

gattgcagat caccgagagg ggatttacca gtaaccacta cagaatctac ccgggcttta 2760

acaagcgctc atttctctcc cttgtcctta gaaaaacttc gcgctggcgt tgatcatatc 2820

gtacttgtag cggcagctta ggggcagcgg aactggtggg gttgtgcgtg cagggggagg 2880

ctgtgaggga gccctgcact ccgcccctcc acccttctgg aggagtggct ttgtttctaa 2940

gggtgccccc ccaacccccg ggtccccact tcaatgtttc tgctctttgt cccaccgccc 3000

gtgaaagctc ggctttcatt tggtcggcga agcctccgac gcccccgagt cccaccctag 3060

cgggccgcgc ggcactgcag ccgggggttc ctgcggactg gcccgacagg gtgcgcggac 3120

ggggacgcgg gccccgagca ccgcgacgcc agggtccttt ggcagggccc aagcacccct 3180

<210> 59
<211> 1038
<212> DNA
<213> Homo sapiens

<400> 59
tggcggccgg cgggcacagc cggctcattg ttctgcacta caaccactcg ggccggctgg 60

ccgggcgcgg ggggccggag gatggcggcc tgggggccct gcgggggctg tcggtggccg 120

ccagctgcct ggtggtgctg gagaacttgc tggtgctggc ggccatcacc agccacatgc 180

ggtcgcgacg ctgggtctac tattgcctgg tgaacatcac gctgagtgac ctgctcacgg 240

gcgcggccta cctggccaac gtgctgctgt cgggggcccg caccttccgt ctggcgcccg 300

cccagtggtt cctacgggag ggcctgctct tcaccgccct ggccgcctcc accttcagcc 360

tgctcttcac tgcaggggag cgctttgcca ccatggtgcg gccggtggcc gagagcgggg 420

ccaccaagac cagccgcgtc tacggcttca tcggcctctg ctggctgctg gccgcgctgc 480

tggggatgct gcctttgctg ggctggaact gcctgtgcgc ctttgaccgc tgctccagcc 540

ttctgcccct ctactccaag cgctacatcc tcttctgcct ggtgatcttc gccggcgtcc 600

tggccaccat catgggcctc tatggggcca tcttccgcct ggtgcaggcc agcgggcaga 660

aggccccacg cccagcggcc cgccgcaagg cccgccgcct gctgaagacg gtgctgatga 720

tcctgctggc cttcctggtg tgctggggcc cactcttcgg gctgctgctg gccgacgtct 780

ttggctccaa cctctgggcc caggagtacc tgcggggcat ggactggatc ctggccctgg 840

ccgtcctcaa ctcggcggtc aaccccatca tctactcctt ccgcagcagg gaggtgtgca 900

gagccgtgct cagcttcctc tgctgcgggt gtctccggct gggcatgcga gggcccgggg 960

actgcctggc ccgggccgtc gaggctcact ccggagcttc caccaccgac agctctctga 1020

ggccaaggga cagctttc 1038

<210> 60
<211> 374
<212> DNA
<213> Homo sapiens

<400> 60
tagtaaggca ccgaggggtg gctcctctcc ctgcagcggc tgtcgcttac catcctgtag 60

accgtgacct cctcacacag cgccaggacg aggatcgcgg tgagccagca ggtgactgcg 120

atcctggagc tggtcgcagc aggccatcct gcacgcggtg gaggcgcccc ctgcaggccg 180

cagcgcatcc ccagcttctg gacgcactgt gagcggttat gcagcagcac gctcatatga 240

gatgccccgc agggtgctat gcaggcccac gtccccacaa agcccatggc aggcgcccgg 300

gtgccggagc acgcacttgg ccccatggat ctctgtgccc agggctcagc caggcatctg 360

gccgctaaag gttt 374

<210> 61
<211> 246
<212> DNA
<213> Homo sapiens

<400> 61
tctcatctga gcgctgtctt tcaccagagc tctgtaggac tgaggcagta gcgctggccc 60

gcctgcgaga gcccgaccgt ggacgatgcg tcgcgccctt cccatcgcgg cctgggcggg 120

cccgcctgcc ctcggctgag cccggtttcc ctaccccggg gcacctcccc tcgcccgcac 180

ccggccccag tccctcccag gcttgcgggt agagcctgtc tttgcccaga aggccgtctc 240

caagct 246

<210> 62
<211> 222
<212> DNA
<213> Homo sapiens

<400> 62
cagtccccga ggccctcccc ggtgactcta accagggatt tcagcgcgcg gcgcggggct 60

gcccccaggc gtgacctcac ccgtgctctc tccctgcaga atctcctacg acccggcgag 120

gtaccccagg tacctgcctg aagcctactg cctgtgccgg ggctgcctga ccgggctgtt 180

cggcgaggag gacgtgcgct tccgcagcgc ccctgtctac at 222

<210> 63
<211> 2209
<212> DNA
<213> Homo sapiens

<400> 63
agagagacat tttccacgga ggccgagttg tggcgcttgg ggttgtgggc gaaggacggg 60

gacacggggg tgaccgtcgt ggtggaggag aaggtctcgg aactgtggcg gcggcggccc 120

ccctgcgggt ctgcgcggat gaccttggcg ccgcggtggg ggtccggggg ctggctggcc 180

tgcaggaagg cctcgactcc cgacacctgc tccatgaggc tcagcctctt cacgcccgac 240

gtcgggctgg ccacgcgggc agcttctggc ttcggggggg ccgcgatagg ttgcggcggg 300

gtggcggcca caccaaaagc catctcggtg tagtcaccat tgtccccggt gtccgaggac 360

aacgatgagg cggcgcccgg gccctgggcg gtggcaacgg ccgaggcggg gggcaggcgg 420

tacagctccc ccggggccgg cggcggtggc ggcggctgca gagacgacga cggggacgcg 480

gacggacgcg ggggcaacgg cggatacggg gaggaggcct cgggggacag gaggccgtcc 540

aaggagccca cggggtggcc gctcggggcg cccggcttag gagacttggg ggagctgaag 600

tcgaggttca tgtagtcgga gagcggagac cgctgccggc tgtcgctgct ggtgcccggg 660

gtgcctgagc ccagcgacga ggccgggctg ctggcggaca agagcgagga ggacgaggcc 720

gccgacgcca gcaggggagg cgcgggcggc gacaggcggg ccccgggctc gccaaagtcg 780

atgttgatgt actcgccggg gctcttgggc tccggtggca gtgggtactc gtgcatgctg 840

ggcaggctgg gcagcccctc caggacaggg cgcgtgggcc tcaccgcccg gccgcgctgg 900

cccaagaagc cctccgggcg gccgccgcta ggccgcacgg gcgaaggcac tacagggtga 960

gggggctgcg tggggccggc cccgaaggcg ctggccgcct ggctgggccc tggcgtggcc 1020

tgaggctcca gacgctcctc ctccaggatg cgccccacgg gggagctcat gagcacgtac 1080

tggtcgctgt ccccgccaca ggtgtagggg gccttgtagg agcggggcaa ggagctgtag 1140

cagcagccgg gaacgcccct gagcggctcc ccgccggggt gcagggctgc ggagaagaag 1200

tcgggcgggg tgcccgtggt gaccgcgtcg ctgggggaca cgttgaggta gtccccgttg 1260

ggcagcagct tgccatctgc atgctccatg gacagcttgg aaccgcacca catgcgcatg 1320

tacccactgt cctcggggga gctctcggcg ggcgagctgg ccttgtagcc gcccccgctc 1380

gccgggaatg tcctgcccgc cgcagaggtg ggtgctggcc ccgcaggccc cgcagaaggc 1440

acggcggcgg cggcggcggc ggcggccctg ggctgcaaga tctgcttggg ggcggacacg 1500

ctggcggggc tcatgggcat gtagtcgtcg ctcctgcagc tgccgctccc actgcccgcg 1560

agggccgcgc cgggcgtcat gggcatgtag ccgtcgtctg cccccaggtt gctgctggag 1620

ctcctgtggg agccgatctc gatgtctccg tagtcctctg ggtaggggtg gtaggccacc 1680

ttgggagagg acgcggggca ggacgggcag aggcggcccg cgctgcccga gaaggtggcc 1740

cgcatcaggg tgtattcatc cagcgaggca gaggagggct ggggcaccgg ccgctgccgg 1800

gctggcgtgg tcagggagta ggtcctcttg cgcagccctc ggtccaggtc ctgggccgcg 1860

tcccccgaga cccggcggta ggagcggcca cagtggctca ggggcctgtc catggtcatg 1920

tacccgtaga actcaccgcc gccgccgccg tctcgggccg ggggcgtctc cgcgatggac 1980

tcgggcgtgt tgcttcggtg gctgcagaag gcgcgcaggt cgcctgggct ggagccgtac 2040

tcgtccaggg acatgaagcc ggggtcgctg ggggagcccg aggcggaggc gctgccgctg 2100

gagggccgct ggccggggcc gtggtgcagc ggatgcggca gaggcgggtg cgggccgggc 2160

ggcggcgggt aggagcccga gccgtggccg ctgctggacg acagggagc 2209

<210> 64
<211> 149
<212> DNA
<213> Homo sapiens

<400> 64

taacctaaag aatgaagtca tgccccggcc tgcacccggg aaactgcaca cagcgaaaga 60

tcgccactga gataaagagc tgaaagctat tccccaattc agctgtttca gccgtgcggt 120

ctcacaatgg gctcacagac ggcagcatc 149

<210> 65
<211> 832
<212> DNA
<213> Homo sapiens

<400> 65
gtttccacaa tccacctcgt agctggggcg tgccgcttgc ctcggcttgt cccggcagaa 60

cactcttacc tttaatggcg actgaaaagt tgccacgagt tcctgatcat tgtggtaggt 120

gctgcgtgaa gctgagacgt gcgtgagcca catcccaggg ggctttgagc ccccaccgcg 180

gcggcggctg aggggaggct tgtcgtactc gcacaggagg acacagggct gcagtgttca 240

ctccagggcc tcttatcatt gggatctgag gaattttccg agaggaagtg cgaattaaca 300

atgatgaaag gtttgtgagt gagtgacagg cacgttctat tgagcactgc atggggcatt 360

atgtgccacc agagacgggg gcagaggtca agagccctcg agggctggga gagttcggag 420

gatagaagtc atcagagcac aatgaagcca gaccctgcag ccgccttccc cttcgggggc 480

ttccttagaa tgcagcattg cggggactga gctgtcccag gtgaaggggg gccgtcacgg 540

tgtgtggacg cccctcggct cagccctcta agagactcgg cagccaggat gggctcaagg 600

catgagccct caaaggaggt taggaaggag cgagggagaa aagatatgct tgtgtgacgt 660

cctggccgaa gtgagaacaa ttgtatcaga taatgagtca tgtcccattg aggggtgccg 720

acaaggactc gggaggaggc cacggagccc tgtactgagg agacgcccac agggagcctc 780

gggggcccag cgtcccggga tcactggatg gtaaagccgc cctgcctggc gt 832

<210> 66
<211> 256
<212> DNA
<213> Homo sapiens

<400> 66
tccagctgca gcgagggcgg ccaggccccc ttctccgacc tgcaggggta gcgcggcctc 60

ggcgccggag acccgcgcgc tgtctggggc tgcggtggcg tggggagggc gcggcccccg 120

gacgccccga ggaaggggca cctcaccgcc cccacccaga gcgcctggcc gtgcgggctg 180

cagaggaccc ctccggggca gaggcaggtt ccacggaaga ccccggcccg ctggggcttc 240

cccggagact ccagag 256

<210> 67
<211> 184
<212> DNA
<213> Homo sapiens

<400> 67
acttactgct tccaaaagcg ctgggcacag ccttatatga ctgaccccgc ccccgagtcc 60

caggccgccc catgcaaccg cccaaccgcc caaccgccac tccaaaggtc accaaccact 120

gctccaggcc acgggctgcc tctccccacg gctctagggc ccttcccctc caccgcaggc 180

tgac 184

<210> 68
<211> 118
<212> DNA
<213> Homo sapiens

<400> 68
tgccacaccc aggtaccgcc cgcccgcgcg agagccgggc aggtgggccg cggatgctcc 60

cagaggccgg cccagcagag cgatggactt ggacaggcta agatggaagt gacctgag 118

<210> 69
<211> 1534
<212> DNA
<213> Homo sapiens

<400> 69
   tcgccagcgc agcgctggtc catgcaggtg ccacccgagg tgagcgcgga ggcaggcgac 60
   gcggcagtgc tgccctgcac cttcacgcac ccgcaccgcc actacgacgg gccgctgacg 120
   gccatctggc gcgcgggcga gccctatgcg ggcccgcagg tgttccgctg cgctgcggcg 180
   cggggcagcg agctctgcca gacggcgctg agcctgcacg gccgcttccg gctgctgggc 240
   aacccgcgcc gcaacgacct ctcgctgcgc gtcgagcgcc tcgccctggc tgacgaccgc 300
   cgctacttct gccgcgtcga gttcgccggc gacgtccatg accgctacga gagccgccac 360
   ggcgtccggc tgcacgtgac aggcgaggcg gcgtgggagc gggtccccgg cctcccttcc 420
   cgccctcccg cctgccccgc cccaagggct acgtgggtgc caggcgctgt gctgagccag 480
   gaagggcaac gagacccagc cctctcctct accccaggga tctcacacct gggggtagtt 540
   taggaccacc tgggagcttg acacaaatgc agaatccagg tcccaggaag ggctgaggtg 600
   ggcccgggaa taggcattgc cgtgactctc gtagagtgac tgtccccagt ggctctcaga 660
   cgaagaggcg agaaagacaa gtgaatggca atcctaaata tgccaagagg tgcaatgtgg 720
   tgtgtgctac cagcccggaa agacactcgc agcccctcta cccaggggtg cacagacagc 780
   ccaccaagta gtgcctagca ctttgccaga ccctgatata caaagatgcc tgaaccaggg 840
   tcccgtccct agagcagtgg ctctccactc tagcccccac cctgctctgc gacaataatg 900
   gccacttagc atttgctagg gagccgggac ctagtccaag cacccacaag catgaatttg 960
   ccaaatcttt tcagcaacct cttaaggcaa ctgctatcat gatcctcact ttacacatgg 1020
   agaagcagaa gcagagatga tagaatcttt cgcccaaggc cacatctgta ttgggacggg 1080
   ggcagcctgg cacccaagtg cccattcctc ccttctgacc agcccccacc cctccggctc 1140
   tggcgtccaa agggctaagg ggaggggtgc ccttgtgaca gtcacccgcc ttctcccctg 1200
   cagccgcgcc gcggatcgtc aacatctcgg tgctgcccag tccggctcac gccttccgcg 1260
   cgctctgcac tgccgaaggg gagccgccgc ccgccctcgc ctggtccggc ccggccctgg 1320
   gcaacagctt ggcagccgtg cggagcccgc gtgagggtca cggccaccta gtgaccgccg 1380
   aactgcccgc actgacccat gacggccgct acacgtgtac ggccgccaac agcctgggcc 1440
   gctccgaggc cagcgtctac ctgttccgct tccatggcgc cagcggggcc tcgacggtcg 1500
   ccctcctgct cggcgctctc ggcttcaagg cgct 1534

<210> 70
<211> 269
<212> DNA
<213> Homo sapiens

<400> 70
atgaacttca agggcgacat catcgtggtc tacgtcagcc agacctcgca ggagggcgcg 60

gcggcggctg cggagcccat gggccgcccg gtgcaggagg agaccctggc gcgccgagac 120

tccttcgcgg ggaacggccc gcgcttcccg gacccgtgcg gcggccccga ggggctgcgg 180

gagccggaga aggcctcgag gccggtgcag gagcaaggcg gggccaaggc ttgagcgccc 240

cccatggctg ggagcccgaa gctcggagc 269

<210> 71
<211> 282
<212> DNA
<213> Homo sapiens

<400> 71
   tcagtgttat gtggggagcg ctagatcgtg cacacagtag gcgtcaggaa gtgttttccc 60
   cagtaattta ttctccatgg tactttgcta aagtcatgaa ataactcaga ttttgttttc 120
   caaggaagga gaaaggccca gaatttaaga gcaggcagac acacaaccgg gcacccccag 180
   accctggccc ttccagcagt caggaattga cttgccttcc aaagccccag cccggagctt 240
   gaggaacgga ctttcctgcg cagggggatc ggggcgcact cg 282

<210> 72
<211> 142
<212> DNA
<213> Homo sapiens

<400> 72
gtggaaacac aacctgcctt ccattgtctg cgcctccaaa acacaccccc cgcgcatccg 60

tgaagctgtg tgtttctgtg ttactacagg ggccggctgt ggaaatccca cgctccagac 120

cgcgtgccgg gcaggcccag cc 142

<210> 73
<211> 741
<212> DNA
<213> Homo sapiens

<400> 73
tccacacctc gggcagtcac taggaaaagg gtcgccaact gaaaggcctg caggaaccag      60

gatgatacct gcgtcagtcc cgcggctgct gcgagtgcgc gctctcctgc cagggggacc     120

tcagaccctc ctttacagca caccgagggc cctgcagaca cgcgagcggg ccttcagttt     180

gcaaaccctg aaagcgggcg cggtccacca ggacgatctg gcagggctct gggtgaggag     240

gccgcgtctt tatttggggt cctcgggcag ccacgttgca gctctggggg aagactgctt     300

aaggaacccg ctctgaactg cgcgctggtg tcctctccgg ccctcgcttc cccgaccccg     360

cacaggctaa cgggagacgc gcaggcccac cccaccggct ggagaccccg gcacggcccg     420

catccgccag gattgaagca gctggcttgg acgcgcgcag ttttcctttg gcgacattgc     480

agcgtcggtg cggccacaat ccgtccactg gttgtgggaa cggttggagg tcccccaaga     540

aggagacacg cagagctctc cagaaccgcc tacatgcgca tggggcccaa acagcctccc     600

aaggagcacc caggtccatg cacccgagcc caaaatcaca gacccgctac gggcttttgc     660

acatcagctc caaacacctg agtccacgtg cacaggctct cgcacagggg actcacgcac     720

ctgagttcgc gctcacagat c                                               741

<210> 74
<211> 4498
<212> DNA
<213> Homo sapiens

<400> 74
   ctgccctcgc ggatctcccc cggcctcgcc ggcctccgcc tgtcctccca ccaccctctc      60
   cgggccagta ccttgaaagc gatgggcagg gtcttgttgc agcgccagtg cgtaggcagc     120
   acggagcaga ggaagttggg gctgtcggtg cgcaccagct cgcccgggtg gtcggccagc     180
   acctccacca tgctgcggtc gccgctcctc agcttgccgg ccagggcagc gccggcgtcc     240
   ggggcgccca gcggcaacgc ctcgctcatc ttgcctgggc tcagcgcggt ggaaggcggc     300
   gtgaagcggc ggctcgtgct ggcatctacg gggatacgca tcacaacaag ccgattgagt     360
   taggaccctg caaacagctc ctaccagacg gcgacagggg cgcggatctt cagcaagcag     420
   ctcccgggag accaacatac acgttcaggg gcctttatta ctgcgggggg tggggggggg     480
   cgggggtggt taggggagga gggagactaa gttactaaca gtccaggagg ggaaaacgtt     540
   ctggttctgc ggatcggcct ctgacccagg atgggctcct agcaaccgat tgcttagtgc     600
   attaaaaagt ggagactatc ttccacgaat cttgcttgca gaggttaagt tctgtctttg     660
   gctgttagaa aagttcctga aggcaaaatt ctcatacact tcctaaaata tttatgcgaa 720
   gagtaaaacg atcagcaaac acattatttg gaagttccag tagttaatgc ctgtcagttt 780
   tttgcaggtg agttttgtct aaagtcccaa cagaacacaa ttatctcccg taacaaggcc 840
   acttttatca tgcaaaactg gcttcagtcc cgaaaagcaa gagctgagac ttccaaaggt 900
   agtgctacta atgtatgtgc acgtatatat aaatatatac atatgctcta cttcataaaa 960
   tatttacaat acaatctgtg gagaatttaa acacaacaga aatccattaa tgtacgctgc 1020
   agattttttt aagtagcctt gaaaatcagc ttcagtagtt ggagcagtgc tgagctagaa 1080
   gtacttgtca tgttctctgt tctctcaatg aattctgtca aaacgctcag tgcagaaaat 1140
   tcagcgtttc agagatcttc agctaatctt aaaacaacaa tcataagaag gcccagtcga 1200
   tgacactcag ggttctacag ctctcccaca tctgtgaact cgggtttggg gatgttggtt 1260
   aagtttgtgg ctggtcctct ggtttgttgg gagttgagca gccgcagagt cacacacatg 1320
   caaacacgca ctcttcggaa ggcagccact gtctacatca gctgggtgac tcagccctga 1380
   ctcgggcagc agcgagacga tactcctcca ccgtcgccca gcacccgccg gttagctgct 1440
   ccgaggcacg aacacccacg agcgccgcgt aaccgcagca ggtggagcgg gccttgaggg 1500
   agggctccgc ggcgcagatc gaaacagatc gggcggctcg ggttacacac gcacgcacat 1560
   cctgccacgc acactgccac gcacacgcaa cttcacggct cgcctcggac cacagagcac 1620
   tttctccccc tgttgtaaaa ggaaaacaat tggggaaaag ttcgcagcca ggaaagaagt 1680
   tgaaaacatc cagccaagaa gccagttaat tcaaaaggaa gaaaggggaa aaacaaaaaa 1740
   aaacaacaaa aaaaggaagg tccaacgcag gccaaggaga agcagcagag gttgacttcc 1800
   ttctggcgtc cctaggagcc ccggaaagaa gtgcctggcg gcgcagggcc gggcagcgtg 1860
   gtgccctggc tgggtccggc cgcggggcgc ccgtcccgcc cgcgcccgct ggctctatga 1920
   atgagagtgc ctggaaatga acgtgctttt actgtaagcc cggccggagg aattccattc 1980
   cctcagctcg tttgcatagg ggcggccggc ggccaatcac aggcctttcc ggtatcagcc 2040
   agggcgcggc tcgccgccgc cggctcctgg aattggcccg cgcgcccccg ccgccgcgcc 2100
   gcgcgctact gtacgcagcc cgggcgggga gtcggaggcc acccccgcgc cccgcatcca 2160
   agcctgcatg ctggcccggg gccccgcccg cgtgcggacc cctttccgca gccacacgca 2220
   ggcttgtgcg gctccgcgag tggccacggt ccggagacct ggaaaaagaa agcaggcccc 2280
   gccggcccga ggaggacccg gccggcgcgc cgcacccgga gaggcccggc cccgcgagcc 2340
   gctgcaggca ggcgcagtgg ccgccacgag gctcccgaac cgggctgcag cccgcggacg 2400
   gccccagatc ctgcgcggcc gcccagggcc aggcctccgc ttccagggcg ggggtgcgat 2460
   ttggccgcgg ggcccggggg agccactccg cgctcctgca ccgtccggct ggcagctgcg 2520
   gcgaagcggc gctgattcct tgcatgaggc cggacggcgt ccgcgcgtgc cgtttgctct 2580
   cagcgtcttc ccttgggtcg gtttctgtaa tgggtgtttt ttaccgctgc gcccgggccg 2640
   cggctcgatc cctccgcgcg tctcacttgc tgcgtgcgtc agcggccagc gaagagtttc 2700
   ctagtcagga aagaccccaa gaacgcgcgg ctggaaggaa agttgaaagc agccacgcgg 2760
   cttgctcccg ggccttgtag cgccggcacc cgcagcagcc ggacagcctg cccgggcccc 2820
   gcgtctcccc tccggctccc cggaagcggc ccccgctcct ctccccgccc ccgtgcgctc 2880
   gagcggcccc aggtgcggaa cccaccccgg cttcgcgtgc gggcggccgc ttccccctgc 2940
   gccggtcccc gcggtgctgc gggcattttc gcggagctcg gagggccccg cccccggtcc 3000
   ggcgtgcgct gccaactccg accccgcccg gcggggctcc ctcccagcgg aggctgctcc 3060
   cgtcaccatg agtccctcca cgccctccct gccgggccct gcacctcccg gggcctctca 3120
   tccaccccgg ggctgcaacc cagtccccgg atcccggccc cgttccaccg cgggctgctt 3180
   tgtggtcccc gcggagcccc tcaattaagc tccccggcgc gggggtccct cgccgacctc 3240
   acggggcccc tgacgcccgc tcctccctcc cccagggcta gggtgctgtg gccgctgccg 3300
   cgcagggact gtccccgggc gttgccgcgg gcccggacgc aggagggggc cggggttgac 3360
   tggcgtggag gcctttcccg ggcgggcccg gactgcgcgg agctgtcggg acgcgccgcg 3420
   ggctctggcg gacgccaggg ggcagcagcc gccctccctg gacgccgcgc gcagtccccg 3480
   gagctcccgg aacgcccccg acggcgcggg gctgtgcggc ccgcctcgtg gccttcgggt 3540
   cgcccgggaa gaactagcgt tcgaggataa aagacaggaa gccgccccag agcccacttg 3600
   agctggaacg gccaaggcgc gtttccgagg ttccaatata gagtcgcagc cggccaggtg 3660
   gggactctcg gaccaggcct ccccgctgtg cggcccggtc ggggtctctt cccgaagccc 3720
   ctgttcctgg ggcttgactc gggccgctct tggctatctg tgcttcagga gcccgggctt 3780
   ccggggggct aaggcgggcg gcccgcggcc tcaaccctct ccgcctccgc tccccctggg 3840
   cactgccagc acccgagttc agttttgttt taatggacct ggggtctcgg aaagaaaact 3900
   tactacattt ttcttttaaa atgatttttt taagcctaat tccagttgta aatccccccc 3960
   tccccccgcc caaacgtcca ctttctaact ctgtccctga gaagagtgca tcgcgcgcgc 4020
   ccgcccgccc gcaggggccg cagcgccttt gcctgcgggt tcggacgcgg cccgctctag 4080
   aggcaagttc tgggcaaggg aaaccttttc gcctggtctc caatgcattt ccccgagatc 4140
   ccacccaggg ctcctggggc cacccccacg tgcatccccc ggaacccccg agatgcggga 4200
   gggagcacga gggtgtggcg gctccaaaag taggcttttg actccagggg aaatagcaga 4260
   ctcgggtgat ttgcccctcg gaaaggtcca gggaggctcc tctgggtctc gggccgcttg 4320
   cctaaaaccc taaaccccgc gacgggggct gcgagtcgga ctcgggctgc ggtctcccag 4380
   gagggagtca agttccttta tcgagtaagg aaagttggtc ccagccttgc atgcaccgag 4440
   tttagccgtc agaggcagcg tcgtgggagc tgctcagcta ggagtttcaa ccgataaa 4498

<210> 75
<211> 453
<212> DNA
<213> Homo sapiens

<400> 75
ttcggaagtg agagttctct gagtcccgca cagagcgagt ctctgtcccc agcccccaag 60

gcagctgccc tggtgggtga gtcaggccag gcccggagac ttcccgagag cgagggaggg 120

acagcagcgc ctccatcaca gggaagtgtc cctgcgggag gccctggccc tgattgggcg 180

ccggggcgga gcggcctttg ctctttgcgt ggtcgcgggg gtataacagc ggcgcgcgtg 240

gctcgcagac cggggagacg ggcgggcgca cagccggcgc ggaggcccca cagccccgcc 300

gggacccgag gccaagcgag gggctgccag tgtcccggga cccaccgcgt ccgccccagc 360

cccgggtccc cgcgcccacc ccatggcgac ggacgcggcg ctacgccggc ttctgaggct 420

gcaccgcacg gagatcgcgg tggccgtgga cag 453

<210> 76
<211> 560
<212> DNA
<213> Homo sapiens

<400> 76
acgcacactg ggggtgtgat ggaaaggggg acgcgatgga taggggtggg cgcacactgg 60

gggacgcgac ggggaggggt gagcacacac tgggggtgtg atggagaggg cgacgcaata 120

gggaggggtg ggcgcacacc agggacgcga tgatggggac gggtgggcgc acaccaggtg 180

gcatgatggg gaggagtggg tacacaccat ggggggcgtg atggggaggc gtgggcgtac 240

accggggggc gcgatgggga ggggtgggcg cacaccgggg gacgcgatgg aggcggtggg 300

tgcacacggg gcgcgatggg tgggagtagg tgcacactga gggcacgatt ggggagacac 360

gaaggagagg ggtgggcgca cactggggga cgcgatggcc gggacacgat gcggagaagt 420

gggtgaatac cggggtcgcg atgggcgccc tggaaggacg gcagtgctgc tcacaggggc 480

caggcccctc agagcgcgcc ccttgggggt aaccccagac gcttgttccc gagccgactc 540

cgtgcactcg acacaggatc 560

<210> 77
<211> 157
<212> DNA
<213> Homo sapiens

<400> 77
ccacagggtg gggtgcgccc acctgccctg tccatgtggc cttgggcctg cgggggagag 60

ggaatcagga cccacagggc gagccccctc cgtagcccgc ggcaccgact ggatctcagt 120

gaacacccgt cagcccatcc agaggctaga aggggga 157

<210> 78
<211> 114
<212> DNA
<213> Homo sapiens

<400> 78
ttgaggtctc tgtgcatgct tgtgcgtacc ctggactttg ccgtgagggg tggccagtgc 60

tctgggtgcc tttgccagac aactggtctg ccgggccgag cattcatgct ggtc 114

<210> 79
<211> 104
<212> DNA
<213> Homo sapiens

<400> 79
tgacgcgccc ctctccccgc agctccacct ggttgcgctc aacagccccc tgtcaggcgg 60

catgcggggc atccgcgggg ccgacttcca gtgcttccag cagg 104

<210> 80
<211> 659
<212> DNA
<213> Homo sapiens

<400> 80
aacacactgt ctcgcactag gtgctcgcgg aagagcgcgg cgtcgatgct gcggctcagg 60

ttgatgggcg atggcggccg cagatccagc tcgctcagcg atggcgccgg tcccacaccg 120

ttgcgggaca gtcccgggcc accctggggt ccgcgaccca acgacgcagc cgagccccag 180

gcgcctgaac tgggcgtggc cagctgccca ctctccgccg ggttgcggat gaggctcttg 240

ctgatgtcca agctgcctgc accaacgttg ctgggccctg catagcagtt attgggtcgc 300

tccggcacct cgctctttcc tgacggcgcc gggcacgcca gacgcatcag cttagcccag 360

caagcgtgct ccgtgggcgg cctgggtctc gcggcagcca ccgcggccaa cgccagggcg 420

agcgcccatg tcagctccag gaggcgcagc cagaagtgga caccccacca ggcccacgag 480

aagcggccca cgcggcctgg gcccgggtac agccagagcg cagccgccag ctgcaagccg 540

ctagccagca gccccagcgc gcccgccaca gccaacagcc gagggcccgg gctggcatcc 600

cagccccgtg ggccgtccag caggcggcga cggcacaggc agagcgtgcc cagagccac 659

<210> 81
<211> 813
<212> DNA
<213> Homo sapiens

<400> 81
gtctgcacga agcccgcggc ggcctgcagg gggcccagcg actcgtccag ggaaccggtg 60

cgcaggagca gccgggggcg cggcgcgccg gccgcccttg ggggactctg gggccggggg 120

cgcagctcga tctgacgctt gggcactgtc cggggcctgg cgggcgcggc gccctcctcc 180

agagccacct ccacacactc gaactgcgct ggggcggcag gacttggccc acggggccgc 240

agctctaggt aggtggccca gcgggagcca ccatcgggga cctgggactg gcgtgggacc 300

gcggcgggag acgctggccc cggcggcaag gggctgatga aggccggctc cgtgaactgt 360

tgttgcgcct cgcgatcgtc tgcgccggag cagccgaaca ggggtccgac gccgaagatg 420

acttccatct cccccgacgg cagcgtgcgc agctggggct ggggtggccg tgggccggaa 480

cctgggcctc gcgggaaacc cgagccgggc ccgtgccgct ggcggctatt ctgggcgctg 540

acggacaggc gaggctgcgc gcccgccccc cgcccaggag ccacccaggg ccaattcgct 600

gggcctttcg cgtccggccc aacgtccggg ggctccggag aacctggagc cgtgtagtag 660

gagcctgacg aaccggagga gtcctggcgc cgcgcggggg ccgtgggcag ctgcctcggg 720

atcccaggca gggctggcgg ggcgagcgcg gtcagcatgg tggggccgga cgccgtgcac 780

tatctccctc gcattcgcct ccgctggtgg cgc 813

<210> 82
<211> 440
<212> DNA
<213> Homo sapiens

<400> 82
ctggagagaa ctatacgggc tgtgggagtc accgggcgac tatcaccggg cctcctttcc 60

acatcctcct ccgggaaggg accccgttcc gggcctcgac cggcgcagac tgggctgacc 120

cactttcttg ggcccactga gtcacctcga aacctccagg ccggtagcgg ggaggagagg 180

aggagcaggc gggggtgcca aggtgtgggc tgcgccctgg ttagggggcg agcccggctt 240

gtttatgagg aggagcgcgg aggaggatcc agacacacag gcttgcgcgc ccagactcgc 300

ccggccagcg gctggcggcc tccgacgtca ccaaaccggt tgggtgagag ggcagagagc 360

agggggaagg gccgcagtcc cgcccgcgcc ccccggcacg caccgtacat cttgccctcg 420

tctgacagga tgatcttccg 440

<210> 83
<211> 252
<212> DNA
<213> Homo sapiens

<400> 83
gagtgcggag tgaaggggtg cactgggcac tcagcgcggc ccttgggagg cagggccgcc 60

ccagcctgcc ctcctgtctg ggaaggccgt ccagaagcag gagccccggg gaaaacaact 120

ggctggacgg ggcggccttc agtgtctctc ccagcctgag agtcgcttcc caccacctgg 180

gcacgaacct gctctgcgat ctccggcaag ttcctgcgcc tcctgtcggt aaaatgcaga 240

tcgtggcgtc tt                                                          252

<210> 84
<211> 1539
<212> DNA
<213> Homo sapiens

<400> 84
tcttctttcc gcccctaggg ggcacaagcg ggcatgtcca agcgcctagg agcccgtacc      60

gctggggacc tccccttccg cgaaccccga gcgggtagac ccagagcaat ccgagtgtgg     120

aaacaatgga gagggggcgt gttgagctgg ggtctccatg cctcgttggg gagagggagg     180

tgagtttgtg tcttctggaa ggcgtggggg ctgtgccctc gtgggggtag gaagtgctcc     240

cgtggggcgg ggtgcggatc ggagaggtga gtgggtgcgt ctgtccagcg gtccgcccgg     300

tgtggtcgtg cccggcccgc gtggggatgg gggtgtctct cccgctgggc aactatacca     360

gcgcaaccgg ggcgtcggcg cggcccacgc tagcggcgct gctccggcgg cgggggctgg     420

gcgtggcggt gatgctgggc gtggtggccg cgctgggcgt ggtggccgcg ctgccgccct     480

cacccgggca gccgtgctgg agaaggatgt cggcgcacag ctggcttcca gcctggcggg     540

cgtagaacag cgccgtgcgg ccctgggcgt cacgggccgc cacgtccgcg ccgtactaga     600

gggcggaaac ggccgcgtga ccgcgcgtcc ccagggcgcc cacacccggc gccgcctccc     660

ccacatggcc aagcctactt ccggggtccc tctgggaatt tcgggctttc ccgcgccagg     720

cgttttccga gatgaagcct caaagacccc ctttcctccc cccagctcac gtacccacag     780

cagcagttgc gtgatgacga cgtgggcgag ctcggccgcc aggtggagtg gggagcgcag     840

ctgtgggtcc tctacgctgg tgtcgagcgg cccgtgtcgc gcatgggcca aaagcaggag     900

aacggtagcc acgtcctggg cctgcacggc ggcccacagc tggcggccca gcggctcctc     960

cgaggtgctc agcggcgcca ggaacagtag ctgctcgtac ttggcgcgaa tccacgactc    1020

gcgctcctcc ctgcaagacc agggatcaac ggaaaaggct ctagggaccc ccagccagga    1080

cttctgcccc tacccacggg accgtctcag gttcgcacac cctcagcaac cctccccccg    1140

ctctgttccc tcacgcttac cgcgaagagt cccgcgaggg cttggcacgg cctcgcgtgt    1200

cgctttccca cacgcggttg gccgtgtcgt tgccaatagc cgtcagcacc agggtcagct    1260

cccgtggcca gtcgtccaag tccagcgagc gaacgcggga caggtgtgtg cccaggttgc    1320

ggtggatgcc agaacactcg atgcagatga gggcgcccag gttcaagctg gcccacgtgg    1380

ggtctgcgga aggagcgtag aggtcggctc ccagccgggc agcacaggca ccccggcatt    1440

cactacactc cctagcccct ccgctgcctc ctggcactca ctgggggccc cgcagtccac    1500

gcagattgaa ttccccttgg cgttccggat cgcctggat                           1539

<210> 85

<211> 2648
<212> DNA
<213> Homo sapiens

<400> 85
agccaggtcc agcccccgcg cctgacaccg gccggacgtt cccggggcgc cgcagctgcg 60

gcgggaactc tgggatccgg agccatctgc tcccacccgc tccggagcca aaccccgggg 120

gccgcctccg ctcccggacc cgcctcctct cccgggagtg tgagccgaac caagagtctc 180

ctgcctatct cctccagtag gaaaatagta ataataatag acaccctgcc cccgtaaaaa 240

acactacctt ccccgtaccg cctcccaagt ctcccggggt acggattgcc tttgcagcag 300

ttccgcccca cctgactcac tccagggtca gccccgggtg ggtttcaatg cggctctggg 360

gagggggtgg gcagtggggg aagtgaggct tcctatccgc cccctctcac ttcacattta 420

aatattctgc acgttccagc ccccgcggac tcgcgtaccg cccaatccgc cttcaccgca 480

cgaaaaacat cactagcctg ctctcagccc aggggacgac tagtccctgg cgagaagctg 540

cctgcaaggt cactgtcatg ccacctgccc caagtgctca ggggaaactg aggcttcctc 600

atccccttca ccttcaacgt cgctctaaac acggcaaagc cccgtttcca tgctcccaga 660

gttcagctga ggctggaagt ggggtcctgg gcttctctgg gagcaatttt ctagtcactc 720

tgatcaagga cgttactttc ccagaaagct ctgaggctga gtccctctga aatcaagtcc 780

tttctcctgt cgcacaatgt agctactcgc cccgcttcag gactcctatt ctttgcccca 840

atccttgaca gaggggtgag cttggttcat ccgcccaccc cagagaaaag cttccctagt 900

ttcctggacc tcgctcctcc accccaagct gagcattcca ggtacccttc cctccctgtt 960

ctcaagccct gactcaactc actaggggaa gcgcggagct cggcgcccag cagctccctg 1020

gacccgctgc cagaagacag gctggggggt ccgggaaggg gcccggagcc aggaggccct 1080

cctgtgctct tggtgaagat gccgctgata aacttgagca tcttgcggtc acgagtggat 1140

gctcggcccc cctcccggcc ccgtttcagc cccggagctg gaggctccag agtgattgga 1200

ggtgcaggcc cggggggctg cgcggaagca gcggtgacag cagtggctgg actcggagtt 1260

ggtgggaggg ttagcggagg aggagagccg gcaggcggtc ccggatgcaa gtcactgttg 1320

tccaaggtct tactcttgcc tttccgaggg gacaacttcc ctcgggctcc agccccagcc 1380

ccgaccccac cagaggtcga agctgtagag ccccctcccc cggcggcggc ggcggtggcg 1440

gcggcagaga ccgaagctcc agtcccggcg ctgctctttg accccttgac cctgggcttg 1500

ccctcgcttt cgggccatga caggcggcta cccgcgccct tgcccccgcc ggctttggct 1560

ccactcgtgg tcacggtctt gcaaggcttg ggagccggcg gaggaggcgc caccttgagc 1620

ctccggctgc cggtgccagg gtgcggagag gatgagccag ggatgccgcc gcccgcccgg 1680

ccttcgggct ccgggccgcc ccagctcggg ctgctgagca gggggcgccg ggaggaggtg 1740

ggggcgcccc caggcttggg gtcggggctc agtcccccgg agagcggggg tcccggaggg 1800

acggcccaga gggagaggcg gcggccggga gcgggggaga ctgggcgggc cggactggcc 1860

ggagccgggg acagggctgg gggctccgcg cccccggtgc ccgcgctgct cgtgctgatc 1920

cacagcgcat cctgccggtg gaagagacgt tcgtgccgct tcttgcccgg ctcctccgcg 1980

cctcgggggc tgccaggatc cccagtctcg gagcctctgg caccggcggc gccggccgcg 2040

gccgcagacg gagaaggcgg cggcggaggc accgactcga gcttaaccag ggtcagcgag 2100

atgaggtagg tcgttgtccg gcgctgaagc gcgcccgcgc cccggctcat ggggcccgga 2160

gacccccgag ctggggaggg gaggggactc ccccggactg cctcaggggg gcccggccat 2220

ggggccgccc tgctcgctgc ccccagcccc cggaccccgc tgagcccccg gcccggctcc 2280

gctgtcgccg ccgcctccgc cgcctccgct tgcgcccccc tcccatcaca tggggcgccc 2340

cctccccatg ctccccgccc tgcgccccca ccctcttgga gccccgggac cttggtgctg 2400

ctccagggag gcgcgccgga ccgtccaccc cggcctgggt gggggcgctg agatgggtgg 2460

gggagggcgg ggaggacagt agtgggggca aatgggggag agagaggaaa agggagcaga 2520

aaaggggacc ggaggctagg ggaaacgaac ctgtgcgggg gaggcagggg cggggaattg 2580

ggactcaagg gacaggggcc gcggatgcgg tcggaaagag ggtctagagg agggtgggaa 2640

gctagtgg 2648

<210> 86
<211> 452
<212> DNA
<213> Homo sapiens

<400> 86
aggagcgcaa ggcttgcagg gcatgctggg agagcgcagg gaacgctggg agagcgcggg 60

aaatactggg attggctccc gagggctgtg aggagggcac gaggggacac tccgatgaag 120

gcagggcacg cggggcgagc cgggagcgtc tcctgagggc agcgaggagg gagctgaggc 180

acgcgggctc tcaatcgacg ccccacagag accaagaggc ctggccttgg ggggcagctg 240

cttgaaggag gcagagcgga agcgagggag actgctggag gccctgccgc ccacccgccc 300

tttcctcccc ctgaggagac gcctgacgca tctgcagtgc aggaggccgt gggcgttaga 360

agtgttgctt ttccagtttg taagaccatt ttcctgattc tcttccccac ggttgcggag 420

gagcaggtca gggccgccat gagggcagga tc 452

<210> 87
<211> 232
<212> DNA
<213> Homo sapiens

<400> 87
tcgaccgcta ctattatgaa aacagcgacc agcccattga cttaaccaag tccaagaaca 60

agccgctggt gtccagcgtg gctgattcgg tggcatcacc tctgcgggag agcgcactca 120

tggacatctc cgacatggtg aaaaacctca caggccgcct gacgcccaag tcctccacgc 180

cctccacagt ttcagagaag tccgatgctg atggcagcag ctttgaggag gc 232

<210> 88
<211> 221
<212> DNA
<213> Homo sapiens

<400> 88
tgtgccgtcg cacacagacg ccctcaacgt cggagagctg tgagcggggc cgtgctcttg 60

ggatgggagc ccccgggaga gctgcccgcc aacaccactc cgacgtgatc catgctggac 120

ataaagtgct cttccctccg ctagtcatcg gccgagcggg cccctcgctc ctgggtgtaa 180

gttctttctg tgcgtccttc tcccatctcc gtgcagttca g 221

<210> 89
<211> 477
<212> DNA
<213> Homo sapiens

<400> 89
ccatgcgccg ctgcgcgcgc gagttcgggc tgctgctgct gttcctctgc gtggccatgg 60

cgctcttcgc gccactggtg cacctggccg agcgcgagct gggcgcgcgc cgcgacttct 120

ccagcgtgcc cgccagctat tggtgggccg tcatctccat gaccaccgtg ggctacggcg 180

acatggtccc gcgcagcctg cccgggcagg tggtggcgct cagcagcatc ctcagcggca 240

tcctgctcat ggccttcccg gtcacctcca tcttccacac cttttcgcgc tcctactccg 300

agctcaagga gcagcagcag cgcgcggcca gccccgagcc ggccctgcag gaggacagca 360

cgcactcggc cacagccacc gaggacagct cgcagggccc cgacagcgcg ggcctggccg 420

acgactccgc ggatgcgctg tgggtgcggg cagggcgctg acgcctgcgc cgcccac 477

<210> 90
<211> 88
<212> DNA
<213> Homo sapiens

<400> 90
gattgacagt ttctccttcc ccagactggc caatcacagg caggaagatg aaggttctgt 60

gggctgcgtt gctggtcaca ttcctggc 88

<210> 91
<211> 30
<212> DNA
<213> Artificial Sequence

<220>

<223> Description of Artificial Sequence: Synthetic
      primer

<400> 91
acgttggatg ttgacagttt ctccttcccc                                        30


<210> 92
<211> 30
<212> DNA
<213> Artificial Sequence

<220>
<223> Description of Artificial Sequence: Synthetic
      primer

<400> 92
acgttggatg gaatgtgacc agcaacgcag                                        30


<210> 93
<211> 18
<212> DNA
<213> Artificial Sequence

<220>
<223> Description of Artificial Sequence: Synthetic
      primer

<400> 93
gcaggaagat gaaggtty                                                     18


<210> 94
<211> 88
<212> DNA
<213> Artificial Sequence

<220>
<223> Description of Artificial Sequence: Synthetic
      oligonucleotide

<400> 94
gattgacagt ttctccttcc ccagactggc caatcacagg caggaagatg aaggttttgt       60

gggctgcgtt gctggtcaca ttcctggc                                          88


<210> 95
<211> 90
<212> DNA
<213> Homo sapiens

<400> 95
gagttttgga tagtaaaata agtttcgaac tctggcacct ttcaattttg tcgcactctc       60

cttgtttttg acaatgcaat catatgcttc                                        90


<210> 96
<211> 31
<212> DNA
<213> Artificial Sequence

<220>
<223> Description of Artificial Sequence: Synthetic
      primer

<400> 96
acgtggatag taaaataagt ttcgaactct g                                    31


<210> 97
<211> 27
<212> DNA
<213> Artificial Sequence

<220>
<223> Description of Artificial Sequence: Synthetic
      primer

<400> 97
gaagcatatg attgcattgt caaaaac                                         27


<210> 98
<211> 20
<212> DNA
<213> Artificial Sequence

<220>
<223> Description of Artificial Sequence: Synthetic
      primer

<400> 98
atttcaattt tgtcgcacty                                                 20


<210> 99
<211> 90
<212> DNA
<213> Artificial Sequence

<220>
<223> Description of Artificial Sequence: Synthetic
      oligonucleotide

<400> 99
gagttttgga tagtaaaata agtttcgaac tctggcacct ttcaattttg tcgcactttc     60

cttgtttttg acaatgcaat catatgcttc                                      90


<210> 100
<211> 97
<212> DNA
<213> Homo sapiens

<400> 100
   gaactcctct ttgtctctgc gtgcccggcg cgcccccctc ccggtgggtg ataaacccac      60
   tctggcgccg gccatgcgct gggtgattaa tttgcga                              97


<210> 101
<211> 29

<212> DNA
<213> Artificial Sequence

<220>
<223> Description of Artificial Sequence: Synthetic
      primer

<400> 101
acgttggatg tctttgtctc tgcgtgccc                                         29


<210> 102
<211> 30
<212> DNA
<213> Artificial Sequence

<220>
<223> Description of Artificial Sequence: Synthetic
      primer

<400> 102
acgttggatg ttaatcaccc agcgcatggc                                        30


<210> 103
<211> 22
<212> DNA
<213> Artificial Sequence

<220>
<223> Description of Artificial Sequence: Synthetic
      primer

<400> 103
cccctcccgg tgggtgataa ay                                                22


<210> 104
<211> 97
<212> DNA
<213> Artificial Sequence

<220>
<223> Description of Artificial Sequence: Synthetic
      oligonucleotide

<400> 104
gaactcctct ttgtctctgc gtgcccggcg cgcccccctc ccggtgggtg ataaatccac       60

tctggcgccg gccatgcgct gggtgattaa tttgcga                                97


<210> 105
<211> 86
<212> DNA
<213> Homo sapiens

<400> 105
ccattggccg tccgccgtgg cagtgcgggc gggagcgcag ggagagaacc acagctggaa       60

tccgattccc accccaaaac ccagga                                            86

<210> 106
<211> 29
<212> DNA
<213> Artificial Sequence

<220>
<223> Description of Artificial Sequence: Synthetic
      primer

<400> 106
acgttggatg ccattggccg tccgccgtg                                          29


<210> 107
<211> 31
<212> DNA
<213> Artificial Sequence

<220>
<223> Description of Artificial Sequence: Synthetic
      primer

<400> 107
acgttggatg tcctgggttt tggggtggga a                                       31


<210> 108
<211> 19
<212> DNA
<213> Artificial Sequence

<220>
<223> Description of Artificial Sequence: Synthetic
      primer

<400> 108
ttccagctgt ggttctctc                                                     19


<210> 109
<211> 86
<212> DNA
<213> Artificial Sequence

<220>
<223> Description of Artificial Sequence: Synthetic
      oligonucleotide

<400> 109
ccattggccg tccgccgtgg cagtgcgggc gggagcgcag agagagaacc acagctggaa        60

tccgattccc accccaaaac ccagga                                             86


<210> 110
<211> 31
<212> DNA
<213> Artificial Sequence

<220>
<223> Description of Artificial Sequence: Synthetic
      primer

<400> 110
acgttggatg acatggtcgg ccccacggaa t                                    31


<210> 111
<211> 30
<212> DNA
<213> Artificial Sequence

<220>
<223> Description of Artificial Sequence: Synthetic
      primer

<400> 111
acgttggatg acccattggc cgtccgccgt                                      30


<210> 112
<211> 31
<212> DNA
<213> Artificial Sequence

<220>
<223> Description of Artificial Sequence: Synthetic
      primer

<400> 112
acgttggatg gaactcctct ttgtctctgc g                                    31


<210> 113
<211> 31
<212> DNA
<213> Artificial Sequence

<220>
<223> Description of Artificial Sequence: Synthetic
      primer

<400> 113
acgttggatg cgcagcaacg ggaccgctac a                                    31


<210> 114
<211> 29
<212> DNA
<213> Artificial Sequence

<220>
<223> Description of Artificial Sequence: Synthetic
      primer

<400> 114
acgttgcgta gcaacctgtt acatattaa                                       29


<210> 115
<211> 31
<212> DNA
<213> Artificial Sequence

<220>
<223> Description of Artificial Sequence: Synthetic

primer

<400> 115
acgttggatg catagaggcc catgatggtg g                                    31


<210> 116
<211> 33
<212> DNA
<213> Artificial Sequence

<220>
<223> Description of Artificial Sequence: Synthetic
      primer

<400> 116
acgttggatg gtgtggtcag ctcttccctt cat                                  33


<210> 117
<211> 32
<212> DNA
<213> Artificial Sequence

<220>
<223> Description of Artificial Sequence: Synthetic
      primer

<400> 117
acgttggatg ctccttccta gtgtgagaac cg                                   32


<210> 118
<211> 31
<212> DNA
<213> Artificial Sequence

<220>
<223> Description of Artificial Sequence: Synthetic
      primer

<400> 118
   acgttggatg ttttgggtgg ggaatcggat t                                     31


<210> 119
<211> 29
<212> DNA
<213> Artificial Sequence

<220>
<223> Description of Artificial Sequence: Synthetic
      primer

<400> 119
acgttggatg tggcatggcc ggcgccaga                                       29


<210> 120
<211> 32
<212> DNA
<213> Artificial Sequence

<220>
<223> Description of Artificial Sequence: Synthetic
      primer

<400> 120
acgttggcat ctaggtaggt ctttgtagcc aa                                    32


<210> 121
<211> 32
<212> DNA
<213> Artificial Sequence

<220>
<223> Description of Artificial Sequence: Synthetic
      primer

<400> 121
acgttggatc tgagcaaagg caatcaacac cc                                    32


<210> 122
<211> 32
<212> DNA
<213> Artificial Sequence

<220>
<223> Description of Artificial Sequence: Synthetic
      primer

<400> 122
acgttggatg accttctgcc cctctactcc aa                                    32


<210> 123
<211> 33
<212> DNA
<213> Artificial Sequence

<220>
<223> Description of Artificial Sequence: Synthetic
      primer

<400> 123
acgttggccc acatgtaatg tgttgaaaaa gca                                   33


<210> 124
<211> 18
<212> DNA
<213> Artificial Sequence

<220>
<223> Description of Artificial Sequence: Synthetic
      primer

<400> 124
caggttccgg ggcttggg                                                    18


<210> 125
<211> 19
<212> DNA

<213> Artificial Sequence

<220>
<223> Description of Artificial Sequence: Synthetic
      primer

<400> 125
cgcagggaga gaaccacag                                                    19


<210> 126
<211> 21
<212> DNA
<213> Artificial Sequence

<220>
<223> Description of Artificial Sequence: Synthetic
      primer

<400> 126
cccctcccgg tgggtgataa a                                                 21


<210> 127
<211> 21
<212> DNA
<213> Artificial Sequence

<220>
<223> Description of Artificial Sequence: Synthetic
      primer

<400> 127
aaagctgtag gacaatcggg t                                                 21


<210> 128
<211> 22
<212> DNA
<213> Artificial Sequence

<220>
<223> Description of Artificial Sequence: Synthetic
      primer

<400> 128
   catttttcta catcctttgt tt                                                 22


<210> 129
<211> 23
<212> DNA
<213> Artificial Sequence

<220>
<223> Description of Artificial Sequence: Synthetic
      primer

<400> 129
agaagatcac caggcagaag agg                                               23


<210> 130

<211> 25
<212> DNA
<213> Artificial Sequence

<220>
<223> Description of Artificial Sequence: Synthetic
      primer

<400> 130
acttggagaa caaaggacac cgtta                                          25


<210> 131
<211> 81
<212> DNA
<213> Artificial Sequence

<220>
<223> Description of Artificial Sequence: Synthetic
      oligonucleotide

<400> 131
ggtcggcccc acggaatccc ggctctgtgt gcgcccaggt tccggggctt gggtgttgcc    60

ggttctcaca ctaggaagga g                                              81


<210> 132
<211> 79
<212> DNA
<213> Artificial Sequence

<220>
<223> Description of Artificial Sequence: Synthetic
      oligonucleotide

<400> 132
ccattggccg tccgccgtgg cagtgcgggc gggagcgcag agagagaacc acagctggaa    60

tccgattccc accccaaaa                                                 79


<210> 133
<211> 77
<212> DNA
<213> Artificial Sequence

<220>
<223> Description of Artificial Sequence: Synthetic
      oligonucleotide

<400> 133
gaactcctct ttgtctctgc gtgcccggcg cgcccccctc ccggtgggtg ataaatccac     60

tctggcgccg gccatgc                                                   77


<210> 134
<211> 81
<212> DNA
<213> Artificial Sequence

<220>

<223> Description of Artificial Sequence: Synthetic
      oligonucleotide

<400> 134
gcagcaacgg gaccgctaca gccactggac aaagccgtag gacaatcggg taacattggc      60

tacaaagacc tacctagatg c                                                81


<210> 135
<211> 95
<212> DNA
<213> Artificial Sequence

<220>
<223> Description of Artificial Sequence: Synthetic
      oligonucleotide

<400> 135
gcgtagcaac ctgttacata ttaaagtttt attatactac atttttctac atcctttgtt      60

tcagagtgtt gattgccttt gctcagtatc ttcag                                 95


<210> 136
<211> 86
<212> DNA
<213> Artificial Sequence

<220>
<223> Description of Artificial Sequence: Synthetic
      oligonucleotide

<400> 136
ccttctgccc ctctactcca agcgctacac cctcttctgc ctggtgatct ttgccggcgt      60

cctggccacc atcatgggcc tctatg                                           86


<210> 137
<211> 83
<212> DNA
<213> Artificial Sequence

<220>
<223> Description of Artificial Sequence: Synthetic
      oligonucleotide

<400> 137
gtgtggtcag ctcttccctt catcacatac ttggagaaca aaggacaccg ttatccatgc      60

tttttcaaca cattacatgt ggg                                              83


<210> 138
<211> 30
<212> DNA
<213> Artificial Sequence

<220>
<223> Description of Artificial Sequence: Synthetic
      primer

<400> 138
acgttggatg ttctgcccct ctactccaag                                        30


<210> 139
<211> 30
<212> DNA
<213> Artificial Sequence

<220>
<223> Description of Artificial Sequence: Synthetic
      primer

<400> 139
acgttggatg tcagctcttc ccttcatcac                                        30


<210> 140
<211> 30
<212> DNA
<213> Artificial Sequence

<220>
<223> Description of Artificial Sequence: Synthetic
      primer

<400> 140
acgttggatg ttgacagttt ctccttcccc                                        30


<210> 141
<211> 28
<212> DNA
<213> Artificial Sequence

<220>
<223> Description of Artificial Sequence: Synthetic
      primer

<400> 141
acgttggatg cggtcggccc cacggaat                                          28


<210> 142
<211> 31
<212> DNA
<213> Artificial Sequence

<220>
<223> Description of Artificial Sequence: Synthetic
      primer

<400> 142
acgtggatag taaaataagt ttcgaactct g                                      31


<210> 143
<211> 30
<212> DNA
<213> Artificial Sequence

<220>
<223> Description of Artificial Sequence: Synthetic

primer

<400> 143
acgttggatg cacagctcac cgcagcaacg                                        30


<210> 144
<211> 29
<212> DNA
<213> Artificial Sequence

<220>
<223> Description of Artificial Sequence: Synthetic
      primer

<400> 144
acgttggatg tctttgtctc tgcgtgccc                                         29


<210> 145
<211> 29
<212> DNA
<213> Artificial Sequence

<220>
<223> Description of Artificial Sequence: Synthetic
      primer

<400> 145
acgttggatg gactgagccc cagaactcg                                         29


<210> 146
<211> 29
<212> DNA
<213> Artificial Sequence

<220>
<223> Description of Artificial Sequence: Synthetic
      primer

<400> 146
acgttggatg aagccaagtt tccctccgc                                         29


<210> 147
<211> 30
<212> DNA
<213> Artificial Sequence

<220>
<223> Description of Artificial Sequence: Synthetic
      primer

<400> 147
acgttagcgt agcaacctgt tacatattaa                                        30


<210> 148
<211> 30
<212> DNA
<213> Artificial Sequence

<220>
<223> Description of Artificial Sequence: Synthetic
      primer

<400> 148
acgttggatg catagaggcc catgatggtg                                        30


<210> 149
<211> 31
<212> DNA
<213> Artificial Sequence

<220>
<223> Description of Artificial Sequence: Synthetic
      primer

<400> 149
acgttggatg cctacctccc acatgtaatg t                                      31


<210> 150
<211> 30
<212> DNA
<213> Artificial Sequence

<220>
<223> Description of Artificial Sequence: Synthetic
      primer

<400> 150
acgttggatg gaatgtgacc agcaacgcag                                        30


<210> 151
<211> 32
<212> DNA
<213> Artificial Sequence

<220>
<223> Description of Artificial Sequence: Synthetic
      primer

<400> 151
acgttggatg ctccttccta gtgtgagaac cg                                     32


<210> 152
<211> 27
<212> DNA
<213> Artificial Sequence

<220>
<223> Description of Artificial Sequence: Synthetic
      primer

<400> 152
gaagcatatg attgcattgt caaaaac                                           27


<210> 153
<211> 32
<212> DNA

<213> Artificial Sequence

<220>
<223> Description of Artificial Sequence: Synthetic
      primer

<400> 153
acgttggatg ctaggtaggt ctttgtagcc aa                                    32


<210> 154
<211> 30
<212> DNA
<213> Artificial Sequence

<220>
<223> Description of Artificial Sequence: Synthetic
      primer

<400> 154
acgttggatg ttaatcaccc agcgcatggc                                       30


<210> 155
<211> 30
<212> DNA
<213> Artificial Sequence

<220>
<223> Description of Artificial Sequence: Synthetic
      primer

<400> 155
acgttggatg gtgggtttgt gctttccacg                                       30


<210> 156
<211> 30
<212> DNA
<213> Artificial Sequence

<220>
<223> Description of Artificial Sequence: Synthetic
      primer

<400> 156
acgttggatg cttttgcttt cccagccagg                                       30


<210> 157
<211> 30
<212> DNA
<213> Artificial Sequence

<220>
<223> Description of Artificial Sequence: Synthetic
      primer

<400> 157
acgttggatg ctgagcaaag gcaatcaaca                                       30


<210> 158

<211> 17
<212> DNA
<213> Artificial Sequence

<220>
<223> Description of Artificial Sequence: Synthetic
      primer

<400> 158
ttctgcctgg tgatctt                                                    17


<210> 159
<211> 17
<212> DNA
<213> Artificial Sequence

<220>
<223> Description of Artificial Sequence: Synthetic
      primer

<400> 159
aacaaaggac accgtta                                                    17


<210> 160
<211> 17
<212> DNA
<213> Artificial Sequence

<220>
<223> Description of Artificial Sequence: Synthetic
      primer

<400> 160
gcaggaagat gaaggtt                                                    17


<210> 161
<211> 18
<212> DNA
<213> Artificial Sequence

<220>
<223> Description of Artificial Sequence: Synthetic
      primer

<400> 161
aaggttccgg ggcttggg                                                   18


<210> 162
<211> 19
<212> DNA
<213> Artificial Sequence

<220>
<223> Description of Artificial Sequence: Synthetic
      primer

<400> 162
atttcaattt tgtcgcact                                                  19

<210> 163
<211> 19
<212> DNA
<213> Artificial Sequence

<220>
<223> Description of Artificial Sequence: Synthetic
      primer

<400> 163
agctgtagga caatcgggt                                                    19


<210> 164
<211> 20
<212> DNA
<213> Artificial Sequence

<220>
<223> Description of Artificial Sequence: Synthetic
      primer

<400> 164
ccctcccggt gggtgataaa                                                   20


<210> 165
<211> 20
<212> DNA
<213> Artificial Sequence

<220>
<223> Description of Artificial Sequence: Synthetic
      primer

<400> 165
agggccgggg tctgcgcgtg                                                   20


<210> 166
<211> 21
<212> DNA
<213> Artificial Sequence

<220>
<223> Description of Artificial Sequence: Synthetic
      primer

<400> 166
gaggcactgc ccggacaaac c                                                 21


<210> 167
<211> 22
<212> DNA
<213> Artificial Sequence

<220>
<223> Description of Artificial Sequence: Synthetic
      primer

<400> 167
   catttttcta catcctttgt tt                                                22

<210> 168
<211> 86
<212> DNA
<213> Artificial Sequence

<220>
<223> Description of Artificial Sequence: Synthetic
      oligonucleotide

<400> 168
ccttctgccc ctctactcca agcgctacac cctcttctgc ctggtgatct ttgccggcgt        60

cctggccacc atcatgggcc tctatg                                            86

<210> 169
<211> 90
<212> DNA
<213> Artificial Sequence

<220>
<223> Description of Artificial Sequence: Synthetic
      oligonucleotide

<400> 169
gtgtggtcag ctcttccctt catcacatac ttggagaaca aaggacaccg ttatccatgc        60

tttttcaaca cattacatgt gggaggtagg                                        90

<210> 170
<211> 88
<212> DNA
<213> Artificial Sequence

<220>
<223> Description of Artificial Sequence: Synthetic
      oligonucleotide

<400> 170
gattgacagt ttctccttcc ccagactggc caatcacagg caggaagatg aaggttttgt        60

gggctgcgtt gctggtcaca ttcctggc                                          88

<210> 171
<211> 98
<212> DNA
<213> Artificial Sequence

<220>
<223> Description of Artificial Sequence: Synthetic
      oligonucleotide

<400> 171
aaaaccagag attcgcggtc ggccccacgg aatcccggct ctgtgtgcgc ccaggttccg        60

gggcttgggt gttgccggtt ctcacactag gaaggagc                               98

<210> 172
<211> 90
<212> DNA
<213> Artificial Sequence

<220>
<223> Description of Artificial Sequence: Synthetic
      oligonucleotide

<400> 172
gagttttgga tagtaaaata agtttcgaac tctggcacct ttcaattttg tcgcactttc      60

cttgtttttg acaatgcaat catatgcttc                                        90


<210> 173
<211> 95
<212> DNA
<213> Artificial Sequence

<220>
<223> Description of Artificial Sequence: Synthetic
      oligonucleotide

<400> 173
gcagccagct caccgcagca acgggaccgc tacagccact ggacaaagct gtaggacaat      60

cgggtgacat tggctacaaa gacctaccta gatgc                                 95


<210> 174
<211> 97
<212> DNA
<213> Artificial Sequence

<220>
<223> Description of Artificial Sequence: Synthetic
      oligonucleotide

<400> 174
gaactcctct ttgtctctgc gtgcccggcg cgcccccctc ccggtgggtg ataaatccac      60

tctggcgccg gccatgcgct gggtgattaa tttgcga                               97


<210> 175
<211> 86
<212> DNA
<213> Artificial Sequence

<220>
<223> Description of Artificial Sequence: Synthetic
      oligonucleotide

<400> 175
gtgggtttgt gctttccacg cgtgcacaca cacgcgcaga ccccggccct tgccccgcct      60

acctccccga gttctggggc tcagtc                                           86


<210> 176
<211> 89
<212> DNA

<213> Artificial Sequence

<220>
<223> Description of Artificial Sequence: Synthetic
      oligonucleotide

<400> 176
gcgccagctt ttgctttccc agccagggcg cggtgaggtt tgtccgggca gtgcctcgag      60

caactgggaa ggccaaggcg gagggaaac                                        89


<210> 177
<211> 96
<212> DNA
<213> Artificial Sequence

<220>
<223> Description of Artificial Sequence: Synthetic
      oligonucleotide

<400> 177
gcgtagcaac ctgttacata ttaaagtttt attatactac atttttctac atcctttgtt      60

ttagggtgtt gattgccttt gctcagtatc ttcagc                                96


<210> 178
<211> 10
<212> DNA
<213> Artificial Sequence

<220>
<223> Description of Artificial Sequence: Synthetic
      oligonucleotide

<400> 178
cgcaaccact                                                             10


<210> 179
<211> 10
<212> DNA
<213> Artificial Sequence

<220>
<223> Description of Artificial Sequence: Synthetic
      oligonucleotide

<400> 179
cgcgaccact                                                             10

## Description

### RELATED PATENT APPLICATION

This patent application claims the benefit of U.S. Provisional Patent Application No. 61/192,264 filed on September 16, 2008, entitled PROCESSES AND COMPOSITIONS FOR METHYLATION-BASED ENRICHMENT OF FETAL NUCLEIC ACID FROM A MATERNAL SAMPLE USEFUL FOR NON INVASIVE PRENATAL DIAGNOSES, naming Mathias Ehrich as an inventor, and having Attorney Docket No. SEQ-6022-PV. The entire content of the foregoing patent application is incorporated herein by reference, including all text, tables and drawings.

### FIELD

Provided in certain embodiments are biomarkers. In some embodiments, biomarkers provided are useful for noninvasive detection of fetal genetic traits. Certain fetal genetic traits include but are not limited to presence or absence of fetal nucleic acid.

### BACKGROUND

Non-invasive prenatal testing is becoming a field of rapidly growing interest. Early detection of pregnancy-related conditions, including complications during pregnancy and genetic defects of the fetus is of crucial importance, as it allows early medical intervention necessary for the safety of both the mother and the fetus. Prenatal diagnosis has been conducted using cells isolated from the fetus through procedures such as chorionic villus sampling (CVS) or amniocentesis. However, these conventional methods are invasive and present an appreciable risk to both the mother and the fetus. The National Health Service currently cites a miscarriage rate of between 1 and 2 per cent following the invasive amniocentesis and chorionic villus sampling (CVS) tests.

An alternative to these invasive approaches has been developed for prenatal screening, e.g., to detecting fetal abnormalities, following the discovery that circulating cell-free fetal nucleic acid can be detected in maternal plasma and serum (Lo et al., Lancet 350:485-487, 1997; and U.S. Patent 6,258,540). Circulating cell free fetal nucleic acid (cffNA) has several advantages making it more applicable for non-invasive prenatal testing. For example, cell free nucleic acid is present at higher levels than fetal cells and at concentrations sufficient for genetic analysis. Also, cffNA is cleared from the maternal bloodstream within hours after delivery, preventing contamination from previous pregnancies.

Examples of prenatal tests performed by detecting fetal DNA in maternal plasma or serum include fetal rhesus D (RhD) genotyping (Lo et al., N. Engl. J. Med. 339:1734-1738, 1998), fetal sex determination (Costa et al., N. Engl. J. Med. 346:1502, 2002), and diagnosis of several fetal disorders (Amicucci et al., Clin. Chem. 46:301-302, 2000; Saito et al., Lancet 356:1170, 2000; and Chiu et al., Lancet 360:998-1000, 2002). In addition, quantitative abnormalities of fetal DNA in maternal plasma/serum have been reported in preeclampsia (Lo et al., Clin. Chem. 45:184-188, 1999 and Zhong et al., Am. J. Obstet. Gynecol. 184:414-419, 2001), fetal trisomy 21 (Lo et al., Clin. Chem. 45:1747-1751, 1999 and Zhong et al., Prenat. Diagn. 20:795-798, 2000) and hyperemesis gravidarum (Sekizawa et al., Clin. Chem. 47:2164-2165, 2001).

### SUMMARY

The technology provides *inter alia* human epigenetic biomarkers that are useful for the noninvasive detection of fetal genetic traits, including, but not limited to, the presence or absence of fetal nucleic acid, the absolute or relative amount of fetal nucleic acid, fetal sex, and fetal chromosomal abnormalities such as aneuploidy. The human epigenetic biomarkers of the technology represent genomic DNA that display differential CpG methylation patterns between the fetus and mother. The compositions and processes of the technology allow for the detection and quantification of fetal nucleic acid in a maternal sample based on the methylation status of the nucleic acid in said sample. More specifically, the amount of fetal nucleic acid from a maternal sample can be determined relative to the total amount of nucleic acid present, thereby providing the percentage of fetal nucleic acid in the sample. Further, the amount of fetal nucleic acid can be determined in a sequence-specific (or locus-specific) manner and with sufficient sensitivity to allow for accurate chromosomal dosage analysis (for example, to detect the presence or absence of a fetal aneuploidy).

In an aspect of the technology, a method is provided for enriching fetal nucleic acids from a maternal biological sample, based on differential methylation between fetal and maternal nucleic acid comprising the steps of: (a) binding a target nucleic acid, from a sample, and a control nucleic acid, from the sample, to a methylation-specific binding protein; and (b) eluting the bound nucleic acid based on methylation status, wherein differentially methylated nucleic acids elute at least partly into separate fractions. In an embodiment, the nucleic acid sequence includes one or more of the polynucleotide sequences of SEQ ID NOs: 1-89. SEQ ID NOs: 1-89 are provided in Table 4. The technology includes the sequences of SEQ ID NOs: 1-89, and variations thereto. In another embodiment, a control nucleic acid is not included in step (a).

In a related embodiment, a method is provided for enriching fetal nucleic acid from a maternal sample, which comprises the following steps: (a) obtaining a biological sample from a woman; (b) separating fetal and maternal nucleic acid based on the methylation status of a CpG-containing genomic sequence in the sample, wherein the genomic sequence from the fetus and the genomic sequence from the woman are differentially methylated, thereby distinguishing the genomic sequence from the woman and the genomic sequence from the fetus in the sample. In another embodiment, the genomic sequence is at least 15 nucleotides in length, comprising at least one cytosine, further wherein the region has (1) a genomic locus selected from Table 1; and (2) a DNA sequence of no more than 10 kb upstream and/or downstream from the locus. In embodiments, obtaining a biological sample from a woman does not limit the scope of the technology. This obtaining can refer to actually collecting a sample from a woman (e.g., a blood draw) or to receiving a sample from elsewhere (e.g., from a clinic or hospital) and performing steps of a method.

In another related embodiment, a method is provided for enriching fetal nucleic acid from a maternal sample, which comprises the following steps: (a) obtaining a biological sample from the woman; (b) digesting or removing maternal nucleic acid based on the methylation status of a CpG-containing genomic sequence in the sample, wherein the genomic sequence from the fetus and the genomic sequence from the woman are differentially methylated, thereby enriching for the genomic sequence from the fetus in the sample. Maternal nucleic acid may be digested using one or more methylation sensitive restriction enzymes that selectively digest or cleave maternal nucleic acid based on its methylation status. In another embodiment, the genomic sequence is at least 15 nucleotides in length, comprising at least one cytosine, further wherein the region consists of (1) a genomic locus selected from Table 1; and (2) a DNA sequence of no more than 10 kb upstream and/or downstream from the locus.

In another aspect of the technology, a method is provided for preparing nucleic acid having a nucleotide sequence of a fetal nucleic acid, which comprises the following steps: (a) providing a sample from a pregnant female; (b) separating fetal nucleic acid from maternal nucleic acid from the sample of the pregnant female according to a different methylation state between the fetal nucleic acid and the maternal nucleic acid counterpart, wherein the nucleotide sequence of the fetal nucleic acid comprises one or more CpG sites from one or more of the polynucleotide sequences of SEQ ID NOs: 1-89 within a polynucleotide sequence from a gene or locus that contains one of the polynucleotide sequences of SEQ ID NOs: 1-89; and (c) preparing nucleic acid comprising a nucleotide sequence of the fetal nucleic acid by an amplification process in which fetal nucleic acid separated in part (b) is utilized as a template. In another embodiment, a method is provided for preparing nucleic acid having a nucleotide sequence of a fetal nucleic acid, which comprises the following steps: (a) providing a sample from a pregnant female; (b) digesting or removing maternal nucleic acid from the sample of the pregnant female according to a different methylation state between the fetal nucleic acid and the maternal nucleic acid counterpart, wherein the nucleotide sequence of the fetal nucleic acid comprises one or more CpG sites from one or more of the polynucleotide sequences of SEQ ID NOs: 1-89 within a polynucleotide sequence from a gene that contains one of the polynucleotide sequences of SEQ ID NOs: 1-89; and (c) preparing nucleic acid comprising a nucleotide sequence of the fetal nucleic acid. The preparing process of step (c) may be a hybridization process, a capture process, or an amplification process in which fetal nucleic acid separated in part (b) is utilized as a template. Also, in the above embodiment wherein maternal nucleic acid is digested, the maternal nucleic acid may be digested using one or more methylation sensitive restriction enzymes that selectively digest or cleave maternal nucleic acid based on its methylation status. In either embodiment, the polynucleotide sequences of SEQ ID NOs: 1-89 may be within a polynucleotide sequence from a CpG island that contains one of the polynucleotide sequences of SEQ ID NOs: 1-89. The polynucleotide sequences of SEQ ID NOs: 1-89 are further characterized in Tables 1-3 herein, including the identification of CpG islands that overlap with the polynucleotide sequences provided in SEQ ID NOs: 1-89. In another embodiment, the nucleic acid prepared by part (c) is in solution. In yet another embodiment, the method further comprises quantifying the fetal nucleic acid from the amplification process of step (c).

In another aspect of the technology, a method is provided for enriching fetal nucleic acid from a sample from a pregnant female with respect to maternal nucleic acid, which comprises the following steps: (a) providing a sample from a pregnant female; and (b) separating or capturing fetal nucleic acid from maternal nucleic acid from the sample of the pregnant female according to a different methylation state between the fetal nucleic acid and the maternal nucleic acid, wherein the nucleotide sequence of the fetal nucleic acid comprises one or more CpG sites from one or more of the polynucleotide sequences of SEQ ID NOs: 1-89 within a polynucleotide sequence from a gene that contains one of the polynucleotide sequences of SEQ ID NOs: 1-89. In another embodiment, the polynucleotide sequences of SEQ ID NOs: 1-89 may be within a polynucleotide sequence from a CpG island that contains one of the polynucleotide sequences of SEQ ID NOs: 1-89. The polynucleotide sequences of SEQ ID NOs: 1-89 are characterized in Table 1 herein. In another embodiment, the nucleic acid separated by part (b) is in solution. In yet another embodiment, the method further comprises amplifying and/or quantifying the fetal nucleic acid from the separation process of step (b).

In another aspect of the technology, a composition is provided comprising an isolated nucleic acid from a fetus of a pregnant female, wherein the nucleotide sequence of the nucleic acid comprises one or more of the polynucleotide sequences of SEQ ID NOs: 1-89. In one embodiment, the nucleotide sequence consists essentially of a nucleotide sequence of a gene, or portion thereof. In another embodiment, the nucleotide sequence consists essentially of a nucleotide sequence of a CpG island, or portion thereof. The polynucleotide sequences of SEQ ID NOs: 1-89 are further characterized in Table 1. In another embodiment, the nucleic acid is in solution. In another embodiment, the nucleic acid from the fetus is enriched relative to maternal nucleic acid. In another embodiment, the composition further comprises an agent that binds to methylated nucleotides. For example, the agent may be a methyl-CpG binding protein (MBD) or fragment thereof.

In another aspect of the technology, a composition is provided comprising an isolated nucleic acid from a fetus of a pregnant female, wherein the nucleotide sequence of the nucleic acid comprises one or more CpG sites from one or more of the polynucleotide sequences of SEQ ID NOs: 1-89 within a polynucleotide sequence from a gene, or portion thereof, that contains one of the polynucleotide sequences of SEQ ID NOs: 1-89. In another embodiment, the nucleotide sequence of the nucleic acid comprises one or more CpG sites from one or more of the polynucleotide sequences of SEQ ID NOs: 1-89 within a polynucleotide sequence from a CpG island, or portion thereof, that contains one of the polynucleotide sequences of SEQ ID NOs: 1-89. The polynucleotide sequences of SEQ ID NOs: 1-89 are further characterized in Table 1. In another embodiment, the nucleic acid is in solution. In another embodiment, the nucleic acid from the fetus is enriched relative to maternal nucleic acid. Hyper- and hypomethylated nucleic acid sequences of the technology are identified in Table 1. In another embodiment, the composition further comprises an agent that binds to methylated nucleotides. For example, the agent may be a methyl-CpG binding protein (MBD) or fragment thereof.

In some embodiments, a nucleotide sequence of the technology includes three or more of the CpG sites. In another embodiment, the nucleotide sequence includes five or more of the CpG sites. In another embodiment, the nucleotide sequence is from a gene region that comprises a PRC2 domain (see Table 3). In another embodiment, the nucleotide sequence is from a gene region involved with development. For example, SOX14 - which is an epigenetic marker of the present technology (See Table 1) - is a member of the SOX (SRY-related HMG-box) family of transcription factors involved in the regulation of embryonic development and in the determination of cell fate.

In some embodiments, the genomic sequence from the woman is methylated and the genomic sequence from the fetus is unmethylated. In other embodiments, the genomic sequence from the woman is unmethylated and the genomic sequence from the fetus is methylated. In another embodiment, the genomic sequence from the fetus is hypermethylated relative to the genomic sequence from the mother. Fetal genomic sequences found to be hypermethylated relative to maternal genomic sequence are provided in SEQ ID NOs: 1-59. Alternatively, the genomic sequence from the fetus is hypomethylated relative to the genomic sequence from the mother. Fetal genomic sequences found to be hypomethylated relative to maternal genomic sequence are provided in SEQ ID NOs: 60-85. Methylation sensitive restriction enzymes of the technology may be sensitive to hypo- or hyper- methylated nucleic acid.

In another embodiment, the fetal nucleic acid is extracellular nucleic acid. Generally the extracellular fetal nucleic acid is about 500, 400, 300, 250, 200 or 150 (or any number there between) nucleotide bases or less. In another embodiment, the digested maternal nucleic acid is less than about 90, 100, 110, 120, 130, 140 or 150 base pairs. In a related embodiment, the fetal nucleic acid is selectively amplified, captured or separated from or relative to the digested maternal nucleic acid based on size. For example, PCR primers may be designed to amplify nucleic acid greater than about 75, 80, 85, 90, 95, 100, 105, 110, 115 or 120 (or any number there between) base pairs thereby amplifying fetal nucleic acid and not digested maternal nucleic acid. In another embodiment, the nucleic acid is subjected to fragmentation prior to certain methodss of the technology. Examples of methods of fragmenting nucleic acid, include but are not limited to sonication and restriction enzyme digestion. In some embodiments the fetal nucleic acid is derived from the placenta. In other embodiments the fetal nucleic acid is apoptotic.

In some embodiments, the present technology provides a method in which the sample is a member selected from the following: maternal whole blood, maternal plasma or serum, amniotic fluid, a chorionic villus sample, biopsy material from a pre-implantation embryo, fetal nucleated cells or fetal cellular remnants isolated from maternal blood, maternal urine, maternal saliva, washings of the female reproductive tract and a sample obtained by celocentesis or lung lavage. In certain embodiments, the biological sample is maternal blood. In some embodiments, the biological sample is a chorionic villus sample. In certain embodiments, the maternal sample is enriched for fetal nucleic acid prior to certain methodss of the present technology. Examples of fetal enrichment methods are provided in PCT Publication Nos. WO/2007140417A2, WO2009/032781A2 and US Publication No. 20050164241.

In some embodiments, nucleated and anucleated cell populations are removed from the sample prior to practicing certain methodss of the technology (e.g., substantially all nucleated and anucleated cell populations are removed). In some embodiments, the sample is collected, stored or transported in a manner known to the person of ordinary skill in the art to minimize degradation or the quality of fetal nucleic acid present in the sample.

The sample can be from any animal, including but not limited, human, non-human, mammal, reptile, cattle, cat, dog, goat, swine, pig, monkey, ape, gorilla, bull, cow, bear, horse, sheep, poultry, mouse, rat, fish, dolphin, whale, and shark, or any animal or organism that may have a detectable pregnancy-associated disorder or chromosomal abnormality.

In some embodiments, the sample is treated with a reagent that differentially modifies methylated and unmethylated DNA. For example, the reagent may comprise bisulfite; or the reagent may comprise one or more enzymes that preferentially cleave methylated DNA; or the reagent may comprise one or more enzymes that preferentially cleave unmethylated DNA. Examples of methylation sensitive restriction enzymes include, but are not limited to, Hhal and Hpall.

In one embodiment, the fetal nucleic acid is separated from the maternal nucleic acid by an agent that specifically binds to methylated nucleotides in the fetal nucleic acid. In another embodiment, the fetal nucleic acid is separated or removed from the maternal nucleic acid by an agent that specifically binds to methylated nucleotides in the maternal nucleic acid counterpart. In an embodiment, the agent that binds to methylated nucleotides is a methyl-CpG binding protein (MBD) or fragment thereof.

In another aspect of the technology, a method is provided for determining the amount or copy number of fetal DNA in a maternal sample that comprises differentially methylated maternal and fetal DNA. The method is performed by a) distinguishing between the maternal and fetal DNA based on differential methylation status; and b) quantifying the fetal DNA of step a). In a specific embodiment, the method comprises a) digesting the maternal DNA in a maternal sample using one or more methylation sensitive restriction enzymes thereby enriching the fetal DNA; and b) determining the amount of fetal DNA from step a). The amount of fetal DNA can be used *inter alia* to confirm the presence or absence of fetal nucleic acid, determine fetal sex, diagnose fetal disease or be used in conjunction with other fetal diagnostic methods to improve sensitivity or specificity. In one embodiment, the method for determining the amount of fetal DNA does not require the use of a polymorphic sequence. For example, an allelic ratio is not used to quantify the fetal DNA in step b). In another embodiment, the method for determining the amount of fetal DNA does not require the treatment of DNA with bisulfite to convert cytosine residues to uracil. Bisulfite is known to degrade DNA, thereby, further reducing the already limited fetal nucleic acid present in maternal samples. In one embodiment, determining the amount of fetal DNA in step b) is done by introducing one or more competitors at known concentrations. In another embodiment, determining the amount of fetal DNA in step b) is done by RT-PCR, primer extension, sequencing or counting. In a related embodiment, the amount of nucleic acid is determined using BEAMing technology as described in US Patent Publication No. US20070065823. In another embodiment, the restriction efficiency is determined and the efficiency rate is used to further determine the amount of fetal DNA. Exemplary differentially methylated nucleic acids are provided in SEQ ID NOs: 1-89.

In another aspect of the technology, a method is provided for determining the concentration of fetal DNA in a maternal sample, wherein the maternal sample comprises differentially methylated maternal and fetal DNA, comprising a) determining the total amount of DNA present in the maternal sample; b) selectively digesting the maternal DNA in a maternal sample using one or more methylation sensitive restriction enzymes thereby enriching the fetal DNA; c) determining the amount of fetal DNA from step b); and d) comparing the amount of fetal DNA from step c) to the total amount of DNA from step a), thereby determining the concentration of fetal DNA in the maternal sample. The concentration of fetal DNA can be used *inter alia* in conjunction with other fetal diagnostic methods to improve sensitivity or specificity. In one embodiment, the method for determining the amount of fetal DNA does not require the use of a polymorphic sequence. For example, an allelic ratio is not used to quantify the fetal DNA in step b). In another embodiment, the method for determining the amount of fetal DNA does not require the treatment of DNA with bisulfite to convert cytosine residues to uracil. In one embodiment, determining the amount of fetal DNA in step b) is done by introducing one or more competitors at known concentrations.
In another embodiment, determining the amount of fetal DNA in step b) is done by RT-PCR, sequencing or counting. In another embodiment, the restriction efficiency is determined and used to further determine the amount of total DNA and fetal DNA. Exemplary differentially methylated nucleic acids are provided in SEQ ID NOs: 1-89.

In another aspect of the technology, a method is provided for determining the presence or absence of a fetal aneuploidy using fetal DNA from a maternal sample, wherein the maternal sample comprises differentially methylated maternal and fetal DNA, comprising a) selectively digesting the maternal DNA in a maternal sample using one or more methylation sensitive restriction enzymes thereby enriching the fetal DNA; b) determining the amount of fetal DNA from a target chromosome; c) determining the amount of fetal DNA from a reference chromosome; and d) comparing the amount of fetal DNA from step b) to step c), wherein a biologically or statistically significant difference between the amount of target and reference fetal DNA is indicative of the presence of a fetal aneuploidy. In one embodiment, the method for determining the amount of fetal DNA does not require the use of a polymorphic sequence. For example, an allelic ratio is not used to quantify the fetal DNA in step b). In another embodiment, the method for determining the amount of fetal DNA does not require the treatment of DNA with bisulfite to convert cytosine residues to uracil. In one embodiment, determining the amount of fetal DNA in steps b) and c) is done by introducing one or more competitors at known concentrations. In another embodiment, determining the amount of fetal DNA in steps b) and c) is done by RT-PCR, sequencing or counting. In another embodiment, the amount of fetal DNA from a target chromosome determined in step b) is compared to a standard control, for example, the amount of fetal DNA from a target chromosome from euploid pregnancies. In another embodiment, the restriction efficiency is determined and used to further determine the amount of fetal DNA from a target chromosome and from a reference chromosome. Exemplary differentially methylated nucleic acids are provided in SEQ ID NOs: 1-89.

In another aspect of the technology, a method is provided for detecting the presence or absence of a chromosomal abnormality by analyzing the amount or copy number of target nucleic acid and control nucleic acid from a sample of differentially methylated nucleic acids comprising the steps of: (a) enriching a target nucleic acid, from a sample, and a control nucleic acid, from the sample, based on its methylation state; (b) performing a copy number analysis of the enriched target nucleic acid in at least one of the fractions; (c) performing a copy number analysis of the enriched control nucleic acid in at least one of the fractions; (d) comparing the copy number from step (b) with the copy number from step (c); and (e) determining if a chromosomal abnormality exists based on the comparison in step (d), wherein the target nucleic acid and control nucleic acid have the same or substantially the same methylation status. In a related embodiment, a method is provided for detecting the presence or absence of a chromosomal abnormality by analyzing the amount or copy number of target nucleic acid and control nucleic acid from a sample of differentially methylated nucleic acids comprising the steps of: (a) binding a target nucleic acid, from a sample, and a control nucleic acid, from the sample, to a binding agent; (b) eluting the bound nucleic acid based on methylation status, wherein differentially methylated nucleic acids elute at least partly into separate fractions; (c) performing a copy number analysis of the eluted target nucleic acid in at least one of the fractions; (d) performing a copy number analysis of the eluted control nucleic acid in at least one of the fractions; (e) comparing the copy number from step (c) with the copy number from step (d); and (f) determining if a chromosomal abnormality exists based on the comparison in step (e), wherein the target nucleic acid and control nucleic acid have the same or substantially the same methylation status. Differentially methylated nucleic acids are provided in SEQ ID NOs: 1-89.

In another aspect of the technology, a method is provided for detecting the presence or absence of a chromosomal abnormality by analyzing the allelic ratio of target nucleic acid and control nucleic acid from a sample of differentially methylated nucleic acids comprising the steps of: (a) binding a target nucleic acid, from a sample, and a control nucleic acid, from the sample, to a binding agent; (b) eluting the bound nucleic acid based on methylation status, wherein differentially methylated nucleic acids elute at least partly into separate fractions; (c) performing an allelic ratio analysis of the eluted target nucleic acid in at least one of the fractions; (d) performing an allelic ratio analysis of the eluted control nucleic acid in at least one of the fractions; (e) comparing the allelic ratio from step c with the allelic ratio from step d; and (f) determining if a chromosomal abnormality exists based on the comparison in step (e), wherein the target nucleic acid and control nucleic acid have the same or substantially the same methylation status. Differentially methylated nucleic acids are provided in SEQ ID NOs: 1-89, and SNPs within the differentially methylated nucleic acids are provided in Table 2. The methods may also be useful for detecting a pregnancy-associated disorder.

In another aspect of the technology, the amount of maternal nucleic acid is determined using the methylation-based methods of the technology. For example, fetal nucleic acid can be separated (for example, digested using a methylation-sensitive enzyme) from the maternal nucleic acid in a sample, and the maternal nucleic acid can be quantified using the methods of the technology. Once the amount of maternal nucleic acid is determined, that amount can subtracted from the total amount of nucleic acid in a sample to determine the amount of fetal nucleic acid. The amount of fetal nucleic acid can be used to detect fetal traits, including fetal aneuploidy, as described herein.

For aspects and embodiments of the technology described herein, the methods may also be useful for detecting a pregnancy-associated disorder. In some embodiments, the sample comprises fetal nucleic acid, or fetal nucleic acid and maternal nucleic acid. In the case when the sample comprises fetal and maternal nucleic acid, the fetal nucleic acid and the maternal nucleic acid may have a different methylation status. Nucleic acid species with a different methylation status can be differentiated by any method known in the art. In an embodiment, the fetal nucleic acid is enriched by the selective digestion of maternal nucleic acid by a methylation sensitive restriction enzyme. In another embodiment, the fetal nucleic acid is enriched by the selective digestion of maternal nucleic acid using two or more methylation sensitive restriction enzymes in the same assay. In an embodiment, the target nucleic acid and control nucleic acid are both from the fetus. In another embodiment, the average size of the fetal nucleic acid is about 100 bases to about 500 bases in length. In another embodiment the chromosomal abnormality is an aneuploidy, such as trisomy 21. In some embodiments, the target nucleic acid is at least a portion of a chromosome which may be abnormal and the control nucleic acid is at least a portion of a chromosome which is very rarely abnormal. For example, when the target nucleic acid is from chromosome 21, the control nucleic acid is from a chromosome other than chromosome 21- preferably another autosome. In another embodiment, the binding agent is a methylation-specific binding protein such as MBD-Fc. Also, the enriched or eluted nucleic acid is amplified and/or quantified by any method known in the art. In an embodiment, the fetal DNA is quantified using a method that does not require the use of a polymorphic sequence. For example, an allelic ratio is not used to quantify the fetal DNA. In another embodiment, the method for quantifying the amount of fetal DNA does not require the treatment of DNA with bisulfite to convert cytosine residues to uracil.

In some embodiments, the methods of the technology include the additional step of determining the amount of one or more Y-chromosome-specific sequences in a sample. In a related embodiment, the amount of fetal nucleic acid in a sample as determined by using the methylation-based methods of the technology is compared to the amount of Y-chromosome nucleic acid present.

Methods for differentiating nucleic acid based on methylation status include, but are not limited to, methylation sensitive capture, for example using, MBD2-Fc fragment; bisulfite conversion methods, for example, MSP (methylation-sensitive PCR), COBRA, methylation-sensitive single nucleotide primer extension (Ms-SNuPE) or Sequenom MassCLEAVE™ technology; and the use of methylation sensitive restriction enzymes. Except where explicitly stated, any method for differentiating nucleic acid based on methylation status can be used with the compositions and methods of the technology.

In some embodiments, methods of the technology may further comprise an amplification step. The amplification step can be performed by PCR, such as methylation-specific PCR. In another embodiment, the amplification reaction is performed on single molecules, for example, by digital PCR, which is further described in US Patent Nos 6,143,496 and 6,440,706, both of which are hereby incorporated by reference. In other embodiments, the method does not require amplification. For example, the amount of enriched fetal DNA may be determined by counting the fetal DNA (or sequence tags attached thereto) with a flow cytometer or by sequencing means that do not require amplification. In another embodiment, the amount of fetal DNA is determined by an amplification reaction that generates amplicons larger than the digested maternal nucleic acid, thereby further enriching the fetal nucleic acid.

For embodiments that require sequence analysis, any one of the following sequencing technologies may be used: a primer extension method (e.g., iPLEX®; Sequenom, Inc.), direct DNA sequencing, restriction fragment length polymorphism (RFLP analysis), allele specific oligonucleotide (ASO) analysis, methylation-specific PCR (MSPCR), pyrosequencing analysis, acycloprime analysis, Reverse dot blot, GeneChip microarrays, Dynamic allele-specific hybridization (DASH), Peptide nucleic acid (PNA) and locked nucleic acids (LNA) probes, TaqMan, Molecular Beacons, Intercalating dye, FRET primers, fluorescence tagged dNTP/ddNTPs, AlphaScreen, SNPstream, genetic bit analysis (GBA), Multiplex minisequencing, SNaPshot, GOOD assay, Microarray miniseq, arrayed primer extension (APEX), Microarray primer extension, Tag arrays, Coded microspheres, Template-directed incorporation (TDI), fluorescence polarization, Colorimetric oligonucleotide ligation assay (OLA), Sequence-coded OLA, Microarray ligation, Ligase chain reaction, Padlock probes, Invader™ assay, hybridization using at least one probe, hybridization using at least one fluorescently labeled probe, electrophoresis, cloning and sequencing, for example as performed on the 454 platform (Roche) (Margulies, M. et al. 2005 Nature 437, 376-380), Illumina Genome Analyzer (or Solexa platform) or SOLiD System (Applied Biosystems) or the Helicos True Single Molecule DNA sequencing technology (Harris T D et al. 2008 Science, 320, 106-109), the single molecule, real-time (SMRT.TM.) technology of Pacific Biosciences, or nanopore-based sequencing (Soni GV and Meller A. 2007 Clin Chem 53: 1996-2001) and combinations thereof. Nanopore-based methods may include sequencing nucleic acid using a nanopore, or counting nucleic acid molecules using a nanopore, for example, based on size wherein sequence information is not determined.

The absolute copy number of one or more nucleic acids can be determined, for example, using mass spectrometry, a system that uses a competitive PCR approach for absolute copy number measurements. See for example, Ding C, Cantor CR (2003) A high-throughput gene expression analysis technique using competitive PCR and matrix-assisted laser desorption ionization time-of-flight MS. Proc Natl Acad Sci U S A 100:3059-3064, and US Patent Application No. 10/655762, which published as US Patent Publication No. 20040081993, both of which are hereby incorporated by reference.

In some embodiments, the amount of the genomic sequence is compared with a standard control, wherein an increase or decrease from the standard control indicates the presence or progression of a pregnancy-associated disorder. For example, the amount of fetal nucleic acid may be compared to the total amount of DNA present in the sample. Or when detecting the presence or absence of fetal aneuploidy, the amount of fetal nucleic acid from target chromosome may be compared to the amount of fetal nucleic acid from a reference chromosome. Preferably the reference chromosome is another autosome that has a low rate of aneuploidy. The ratio of target fetal nucleic acid to reference fetal nucleic acid may be compared to the same ratio from a normal, euploid pregnancy. For example, a control ratio may be determined from a DNA sample obtained from a female carrying a healthy fetus who does not have a chromosomal abnormality. Preferably, one uses a panel of control samples. Where certain chromosome anomalies are known, one can also have standards that are indicative of a specific disease or condition. Thus, for example, to screen for three different chromosomal aneuploidies in a maternal plasma of a pregnant female, one preferably uses a panel of control DNAs that have been isolated from mothers who are known to carry a fetus with, for example, chromosome 13, 18, or 21 trisomy, and a mother who is pregnant with a fetus who does not have a chromosomal abnormality.

In some embodiments, the present technology provides a method in which the alleles from the target nucleic acid and control nucleic acid are differentiated by sequence variation. The sequence variation may be a single nucleotide polymorphism (SNP) or an insertion/deletion polymorphism. In an embodiment, the fetal nucleic acid should comprise at least one high frequency heterozygous polymorphism (e.g., about 2%, 3%, 4%, 5%, 6%, 7%, 8%, 9%, 10%, 11%, 12%, 13%, 14%, 15%, 16%, 17%, 18%, 19%, 20%, 25% or more frequency rate), which allows the determination of the allelic-ratio of the nucleic acid in order to assess the presence or absence of the chromosomal abnormality. A list of exemplary SNPs is provided in Table 2, however, this does not represent a complete list of polymorphic alleles that can be used as part of the technology. Any SNP meeting the following criteria may also be considered: (a) the SNP has a heterozygosity frequency greater than about 2% (preferably across a range of different populations), (b) the SNP is a heterozygous locus; and (c)(i) the SNP is within nucleic acid sequence described herein, or (c)(iii) the SNP is within about 5 to about 2000 base pairs of a SNP described herein (e.g., within about 5, 10, 15, 20, 25, 30, 40, 50, 60, 70, 80, 90, 100, 200, 300, 400, 500, 600, 700, 800, 900, 1000, 1250, 1500, 1750 or 2000 base pairs of a SNP described herein).

In other embodiments, the sequence variation is a short tandem repeat (STR) polymorphism. In some embodiments, the sequence variation falls in a restriction site, whereby one allele is susceptible to digestion by a restriction enzyme and the one or more other alleles are not. In some embodiments, the sequence variation is a methylation site.

In some embodiments, performing an allelic ratio analysis comprises determining the ratio of alleles of the target nucleic acid and control nucleic acid from the fetus of a pregnant woman by obtaining an nucleic acid-containing biological sample from the pregnant woman, wherein the biological sample contains fetal nucleic acid, partially or wholly separating the fetal nucleic acid from the maternal nucleic acid based on differential methylation, discriminating the alleles from the target nucleic acid and the control nucleic acid, followed by determination of the ratio of the alleles, and detecting the presence or absence of a chromosomal disorder in the fetus based on the ratio of alleles, wherein a ratio above or below a normal, euploid ratio is indicative of a chromosomal disorder. In one embodiment, the target nucleic acid is from a suspected aneuploid chromosome (e.g., chromosome 21) and the control nucleic acid is from a euploid chromosome from the same fetus.

In some embodiments, the present technology is combined with other fetal markers to detect the presence or absence of multiple chromosomal abnormalities, wherein the chromosomal abnormalities are selected from the following: trisomy 21, trisomy 18 and trisomy 13, or combinations thereof. In some embodiments, the chromosomal disorder involves the X chromosome or the Y chromosome.

In some embodiments, the compositions or processes may be multiplexed in a single reaction. For example, the amount of fetal nucleic acid may be determined at multiple loci across the genome. Or when detecting the presence or absence of fetal aneuploidy, the amount of fetal nucleic acid may be determined at multiple loci on one or more target chromosomes (e.g., chromosomes 13, 18 or 21) and on one or more reference chromosomes. If an allelic ratio is being used, one or more alleles from Table 2 can be detected and discriminated simultaneously. When determining allelic ratios, multiplexing embodiments are particularly important when the genotype at a polymorphic locus is not known. In some instances, for example when the mother and child are homozygous at the polymorphic locus, the assay may not be informative. In one embodiment, greater than 1, 2, 3, 4, 5, 6, 7, 8, 9, 10, 11,12, 13, 14, 15, 16, 17, 18, 19, 20, 25, 30, 35, 40, 50, 100, 200, 300 or 500, and any intermediate levels, polynucleotide sequences of the technology are enriched, separated and/or examined according the methods of the technology. When detecting a chromosomal abnormality by analyzing the copy number of target nucleic acid and control nucleic acid, less than 3, 4, 5, 6, 7, 8, 9, 10, 11, 12, 13, or 14 polynucleotide sequences may need to be analyzed to accurately detect the presence or absence of a chromosomal abnormality. In another embodiment, the compositions or processes of the technology may be used to assay samples that have been divided into 2, 3, 4, 5, 6, 7, 8, 9, 10, 11, 12, 13, 14, 15, 16, 17, 18, 19, 20, 25, 30, 35, 40, 50, 100 or more replicates, or into single molecule equivalents. Methods for analyzing fetal nucleic acids from a maternal sample in replicates, including single molecule analyses, are provided in US Application No, 11/364,294, which published as US Patent Publication No. US 2007-0207466 A1, which is hereby incorporated by reference.

In a further embodiment, the present technology provides a method wherein a comparison step shows an increased risk of a fetus having a chromosomal disorder if the ratio of the alleles or absolute copy number of the target nucleic acid is higher or lower by 1 standard deviation from the standard control sequence. In some embodiments, the comparison step shows an increased risk of a fetus having a chromosomal disorder if the ratio of the alleles or absolute copy number of the target nucleic acid is higher or lower by 2 standard deviation from the standard control sequence. In some other embodiments, the comparison step shows an increased risk of a fetus having a chromosomal disorder if the ratio of the alleles or absolute copy number of the target nucleic acid is higher or lower by 3 standard deviation from the standard control sequence. In some embodiments, the comparison step shows an increased risk of a fetus having a chromosomal disorder if the ratio of the alleles or absolute copy number of the target nucleic acid is higher or lower than a statistically significant standard deviation from the control. In one embodiment, the standard control is a maternal reference, and in another embodiment the standard control is a fetal reference chromosome (e.g., non-trisomic autosome).

In some embodiments, the methods of the technology may be combined with other methods for diagnosing a chromosomal abnormality. For example, a noninvasive diagnostic method may require confirmation of the presence or absence of fetal nucleic acid, such as a sex test for a female fetus or to confirm an RhD negative female fetus in an RhD negative mother. In another embodiment, the compositions and methods of the technology may be used to determine the percentage of fetal nucleic acid in a maternal sample in order to enable another diagnostic method that requires the percentage of fetal nucleic acid be known. For example, does a sample meet certain threshold concentration requirements? When determining an allelic ratio to diagnose a fetal aneuploidy from a maternal sample, the amount or concentration of fetal nucleic acid may be required to make a diagnose with a given sensitivity and specificity. In other embodiments, the compositions and methods of the technology for detecting a chromosomal abnormality can be combined with other known methods thereby improving the overall sensitivity and specificity of the detection method. For example, mathematical models have suggested that a combined first-trimester screening program utilizing maternal age (MA), nuchal translucency (NT) thickness, serum-free beta-hCG, and serum PAPP-A will detect more than 80% of fetuses with Down's syndrome for a 5% invasive testing rate (Wald and Hackshaw, Prenat Diagn 17(9):921-9 (1997)). However, the combination of commonly used aneuploidy detection methods combined with the non-invasive free fetal nucleic acid-based methods described herein may offer improved accuracy with a lower false positive rate. Examples of combined diagnostic methods are provided in PCT Publication Number WO2008157264A2 (assigned to the Applicant), which is hereby incorporated by reference. In some embodiments, the methods of the technology may be combined with cell-based methods, wherein fetal cells are procured invasively or non-invasively.

In certain embodiments, an increased risk for a chromosomal abnormality is based on the outcome or result(s) produced from the compositions or methods provided herein. An example of an outcome is a deviation from the euploid absolute copy number or allelic ratio, which indicates the presence of chromosomal aneuploidy. This increase or decrease in the absolute copy number or ratio from the standard control indicates an increased risk of having a fetus with a chromosomal abnormality (e.g., trisomy 21). Information pertaining to a method described herein, such as an outcome, result, or risk of trisomy or aneuploidy, for example, may be transfixed, renditioned, recorded and/or displayed in any suitable medium. For example, an outcome may be transfixed in a medium to save, store, share, communicate or otherwise analyze the outcome. A medium can be tangible (e.g., paper) or intangible (e.g., electronic medium), and examples of media include, but are not limited to, computer media, databases, charts, patient charts, records, patient records, graphs and tables, and any other medium of expression. The information sometimes is stored and/or renditioned in computer readable form and sometimes is stored and organized in a database. In certain embodiments, the information may be transferred from one location to another using a physical medium (e.g., paper) or a computer readable medium (e.g., optical and/or magnetic storage or transmission medium, floppy disk, hard disk, random access memory, computer processing unit, facsimile signal, satellite signal, transmission over an internet or transmission over the world-wide web).

A CpG island may be used as the CpG-containing genomic sequence in some cases, whereas in other cases the CpG-containing genomic sequence may not be a CpG island. In some embodiments, the present technology provides a kit for performing the methods of the technology. One component of the kit is a methylation-sensitive binding agent.

### BRIEF DESCRIPTION OF THE DRAWINGS

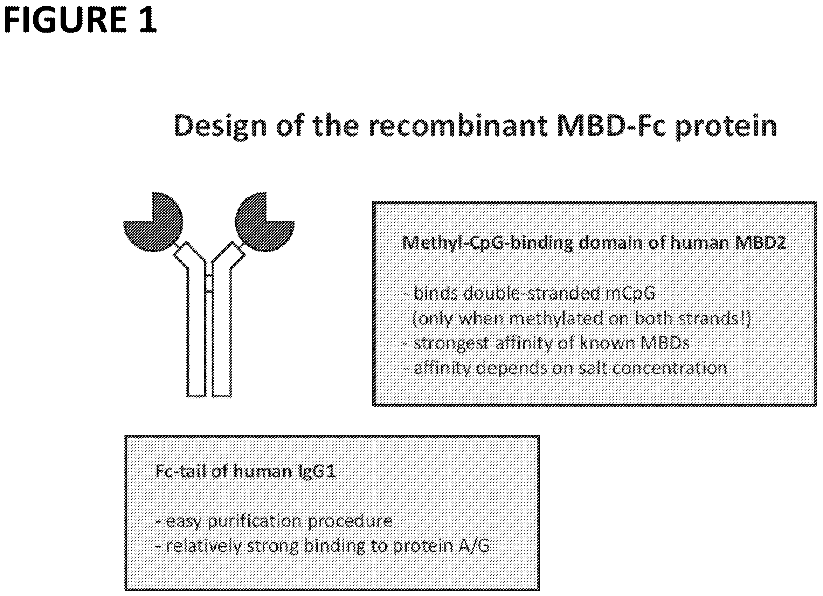

FIGURE 1: Shows the design of the recombinant MBD-Fc protein used to separate differentially methylated DNA.

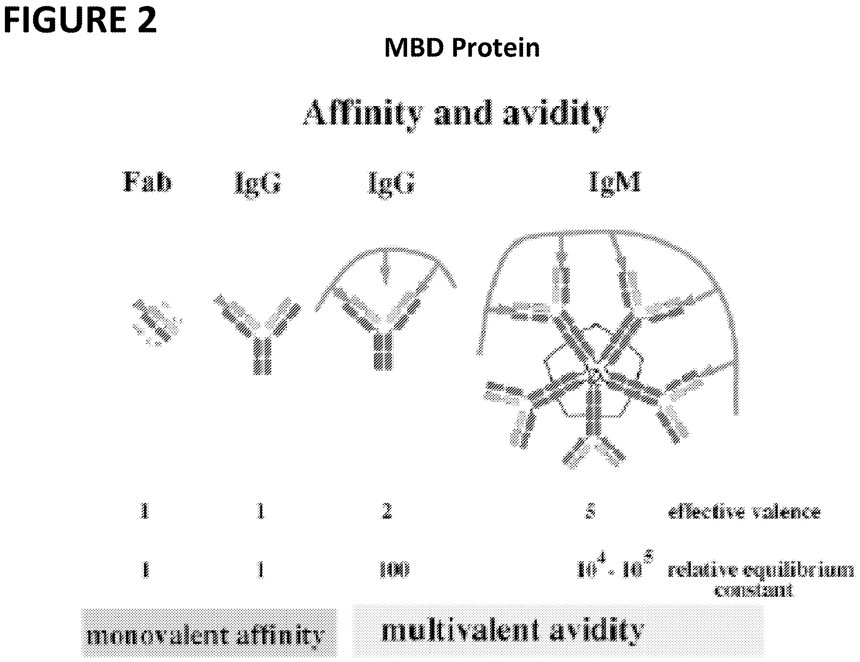

FIGURE 2: Shows the methyl-CpG-binding, antibody-like protein has a high affinity and high avidity to its "antigen", which is preferably DNA that is methylated at CpG di-nucleotides.

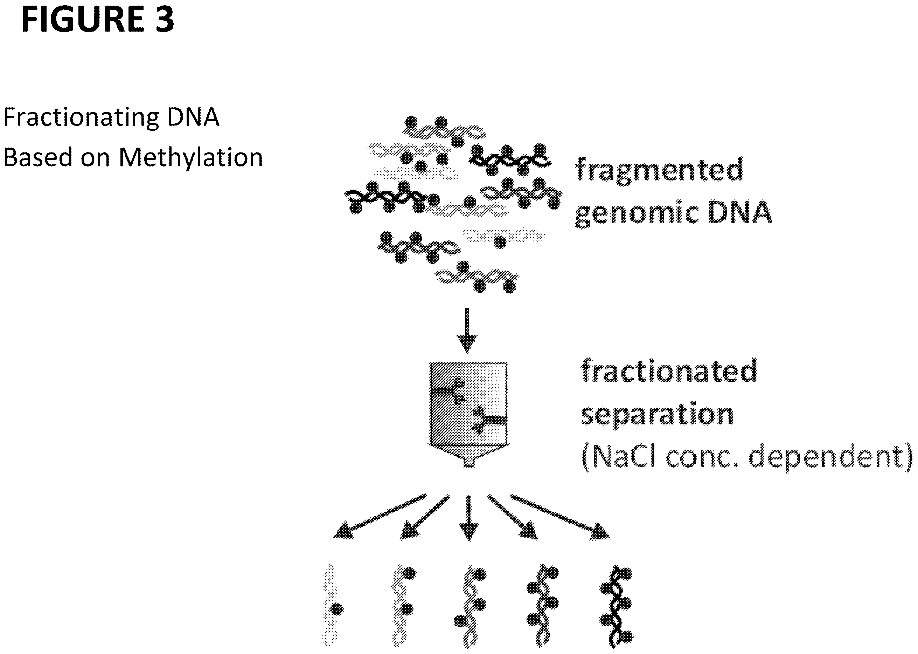

FIGURE 3: Shows the methyl binding domain of MBD-FC binds DNA molecules regardless of their methylation status. The strength of this protein/DNA interaction is defined by the level of DNA methylation. After binding genomic DNA, eluate solutions of increasing salt concentrations can be used to fractionate non-methylated and methylated DNA allowing for a controlled separation.

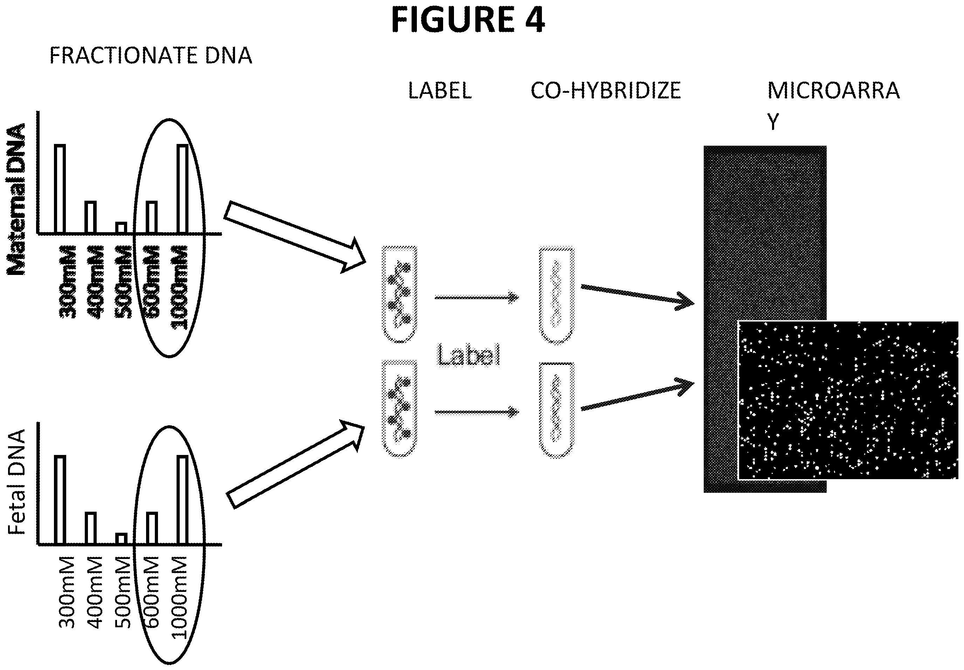

FIGURE 4: Shows the experiment used to identify differentially methylated DNA from a fetus and mother using the recombinant MBD-Fc protein and a microarray.

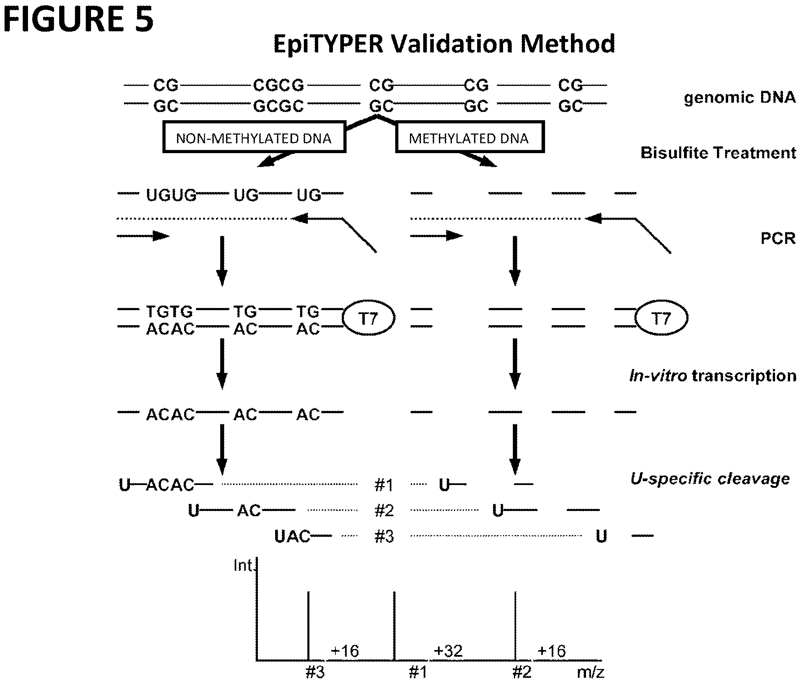

FIGURE 5: Shows typical results generated by Sequenom® EpiTYPER™ method, which was used to validate the results generated from the experiment illustrated in Figure 4.

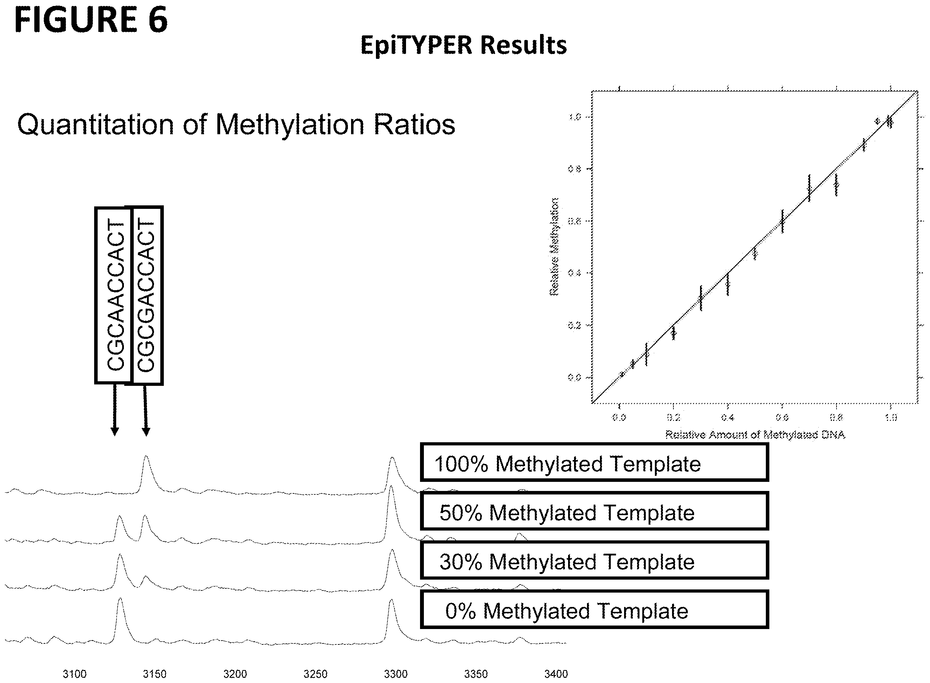

FIGURE 6: Shows the correlation between the log ratios derived from microarray analysis (x axis) and methylation differences obtained by EpiTYPER analysis (y axis). Each data point represents the average for one region across all measured samples. The microarray analysis is comparative in nature because the highly methylated fraction of the maternal DNA is hybridized together with the highly methylated fraction of placenta DNA. Positive values indicate higher methylation of the placenta samples. In mass spectrometry each samples is measured individually. We first calculated difference in methylation by subtracting the maternal methylation values from the placenta methylation value. To compare the results with the microarray data we calculated the average of the differences for all maternal / placenta DNA pairs.

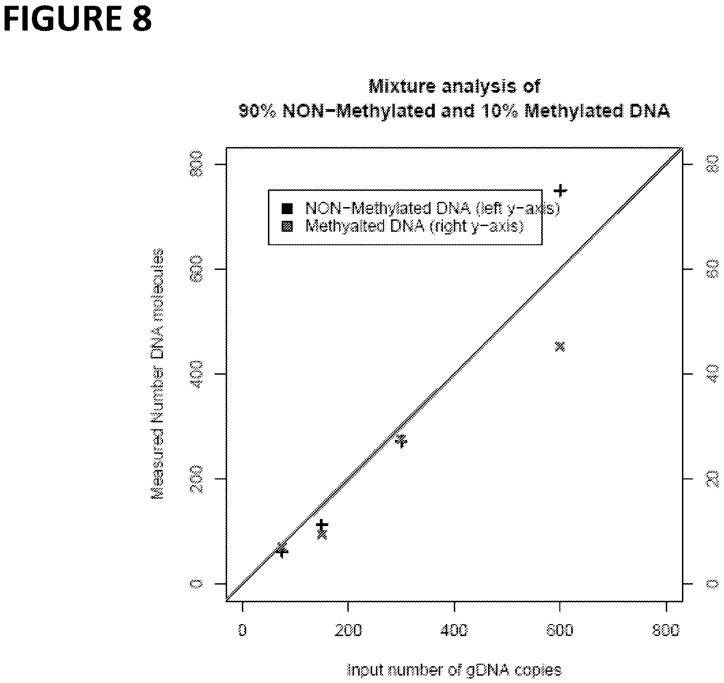

FIGURE 8: Shown is the correlation between the number of gDNA molecules that were expected and the number of molecules measured by competitive PCR in combination with mass spectrometry analysis. In this experiment we used DNA derived from whole blood (black plus signs) and commercially available fully methylated DNA(red crosses) in a 90 to 10 ratio. We used the MBD-FC fusion protein to separate the non-methylated and the methylated fraction of DNA. Each fraction was subject to competitive PCR analysis with mass spectrometry readout. The method has been described earlier for the analysis of copy number variations and is commercially available for gene expression analysis. The approach allows absolute quantification of DNA molecules with the help of a synthetic oligonucleotides of know concentration. In this experiment we targeted the MGMT locus, which was not methylated in the whole blood sample used here. Using an input of 300 total gDNA copies we expect to see 270 copies of non-methylated DNA and 30 copies of methylated DNA. The measured copy numbers are largely in agreement with the expected values. The data point at 600 copies of input DNA indicates a bias in the reaction and shows that this initial proof of concept experiment needs to be followed up with more development work, before the assay can be used. However, this initial data indicates the feasibility of the approach for capturing and quantifying of a few copies of methylated DNA in the presence of an excess of unmethylated DNA species.

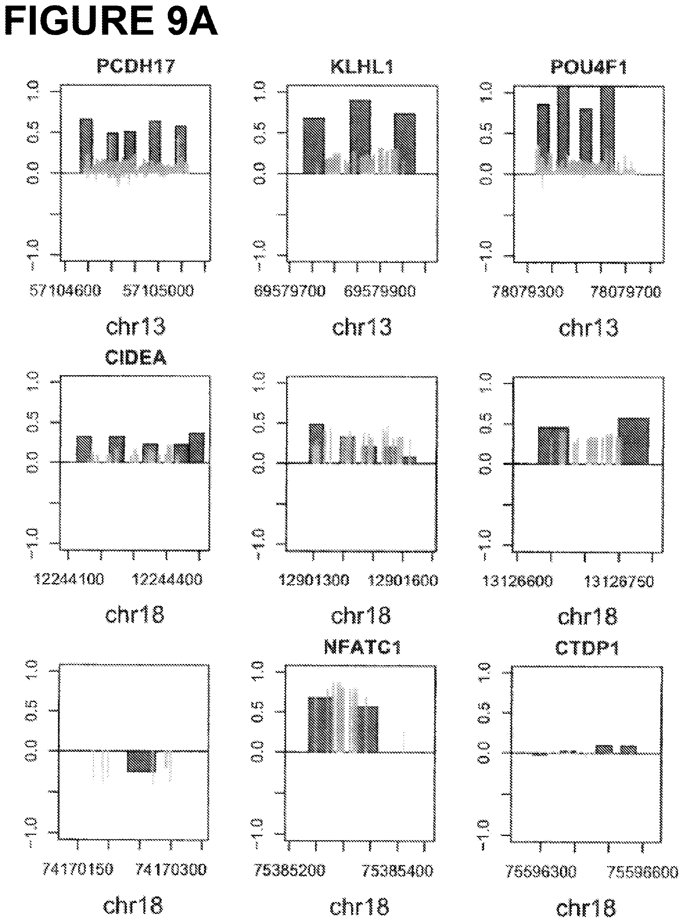

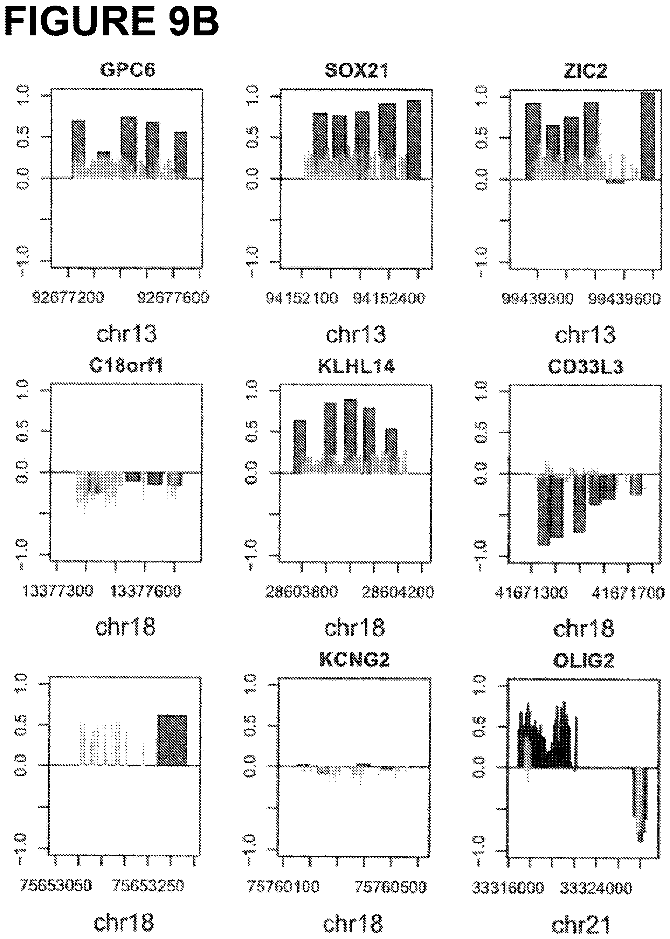

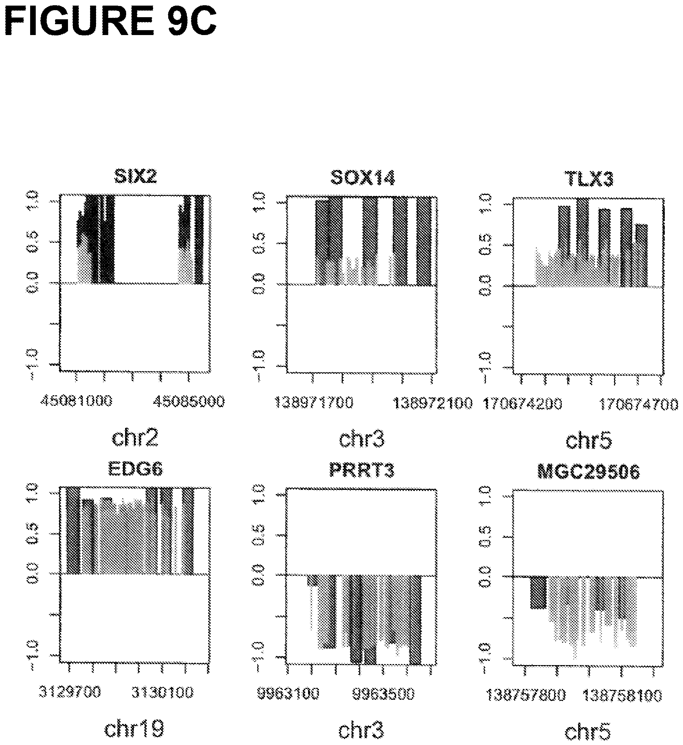

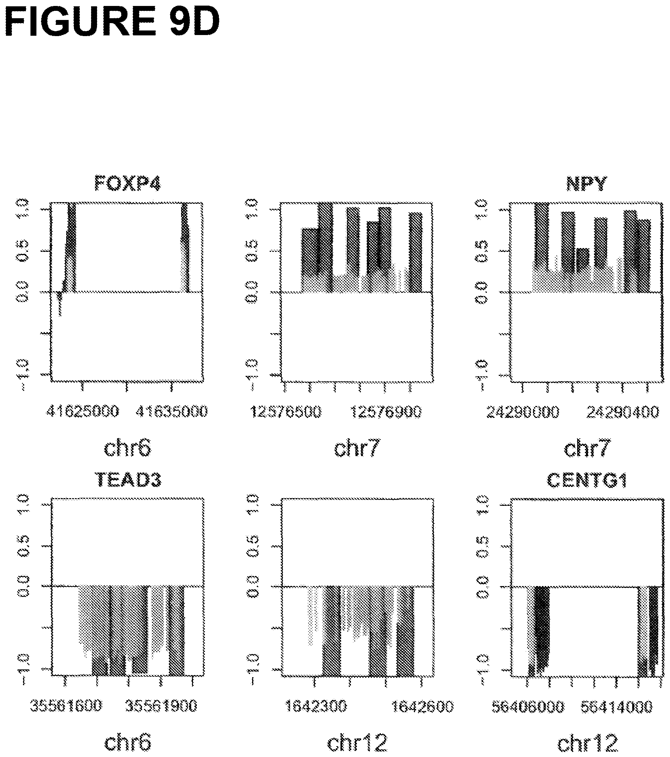

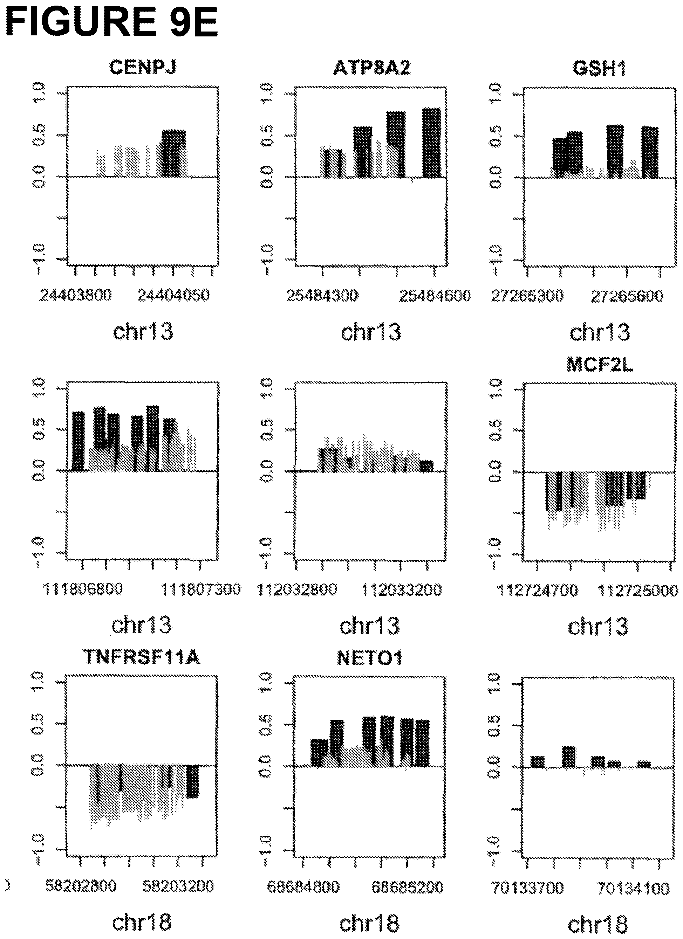

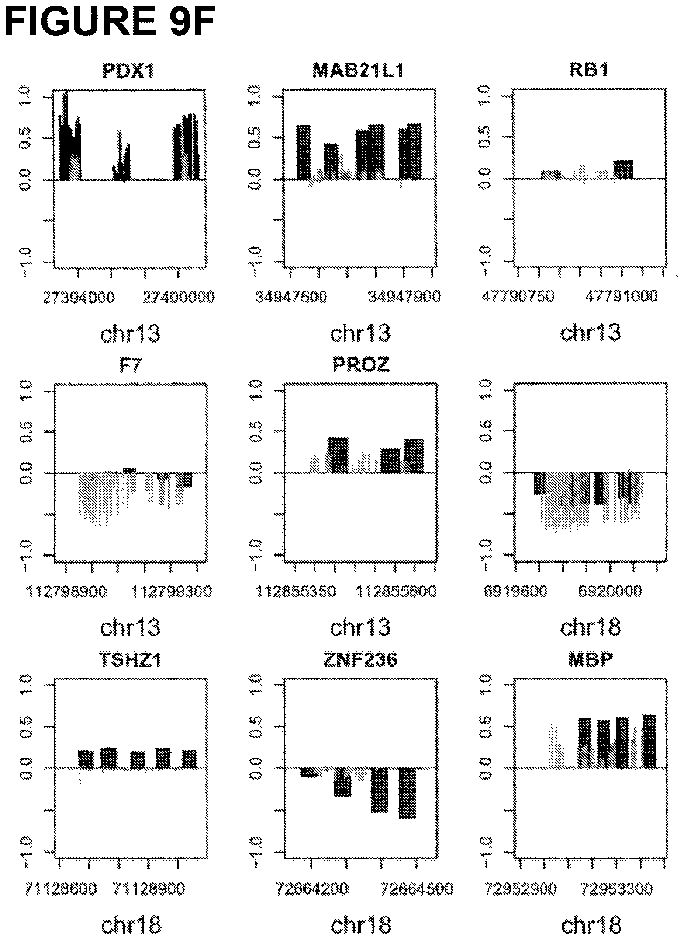

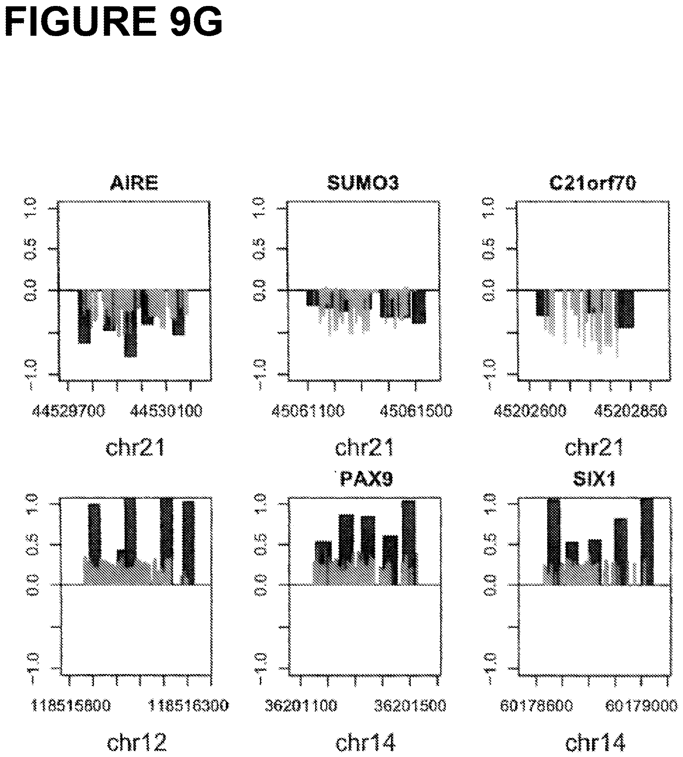

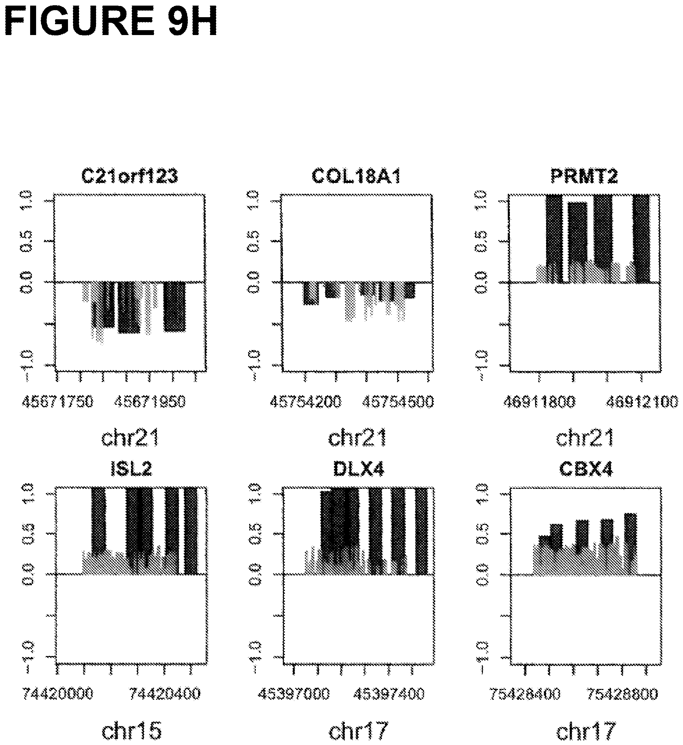

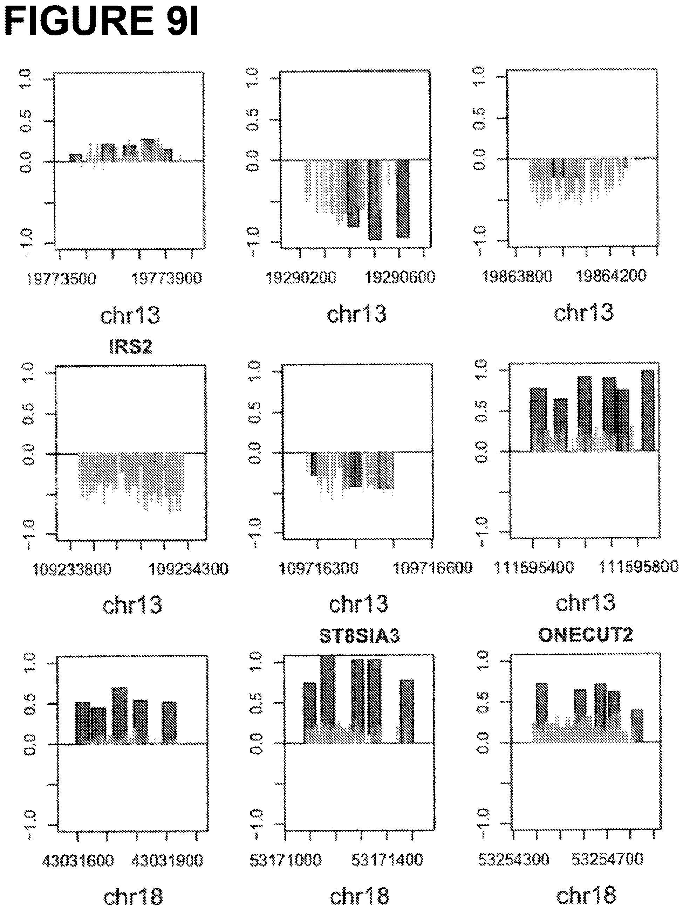

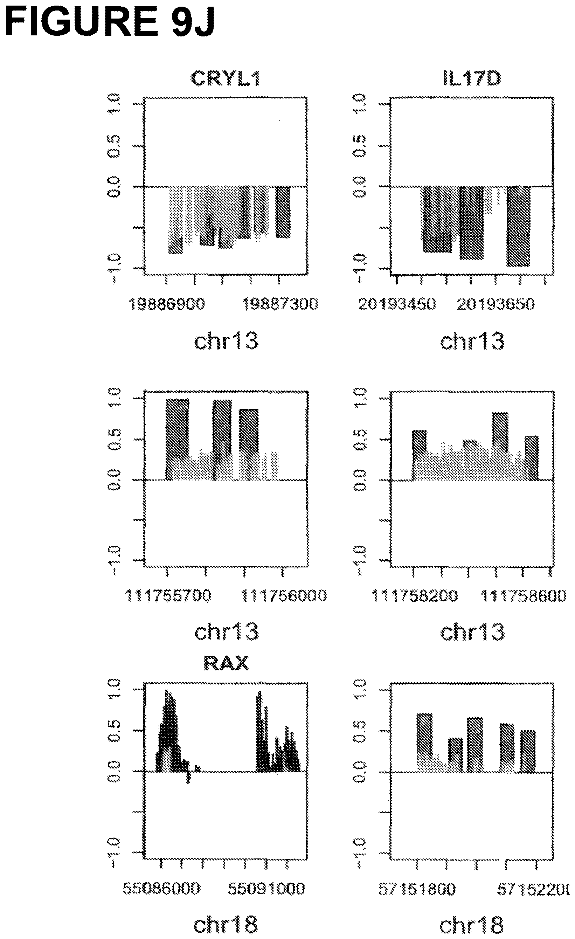

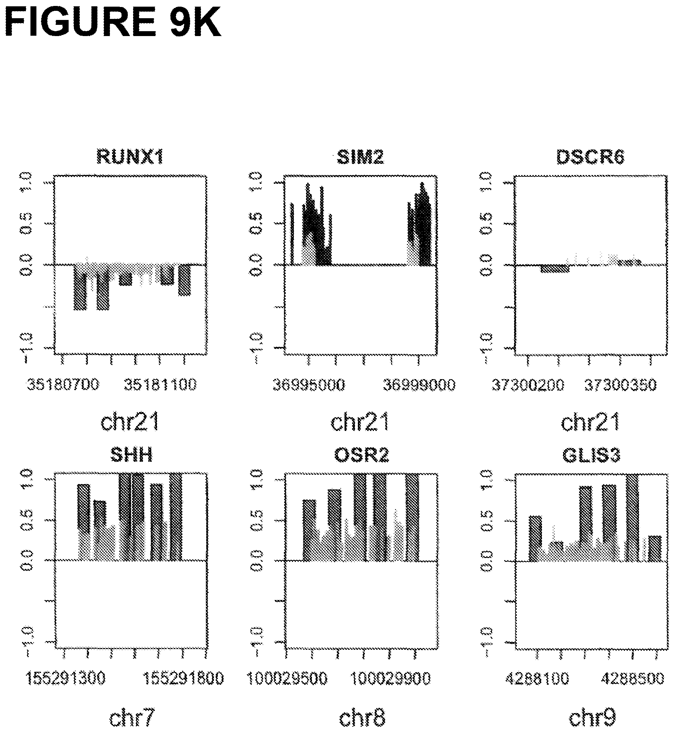

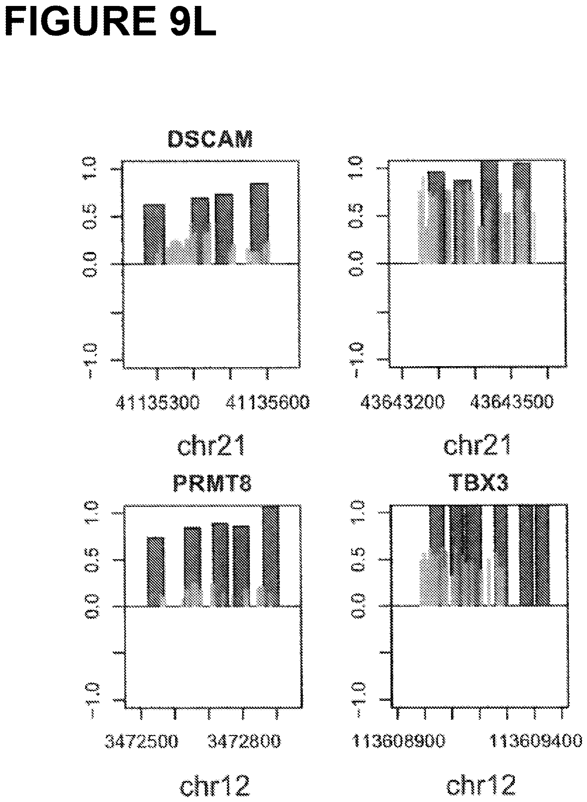

FIGURE 9A-9C: Shown are bar graph plots of the methylation differences obtained from the microarray analysis (dark bars) and the mass spectrometry analysis (light grey bars) with respect to their genomic location. For each of the 85 region that were identified to be differentially methylated by microarray an individual plot is provided. The x axis for each plot shows the chromosomal position of the region. The y axis depicts the log ration (in case of the microarrays) and the methylation differences (in case of the mass spectrometry results). For the microarrays each hybridization probe in the area is shown as a single black (or dark grey) bar. For the mass spectrometry results each CpG site, is shown as a light grey bar. Bars showing values greater than zero indicate higher DNA methylation in the placenta samples compared to the maternal DNA. For some genes the differences are small (i.e. RB1 or DSCR6) but still statistically significant. Those regions would be less suitable for a fetal DNA enrichment strategy.

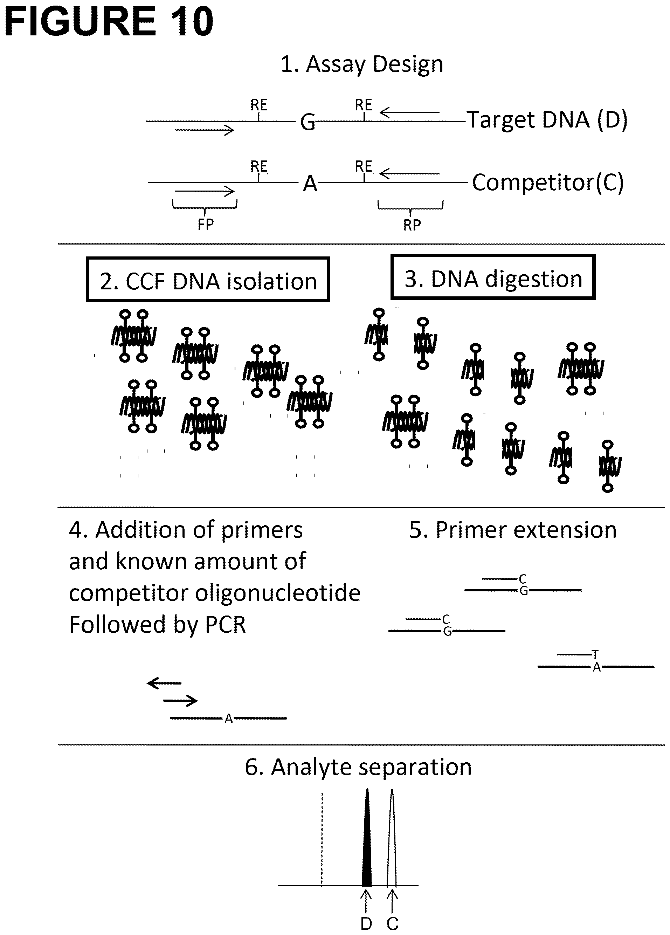

FIGURE 10: Shows one embodiment of the Fetal Quantifier Method. Maternal nucleic acid is selectively digested and the remaining fetal nucleic acid is quantified using a competitor of known concentration. In this schema, the analyte is separated and quantified by a mass spectromter.

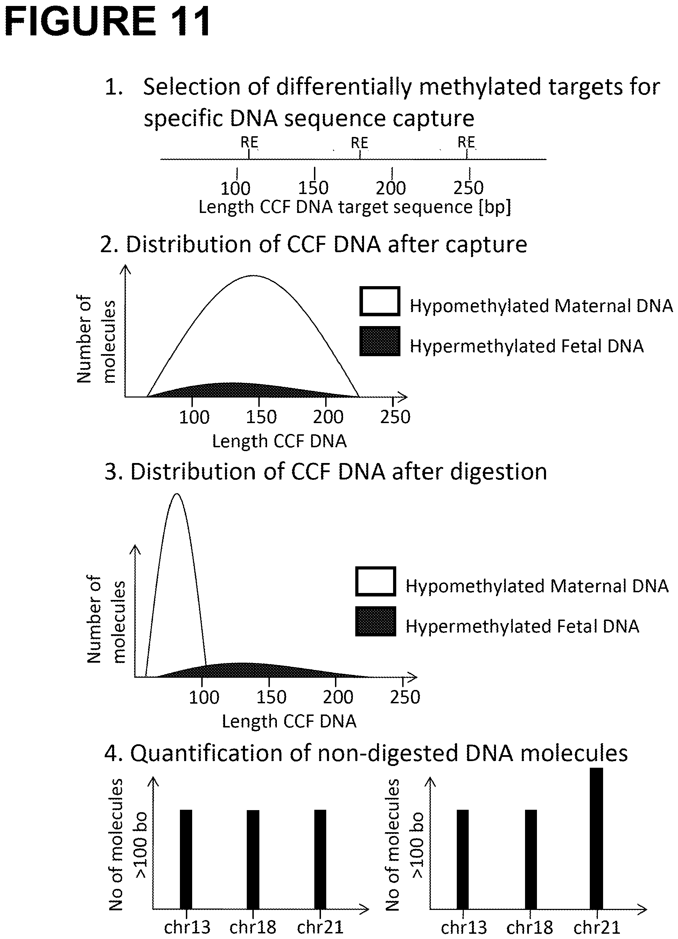

FIGURE 11: Shows one embodiment of the Methylation-Based Fetal Diagnostic Method. Maternal nucleic acid is selectively digested and the remaining fetal nucleic acid is quantified for three different chromosomes (13, 18 and 21). Parts 2 and 3 of the Figure illustrate the size distribution of the nucleic acid in the sample before and after digestion. The amplification reactions can be size-specific (e.g., greater than 100 base pair amplicons) such that they favor the longer, non-digested fetal nucleic acid over the digested maternal nucleic acid, thereby further enriching the fetal nucleic acid. The spectra at the bottom of the Figure show an increased amount of chromosome 21 fetal nucleic acid indicative of trisomy 21.

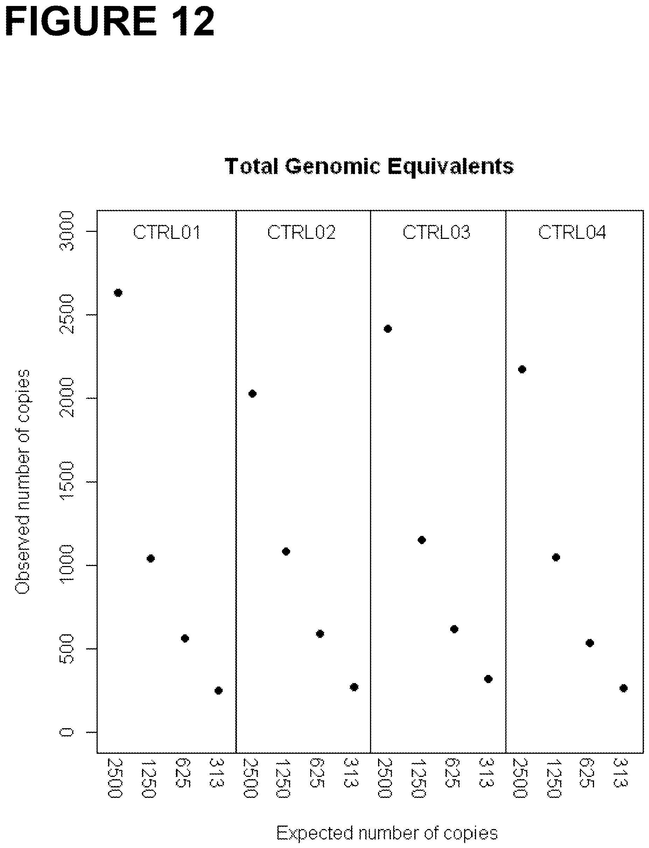

FIGURE 12: Shows the total number of amplifiable genomic copies from four different DNA samples isolated from the blood of non-pregnant women. Each sample was diluted to contain approximately 2500, 1250, 625 or 313 copies per reaction. Each measurement was obtained by taking the mean DNA/competitor ratio obtained from two total copy number assays (ALB and RNAseP in Table X). As Figure 12 shows, the total copy number is accurate and stable across the different samples, thus validating the usefulness of the competitor-based approach.

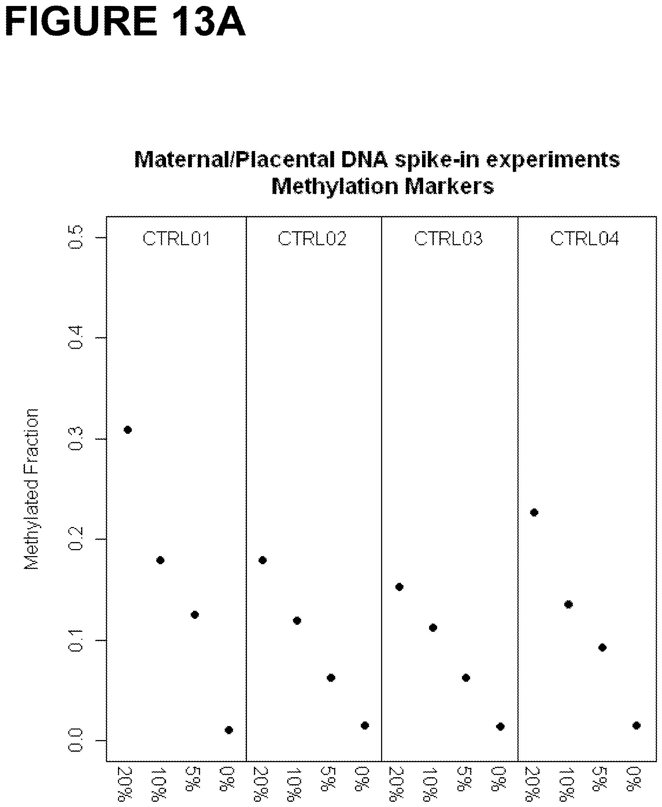

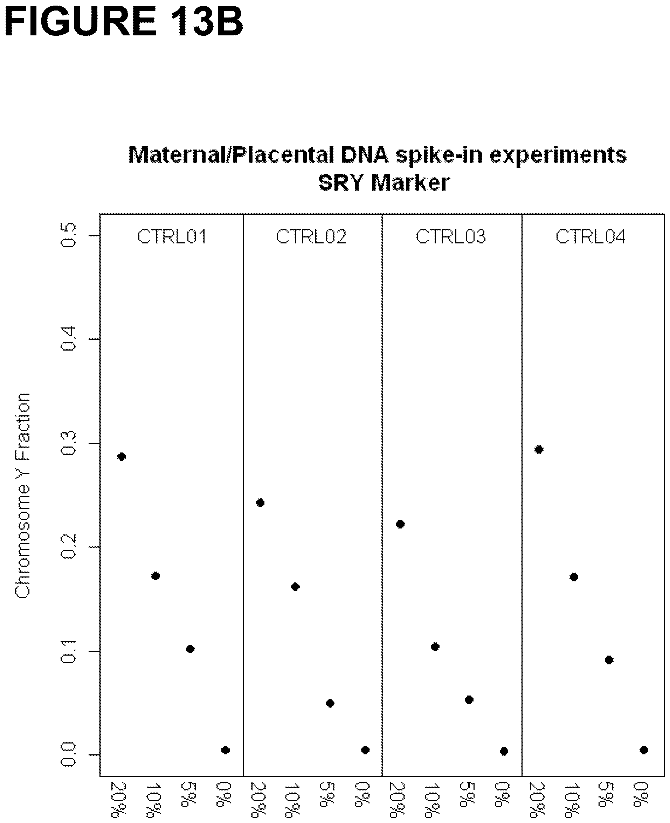

FIGURES 13A and B: A model system was created that contained a constant number of maternal non-methylated DNA with varying amounts of male placental methylated DNA spiked-in. The samples were spiked with male placental amounts ranging from approximately 0 to 25% relative to the maternal non-methylated DNA. The fraction of placental DNA was calculated using the ratios obtained from the methylation assays (Figure 13A) and the Y-chromosome marker (Figure 13B) as compared to the total copy number assay. The methylation and Y-chromosome markers are provided in Table X.

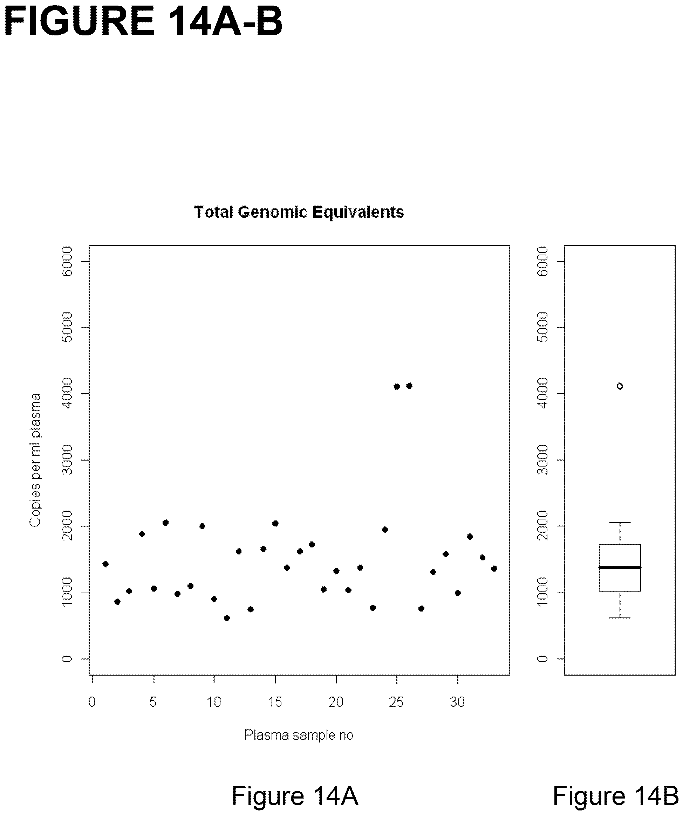

FIGURES 14 A and B: Show the results of the total copy number assay from plasma samples. In Figure 14A, the copy number for each sample is shown. Two samples (no 25 and 26) have a significantly higher total copy number than all the other samples. A mean of approximately 1300 amplifiable copies/ml plasma was obtained (range 766-2055). Figure 14B shows a box-and-whisker plot of the given values, summarizing the results.

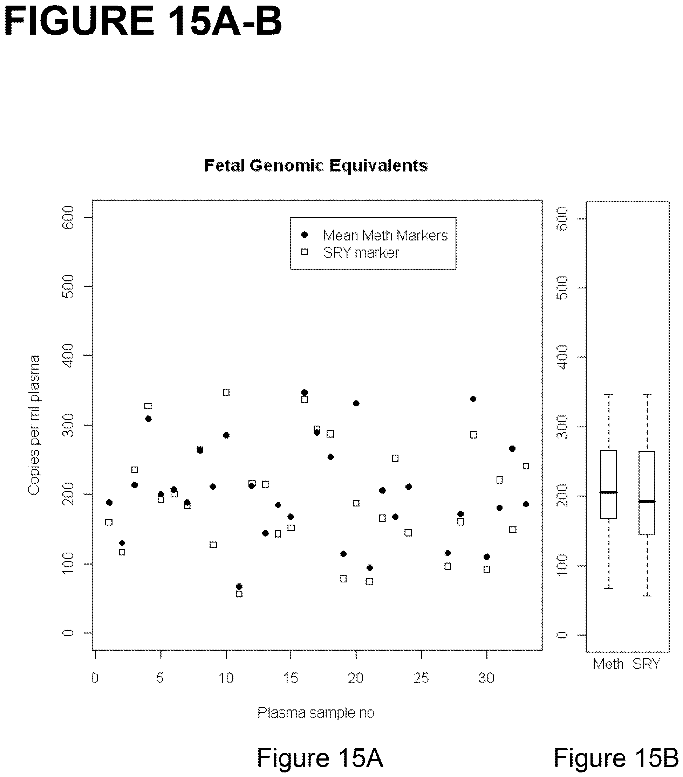

FIGURES 15A and B: The amount (or copy numbers) of fetal nucleic acid from 33 different plasma samples taken from pregnant women with male fetuses are plotted. The copy numbers obtained were calculated using the methylation markers and the Y-chromosome-specific markers using the assays provided in Table X. As can be seen in Figure 15B, the box-and-whisker plot of the given values indicated minimal difference between the two different measurements, thus validating the accuracy and stability of the method.

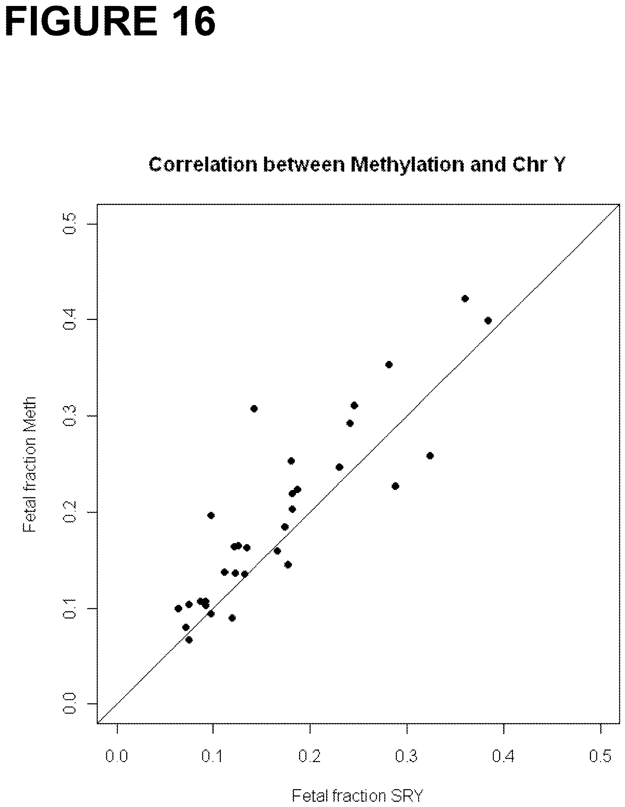

FIGURE 16: Shows a paired correlation between the results obtained using the methylation markers versus the Y-chromosome marker from Figure 15A.

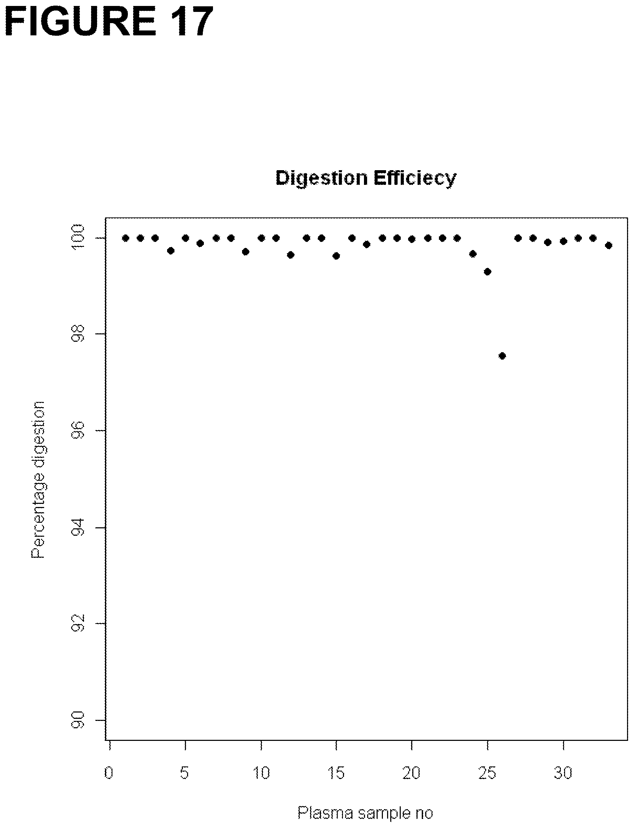

FIGURE 17: Shows the digestion efficiency of the restriction enzymes using the ratio of digestion for the control versus the competitor and comparing this value to the mean total copy number assays. Apart from sample 26 all reactions indicate the efficiency to be above about 99%.

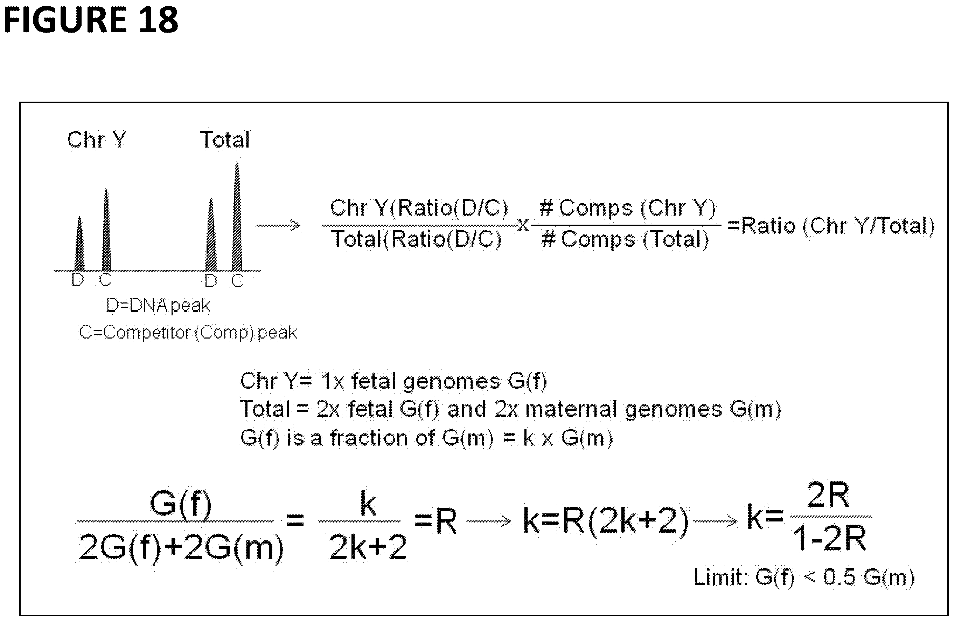

FIGURE 18: Provides a specific method for calculating fetal DNA fraction (or concentration) in a sample using the Y-chromosome-specific markers for male pregnancies and the mean of the methylated fraction for all pregnancies (regardless of fetal sex).

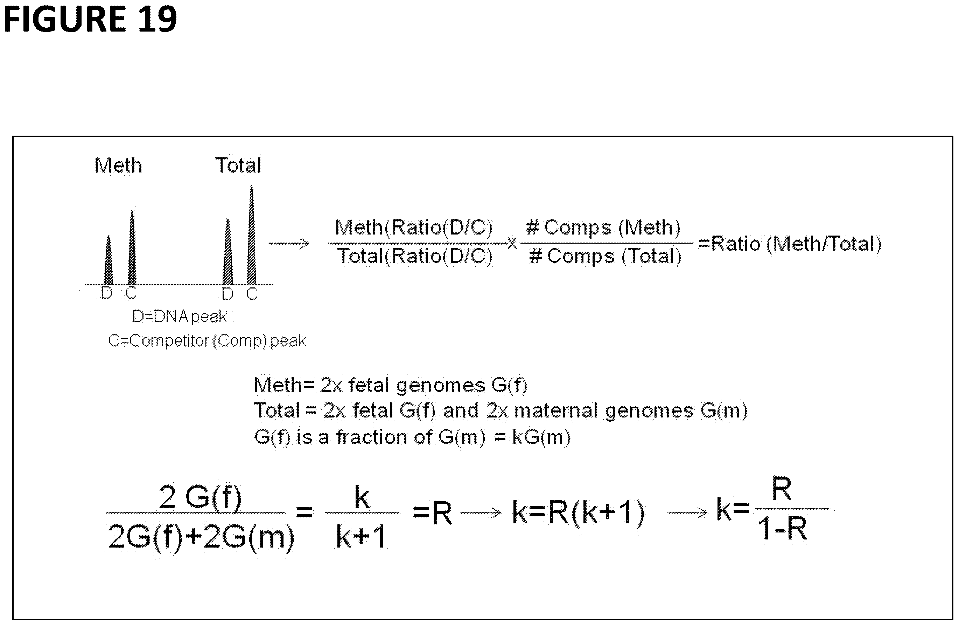

FIGURE 19: Provides a specific method for calculating fetal DNA fraction (or concentration) in a sample without the Y-chromosome-specific markers. Instead, only the Assays for Methylation Quantification were used to determine the concentration of fetal DNA.

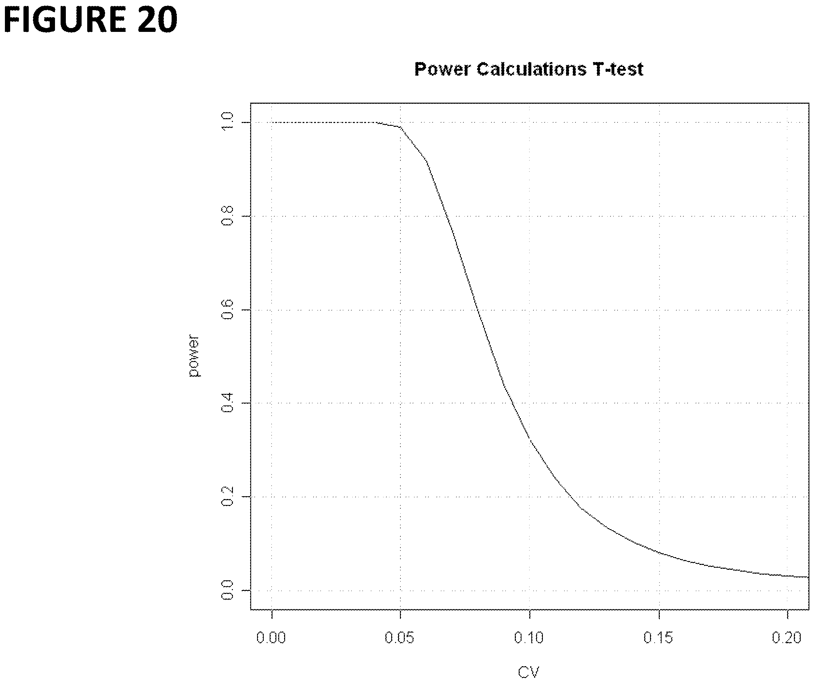

FIGURE 20: Shows a power calculation t-test for a simulated trisomy 21 diagnosis using the methods of the technology. The Figure shows the relationship between the coefficient of variation (CV) on the x-axis and the power to discriminate the assay populations using a simple t-test (y-axis). The data indicates that in 99% of all cases, one can discriminate the two population (euploid vs. aneuploid) on a significance level of 0.001 provided a CV of 5% or less.

### DEFINITIONS

The term "pregnancy-associated disorder," as used in this application, refers to any condition or disease that may affect a pregnant woman, the fetus, or both the woman and the fetus. Such a condition or disease may manifest its symptoms during a limited time period, e.g., during pregnancy or delivery, or may last the entire life span of the fetus following its birth. Some examples of a pregnancy-associated disorder include ectopic pregnancy, preeclampsia, preterm labor, RhD incompatibility, fetal chromosomal abnormalities such as trisomy 21, and genetically inherited fetal disorders such as cystic fibrosis, beta-thalassemia or other monogenic disorders. The ability to enrich fetal nucleic from a maternal sample may prove particularly useful for the noninvasive prenatal diagnosis of autosomal recessive diseases such as the case when a mother and father share an identical disease causing mutation, an occurrence previously perceived as a challenge for maternal plasma-based non-trisomy prenatal diagnosis.

The term "chromosomal abnormality" or "aneuploidy" as used herein refers to a deviation between the structure of the subject chromosome and a normal homologous chromosome. The term "normal" refers to the predominate karyotype or banding pattern found in healthy individuals of a particular species, for example, a euploid genome (in humans, 46XX or 46XY). A chromosomal abnormality can be numerical or structural, and includes but is not limited to aneuploidy, polyploidy, inversion, a trisomy, a monosomy, duplication, deletion, deletion of a part of a chromosome, addition, addition of a part of chromosome, insertion, a fragment of a chromosome, a region of a chromosome, chromosomal rearrangement, and translocation. Chromosomal abnormality may also refer to a state of chromosomal abnormality where a portion of one or more chromosomes is not an exact multiple of the usual haploid number due to, for example, chromosome translocation. Chromosomal translocation (e.g. translocation between chromosome 21 and 14 where some of the 14th chromosome is replaced by extra 21st chromosome) may cause partial trisomy 21. A chromosomal abnormality can be correlated with presence of a pathological condition or with a predisposition to develop a pathological condition. A chromosomal abnormality may be detected by quantitative analysis of nucleic acid.

The terms "nucleic acid" and "nucleic acid molecule" may be used interchangeably throughout the disclosure. The terms refer to nucleic acids of any composition from, such as DNA (e.g., complementary DNA (cDNA), genomic DNA (gDNA) and the like), RNA (e.g., message RNA (mRNA), short inhibitory RNA (siRNA), ribosomal RNA (rRNA), tRNA, microRNA, RNA highly expressed by the fetus or placenta, and the like), and/or DNA or RNA analogs (e.g., containing base analogs, sugar analogs and/or a non-native backbone and the like), RNA/DNA hybrids and polyamide nucleic acids (PNAs), all of which can be in single-or double-stranded form, and unless otherwise limited, can encompass known analogs of natural nucleotides that can function in a similar manner as naturally occurring nucleotides. For example, the nucleic acids provided in SEQ ID Nos: 1-89 (see Table 4) can be in any form useful for conducting processes herein (e.g., linear, circular, supercoiled, single-stranded, double-stranded and the like) or may include variations (e.g., insertions, deletions or substitutions) that do not alter their utility as part of the present technology. A nucleic acid may be, or may be from, a plasmid, phage, autonomously replicating sequence (ARS), centromere, artificial chromosome, chromosome, or other nucleic acid able to replicate or be replicated in vitro or in a host cell, a cell, a cell nucleus or cytoplasm of a cell in certain embodiments. A template nucleic acid in some embodiments can be from a single chromosome (e.g., a nucleic acid sample may be from one chromosome of a sample obtained from a diploid organism). Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)). The term nucleic acid is used interchangeably with locus, gene, cDNA, and mRNA encoded by a gene. The term also may include, as equivalents, derivatives, variants and analogs of RNA or DNA synthesized from nucleotide analogs, single-stranded ("sense" or "antisense", "plus" strand or "minus" strand, "forward" reading frame or "reverse" reading frame) and double-stranded polynucleotides. Deoxyribonucleotides include deoxyadenosine, deoxycytidine, deoxyguanosine and deoxythymidine. For RNA, the base cytosine is replaced with uracil. A template nucleic acid may be prepared using a nucleic acid obtained from a subject as a template.

A "nucleic acid comprising one or more CpG sites" or a "CpG-containing genomic sequence" as used herein refers to a segment of DNA sequence at a defined location in the genome of an individual such as a human fetus or a pregnant woman. Typically, a "CpG-containing genomic sequence" is at least 15 nucleotides in length and contains at least one cytosine. Preferably, it can be at least 30, 50, 80, 100, 150, 200, 250, or 300 nucleotides in length and contains at least 2, 5, 10, 15, 20, 25, or 30 cytosines. For anyone "CpG-containing genomic sequence" at a given location, e.g., within a region centering around a given genetic locus (see Table 1), nucleotide sequence variations may exist from individual to individual and from allele to allele even for the same individual. Typically, such a region centering around a defined genetic locus (e.g., a CpG island) contains the locus as well as upstream and/or downstream sequences. Each of the upstream or downstream sequence (counting from the 5' or 3' boundary of the genetic locus, respectively) can be as long as 10 kb, in other cases may be as long as 5 kb, 2 kb, 1 kb, 500 bp, 200 bp, or 100 bp. Furthermore, a "CpG-containing genomic sequence" may encompass a nucleotide sequence transcribed or not transcribed for protein production, and the nucleotide sequence can be an inter-gene sequence, intra-gene sequence, protein-coding sequence, a non protein-coding sequence (such as a transcription promoter), or a combination thereof.

As used herein, a "methylated nucleotide" or a "methylated nucleotide base" refers to the presence of a methyl moiety on a nucleotide base, where the methyl moiety is not present in a recognized typical nucleotide base. For example, cytosine does not contain a methyl moiety on its pyrimidine ring, but 5-methylcytosine contains a methyl moiety at position 5 of its pyrimidine ring. Therefore, cytosine is not a methylated nucleotide and 5-methylcytosine is a methylated nucleotide. In another example, thymine contains a methyl moiety at position 5 of its pyrimidine ring, however, for purposes herein, thymine is not considered a methylated nucleotide when present in DNA since thymine is a typical nucleotide base of DNA. Typical nucleoside bases for DNA are thymine, adenine, cytosine and guanine. Typical bases for RNA are uracil, adenine, cytosine and guanine. Correspondingly a "methylation site" is the location in the target gene nucleic acid region where methylation has, or has the possibility of occurring. For example a location containing CpG is a methylation site wherein the cytosine may or may not be methylated.

As used herein, a "CpG site" or "methylation site" is a nucleotide within a nucleic acid that is susceptible to methylation either by natural occurring events in vivo or by an event instituted to chemically methylate the nucleotide in vitro.

As used herein, a "methylated nucleic acid molecule" refers to a nucleic acid molecule that contains one or more methylated nucleotides that is/are methylated.

A "CpG island" as used herein describes a segment of DNA sequence that comprises a functionally or structurally deviated CpG density. For example, Yamada et al. (Genome Research 14:247-266, 2004) have described a set of standards for determining a CpG island: it must be at least 400 nucleotides in length, has a greater than 50% GC content, and an OCF/ECF ratio greater than 0.6. Others (Takai et al., Proc. Natl. Acad. Sci. U.S.A. 99:3740-3745, 2002) have defined a CpG island less stringently as a sequence at least 200 nucleotides in length, having a greater than 50% GC content, and an OCF/ECF ratio greater than 0.6.

The term "epigenetic state" or "epigenetic status" as used herein refers to any structural feature at a molecular level of a nucleic acid (e.g., DNA or RNA) other than the primary nucleotide sequence. For instance, the epigenetic state of a genomic DNA may include its secondary or tertiary structure determined or influenced by, e.g., its methylation pattern or its association with cellular proteins.

The term "methylation profile" "methylation state" or "methylation status," as used herein to describe the state of methylation of a genomic sequence, refers to the characteristics of a DNA segment at a particular genomic locus relevant to methylation. Such characteristics include, but are not limited to, whether any of the cytosine (C) residues within this DNA sequence are methylated, location of methylated C residue(s), percentage of methylated C at any particular stretch of residues, and allelic differences in methylation due to, e.g., difference in the origin of the alleles. The term "methylation" profile" or "methylation status" also refers to the relative or absolute concentration of methylated C or unmethylated C at any particular stretch of residues in a biological sample. For example, if the cytosine (C) residue(s) within a DNA sequence are methylated it may be referred to as "hypermethylated"; whereas if the cytosine (C) residue(s) within a DNA sequence are not methylated it may be referred to as "hypomethylated". Likewise, if the cytosine (C) residue(s) within a DNA sequence (e.g., fetal nucleic acid) are methylated as compared to another sequence from a different region or from a different individual (e.g., relative to maternal nucleic acid), that sequence is considered hypermethylated compared to the other sequence. Alternatively, if the cytosine (C) residue(s) within a DNA sequence are not methylated as compared to another sequence from a different region or from a different individual (e.g., the mother), that sequence is considered hypomethylated compared to the other sequence. These sequences are said to be "differentially methylated", and more specifically, when the methylation status differs between mother and fetus, the sequences are considered "differentially methylated maternal and fetal nucleic acid".

The term "agent that binds to methylated nucleotides" as used herein refers to a substance that is capable of binding to methylated nucleic acid. The agent may be naturally-occurring or synthetic, and may be modified or unmodified. In one embodiment, the agent allows for the separation of different nucleic acid species according to their respective methylation states. An example of an agent that binds to methylated nucleotides is described in PCT Patent Application No. PCT/EP2005/012707, which published as WO06056480A2 and is hereby incorporated by reference. The described agent is a bifunctional polypeptide comprising the DNA-binding domain of a protein belonging to the family of Methyl-CpG binding proteins (MBDs) and an Fc portion of an antibody (see Figure 1). The recombinant methyl-CpG-binding, antibody-like protein can preferably bind CpG methylated DNA in an antibody-like manner. That means, the methyl-CpG-binding, antibody-like protein has a high affinity and high avidity to its "antigen", which is preferably DNA that is methylated at CpG dinucleotides. The agent may also be a multivalent MBD (see Figure 2).

The term "polymorphism" as used herein refers to a sequence variation within different alleles of the same genomic sequence. A sequence that contains a polymorphism is considered "polymorphic sequence". Detection of one or more polymorphisms allows differentiation of different alleles of a single genomic sequence or between two or more individuals. As used herein, the term "polymorphic marker" or "polymorphic sequence" refers to segments of genomic DNA that exhibit heritable variation in a DNA sequence between individuals. Such markers include, but are not limited to, single nucleotide polymorphisms (SNPs), restriction fragment length polymorphisms (RFLPs), short tandem repeats, such as di-, tri- or tetra-nucleotide repeats (STRs), and the like. Polymorphic markers according to the present technology can be used to specifically differentiate between a maternal and paternal allele in the enriched fetal nucleic acid sample.

The terms "single nucleotide polymorphism" or "SNP" as used herein refer to the polynucleotide sequence variation present at a single nucleotide residue within different alleles of the same genomic sequence. This variation may occur within the coding region or non-coding region (i.e., in the promoter or intronic region) of a genomic sequence, if the genomic sequence is transcribed during protein production. Detection of one or more SNP allows differentiation of different alleles of a single genomic sequence or between two or more individuals.

The term "allele" as used herein is one of several alternate forms of a gene or non-coding regions of DNA that occupy the same position on a chromosome. The term allele can be used to describe DNA from any organism including but not limited to bacteria, viruses, fungi, protozoa, molds, yeasts, plants, humans, non-humans, animals, and archeabacteria.

The terms "ratio of the alleles" or "allelic ratio" as used herein refer to the ratio of the population of one allele and the population of the other allele in a sample. In some trisomic cases, it is possible that a fetus may be tri-allelic for a particular locus. In such cases, the term "ratio of the alleles" refers to the ratio of the population of any one allele against one of the other alleles, or any one allele against the other two alleles.

The term "non-polymorphism-based quantitative method" as used herein refers to a method for determining the amount of an analyte (e.g., total nucleic acid, Y-chromosome nucleic acid, or fetal nucleic acid) that does not require the use of a polymorphic marker or sequence. Although a polymorphism may be present in the sequence, said polymorphism is not required to quantify the sequence. Examples of non-polymorphism-based quantitative methods include, but are not limited to, RT-PCR, digital PCR, array-based methods, sequencing methods, nanopore-based methods, nucleic acid-bound bead-based counting methods and competitor-based methods wherein one or more competitors are introduced at a known concentration(s) to determine the amount of one or more analytes. In some embodiments, some of the above exemplary methods (for example, sequencing) may need to be actively modified or designed such that one or more polymorphisms are not interrogated.

The terms "absolute amount" or "copy number" as used herein refers to the amount or quantity of an analyte (e.g., total nucleic acid or fetal nucleic acid). The present technology provides compositions and processes for determining the absolute amount of fetal nucleic acid in a mixed maternal sample. Absolute amount or copy number represents the number of molecules available for detection, and may be expressed as the genomic equivalents per unit. The term "concentration" refers to the amount or proportion of a substance in a mixture or solution (e.g., the amount of fetal nucleic acid in a maternal sample that comprises a mixture of maternal and fetal nucleic acid). The concentration may be expressed as a percentage, which is used to express how large/small one quantity is, relative to another quantity as a fraction of 100. Platforms for determining the quantity or amount of an analyte (e.g., target nucleic acid) include, but are not limited to, mass spectrometery, digital PCR, sequencing by synthesis platforms (e.g., pyrosequencing), fluorescence spectroscopy and flow cytometry.

The term "sample" as used herein refers to a specimen containing nucleic acid. Examples of samples include, but are not limited to, tissue, bodily fluid (for example, blood, serum, plasma, saliva, urine, tears, peritoneal fluid, ascitic fluid, vaginal secretion, breast fluid, breast milk, lymph fluid, cerebrospinal fluid or mucosa secretion), umbilical cord blood, chorionic villi, amniotic fluid, an embryo, a two-celled embryo, a four-celled embryo, an eight-celled embryo, a 16-celled embryo, a 32-celled embryo, a 64-celled embryo, a 128-celled embryo, a 256-celled embryo, a 512-celled embryo, a 1024-celled embryo, embryonic tissues, lymph fluid, cerebrospinal fluid, mucosa secretion, or other body exudate, fecal matter, an individual cell or extract of the such sources that contain the nucleic acid of the same, and subcellular structures such as mitochondria, using protocols well established within the art.

Fetal DNA can be obtained from sources including but not limited to maternal blood, maternal serum, maternal plasma, fetal cells, umbilical cord blood, chorionic villi, amniotic fluid, urine, saliva, lung lavage, cells or tissues.

The term "blood" as used herein refers to a blood sample or preparation from a pregnant woman or a woman being tested for possible pregnancy. The term encompasses whole blood or any fractions of blood, such as serum and plasma as conventionally defined.

The term "bisulfite" as used herein encompasses any suitable types of bisulfites, such as sodium bisulfite, that are capable of chemically converting a cytosine (C) to a uracil (U) without chemically modifying a methylated cytosine and therefore can be used to differentially modify a DNA sequence based on the methylation status of the DNA.

As used herein, a reagent that "differentially modifies" methylated or non-methylated DNA encompasses any reagent that modifies methylated and/or unmethylated DNA in a process through which distinguishable products result from methylated and non-methylated DNA, thereby allowing the identification of the DNA methylation status. Such processes may include, but are not limited to, chemical reactions (such as a C.fwdarw.U conversion by bisulfite) and enzymatic treatment (such as cleavage by a methylation-dependent endonuclease). Thus, an enzyme that preferentially cleaves or digests methylated DNA is one capable of cleaving or digesting a DNA molecule at a much higher efficiency when the DNA is methylated, whereas an enzyme that preferentially cleaves or digests unmethylated DNA exhibits a significantly higher efficiency when the DNA is not methylated.

The terms "non-bisulfite-based method" and "non-bisulfite-based quantitative method" as used herein refer to any method for quantifying methylated or non-methylated nucleic acid that does not require the use of bisulfite. The terms also refer to methods for preparing a nucleic acid to be quantified that do not require bisulfite treatment. Examples of non-bisulfite-based methods include, but are not limited to, methods for digesting nucleic acid using one or more methylation sensitive enzymes and methods for separating nucleic acid using agents that bind nucleic acid based on methylation status.

The terms "methyl-sensitive enzymes" and "methylation sensitive restriction enzymes" are DNA restriction endonucleases that are dependent on the methylation state of their DNA recognition site for activity. For example, there are methyl-sensitive enzymes that cleave or digest at their DNA recognition sequence only if it is not methylated. Thus, an unmethylated DNA sample will be cut into smaller fragments than a methylated DNA sample. Similarly, a hypermethylated DNA sample will not be cleaved. In contrast, there are methyl-sensitive enzymes that cleave at their DNA recognition sequence only if it is methylated. As used herein, the terms "cleave", "cut" and "digest" are used interchangeably.

The term "target nucleic acid" as used herein refers to a nucleic acid examined using the methods disclosed herein to determine if the nucleic acid is part of a pregnancy-related disorder or chromosomal abnormality. For example, a target nucleic acid from chromosome 21 could be examined using the methods of the technology to detect Down's Syndrome.

The term "control nucleic acid" as used herein refers to a nucleic acid used as a reference nucleic acid according to the methods disclosed herein to determine if the nucleic acid is part of a chromosomal abnormality. For example, a control nucleic acid from a chromosome other than chromosome 21 (herein referred to as a "reference chromosome") could be as a reference sequence to detect Down's Syndrome. In some embodiments, the control sequence has a known or predetermined quantity.

The term "sequence-specific" or "locus-specific method" as used herein refers to a method that interrogates (for example, quantifies) nucleic acid at a specific location (or locus) in the genome based on the sequence composition. Sequence-specific or locus-specific methods allow for the quantification of specific regions or chromosomes.

The term "gene" means the segment of DNA involved in producing a polypeptide chain; it includes regions preceding and following the coding region (leader and trailer) involved in the transcription/translation of the gene product and the regulation of the transcription/translation, as well as intervening sequences (introns) between individual coding segments (exons).

In this application, the terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins (i.e., antigens), wherein the amino acid residues are linked by covalent peptide bonds.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, .gamma.-carboxyglutamate, and O-phosphoserine.

Amino acids may be referred to herein by either the commonly known three letter symbols or by the one-letter symbols recommended by the IUPAC-IUB Biochemical Nomenclature Commission. Nucleotides, likewise, may be referred to by their commonly accepted single-letter codes.

"Primers" as used herein refer to oligonucleotides that can be used in an amplification method, such as a polymerase chain reaction (PCR), to amplify a nucleotide sequence based on the polynucleotide sequence corresponding to a particular genomic sequence, e.g., one located within the CpG island CGI137, PDE9A, or CGI009 on chromosome 21, in various methylation status. At least one of the PCR primers for amplification of a polynucleotide sequence is sequence-specific for the sequence.

The term "template" refers to any nucleic acid molecule that can be used for amplification in the technology. RNA or DNA that is not naturally double stranded can be made into double stranded DNA so as to be used as template DNA. Any double stranded DNA or preparation containing multiple, different double stranded DNA molecules can be used as template DNA to amplify a locus or loci of interest contained in the template DNA.

The term "amplification reaction" as used herein refers to a process for copying nucleic acid one or more times. In embodiments, the method of amplification includes but is not limited to polymerase chain reaction, self-sustained sequence reaction, ligase chain reaction, rapid amplification of cDNA ends, polymerase chain reaction and ligase chain reaction, Q-beta phage amplification, strand displacement amplification, or splice overlap extension polymerase chain reaction. In some embodiments, a single molecule of nucleic acid is amplified, for example, by digital PCR.

The term "sensitivity" as used herein refers to the number of true positives divided by the number of true positives plus the number of false negatives, where sensitivity (sens) may be within the range of 0 ≤ sens ≤ 1. Ideally, method embodiments herein have the number of false negatives equaling zero or close to equaling zero, so that no subject is wrongly identified as not having at least one chromosome abnormality or other genetic disorder when they indeed have at least one chromosome abnormality or other genetic disorder. Conversely, an assessment often is made of the ability of a prediction algorithm to classify negatives correctly, a complementary measurement to sensitivity. The term "specificity" as used herein refers to the number of true negatives divided by the number of true negatives plus the number of false positives, where sensitivity (spec) may be within the range of 0 ≤ spec ≤ 1. Ideally, methods embodiments herein have the number of false positives equaling zero or close to equaling zero, so that no subject wrongly identified as having at least one chromosome abnormality other genetic disorder when they do not have the chromosome abnormality other genetic disorder being assessed. Hence, a method that has sensitivity and specificity equaling one, or 100%, sometimes is selected.

One or more prediction algorithms may be used to determine significance or give meaning to the detection data collected under variable conditions that may be weighed independently of or dependently on each other. The term "variable" as used herein refers to a factor, quantity, or function of an algorithm that has a value or set of values. For example, a variable may be the design of a set of amplified nucleic acid species, the number of sets of amplified nucleic acid species, percent fetal genetic contribution tested, percent maternal genetic contribution tested, type of chromosome abnormality assayed, type of genetic disorder assayed, type of sex-linked abnormalities assayed, the age of the mother and the like. The term "independent" as used herein refers to not being influenced or not being controlled by another. The term "dependent" as used herein refers to being influenced or controlled by another. For example, a particular chromosome and a trisomy event occurring for that particular chromosome that results in a viable being are variables that are dependent upon each other.

One of skill in the art may use any type of method or prediction algorithm to give significance to the data of the present technology within an acceptable sensitivity and/or specificity. For example, prediction algorithms such as Chi-squared test, z-test, t-test, ANOVA (analysis of variance), regression analysis, neural nets, fuzzy logic, Hidden Markov Models, multiple model state estimation, and the like may be used. One or more methods or prediction algorithms may be determined to give significance to the data having different independent and/or dependent variables of the present technology. And one or more methods or prediction algorithms may be determined not to give significance to the data having different independent and/or dependent variables of the present technology. One may design or change parameters of the different variables of methods described herein based on results of one or more prediction algorithms (e.g., number of sets analyzed, types of nucleotide species in each set). For example, applying the Chi-squared test to detection data may suggest that specific ranges of maternal age are correlated to a higher likelihood of having an offspring with a specific chromosome abnormality, hence the variable of maternal age may be weighed differently verses being weighed the same as other variables.

In certain embodiments, several algorithms may be chosen to be tested. These algorithms can be trained with raw data. For each new raw data sample, the trained algorithms will assign a classification to that sample (i.e. trisomy or normal). Based on the classifications of the new raw data samples, the trained algorithms' performance may be assessed based on sensitivity and specificity. Finally, an algorithm with the highest sensitivity and/or specificity or combination thereof may be identified.

### DETAILED DESCRIPTION

### Introduction

The presence of fetal nucleic acid in maternal plasma was first reported in 1997 and offers the possibility for non-invasive prenatal diagnosis simply through the analysis of a maternal blood sample (Lo et al., Lancet 350:485-487, 1997). To date, numerous potential clinical applications have been developed. In particular, quantitative abnormalities of fetal nucleic acid, for example DNA, concentrations in maternal plasma have been found to be associated with a number of pregnancy-associated disorders, including preeclampsia, preterm labor, antepartum hemorrhage, invasive placentation, fetal Down syndrome, and other fetal chromosomal aneuploidies. Hence, fetal nucleic acid analysis in maternal plasma represents a powerful mechanism for the monitoring of fetomaternal well-being.

However, fetal DNA co-exists with background maternal DNA in maternal plasma. Hence, most reported applications have relied on the detection of Y-chromosome sequences as these are most conveniently distinguishable from maternal DNA. Such an approach limits the applicability of the existing assays to only 50% of all pregnancies, namely those with male fetuses. Thus, there is much need for the development of sex-independent compositions and methods for enriching and analyzing fetal nucleic acid from a maternal sample. Also, methods that rely on polymorphic markers to quantify fetal nucleic acid may be susceptible to varying heterozygosity rates across different ethnicities thereby limiting their applicability (e.g., by increasing the number of markers that are needed).

It was previously demonstrated that fetal and maternal DNA can be distinguished by their differences in methylation status (U.S. Patent No. 6,927,028, which is hereby incorporated by reference). Methylation is an epigenetic phenomenon, which refers to processes that alter a phenotype without involving changes in the DNA sequence. By exploiting the difference in the DNA methylation status between mother and fetus, one can successfully detect and analyze fetal nucleic acid in a background of maternal nucleic acid.

The present inventors provides novel genomic polynucleotides that are differentially methylated between the fetal DNA from the fetus (e.g., from the placenta) and the maternal DNA from the mother, for example from peripheral blood cells. This discovery thus provides a new approach for distinguishing fetal and maternal genomic DNA and new methods for accurately quantifying fetal nucleic which may be used for non-invasive prenatal diagnosis.

### Methodology

Practicing the technology utilizes routine techniques in the field of molecular biology. Basic texts disclosing the general methods of use in the technology include Sambrook and Russell, Molecular Cloning, A Laboratory Manual (3rd ed. 2001); Kriegler, Gene Transfer and Expression: A Laboratory Manual (1990); and Current Protocols in Molecular Biology (Ausubel et al., eds., 1994)).

For nucleic acids, sizes are given in either kilobases (kb) or base pairs (bp). These are estimates derived from agarose or acrylamide gel electrophoresis, from sequenced nucleic acids, or from published DNA sequences. For proteins, sizes are given in kilodaltons (kDa) or amino acid residue numbers. Protein sizes are estimated from gel electrophoresis, from sequenced proteins, from derived amino acid sequences, or from published protein sequences.

Oligonucleotides that are not commercially available can be chemically synthesized, e.g., according to the solid phase phosphoramidite triester method first described by Beaucage & Caruthers, Tetrahedron Lett. 22: 1859-1862 (1981), using an automated synthesizer, as described in Van Devanter et. al., Nucleic Acids Res. 12: 6159-6168 (1984). Purification of oligonucleotides is performed using any art-recognized strategy, e.g., native acrylamide gel electrophoresis or anion-exchange high performance liquid chromatography (HPLC) as described in Pearson & Reanier, J. Chrom. 255: 137-149 (1983).

### Acquisition of Blood Samples and Extraction of DNA

The present technology relates to separating, enriching and analyzing fetal DNA found in maternal blood as a non-invasive means to detect the presence and/or to monitor the progress of a pregnancy-associated condition or disorder. Thus, the first steps of practicing the technology are to obtain a blood sample from a pregnant woman and extract DNA from the sample.

### A. Acquisition of Blood Samples

A blood sample is obtained from a pregnant woman at a gestational age suitable for testing using a method of the present technology. The suitable gestational age may vary depending on the disorder tested, as discussed below. Collection of blood from a woman is performed in accordance with the standard protocol hospitals or clinics generally follow. An appropriate amount of peripheral blood, e.g., typically between 5-50 ml, is collected and may be stored according to standard procedure prior to further preparation. Blood samples may be collected, stored or transported in a manner known to the person of ordinary skill in the art to minimize degradation or the quality of nucleic acid present in the sample.

### B. Preparation of Blood Samples

The analysis of fetal DNA found in maternal blood according to the present technology may be performed using, e.g., the whole blood, serum, or plasma. The methods for preparing serum or plasma from maternal blood are well known among those of skill in the art. For example, a pregnant woman's blood can be placed in a tube containing EDTA or a specialized commercial product such as Vacutainer SST (Becton Dickinson, Franklin Lakes, N.J.) to prevent blood clotting, and plasma can then be obtained from whole blood through centrifugation. On the other hand, serum may be obtained with or without centrifugation-following blood clotting. If centrifugation is used then it is typically, though not exclusively, conducted at an appropriate speed, e.g., 1,500-3,000 times g. Plasma or serum may be subjected to additional centrifugation steps before being transferred to a fresh tube for DNA extraction.

In addition to the acellular portion of the whole blood, DNA may also be recovered from the cellular fraction, enriched in the buffy coat portion, which can be obtained following centrifugation of a whole blood sample from the woman and removal of the plasma.

### C. Extraction of DNA

There are numerous known methods for extracting DNA from a biological sample including blood. The general methods of DNA preparation (e.g., described by Sambrook and Russell, Molecular Cloning: A Laboratory Manual 3d ed., 2001) can be followed; various commercially available reagents or kits, such as Qiagen's QIAamp Circulating Nucleic Acid Kit, QiaAmp DNA Mini Kit or QiaAmp DNA Blood Mini Kit (Qiagen, Hilden, Germany), GenomicPrep™ Blood DNA Isolation Kit (Promega, Madison, Wis.), and GFX™ Genomic Blood DNA Purification Kit (Amersham, Piscataway, N.J.), may also be used to obtain DNA from a blood sample from a pregnant woman. Combinations of more than one of these methods may also be used.

In some embodiments, the sample may first be enriched or relatively enriched for fetal nucleic acid by one or more methods. For example, the discrimination of fetal and maternal DNA can be performed using the compositions and processes of the present technology alone or in combination with other discriminating factors. Examples of these factors include, but are not limited to, single nucleotide differences between chromosome X and Y, chromosome Y-specific sequences, polymorphisms located elsewhere in the genome, size differences between fetal and maternal DNA and differences in methylation pattern between maternal and fetal tissues.

Other methods for enriching a sample for a particular species of nucleic acid are described in PCT Patent Application Number PCT/US07/69991, filed May 30, 2007, PCT Patent Application Number PCT/US2007/071232, filed June 15, 2007, US Provisional Application Numbers 60/968,876 and 60/968,878 (assigned to the Applicant), (PCT Patent Application Number PCT/EP05/012707, filed November 28, 2005) which are all hereby incorporated by reference. In certain embodiments, maternal nucleic acid is selectively removed (either partially, substantially, almost completely or completely) from the sample.

### Methylation Specific Separation of Nucleic Acid

The methods provided herein offer an alternative approach for the enrichment of fetal DNA based on the methylation-specific separation of differentially methylated DNA. It has recently been discovered that many genes involved in developmental regulation are controlled through epigenetics in embryonic stem cells.

Consequently, multiple genes can be expected to show differential DNA methylation between nucleic acid of fetal origin and maternal origin. Once these regions are identified, a technique to capture methylated DNA can be used to specifically enrich fetal DNA. For identification of differentially methylated regions, a novel approach was used to capture methylated DNA. This approach uses a protein, in which the methyl binding domain of MBD2 is fused to the Fc fragment of an antibody (MBD-FC) (Gebhard C, Schwarzfischer L, Pham TH, Schilling E, Klug M, Andreesen R, Rehli M (2006) Genomewide profiling of CpG methylation identifies novel targets of aberrant hypermethylation in myeloid leukemia. Cancer Res 66:6118-6128). This fusion protein has several advantages over conventional methylation specific antibodies. The MBD-FC has a higher affinity to methylated DNA and it binds double stranded DNA. Most importantly the two proteins differ in the way they bind DNA. Methylation specific antibodies bind DNA stochastically, which means that only a binary answer can be obtained. The methyl binding domain of MBD-FC on the other hand binds DNA molecules regardless of their methylation status. The strength of this protein - DNA interaction is defined by the level of DNA methylation. After binding genomic DNA, eluate solutions of increasing salt concentrations can be used to fractionate non-methylated and methylated DNA allowing for a more controlled separation (Gebhard C, Schwarzfischer L, Pham TH, Andreesen R, Mackensen A, Rehli M (2006) Rapid and sensitive detection of CpG-methylation using methyl-binding (MB)-PCR. Nucleic Acids Res 34:e82). Consequently this method, called Methyl-CpG immunoprecipitation (MCIP), cannot only enrich, but also fractionate genomic DNA according to methylation level, which is particularly helpful when the unmethylated DNA fraction should be investigated as well.

### Methylation Sensitive Restriction Enzyme Digestion

The technology also provides compositions and processes for determining the amount of fetal nucleic acid from a maternal sample. The technology allows for the enrichment of fetal nucleic acid regions in a maternal sample by selectively digesting nucleic acid from said maternal sample with an enzyme that selectively and completely or substantially digests the maternal nucleic acid to enrich the sample for at least one fetal nucleic acid region. Preferably, the digestion efficiency is greater than about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. Following enrichment, the amount of fetal nucleic acid can be determined by quantitative methods that do not require polymorphic sequences or bisulfite treatment, thereby, offering a solution that works equally well for female fetuses and across different ethnicities and preserves the low copy number fetal nucleic acid present in the sample.

For example, there are methyl-sensitive enzymes that preferentially or substantially cleave or digest at their DNA recognition sequence if it is non-methylated. Thus, an unmethylated DNA sample will be cut into smaller fragments than a methylated DNA sample. Similarly, a hypermethylated DNA sample will not be cleaved. In contrast, there are methyl-sensitive enzymes that cleave at their DNA recognition sequence only if it is methylated.

Methyl-sensitive enzymes that digest unmethylated DNA suitable for use in methods of the technology include, but are not limited to, Hpall, Hhal, Maell, BstUI and Acil. An enzyme that can be used is Hpall that cuts only the unmethylated sequence CCGG. Another enzyme that can be used is Hhal that cuts only the unmethylated sequence GCGC. Both enzymes are available from New England BioLabs®, Inc. Combinations of two or more methyl-sensitive enzymes that digest only unmethylated DNA can also be used. Suitable enzymes that digest only methylated DNA include, but are not limited to, Dpnl, which cuts at a recognition sequence GATC, and McrBC, which belongs to the family of AAA.sup.+ proteins and cuts DNA containing modified cytosines and cuts at recognition site 5' ... Pu.sup.mC(N.sub.40-3000) Pu.sup.mC ... 3' (New England BioLabs, Inc., Beverly, Mass.).

Cleavage methods and procedures for selected restriction enzymes for cutting DNA at specific sites are well known to the skilled artisan. For example, many suppliers of restriction enzymes provide information on conditions and types of DNA sequences cut by specific restriction enzymes, including New England BioLabs, Pro-Mega Biochems, Boehringer-Mannheim, and the like. Sambrook et al. (See Sambrook et al., Molecular Biology: A laboratory Approach, Cold Spring Harbor, N.Y. 1989) provide a general description of methods for using restriction enzymes and other enzymes. In methods of the present technology enzymes often are used under conditions that will enable cleavage of the maternal DNA with about 95%-100% efficiency, preferably with about 98%-100% efficiency.

### Other Methods for Methylation Analysis

Various methylation analysis procedures are known in the art, and can be used in conjunction with the present technology. These assays allow for determination of the methylation state of one or a plurality of CpG islands within a DNA sequence. In addition, the methods maybe used to quantify methylated nucleic acid. Such assays involve, among other techniques, DNA sequencing of bisulfite-treated DNA, PCR (for sequence-specific amplification), Southern blot analysis, and use of methylation-sensitive restriction enzymes.

Genomic sequencing is a technique that has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA may be used, e.g., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

COBRA analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the interested CpG islands, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples. Typical reagents (e.g., as might be found in a typical COBRA-based kit) for COBRA analysis may include, but are not limited to: PCR primers for specific gene (or methylation-altered DNA sequence or CpG island); restriction enzyme and appropriate buffer; gene-hybridization oligo; control hybridization oligo; kinase labeling kit for oligo probe; and radioactive nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

The MethyLight™ assay is a high-throughput quantitative methylation assay that utilizes fluorescence-based real-time PCR (TaqMan.RTM.) technology that requires no further manipulations after the PCR step (Eads et al., Cancer Res. 59:2302-2306, 1999). Briefly, the MethyLight.TM. process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight process can by used with a "TaqMan" probe in the amplification process. For example, double-stranded genomic DNA is treated with sodium bisulfite and subjected to one of two sets of PCR reactions using TaqMan.RTM. probes; e.g., with either biased primers and TaqMan.RTM. probe, or unbiased primers and TaqMan.RTM. probe. The TaqMan.RTM. probe is dual-labeled with fluorescent "reporter" and "quencher" molecules, and is designed to be specific for a relatively high GC content region so that it melts out at about 10.degree. C. higher temperature in the PCR cycle than the forward or reverse primers. This allows the TaqMan.RTM. probe to remain fully hybridized during the PCR annealing/extension step. As the Taq polymerase enzymatically synthesizes a new strand during PCR, it will eventually reach the annealed TaqMan.RTM. probe. The Taq polymerase 5' to 3' endonuclease activity will then displace the TaqMan.RTM. probe by digesting it to release the fluorescent reporter molecule for quantitative detection of its now unquenched signal using a real-time fluorescent detection system.

Typical reagents (e.g., as might be found in a typical MethyLight.TM.-based kit) for MethyLight.TM. analysis may include, but are not limited to: PCR primers for specific gene (or methylation-altered DNA sequence or CpG island); TaqMan.RTM. probes; optimized PCR buffers and deoxynucleotides; and Taq polymerase.

The Ms-SNuPE technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997).

Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest.

Small amounts of DNA can be analyzed (e.g., microdissected pathology sections), and it avoids utilization of restriction enzymes for determining the methylation status at CpG sites.

Typical reagents (e.g., as might be found in a typical Ms-SNuPE-based kit) for Ms-SNuPE analysis may include, but are not limited to: PCR primers for specific gene (or methylation-altered DNA sequence or CpG island); optimized PCR buffers and deoxynucleotides; gel extraction kit; positive control primers; Ms-SNuPE primers for specific gene; reaction buffer (for the Ms-SNuPE reaction); and radioactive nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

MSP (methylation-specific PCR) allows for assessing the methylation status of virtually any group of CpG sites within a CpG island, independent of the use of methylation-sensitive restriction enzymes (Herman et al. Proc. Nat. Acad. Sci. USA 93:9821-9826, 1996; U.S. Pat. No. 5,786,146). Briefly, DNA is modified by sodium bisulfite converting unmethylated, but not methylated cytosines to uracil, and subsequently amplified with primers specific for methylated versus umnethylated DNA. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific gene (or methylation-altered DNA sequence or CpG island), optimized PCR buffers and deoxynucleotides, and specific probes.

The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (e.g., as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

Another method for analyzing methylation sites is a primer extension assay, including an optimized PCR amplification reaction that produces amplified targets for subsequent primer extension genotyping analysis using mass spectrometry. The assay can also be done in multiplex. This method (particularly as it relates to genotyping single nucleotide polymorphisms) is described in detail in PCT publication WO05012578A1 and US publication US20050079521A1. For methylation analysis, the assay can be adopted to detect bisulfite introduced methylation dependent C to T sequence changes. These methods are particularly useful for performing multiplexed amplification reactions and multiplexed primer extension reactions (e.g., multiplexed homogeneous primer mass extension (hME) assays) in a single well to further increase the throughput and reduce the cost per reaction for primer extension reactions.

Four additional methods for DNA methylation analysis include restriction landmark genomic scanning (RLGS, Costello et al., 2000), methylation-sensitive-representational difference analysis (MS-RDA), methylation-specific AP-PCR (MS-AP-PCR) and methyl-CpG binding domain column/segregation of partly melted molecules (MBD/SPM).

Additional methylation analysis methods that may be used in conjunction with the present technology are described in the following papers: Laird, P.W. Nature Reviews Cancer 3, 253-266 (2003); Biotechniques; Uhlmann, K. et al. Electrophoresis 23:4072-4079 (2002) - PyroMeth; Colella et al. Biotechniques. 2003 Jul;35(1):146-50; Dupont JM, Tost J, Jammes H, and Gut IG. Anal Biochem, Oct 2004; 333(1): 119-27; and Tooke N and Pettersson M. IVDT. Nov 2004; 41.

### Polynucleotide Sequence Amplification and Determination

Following separation of nucleic acid in a methylation-differential manner, the nucleic acid may be subjected to sequence-based analysis. Furthermore, once it is determined that one particular genomic sequence of fetal origin is hypermethylated or hypomethylated compared to the maternal counterpart, the amount of this fetal genomic sequence can be determined. Subsequently, this amount can be compared to a standard control value and serve as an indication for the potential of certain pregnancy-associated disorder.

### A. Amplification of Nucleotide Sequences

In many instances, it is desirable to amplify a nucleic acid sequence of the technology using any of several nucleic acid amplification procedures which are well known in the art (listed above and described in greater detail below). Specifically, nucleic acid amplification is the enzymatic synthesis of nucleic acid amplicons (copies) which contain a sequence that is complementary to a nucleic acid sequence being amplified. Nucleic acid amplification is especially beneficial when the amount of target sequence present in a sample is very low. By amplifying the target sequences and detecting the amplicon synthesized, the sensitivity of an assay can be vastly improved, since fewer target sequences are needed at the beginning of the assay to better ensure detection of nucleic acid in the sample belonging to the organism or virus of interest.

A variety of polynucleotide amplification methods are well established and frequently used in research. For instance, the general methods of polymerase chain reaction (PCR) for polynucleotide sequence amplification are well known in the art and are thus not described in detail herein. For a review of PCR methods, protocols, and principles in designing primers, see, e.g., Innis, et al., PCR Protocols: A Guide to Methods and Applications, Academic Press, Inc. N.Y., 1990. PCR reagents and protocols are also available from commercial vendors, such as Roche Molecular Systems.

PCR is most usually carried out as an automated process with a thermostable enzyme. In this process, the temperature of the reaction mixture is cycled through a denaturing region, a primer annealing region, and an extension reaction region automatically. Machines specifically adapted for this purpose are commercially available.

Although PCR amplification of a polynucleotide sequence is typically used in practicing the present technology, one of skill in the art will recognize that the amplification of a genomic sequence found in a maternal blood sample may be accomplished by any known method, such as ligase chain reaction (LCR), transcription-mediated amplification, and self-sustained sequence replication or nucleic acid sequence-based amplification (NASBA), each of which provides sufficient amplification. More recently developed branched-DNA technology may also be used to qualitatively demonstrate the presence of a particular genomic sequence of the technology, which represents a particular methylation pattern, or to quantitatively determine the amount of this particular genomic sequence in the maternal blood. For a review of branched-DNA signal amplification for direct quantitation of nucleic acid sequences in clinical samples, see Nolte, Adv. Clin. Chem. 33:201-235, 1998.

The compositions and processes of the technology are also particularly useful when practiced with digital PCR. Digital PCR was first developed by Kalinina and colleagues (Kalinina et al., "Nanoliter scale PCR with TaqMan detection." Nucleic Acids Research. 25; 1999-2004, (1997)) and further developed by Vogelstein and Kinzler (Digital PCR. Proc Natl Acad Sci USA. 96; 9236-41, (1999)). The application of digital PCR for use with fetal diagnostics was first described by Cantor et al. (PCT Patent Publication No. WO05023091A2) and subsequently described by Quake et al. (US Patent Publication No. US 20070202525), which are both hereby incorporated by reference. Digital PCR takes advantage of nucleic acid (DNA, cDNA or RNA) amplification on a single molecule level, and offers a highly sensitive method for quantifying low copy number nucleic acid. Fluidigm® Corporation offers systems for the digital analysis of nucleic acids.

### B. Determination of Polynucleotide Sequences

Techniques for polynucleotide sequence determination are also well established and widely practiced in the relevant research field. For instance, the basic principles and general techniques for polynucleotide sequencing are described in various research reports and treatises on molecular biology and recombinant genetics, such as Wallace et al., supra; Sambrook and Russell, supra, and Ausubel et al., supra. DNA sequencing methods routinely practiced in research laboratories, either manual or automated, can be used for practicing the present technology. Additional means suitable for detecting changes in a polynucleotide sequence for practicing the methods of the present technology include but are not limited to mass spectrometry, primer extension, polynucleotide hybridization, real-time PCR, and electrophoresis.

Use of a primer extension reaction also can be applied in methods of the technology. A primer extension reaction operates, for example, by discriminating the SNP alleles by the incorporation of deoxynucleotides and/or dideoxynucleotides to a primer extension primer which hybridizes to a region adjacent to the SNP site. The primer is extended with a polymerase. The primer extended SNP can be detected physically by mass spectrometry or by a tagging moiety such as biotin. As the SNP site is only extended by a complementary deoxynucleotide or dideoxynucleotide that is either tagged by a specific label or generates a primer extension product with a specific mass, the SNP alleles can be discriminated and quantified.

Reverse transcribed and amplified nucleic acids may be modified nucleic acids. Modified nucleic acids can include nucleotide analogs, and in certain embodiments include a detectable label and/or a capture agent. Examples of detectable labels include without limitation fluorophores, radioisotopes, colormetric agents, light emitting agents, chemiluminescent agents, light scattering agents, enzymes and the like. Examples of capture agents include without limitation an agent from a binding pair selected from antibody/antigen, antibody/antibody, antibody/antibody fragment, antibody/antibody receptor, antibody/protein A or protein G, hapten/anti-hapten, biotin/avidin, biotin/streptavidin, folic acid/folate binding protein, vitamin B12/intrinsic factor, chemical reactive group/complementary chemical reactive group (e.g., sulfhydryl/maleimide, sulfhydryl/haloacetyl derivative, amine/isotriocyanate, amine/succinimidyl ester, and amine/sulfonyl halides) pairs, and the like. Modified nucleic acids having a capture agent can be immobilized to a solid support in certain embodiments

Mass spectrometry is a particularly effective method for the detection of a polynucleotide of the technology, for example a PCR amplicon, a primer extension product or a detector probe that is cleaved from a target nucleic acid. The presence of the polynucleotide sequence is verified by comparing the mass of the detected signal with the expected mass of the polynucleotide of interest. The relative signal strength, e.g., mass peak on a spectra, for a particular polynucleotide sequence indicates the relative population of a specific allele, thus enabling calculation of the allele ratio directly from the data. For a review of genotyping methods using Sequenom® standard iPLEX™ assay and MassARRAY® technology, see Jurinke, C., Oeth, P., van den Boom, D., "MALDI-TOF mass spectrometry: a versatile tool for high-performance DNA analysis." Mol. Biotechnol. 26, 147-164 (2004); and Oeth, P. et al., "iPLEX™ Assay: Increased Plexing Efficiency and Flexibility for MassARRAY® System through single base primer extension with mass-modified Terminators." SEQUENOM Application Note (2005), both of which are hereby incorporated by reference. For a review of detecting and quantifying target nucleic using cleavable detector probes that are cleaved during the amplification process and detected by mass spectrometry, see US Patent Application Number 11/950,395, which was filed December 4, 2007, and is hereby incorporated by reference.

Sequencing technologies are improving in terms of throughput and cost. Sequencing technologies, such as that achievable on the 454 platform (Roche) (Margulies, M. et al. 2005 Nature 437, 376-380), Illumina Genome Analyzer (or Solexa platform) or SOLiD System (Applied Biosystems) or the Helicos True Single Molecule DNA sequencing technology (Harris T D et al. 2008 Science, 320, 106-109), the single molecule, real-time (SMRT.TM.) technology of Pacific Biosciences, and nanopore sequencing (Soni GV and Meller A. 2007 Clin Chem 53: 1996-2001), allow the sequencing of many nucleic acid molecules isolated from a specimen at high orders of multiplexing in a parallel fashion (Dear Brief Funct Genomic Proteomic 2003; 1: 397-416).

Each of these platforms allow sequencing of clonally expanded or non-amplified single molecules of nucleic acid fragments. Certain platforms involve, for example, (i) sequencing by ligation of dye-modified probes (including cyclic ligation and cleavage), (ii) pyrosequencing, and (iii) single-molecule sequencing. Nucleotide sequence species, amplification nucleic acid species and detectable products generated there from can be considered a "study nucleic acid" for purposes of analyzing a nucleotide sequence by such sequence analysis platforms.

Sequencing by ligation is a nucleic acid sequencing method that relies on the sensitivity of DNA ligase to base-pairing mismatch. DNA ligase joins together ends of DNA that are correctly base paired. Combining the ability of DNA ligase to join together only correctly base paired DNA ends, with mixed pools of fluorescently labeled oligonucleotides or primers, enables sequence determination by fluorescence detection. Longer sequence reads may be obtained by including primers containing cleavable linkages that can be cleaved after label identification. Cleavage at the linker removes the label and regenerates the 5' phosphate on the end of the ligated primer, preparing the primer for another round of ligation. In some embodiments primers may be labeled with more than one fluorescent label (e.g., 1 fluorescent label, 2, 3, or 4 fluorescent labels).

An example of a system that can be used by a person of ordinary skill based on sequencing by ligation generally involves the following steps. Clonal bead populations can be prepared in emulsion microreactors containing study nucleic acid ("template"), amplification reaction components, beads and primers. After amplification, templates are denatured and bead enrichment is performed to separate beads with extended templates from undesired beads (e.g., beads with no extended templates). The template on the selected beads undergoes a 3' modification to allow covalent bonding to the slide, and modified beads can be deposited onto a glass slide. Deposition chambers offer the ability to segment a slide into one, four or eight chambers during the bead loading process. For sequence analysis, primers hybridize to the adapter sequence. A set of four color dye-labeled probes competes for ligation to the sequencing primer. Specificity of probe ligation is achieved by interrogating every 4th and 5th base during the ligation series. Five to seven rounds of ligation, detection and cleavage record the color at every 5th position with the number of rounds determined by the type of library used. Following each round of ligation, a new complimentary primer offset by one base in the 5' direction is laid down for another series of ligations. Primer reset and ligation rounds (5-7 ligation cycles per round) are repeated sequentially five times to generate 25-35 base pairs of sequence for a single tag. With mate-paired sequencing, this process is repeated for a second tag. Such a system can be used to exponentially amplify amplification products generated by a process described herein, e.g., by ligating a heterologous nucleic acid to the first amplification product generated by a process described herein and performing emulsion amplification using the same or a different solid support originally used to generate the first amplification product. Such a system also may be used to analyze amplification products directly generated by a process described herein by bypassing an exponential amplification process and directly sorting the solid supports described herein on the glass slide.

Pyrosequencing is a nucleic acid sequencing method based on sequencing by synthesis, which relies on detection of a pyrophosphate released on nucleotide incorporation. Generally, sequencing by synthesis involves synthesizing, one nucleotide at a time, a DNA strand complimentary to the strand whose sequence is being sought. Study nucleic acids may be immobilized to a solid support, hybridized with a sequencing primer, incubated with DNA polymerase, ATP sulfurylase, luciferase, apyrase, adenosine 5' phosphsulfate and luciferin. Nucleotide solutions are sequentially added and removed. Correct incorporation of a nucleotide releases a pyrophosphate, which interacts with ATP sulfurylase and produces ATP in the presence of adenosine 5' phosphsulfate, fueling the luciferin reaction, which produces a chemiluminescent signal allowing sequence determination.

An example of a system that can be used by a person of ordinary skill based on pyrosequencing generally involves the following steps: ligating an adaptor nucleic acid to a study nucleic acid and hybridizing the study nucleic acid to a bead; amplifying a nucleotide sequence in the study nucleic acid in an emulsion; sorting beads using a picoliter multiwell solid support; and sequencing amplified nucleotide sequences by pyrosequencing methodology (e.g., Nakano et al., "Single-molecule PCR using water-in-oil emulsion;" Journal of Biotechnology 102: 117-124 (2003)). Such a system can be used to exponentially amplify amplification products generated by a process described herein, e.g., by ligating a heterologous nucleic acid to the first amplification product generated by a process described herein.

Certain single-molecule sequencing embodiments are based on the principal of sequencing by synthesis, and utilize single-pair Fluorescence Resonance Energy Transfer (single pair FRET) as a mechanism by which photons are emitted as a result of successful nucleotide incorporation. The emitted photons often are detected using intensified or high sensitivity cooled charge-couple-devices in conjunction with total internal reflection microscopy (TIRM). Photons are only emitted when the introduced reaction solution contains the correct nucleotide for incorporation into the growing nucleic acid chain that is synthesized as a result of the sequencing process. In FRET based single-molecule sequencing, energy is transferred between two fluorescent dyes, sometimes polymethine cyanine dyes Cy3 and Cy5, through long-range dipole interactions. The donor is excited at its specific excitation wavelength and the excited state energy is transferred, non-radiatively to the acceptor dye, which in turn becomes excited. The acceptor dye eventually returns to the ground state by radiative emission of a photon. The two dyes used in the energy transfer process represent the "single pair", in single pair FRET. Cy3 often is used as the donor fluorophore and often is incorporated as the first labeled nucleotide. Cy5 often is used as the acceptor fluorophore and is used as the nucleotide label for successive nucleotide additions after incorporation of a first Cy3 labeled nucleotide. The fluorophores generally are within 10 nanometers of each for energy transfer to occur successfully.

An example of a system that can be used based on single-molecule sequencing generally involves hybridizing a primer to a study nucleic acid to generate a complex; associating the complex with a solid phase; iteratively extending the primer by a nucleotide tagged with a fluorescent molecule; and capturing an image of fluorescence resonance energy transfer signals after each iteration (e.g., U.S. Patent No. 7,169,314; Braslavsky et al., PNAS 100(7): 3960-3964 (2003)). Such a system can be used to directly sequence amplification products generated by processes described herein. In some embodiments the released linear amplification product can be hybridized to a primer that contains sequences complementary to immobilized capture sequences present on a solid support, a bead or glass slide for example. Hybridization of the primer--released linear amplification product complexes with the immobilized capture sequences, immobilizes released linear amplification products to solid supports for single pair FRET based sequencing by synthesis. The primer often is fluorescent, so that an initial reference image of the surface of the slide with immobilized nucleic acids can be generated. The initial reference image is useful for determining locations at which true nucleotide incorporation is occurring. Fluorescence signals detected in array locations not initially identified in the "primer only" reference image are discarded as non-specific fluorescence. Following immobilization of the primer--released linear amplification product complexes, the bound nucleic acids often are sequenced in parallel by the iterative steps of, a) polymerase extension in the presence of one fluorescently labeled nucleotide, b) detection of fluorescence using appropriate microscopy, TIRM for example, c) removal of fluorescent nucleotide, and d) return to step a with a different fluorescently labeled nucleotide.

In some embodiments, nucleotide sequencing may be by solid phase single nucleotide sequencing methods and processes. Solid phase single nucleotide sequencing methods involve contacting sample nucleic acid and solid support under conditions in which a single molecule of sample nucleic acid hybridizes to a single molecule of a solid support. Such conditions can include providing the solid support molecules and a single molecule of sample nucleic acid in a "microreactor." Such conditions also can include providing a mixture in which the sample nucleic acid molecule can hybridize to solid phase nucleic acid on the solid support. Single nucleotide sequencing methods useful in the embodiments described herein are described in United States Provisional Patent Application Serial Number 61/021,871 filed January 17, 2008.

In certain embodiments, nanopore sequencing detection methods include (a) contacting a nucleic acid for sequencing ("base nucleic acid," e.g., linked probe molecule) with sequence-specific detectors, under conditions in which the detectors specifically hybridize to substantially complementary subsequences of the base nucleic acid; (b) detecting signals from the detectors and (c) determining the sequence of the base nucleic acid according to the signals detected. In certain embodiments, the detectors hybridized to the base nucleic acid are disassociated from the base nucleic acid (e.g., sequentially dissociated) when the detectors interfere with a nanopore structure as the base nucleic acid passes through a pore, and the detectors disassociated from the base sequence are detected. In some embodiments, a detector disassociated from a base nucleic acid emits a detectable signal, and the detector hybridized to the base nucleic acid emits a different detectable signal or no detectable signal. In certain embodiments, nucleotides in a nucleic acid (e.g., linked probe molecule) are substituted with specific nucleotide sequences corresponding to specific nucleotides ("nucleotide representatives"), thereby giving rise to an expanded nucleic acid (e.g., U.S. Patent No. 6,723,513), and the detectors hybridize to the nucleotide representatives in the expanded nucleic acid, which serves as a base nucleic acid. In such embodiments, nucleotide representatives may be arranged in a binary or higher order arrangement (e.g., Soni and Meller, Clinical Chemistry 53(11): 1996-2001 (2007)). In some embodiments, a nucleic acid is not expanded, does not give rise to an expanded nucleic acid, and directly serves a base nucleic acid (e.g., a linked probe molecule serves as a non-expanded base nucleic acid), and detectors are directly contacted with the base nucleic acid. For example, a first detector may hybridize to a first subsequence and a second detector may hybridize to a second subsequence, where the first detector and second detector each have detectable labels that can be distinguished from one another, and where the signals from the first detector and second detector can be distinguished from one another when the detectors are disassociated from the base nucleic acid. In certain embodiments, detectors include a region that hybridizes to the base nucleic acid (e.g., two regions), which can be about 3 to about 100 nucleotides in length (e.g., about 4, 5, 6, 7, 8, 9, 10,11, 12, 13, 14, 15, 16, 17, 18, 19, 20, 25, 30, 35, 40, 50, 55, 60, 65, 70, 75, 80, 85, 90, or 95 nucleotides in length). A detector also may include one or more regions of nucleotides that do not hybridize to the base nucleic acid. In some embodiments, a detector is a molecular beacon. A detector often comprises one or more detectable labels independently selected from those described herein. Each detectable label can be detected by any convenient detection process capable of detecting a signal generated by each label (e.g., magnetic, electric, chemical, optical and the like). For example, a CD camera can be used to detect signals from one or more distinguishable quantum dots linked to a detector.

In certain sequence analysis embodiments, reads may be used to construct a larger nucleotide sequence, which can be facilitated by identifying overlapping sequences in different reads and by using identification sequences in the reads. Such sequence analysis methods and software for constructing larger sequences from reads are known to the person of ordinary skill (e.g., Venter et al., Science 291: 1304-1351 (2001)). Specific reads, partial nucleotide sequence constructs, and full nucleotide sequence constructs may be compared between nucleotide sequences within a sample nucleic acid (i.e., internal comparison) or may be compared with a reference sequence (i.e., reference comparison) in certain sequence analysis embodiments. Internal comparisons sometimes are performed in situations where a sample nucleic acid is prepared from multiple samples or from a single sample source that contains sequence variations. Reference comparisons sometimes are performed when a reference nucleotide sequence is known and an objective is to determine whether a sample nucleic acid contains a nucleotide sequence that is substantially similar or the same, or different, than a reference nucleotide sequence. Sequence analysis is facilitated by sequence analysis apparatus and components known to the person of ordinary skill in the art.

Methods provided herein allow for high-throughput detection of nucleic acid species in a plurality of nucleic acids (e.g., nucleotide sequence species, amplified nucleic acid species and detectable products generated from the foregoing). Multiplexing refers to the simultaneous detection of more than one nucleic acid species. General methods for performing multiplexed reactions in conjunction with mass spectrometry, are known (see, e.g., U.S. Pat. Nos. 6,043,031, 5,547,835 and International PCT application No. WO 97/37041). Multiplexing provides an advantage that a plurality of nucleic acid species (e.g., some having different sequence variations) can be identified in as few as a single mass spectrum, as compared to having to perform a separate mass spectrometry analysis for each individual target nucleic acid species. Methods provided herein lend themselves to high-throughput, highly-automated processes for analyzing sequence variations with high speed and accuracy, in some embodiments. In some embodiments, methods herein may be multiplexed at high levels in a single reaction.

In certain embodiments, the number of nucleic acid species multiplexed include, without limitation, about 1 to about 500 (e.g., about 1-3, 3-5, 5-7, 7-9, 9-11, 11-13, 13-15, 15-17, 17-19, 19-21, 21-23, 23-25, 25-27, 27-29, 29-31, 31-33, 33-35, 35-37, 37-39, 39-41, 41-43, 43-45, 45-47, 47-49, 49-51, 51-53, 53-55, 55-57, 57-59, 59-61, 61-63, 63-65, 65-67, 67-69, 69-71, 71-73, 73-75, 75-77, 77-79, 79-81, 81-83, 83-85, 85-87, 87-89, 89-91, 91-93, 93-95, 95-97, 97-101, 101-103, 103-105, 105-107, 107-109, 109-111, 111-113, 113-115, 115-117, 117-119, 121-123, 123-125, 125-127, 127-129, 129-131, 131-133, 133-135, 135-137, 137-139, 139-141, 141-143, 143-145, 145-147, 147-149, 149-151, 151-153, 153-155, 155-157, 157-159, 159-161, 161-163, 163-165, 165-167, 167-169, 169-171, 171-173, 173-175, 175-177, 177-179, 179-181, 181-183, 183-185, 185-187, 187-189, 189-191, 191-193, 193-195, 195-197, 197-199, 199-201, 201-203, 203-205, 205-207, 207-209, 209-211, 211-213, 213-215, 215-217, 217-219, 219-221, 221-223, 223-225, 225-227, 227-229, 229-231, 231-233, 233-235, 235-237, 237-239, 239-241, 241-243, 243-245, 245-247, 247-249, 249-251, 251-253, 253-255, 255-257, 257-259, 259-261, 261-263, 263-265, 265-267, 267-269, 269-271, 271-273, 273-275, 275-277, 277-279, 279-281, 281-283, 283-285, 285-287, 287-289, 289-291, 291-293, 293-295, 295-297, 297-299, 299-301, 301- 303, 303- 305, 305- 307, 307- 309, 309- 311, 311- 313, 313- 315, 315- 317, 317- 319, 319-321, 321-323, 323-325, 325-327, 327-329, 329-331, 331-333, 333- 335, 335-337, 337-339, 339-341, 341-343, 343-345, 345-347, 347-349, 349-351, 351-353, 353-355, 355-357, 357-359, 359-361, 361-363, 363-365, 365-367, 367-369, 369-371, 371-373, 373-375, 375-377, 377-379, 379-381, 381-383, 383-385, 385-387, 387-389, 389-391, 391-393, 393-395, 395-397, 397-401, 401- 403, 403- 405, 405- 407, 407- 409, 409- 411, 411- 413, 413- 415, 415- 417, 417- 419, 419-421, 421-423, 423-425, 425-427, 427-429, 429-431, 431-433, 433- 435, 435-437, 437-439, 439-441, 441-443, 443-445, 445-447, 447-449, 449-451, 451-453, 453-455, 455-457, 457-459, 459-461, 461-463, 463-465, 465-467, 467-469, 469-471, 471-473, 473-475, 475-477, 477-479, 479-481, 481-483, 483-485, 485-487, 487-489, 489-491, 491-493, 493-495, 495-497, 497-501).

Design methods for achieving resolved mass spectra with multiplexed assays can include primer and oligonucleotide design methods and reaction design methods. See, for example, the multiplex schemes provided in Tables X and Y. For primer and oligonucleotide design in multiplexed assays, the same general guidelines for primer design applies for uniplexed reactions, such as avoiding false priming and primer dimers, only more primers are involved for multiplex reactions. For mass spectrometry applications, analyte peaks in the mass spectra for one assay are sufficiently resolved from a product of any assay with which that assay is multiplexed, including pausing peaks and any other by-product peaks. Also, analyte peaks optimally fall within a user-specified mass window, for example, within a range of 5,000-8,500 Da. In some embodiments multiplex analysis may be adapted to mass spectrometric detection of chromosome abnormalities, for example. In certain embodiments multiplex analysis may be adapted to various single nucleotide or nanopore based sequencing methods described herein. Commercially produced micro-reaction chambers or devices or arrays or chips may be used to facilitate multiplex analysis, and are commercially available.

### Detection of Fetal Aneuploidy

For the detection of fetal aneuploidies, some methods rely on measuring the ratio between maternally and paternally inherited alleles. However, the ability to quantify chromosomal changes is impaired by the maternal contribution of cell free nucleic acids, which makes it necessary to deplete the sample from maternal DNA prior to measurement. Promising approaches take advantage of the different size distribution of fetal and maternal DNA or measure RNA that is exclusively expressed by the fetus (see for example, US Patent Application No. 11/384128, which published as US20060252071 and is hereby incorporated by reference). Assuming fetal DNA makes up only about 5% of all cell free DNA in the maternal plasma, there is a decrease of the ratio difference from 1.6% to only about 1.2% between a trisomy sample and a healthy control. Consequently, reliable detection of allele ratio changes requires enriching the fetal fraction of cell free DNA, for example, using the compositions and methods of the present technology.

Some methods rely on measuring the ratio of maternal to paternally inherited alleles to detect fetal chromosomal aneuploidies from maternal plasma. A diploid set yields a 1:1 ratio while trisomies can be detected as a 2:1 ratio. Detection of this difference is impaired by statistical sampling due to the low abundance of fetal DNA, presence of excess maternal DNA in the plasma sample and variability of the measurement technique. The latter is addressed by using methods with high measurement precision, like digital PCR or mass spectrometry. Enriching the fetal fraction of cell free DNA in a sample is currently achieved by either depleting maternal DNA through size exclusion or focusing on fetal-specific nucleic acids, like fetal-expressed RNA. Another differentiating feature of fetal DNA is its DNA methylation pattern. Thus, provided herein are novel compositions and methods for accurately quantifying fetal nucleic acid based on differential methylation between a fetus and mother. The methods rely on sensitive absolute copy number analysis to quantify the fetal nucleic acid portion of a maternal sample, thereby allowing for the prenatal detection of fetal traits. The methods of the technology have identified approximately 3000 CpG rich regions in the genome that are differentially methylated between maternal and fetal DNA. The selected regions showed highly conserved differential methylation across all measured samples. In addition the set of regions is enriched for genes important in developmental regulation, indicating that epigenetic regulation of these areas is a biologically relevant and consistent process (see Table 3). Enrichment of fetal DNA can now be achieved by using our MBD-FC protein to capture cell free DNA (e.g., substantially all cell free DNA) and then elute the highly methylated DNA fraction with high salt concentrations. Using the low salt eluate fractions, the MBD-FC is equally capable of enriching non-methylated fetal DNA.

The present technology provides 63 confirmed genomic regions on chromosomes 13, 18 and 21 with low maternal and high fetal methylation levels. After capturing these regions, SNPs can be used to determine the aforementioned allele ratios. When high frequency SNPs are used around 10 markers have to be measured to achieve a high confidence of finding at least one SNP where the parents have opposite homozygote genotypes and the child has a heterozygote genotype.

In another embodiment, a method for chromosomal abnormality detection is provided that utilizes absolute copy number quantification. A diploid chromosome set will show the same number of copies for differentially methylated regions across all chromosomes, but, for example, a trisomy 21 sample would show 1.5 times more copies for differentially methylated regions on chromosome 21. Normalization of the genomic DNA amounts for a diploid chromosome set can be achieved by using unaltered autosomes as reference (also provided herein - see Table 1). Comparable to other approaches, a single marker is less likely to be sufficient for detection of this difference, because the overall copy numbers are low. Typically there are approximately 100 to 200 copies of fetal DNA from 1 ml of maternal plasma at 10 to 12 weeks of gestation. However, the methods of the present technology offer a redundancy of detectable markers that enables highly reliable discrimination of diploid versus aneuploid chromosome sets.

### Data Processing and Identifying Presence or Absence of a Chromosome Abnormality

The term "detection" of a chromosome abnormality as used herein refers to identification of an imbalance of chromosomes by processing data arising from detecting sets of amplified nucleic acid species, nucleotide sequence species, or a detectable product generated from the foregoing (collectively "detectable product"). Any suitable detection device and method can be used to distinguish one or more sets of detectable products, as addressed herein. An outcome pertaining to the presence or absence of a chromosome abnormality can be expressed in any suitable form, including, without limitation, probability (e.g., odds ratio, p-value), likelihood, percentage, value over a threshold, or risk factor, associated with the presence of a chromosome abnormality for a subject or sample. An outcome may be provided with one or more of sensitivity, specificity, standard deviation, coefficient of variation (CV) and/or confidence level, or combinations of the foregoing, in certain embodiments.

Detection of a chromosome abnormality based on one or more sets of detectable products may be identified based on one or more calculated variables, including, but not limited to, sensitivity, specificity, standard deviation, coefficient of variation (CV), a threshold, confidence level, score, probability and/or a combination thereof. In some embodiments, (i) the number of sets selected for a diagnostic method, and/or (ii) the particular nucleotide sequence species of each set selected for a diagnostic method, is determined in part or in full according to one or more of such calculated variables.

In certain embodiments, one or more of sensitivity, specificity and/or confidence level are expressed as a percentage. In some embodiments, the percentage, independently for each variable, is greater than about 90% (e.g., about 90, 91, 92, 93, 94, 95, 96, 97, 98 or 99%, or greater than 99% (e.g., about 99.5%, or greater, about 99.9% or greater, about 99.95% or greater, about 99.99% or greater)). Coefficient of variation (CV) in some embodiments is expressed as a percentage, and sometimes the percentage is about 10% or less (e.g., about 10, 9, 8, 7, 6, 5, 4, 3, 2 or 1%, or less than 1% (e.g., about 0.5% or less, about 0.1% or less, about 0.05% or less, about 0.01% or less)). A probability (e.g., that a particular outcome determined by an algorithm is not due to chance) in certain embodiments is expressed as a p-value, and sometimes the p-value is about 0.05 or less (e.g., about 0.05, 0.04, 0.03, 0.02 or 0.01, or less than 0.01 (e.g., about 0.001 or less, about 0.0001 or less, about 0.00001 or less, about 0.000001 or less)).

For example, scoring or a score may refer to calculating the probability that a particular chromosome abnormality is actually present or absent in a subject/sample. The value of a score may be used to determine for example the variation, difference, or ratio of amplified nucleic detectable product that may correspond to the actual chromosome abnormality. For example, calculating a positive score from detectable products can lead to an identification of a chromosome abnormality, which is particularly relevant to analysis of single samples.

In certain embodiments, simulated (or simulation) data can aid data processing for example by training an algorithm or testing an algorithm. Simulated data may for instance involve hypothetical various samples of different concentrations of fetal and maternal nucleic acid in serum, plasma and the like. Simulated data may be based on what might be expected from a real population or may be skewed to test an algorithm and/or to assign a correct classification based on a simulated data set. Simulated data also is referred to herein as "virtual" data. Fetal/maternal contributions within a sample can be simulated as a table or array of numbers (for example, as a list of peaks corresponding to the mass signals of cleavage products of a reference biomolecule or amplified nucleic acid sequence), as a mass spectrum, as a pattern of bands on a gel, or as a representation of any technique that measures mass distribution. Simulations can be performed in most instances by a computer program. One possible step in using a simulated data set is to evaluate the confidence of the identified results, i.e. how well the selected positives/negatives match the sample and whether there are additional variations. A common approach is to calculate the probability value (p-value) which estimates the probability of a random sample having better score than the selected one. As p-value calculations can be prohibitive in certain circumstances, an empirical model may be assessed, in which it is assumed that at least one sample matches a reference sample (with or without resolved variations). Alternatively other distributions such as Poisson distribution can be used to describe the probability distribution.

In certain embodiments, an algorithm can assign a confidence value to the true positives, true negatives, false positives and false negatives calculated. The assignment of a likelihood of the occurrence of a chromosome abnormality can also be based on a certain probability model.

Simulated data often is generated in an *in silico* process. As used herein, the term "in silico" refers to research and experiments performed using a computer. In silico methods include, but are not limited to, molecular modeling studies, karyotyping, genetic calculations, biomolecular docking experiments, and virtual representations of molecular structures and/or processes, such as molecular interactions.

As used herein, a "data processing routine" refers to a process, that can be embodied in software, that determines the biological significance of acquired data (i.e., the ultimate results of an assay). For example, a data processing routine can determine the amount of each nucleotide sequence species based upon the data collected. A data processing routine also may control an instrument and/or a data collection routine based upon results determined. A data processing routine and a data collection routine often are integrated and provide feedback to operate data acquisition by the instrument, and hence provide assay-based judging methods provided herein.

As used herein, software refers to computer readable program instructions that, when executed by a computer, perform computer operations. Typically, software is provided on a program product containing program instructions recorded on a computer readable medium, including, but not limited to, magnetic media including floppy disks, hard disks, and magnetic tape; and optical media including CD-ROM discs, DVD discs, magneto-optical discs, and other such media on which the program instructions can be recorded.

Different methods of predicting abnormality or normality can produce different types of results. For any given prediction, there are four possible types of outcomes: true positive, true negative, false positive, or false negative. The term "true positive" as used herein refers to a subject correctly diagnosed as having a chromosome abnormality. The term "false positive" as used herein refers to a subject wrongly identified as having a chromosome abnormality. The term "true negative" as used herein refers to a subject correctly identified as not having a chromosome abnormality. The term "false negative" as used herein refers to a subject wrongly identified as not having a chromosome abnormality. Two measures of performance for any given method can be calculated based on the ratios of these occurrences: (i) a sensitivity value, the fraction of predicted positives that are correctly identified as being positives (e.g., the fraction of nucleotide sequence sets correctly identified by level comparison detection/determination as indicative of chromosome abnormality, relative to all nucleotide sequence sets identified as such, correctly or incorrectly), thereby reflecting the accuracy of the results in detecting the chromosome abnormality; and (ii) a specificity value, the fraction of predicted negatives correctly identified as being negative (the fraction of nucleotide sequence sets correctly identified by level comparison detection/determination as indicative of chromosomal normality, relative to all nucleotide sequence sets identified as such, correctly or incorrectly), thereby reflecting accuracy of the results in detecting the chromosome abnormality.

### EXAM PLES

The following examples are provided by way of illustration only and not by way of limitation. Those of skill in the art will readily recognize a variety of non-critical parameters that could be changed or modified to yield essentially the same or similar results.

In Example 1 below, the Applicants used a new fusion protein that captures methylated DNA in combination with CpG Island array to identify genomic regions that are differentially methylated between fetal placenta tissue and maternal blood. A stringent statistical approach was used to only select regions which show little variation between the samples, and hence suggest an underlying biological mechanism. Eighty-five differentially methylated genomic regions predominantly located on chromosomes 13, 18 and 21 were validated. For this validation, a quantitative mass spectrometry based approach was used that interrogated 261 PCR amplicons covering these 85 regions. The results are in very good concordance (95% confirmation), proving the feasibility of the approach.

Next, the Applicants provide an innovative approach for aneuploidy testing, which relies on the measurement of absolute copy numbers rather than allele ratios.

### Example 1

In the below Example, ten paired maternal and placental DNA samples were used to identify differentially methylated regions. These results were validated using a mass spectrometry-based quantitative methylation assay. First, genomic DNA from maternal buffy coat and corresponding placental tissue was first extracted. Next the MBD-FC was used to capture the methylated fraction of each DNA sample. See Figures 1-3. The two tissue fractions were labeled with different fluorescent dyes and hybridized to an Agilent® CpG Island microarray. See Figure 4. This was done to identify differentially methylated regions that could be utilized for prenatal diagnoses. Therefore, two criteria were employed to select genomic regions as potential enrichment markers: the observed methylation difference had to be present in all tested sample pairs, and the region had to be more than 200 bp in length.

### DNA preparation and fragmentation

Genomic DNA (gDNA) from maternal buffy coat and placental tissue was prepared using the QIAamp DNA Mini Kit™ and QIAamp DNA Blood Mini Kit™, respectively, from Qiagen® (Hilden, Germany). For MClp, gDNA was quantified using the NanoDrop ND 1000™ spectrophotometer (Thermo Fisher®, Waltham, MA,USA). Ultrasonication of 2.5 µg DNA in 500 µl TE buffer to a mean fragment size of 300-500 bp was carried out with the Branson Digital Sonifier 450™ (Danbury, CT, USA) using the following settings: amplitude 20%, sonication time 110 seconds, pulse on/pulse off time 1.4/0.6 seconds. Fragment range was monitored using gel electrophoresis.

### Methyl-CpG Immunoprecipitation

Per sample, 56 µg purified MBD-Fc protein and 150 µl of Protein A Sepharose 4 Fast Flow beads (Amersham Biosciences®, Piscataway, NJ, USA) were rotated in 15 ml TBS overnight at 4°C. Then, theMBD-Fc beads (150 µl/assay) were transferred and dispersed in to 2 ml Ultrafree-CL centrifugal filter devices (Millipore®, Billerica, MA, USA) and spin-washed three times with Buffer A (20 mM Tris-HCI, pH8.0, 2 mM MgCI2, 0.5 mM EDTA 300 mM NaCl, 0.1% NP-40). Sonicated DNA (2 µg) was added to the washed MBD-Fc beads in 2 ml Buffer A and rotated for 3 hours at 4°C. Beads were centrifuged to recover unbound DNA fragments (300 mM fraction) and subsequently washed twice with 600 µl of buffers containing increasing NaCl concentrations (400, 500, 550, 600, and 1000 mM). The flow through of each wash step was collected in separate tubes and desalted using a MinElute PCR Purification Kit™ (Qiagen®). In parallel, 200 ng sonicated input DNA was processed as a control using the MinElute PCR Purification Kit™ (Qiagen®).

### Microarray handling and analysis

To generate fluorescently labeled DNA for microarray hybridization, the 600 mM and 1M NaCl fractions (enriched methylated DNA) for each sample were combined and labeled with either Alexa Fluor 555-aha-dCTP (maternal) or Alexa Fluor 647-aha-dCTP (placental) using the BioPrime Total Genomic Labeling System™ (invitrogen®, Carlsbad, CA, USA). The labeling reaction was carried out according to the manufacturer's manual. The differently labeled genomic DNA fragments of matched maternal/placental pairs were combined to a final volume of 80 µl, supplemented with 50 µg Cot-1 DNA (Invitrogen®), 52 µl of Agilent 10X blocking reagent (Agilent Technologies®, Santa Clara, CA, USA), 78 µl of deionized formamide, and 260 µl Agilent 2X hybridization buffer. The samples were heated to 95°C for 3 min, mixed, and subsequently incubated at 37°C for 30 min. Hybridization on Agilent CpG Island Microarray Kit™ was then carried out at 67°C for 40 hours using an Agilent SureHyb™ chamber and an Agilent hybridization oven. Slides were washed in Wash I (6X SSPE, 0.005% N-lauroylsarcosine) at room temperature for 5 min and in Wash II (0.06X SSPE) at 37°C for an additional 5 min. Next, the slides were submerged in acetonitrile and Agilent Ozone Protection Solution™, respectively, for 30 seconds. Images were scanned immediately and analyzed using an Agilent DNA Microarray Scanner™. Microarray images were processed using Feature Extraction Software v9.5 and the standard CGH protocol.

### Bisulfite Treatment

Genomic DNA sodium bisulfite conversion was performed using EZ-96 DNA Methylation Kit™ (ZymoResearch, Orange County, CA). The manufacturer's protocol was followed using 1ug of genomic DNA and the alternative conversion protocol (a two temperature DNA denaturation).

### Quantitative Methylation Analysis

Sequenom's MassARRAY® System was used to perform quantitative methylation analysis. This system utilizes matrix-assisted laser desorption ionization time-of-flight (MALDI-TOF) mass spectrometry in combination with RNA base specific cleavage (Sequenom® MassCLEAVE™). A detectable pattern is then analyzed for methylation status. PCR primers were designed using Sequenom® *EpiDESIGNER™* (www.epidesigner.com). A total of 261 amplicons, covering 85 target regions, were used for validation (median amplification length = 367 bp, min = 108, max = 500; median number of CpG's per amplicon =23, min = 4, max = 65). For each reverse primer, an additional T7 promoter tag for *in-vivo* transcription was added, as well as a 10mer tag on the forward primer to adjust for melting temperature differences. The MassCLEAVE(tm) biochemistry was performed as previously described (Ehrich M, et al. (2005) Quantitative high-throughput analysis of DNA methylation patterns by base specific cleavage and mass spectrometry. Proc Natl Acad Sci U S A 102:15785-15790). Mass spectra were acquired using a MassARRAY™ Compact MALDI-TOF (Sequenom®, San Diego) and methylation ratios were generated by the EpiTYPER™ software v1.0 (Sequenom®, San Diego).

### Statistical analysis

All statistical calculations were performed using the R statistical software package (www.r-project.org). First, the array probes were grouped based on their genomic location. Subsequent probes that were less than 1000 bp apart were grouped together. To identify differentially methylated regions, a control sample was used as reference. In the control sample, the methylated fraction of a blood derived control DNA was hybridized against itself. Ideally this sample should show log ratios of the two color channels around 0. However because of the variability in hybridization behavior, the probes show a mean log ratio of 0.02 and a standard deviation of 0.18. Next the log ratios observed in our samples were compared to the control sample. A two way, paired t-test was used to test the NULL hypothesis that the groups are identical. Groups that contained less than 4 probes were excluded from the analysis. For groups including four or five probes, all probes were used in a paired t-test. For Groups with six or more probes, a sliding window test consisting of five probes at a time was used, whereby the window was moved by one probe increments. Each test sample was compared to the control sample and the p-values were recorded. Genomic regions were selected as being differentially methylated if eight out of ten samples showed a p value < 0.01, or if six out of ten samples showed a p value < 0.001. The genomic regions were classified as being not differentially methylated when the group showed less than eight samples with a p value < 0.01 and less than six samples with a p value < 0.001. Samples that didn't fall in either category were excluded from the analysis. For a subset of genomic regions that have been identified as differentially methylated, the results were confirmed using quantitative methylation analysis.
The Go analysis was performed using the online GOstat tool (http://gostat.wehi.edu.au/cgibin/-goStat.pl). P values were calculated using Fisher's exact test.

### Microarray-based marker discovery results

To identify differentially methylated regions a standard sample was used, in which the methylated DNA fraction of monocytes was hybridized against itself. This standard provided a reference for the variability of fluorescent measurements in a genomic region. Differentially methylated regions were then identified by comparing the log ratios of each of the ten placental/maternal samples against this standard. Because the goal of this study was to identify markers that allow the reliable separation of maternal and fetal DNA, the target selection was limited to genes that showed a stable, consistent methylation difference over a contiguous stretch of genomic DNA. This focused the analysis on genomic regions where multiple probes indicated differential methylation. The selection was also limited to target regions where all samples showed differential methylation, excluding those with strong inter-individual differences. Two of the samples showed generally lower log ratios in the microarray analysis. Because a paired test was used for target selection, this did not negatively impact the results.
Based on these selection criteria, 3043 genomic regions were identified that were differentially methylated between maternal and fetal DNA. 21778 regions did not show a methylation difference. No interchromosomal bias in the distribution of differentially methylated regions was observed. The differentially methylated regions were located next to or within 2159 known genes. The majority of differentially methylated regions are located in the promoter area (18%) and inside the coding region (68%), while only few regions are located downstream of the gene (7%) or at the transition from promoter to coding region (7%). Regions that showed no differential methylation showed a similar distribution for promoter (13%) and downstream (5%) locations, but the fraction of regions located in the transition of promoter to coding region was higher (39%) and the fraction inside the coding region was lower (43%).

It has been shown in embryonic stem cells (ES) that genes targeted by the polycomb repressive complex2 (PRC2) are enriched for genes regulating development (Lee Tl, et al. (2006) Control of developmental regulators by Polycomb in human embryonic stem cells. Cell 125:301-313). It has also been shown that differentially methylated genes are enriched for genes targeted by PRC2 in many cancer types (Ehrich M, et al. (2008) Cytosine methylation profiling of cancer cell lines. Proc Natl Acad Sci U S A 105:4844-48). The set of genes identified as differentially methylated in this study is also enriched for genes targeted by PRC2 (p-value < 0.001, odds ratio = 3.6, 95% Cl for odds ratio= 3.1 - 4.2). A GO analysis of the set of differentially methylated genes reveals that this set is significantly enriched for functions important during development. Six out of the ten most enriched functions include developmental or morphogenic processes [anatomical structure morphogenesis (GO:0009653, p value =0), developmental process (GO:0032502, p value = 0), multicellular organismal development (GO:0007275, p value = 0), developmental of an organ (GO:0048513, p value = 0), system development (GO:0048731, p value = 0) and development of an anatomical structure (GO:0048856, p value = 0)].

### Validation using Sequenom® EpiTYPER™

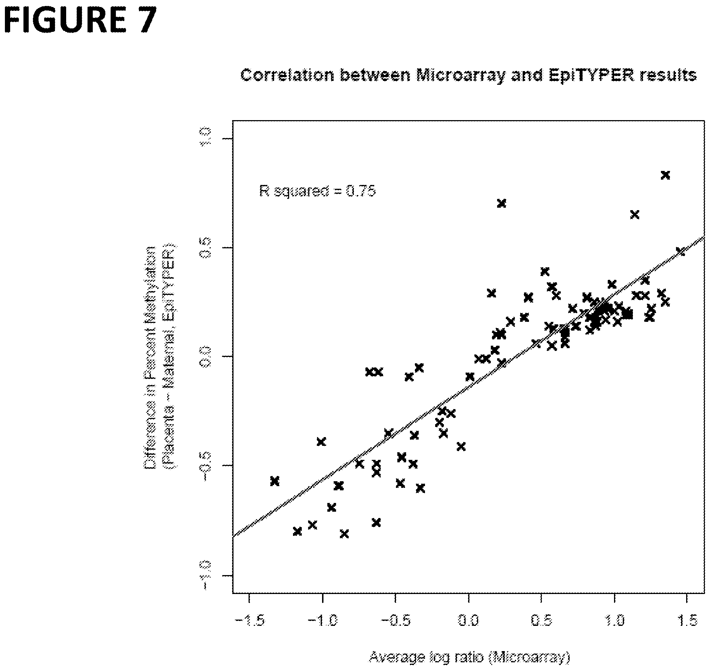

To validate the microarray findings, 63 regions from chromosomes 13, 18 and 21 and an additional 26 regions from other autosomes were selected for confirmation by a different technology. Sequenom EpiTYPER™ technology was used to quantitatively measure DNA methylation in maternal and placental samples. For an explanation of the EpiTYPER™ methods, see Ehrich M, Nelson MR, Stanssens P, Zabeau M, Liloglou T, Xinarianos G, Cantor CR, Field JK, van den Boom D (2005) Quantitative high-throughput analysis of DNA methylation patterns by base specific cleavage and mass spectrometry. Proc Natl Acad Sci U S A 102:15785-15790). For each individual CpG site in a target region the average methylation value across all maternal DNA samples and across all placenta samples was calculated. The difference between average maternal and placenta methylation was then compared to the microarray results. The results from the two technologies were in good concordance (see Figure7). For 85 target regions the quantitative results confirm the microarray results (95% confirmation rate). For 4 target regions, all located on chromosome 18, the results could not be confirmed. The reason for this discrepancy is currently unclear.

In contrast to microarrays, which focus on identification of methylation differences, the quantitative measurement of DNA methylation allowed analysis of absolute methylation values. In the validation set of 85 confirmed differentially methylated regions, a subset of 26 regions is more methylated in the maternal DNA sample and 59 regions are more methylated in the placental sample (see Table 1). Interestingly, genes that are hypomethylated in the placental samples tend to show larger methylation differences than genes that are hypermethylated in the placental sample (median methylation difference for hypomethylated genes = 39%, for hypermethylated genes = 20%).

### Example 2

Example 2 describes a non-invasive approach for detecting the amount of fetal nucleic acid present in a maternal sample (herein referred to as the "Fetal Quantifier Method"), which may be used to detect or confirm fetal traits (e.g., fetal sex of RhD compatibility), or diagnose chromosomal abnormalities such as Trisomy 21 (both of which are herein referred to as the "Methylation-Based Fetal Diagnostic Method"). Figure 10 shows one embodiment of the Fetal Quantifier Method, and Figure 11 shows one embodiment of the Methylation-Based Fetal Diagnostic Method. Both processes use fetal DNA obtained from a maternal sample. The sample comprises maternal and fetal nucleic acid that is differentially methylated. For example, the sample may be maternal plasma or serum. Fetal DNA comprises approximately 2-30% of the total DNA in maternal plasma. The actual amount of fetal contribution to the total nucleic acid present in a sample varies from pregnancy to pregnancy and can change based on a number of factors, including, but not limited to, gestational age, the mother's health and the fetus' health.

As described herein, the technical challenge posed by analysis of fetal DNA in maternal plasma lies in the need to be able to discriminate the fetal DNA from the co-existing background maternal DNA. The methods of the present technology exploit such differences, for example, the differential methylation that is observed between fetal and maternal DNA, as a means to enrich for the relatively small percentage of fetal DNA present in a sample from the mother. The non-invasive nature of the approach provides a major advantage over conventional methods of prenatal diagnosis such as, amniocentesis, chronic villus sampling and cordocentesis, which are associated with a small but finite risk of fetal loss. Also, because the method is not dependent on fetal cells being in any particular cell phase, the method provides a rapid detection means to determine the presence and also the nature of the chromosomal abnormality. Further, the approach is sex-independent (i.e., does not require the presence of a Y-chromosome) and polymorphic-independent (i.e., an allelic ratio is not determined). Thus, the compositions and methods of the technology represent improved universal, noninvasive approaches for accurately determining the amount of fetal nucleic acid present in a maternal sample.

### Assay design and advantages

There is a need for accurate detection and quantification of fetal DNA isolated noninvasively from a maternal sample. The present technology takes advantage of the presence of circulating, cell free fetal nucleic acid (ccfDNA) in maternal plasma or serum. In order to be commercially and clinically practical, the methods of the technology should only consume a small portion of the limited available fetal DNA. For example, less than 50%, 40%, 30%, 25%, 20%, 15%, 10%, 5% or less of the sample. Further, the approach should preferably be developed in a multiplex assay format in which one or more (preferably all) of the following assays are included:
- Assays for the detection of total amount of genomic equivalents present in the sample, i.e., assays recognizing both maternal and fetal DNA species;
- Assays for the detection of fetal DNA isolated from a male pregnancy, i.e., sequences specific for chromosome Y;
- Assays specific for regions identified as differentially methylated between the fetus and mother; or
- Assays specific for regions known to be hypomethylated in all tissues to be investigated, which can serve as a control for restriction efficiency.

Other features of the assay may include one or more of the following:
- For each assay, a target-specific, competitor oligonucleotide that is identical, or substantially identical, to the target sequence apart from a distinguishable feature of the competitor, such as a difference in one or more nucleotides relative to the target sequence. This oligonucleotide when added into the PCR reaction will be co-amplified with the target and a ratio obtained between these two PCR amplicons will indicate the number of target specific DNA sequences (e.g., fetal DNA from a specific locus) present in the maternal sample.
- The amplicon lengths should preferably be of similar length in order not to skew the amplification towards the shorter fragments. However, as long as the amplification efficiency is about equal, different lengths may be used.
- Differentially methylated targets can be selected from Table 1 or from any other targets known to be differentially methylated between mother and fetus. These targets can be hypomethylated in DNA isolated from non-pregnant women and hypermethylated in samples obtained from fetal samples. These assays will serve as controls for the restriction efficiency.
- The results obtained from the different assays can be used to quantify one or more of the following:
   o Total number of amplifiable genomes present in the sample (total amount of genomic equivalents);
   o The fetal fraction of the amplifiable genomes (fetal concentration or percentage); or
   o Differences in copy number between fetally-derived DNA sequences (for example, between fetal chromosome 21 and a reference chromosome such as chromosome 3).

### Examples of assays used in the test

Below is an outline of the reaction steps used to perform a method of the technology, for example, as provided in Figure 10. This outline is not intended to limit the scope of the technology. Rather it provides one embodiment of the technology using the Sequenom® MassARRAY® technology.
1) DNA isolation from plasma samples.
2) Digestion of the DNA targets using methylation sensitive restriction enzymes (for example, Hhal and Hpall).
   For each reaction the available DNA was mixed with water to a final volume of 25 ul.
   10 ul of a reaction mix consisting of 10 units Hhal, 10 units Hpall and a reaction buffer were added. The sample was incubated at an optimal temperature for the restriction enzymes. Hhal and Hpall digest non-methylated DNA (and will not digest hemi- or completely methylated DNA). Following digestion, the enzymes were denatured using a heating step.
3) Genomic Amplification- PCR was performed in a total volume of 50 ul by adding PCR reagents (Buffer, dNTPs, primers and polymerase). Exemplary PCR and extend primers are provided below. In addition, synthetic competitor oligonucleotide was added at known concentrations.
4) Replicates (optional) - Following PCR the 50 ul reaction was split into 5 ul parallel reactions (replicates) in order to minimize variation introduced during the post PCR steps of the test. Post PCR steps include SAP, primer extension (MassEXTEND® technology), resin treatment, dispensing of spectrochip and MassARRAY.
5) Quantification of the Amplifiable Genomes - Sequenom MassARRAY® technology was used to determine the amount of amplification product for each assay. Following PCR, a single base extension assay was used to interrogate the amplified regions (including the competitor oligonucleotides introduced in step 3). Specific extend primers designed to hybridize directly adjacent to the site of interest were introduced. See extend primers provided below. These DNA oligonucleotides are referred to as iPLEX® MassEXTEND® primers. In the extension reaction, the iPLEX primers were hybridized to the complementary DNA templates and extended with a DNA polymerase. Special termination mixtures that contain different combinations of deoxy- and dideoxynucleotide triphosphates along with enzyme and buffer, directed limited extension of the iPLEX primers. Primer extension occurs until a complementary dideoxynucleotide is incorporated.
   The extension reaction generated primer products of varying length, each with a unique molecular weight. As a result, the primer extension products can be simultaneously separated and detected using Matrix Assisted Laser Desorption/Ionization, Time-Of-Flight (MALDI-TOF) mass spectrometry on the MassARRAY® Analyzer Compact. Following this separation and detection, SEQUENOM's proprietary software automatically analyzes the data.
6) Calculating the amount and concentration of fetal nucleic acid - Methods for calculating the total amount of genomic equivalents present in the sample, the amount (and concentration) of fetal nucleic acid isolated from a male pregnancy, and the amount (and concentration) of fetal nucleic based on differentially methylated targets are provided below and in Figures 18 and 19.

The above protocol can be used to perform one or more of the assays described below. In addition to the sequences provided immediately below, a multiplex scheme that interrogates multiple is provided in Table X below.

### 1) Assay for the quantification of the total number of amplifiable genomic equivalents in the sample.

Targets were selected in housekeeping genes not located on the chromosomes 13, 18, 21, X or Y. The targets should be in a single copy gene and not contain any recognition sites for the methylation sensitive restriction enzymes.

Underlined sequences are PCR primer sites, italic is the site for the single base extend primer and bold letter (C) is the nucleotide extended on human DNA
ApoE Chromosome 19:45409835-45409922 DNA target sequence with interrogated nucleotide C in bold. All of the chromosome positions provided in this section are from the February 2009 UCSC Genome Build. 
GATTGACAGTTTCTCCTTCCCCAGACTGGCCAATCACAGGCAGGAAGATGAAGGTTCTGTGGGCTGCGTTGCTGG
TCACATTCCTGGC
ApoE Forward Primer: 5'-ACGTTGGATG-TTGACAGTTTCTCCTTCCCC (Primer contains a 5' 10 bp MassTag separated by a dash)
ApoE Reverse Primer: 5'-ACGTTGGATG-GAATGTGACCAGCAACGCAG (Primer contains a 5' 10 bp MassTag separated by a dash)
ApoE Extension Primer: 5'-GCAGGAAGATGAAGGTT [C/T] Primer extends C on human DNA targets and T on synthetic DNA targets
ApoE synthetic competitor oligonucleotide: 5'-GATTGACAGTTTCTCCTTCCCCAGACTGGCCAATCACAGGCAGGAAGATGAAGGTTTTGTGGGCTGCGTTGCTGG TCACATTCCTGGC (Bold T at position 57 is different from human DNA)

### 2) Assay for the quantification of the total number of chromosome Y sequences in the sample.

Targets specific for the Y-chromosome were selected, with no similar or paralog sequences elsewhere in the genome. The targets should preferably be in a single copy gene and not contain any recognition sites for the methylation sensitive restriction enzyme(s).

Underlined sequences are PCR primer sites, and italic nucleotide(s) is the site for the single-base extend primer and bold letter (C) is the nucleotide extended on human DNA.
SRY on chrY:2655628-2655717 (reverse complement) 
GAGTTTTGGATAGTAAAATAAGTTTCGAACTCTGGCACCTTTCAATTTTGTCGCACTCTCCTTGTTTTTGACAATGC
AATCATATGCTTC
SRY Forward Primer: 5'-ACG-TGGATAGTAAAATAAGTTTCGAACTCTG (Primer contains a 5' 3 bp MassTag separated by a dash)
SRY Reverse Primer: 5'- GAAGCATATGATTGCATTGTCAAAAAC
SRY Extension Primer: 5'-aTTTCAATTTTGTCGCACT [C/T] Primer extends C on human DNA targets and T on synthetic DNA targets. 5' Lower case "a" is a non-complementary nucleotide
SRY synthetic competitor oligonucleotide: 5'-GAGTTTTGGATAGTAAAATAAGTTTCGAACTCTGGCACCTTTCAATTTTGTCGCACTTTCCTTGTTTTTGACAATGC AATCATATGCTTC

### 3) Assay for the quantification of fetal methylated DNA sequences present in the sample.

Targets were selected in regions known to be differentially methylated between maternal and fetal DNA. Sequences were selected to contain several restriction sites for methylation sensitive enzymes. For this study the Hhal (GCGC) and Hpall (CCGG) enzymes were used.

Underlined sequences are PCR primer sites, italic is the site for the single base extend primer and bold letter (C) is the nucleotide extended on human DNA, lower case letter are recognition sites for the methylation sensitive restriction enzymes.
TBX3 on chr12:115124905-115125001 
GAACTCCTCTTTGTCTCTGCGTGCccggcgcgcCCCCCTCccggTGGGTGATAAACCCACTCTGgcgccggCCATgcgcTG
GGTGATTAATTTGCGA
TBX3 Forward Primer: 5'- ACGTTGGATG-TCTTTGTCTCTGCGTGCCC (Primer contains a 5' 10 bp MassTag separated by a dash)
TBX3 Reverse Primer: 5'- ACGTTGGATG-TTAATCACCCAGCGCATGGC (Primer contains a 5' 10 bp MassTag separated by a dash)
TBX3 Extension Primer: 5'- CCCCTCCCGGTGGGTGATAAA [C/T] Primer extends C on human DNA targets and T on synthetic DNA targets. 5' Lower case "a" is a non-complementary nucleotide
TBX3 synthetic competitor oligonucleotide: 5'-GAACTCCTCTTTGTCTCTGCGTGCCCGGCGCGCCCCCCTCCCGGTGGGTGATAAATCCACTCTGGCGCCGGCCATG CGCTGGGTGATTAATTTGCGA

### 4) Control Assay for the enzyme restriction efficiency.

Targets were selected in regions known not to be methylated in any tissue to be investigated. Sequences were selected to contain no more than one site for each restriction enzyme to be used.

Underlined sequences are PCR primer sites, italic nucleotide(s) represent the site for the single-base extend primer and bold letter (G) is the reverse nucleotide extended on human DNA, lower case letter are recognition sites for the methylation sensitive restriction enzymes.
CACNA1G chr17:48637892-48637977 (reverse complement) 
CCATTGGCCGTCCGCCGTGGCAGTGCGGGCGGGAgcgcAGGGAGAGAACCACAGCTGGAATCCGATTCCCACCCC
AAAACCCAGGA
Hhal Forward Primer: 5'- ACGTTGGATG-CCATTGGCCGTCCGCCGTG (Primer contains a 5' 10 bp MassTag separated by a dash)
Hhal Reverse Primer: 5'- ACGTTGGATG-TCCTGGGTTTTGGGGTGGGAA (Primer contains a 5' 10 bp MassTag separated by a dash)
Hhal Extension Primer: 5'- TTCCAGCTGTGGTTCTCTC
Hhal synthetic competitor oligonucleotide: 5'-CCATTGGCCGTCCGCCGTGGCAGTGCGGGCGGGAGCGCAG**A***GAGAGAACCACAGCTGGAAT*CCGATTCCCACCC CAAAACCCAGGA

### Validation experiments

The sensitivity and accuracy of the present technology was measured using both a model system and clinical samples. In the different samples, a multiplex assay was run that contains 2 assays for total copy number quantification, 3 assays for methylation quantification, 1 assay specific for chromosome Y and 1 digestion control assay. See Tables X1 and X2. Another multiplex scheme with additional assays is provided in Tables Y1 and Y2.

**TABLE X1: PCR Primers and Extend Primers**

| Gene ID | * | First Primer | Second Primer | Extend Primer |
|---|---|---|---|---|
| SOX14 | M | ACGTTGGATGACATGGTCGGCCCCACGGAAT | ACGTTGGATGCTCCTTCCTAGTGTGAGAACCG | CAGGTTCCGGGGCTTGGG |
| Hhal_CTRL | D | ACGTTGGATGACCCATTGGCCGTCCGCCGT | ACGTTGGATGTTTTGGGGTGGGAATCGGATT | CGCAGGGAGAGAACCACAG |
| TBX3 | M | ACGTTGGATGGAACTCCTCTTTGTCTCTGCG | ACGTTGGATGTGGCATGGCCGGCGCCAGA | CCCCTCCCGGTGGGTGATAAA |
| SRY | Y | ACGTTGGATGCGCAGCAACGGGACCGCTACA | ACGTTGGCATCTAGGTAGGTCTTTGTAGCCAA | AAAGCTGTAGGACAATCGGGT |
| ALB | T | ACGTTGCGTAGCAACCTGTTACATATTAA | ACGTTGGATCTGAGCAAAGGCAATCAACACCC | CATTTTTCTACATCCTTTGTTT |
| EDG6 | M | ACGTTGGATGCATAGAGGCCCATGATGGTGG | ACGTTGGATGACCTTCTGCCCCTCTACTCCAA | agAAGATCACCAGGCAGAAGAGG |
| RNaseP | T | ACGTTGGATGGTGTGGTCAGCTCTTCCCTTCAT | ACGTTGGCCCACATGTAATGTGTTGAAAAAGCA | ACTTGGAGAACAAAGGACACCGTTA |

**TABLE X2: Competitor Oligonucleotide Sequence**

| Gene ID | * | Competitor Oligonucleotide Sequence |
|---|---|---|
| SOX14 | M | GGTCGGCCCCACGGAA TCCCGGCTCTGTGTGCGCCCAGGTTCCGGGGCTTGGGTGTTGCCGGTTCTCACACT AGGAAGGAG |
| Hhal_CTRL | D | CCATTGGCCGTCCGCCGTGGCAGTGCGGGCGGGAGCGCAGAGAGAGAACCACAGCTGGAATCCGATTCCCACCCCAAAA |
| TBX3 | M | GAACTCCTCTTTGTCTCTGCGTGCCCGGCGCGCCCCCCTCCCGGTGGGTGATAAATCCACTCTGGCGCCGGCCATGC |
| SRY | Y | GCAGCAACGGGACCGCTACAGCCACTGGACAAAGCCGTAGGACAATCGGGTAACATTGGCTACAAAGACCTACCTAGATGC |
| ALB | T | GCGTAGCAACCTGTTACATATTAAAGTTTTATTATACTACATTTTTCTACATCCTTTGTTTCAGAGTGTTGATTGCCTTTGCTCAGTATCTTCAG |
| EDG6 | M | CCTTCTGCCCCTCTACTCCAAGCGCTACACCCTCTTCTGCCTGGTGATCTTTGCCGGCGTCCTGGCCACCATCATGGGCCTCTATG |
| RNaseP | T | GTGTGGTCAGCTCTTCCCTTCATCACATACTTGGAGAACAAAGGACACCGTTATCCATGCTTTTTCAACACATTACATGTGGG |

**TABLE Y1: PCR Primers and Extend Primers**

| Gene ID | * | First Primer | Second Primer | Extend Primer |
|---|---|---|---|---|
| EDG6 | M | ACGTTGGATGTTCTGCCCCTCTACTCCAAG | ACGTTGGATGCATAGAGGCCCATGATGGTG | TTCTGCCTGGTGATCTT |
| RNAseP | T | ACGTTGGATGTCAGCTCTTCCCTTCATCAC | ACGTTGGATGCCTACCTCCCACATGTAATGT | AACAAAGGACACCGTTA |
| ApoE | T | ACGTTGGATGTTGACAGTTTCTCCTTCCCC | ACGTTGGATGGAATGTGACCAGCAACGCAG | GCAGGAAGATGAAGGTT |
| SOX14 | M | ACGTTGGATGCGGTCGGCCCCACGGAAT | ACGTTGGATGCTCCTTCCTAGTGTGAGAACCG | aAGGTTCCGGGGCTTGGG |
| SRY no2 | Y | ACGTGGATAGTAAAATAAGTTTCGAACTCTG | GAAGCATATGATTGCATTGTCAAAAAC | aTTTCAATTTTGTCGCACT |
| SRY no1 | Y | ACGTTGGATGCACAGCTCACCGCAGCAACG | ACGTTGGATGCTAGGTAGGTCTTTGTAGCCAA | AGCTGTAGGACAATCGGGT |
| TBX3 | M | ACGTTGGATGTCTTTGTCTCTGCGTGCCC | ACGTTGGATGTTAATCACCCAGCGCATGGC | CCCTCCCGGTGGGTGATAAA |
| CACNA1G dig CTRL 1 | D | ACGTTGGATGGACTGAGCCCCAGAACTCG | ACGTTGGATGGTGGGTTTGTGCTTTCCACG | AGGGCCGGGGTCTGCGCGTG |
| DAPK1 dig CTRL 2 | D | ACGTTGGATGAAGCCAAGTTTCCCTCCGC | ACGTTGGATGCTTTTGCTTTCCCAGCCAGG | GAGGCACTGCCCGGACAAACC |
| ALB | T | ACGTTAGCGTAGCAACCTGTTACATATTAA | ACGTTGGATGCTGAGCAAAGGCAATCAACA | CATTTTTCTACATCCTTTGTTT |

**TABLE Y2: Competitor Oligonucleotide Sequence**

| Gene ID | * | Competitor |
|---|---|---|
| EDG6 | M | CCTTCTGCCCCTCTACTCCAAGCGCTACACCCTCTTCTGCCTGGTGATCTTTGCCGGCGTCCTGGCCACCATCATGGGCCTCTATG |
| RNAseP | T | GTGTGGTCAGCTCTTCCCTTCATCACATACTTGGAGAACAAAGGACACCGTTATCCATGCTTTTTCAACACATTACATGTGGGAGGTAGG |
| ApoE | T | GATTGACAGTTTCTCCTTCCCCAGACTGGCCAATCACAGGCAGGAAGATGAAGGTTTTGTGGGCTGCGTTGCTGGTCACATTCCTGGC |
| SOX14 | M | AAAACCAGAGATTCGCGGTCGGCCCCACGGAATCCCGGCTCTGTGTGCGCCCAGGTTCCGGGGCTTGGGTGTTGCCGGTTCTCACACTAGGAAGG AGC |
| SRY no2 | Y | GAGTTTTGGATAGTAAAATAAGTTTCGAACTCTGGCACCTTTCAATTTTGTCGCACTTTCCTTGTTTTTGACAATGCAATCATATGCTTC |
| SRY no1 | Y | GCAGCCAGCTCACCGCAGCAACGGGACCGCTACAGCCACTGGACAAAGCTGTAGGACAATCGGGTGACATTGGCTACAAAGACCTACCTAGATGC |
| TBX3 | M | GAACTCCTCTTTGTCTCTGCGTGCCCGGCGCGCCCCCCTCCCGGTGGGTGATAAATCCACTCTGGCGCCGGCCATGCGCTGGGTGATTAATTTGCG A |
| CACNA1G dig CTRL 1 | D | GTGGGTTTGTGCTTTCCACGCGTGCACACACACGCGCAGACCCCGGCCCTTGCCCCGCCTACCTCCCCGAGTTCTGGGGCTCAGTC |
| DAPK1 dig CTRL 2 | D | GCGCCAGCTTTTGCTTTCCCAGCCAGGGCGCGGTGAGGTTTGTCCGGGCAGTGCCTCGAGCAACTGGGAAGGCCAAGGCGGAGGGAAAC |
| ALB | T | GCGTAGCAACCTGTTACATATTAAAGTTTTATTATACTACATTTTTCTACATCCTTTGTTTTAGGGTGTTGATTGCCTTTGCTCAGTATCTTCAGC |

| | | |
|---|---|---|
| T=Assay for Total Amount; M=Assay for Methylation quantification; Y= Y-Chromosome Specific Assay; D=Digestion control | | |

### Model system using genomic DNA

In order to determine the sensitivity and accuracy of the method when determining the total number of amplifiable genomic copies in a sample, a subset of different DNA samples isolated from the blood of non-pregnant women was tested. Each sample was diluted to contain approximately 2500, 1250, 625 or 313 copies per reaction. The total number of amplifiable genomic copies was obtained by taking the mean DNA/competitor ratio obtained from the three total copy number assays. The results from the four different samples are shown in Figure 12.

To optimize the reaction, a model system was developed to simulate DNA samples isolated from plasma. These samples contained a constant number of maternal non-methylated DNA and were spiked with different amounts of male placental methylated DNA. The samples were spiked with amounts ranging from approximately 0 to 25% relative to the maternal non-methylated DNA. The results are shown in Figures 13A and B. The fraction of placental DNA was calculated using the ratios obtained from the methylation assays (Figure 13A), the SRY markers (Figure 13B) and the total copy number assays. The primer sequences for the methylation assays (TBX), Y-chromosome assays (SRY) and total copy number (APOE) are provided above. The model system demonstrated that the methylation-based method performed equal to the Y-chromosome method (SRY markers), thus validating the methylation-based method as a sex-independent fetal quantifier.

### Plasma samples

To investigate the sensitivity and accuracy of the methods in clinical samples, 33 plasma samples obtained from women pregnant with a male fetus were investigated using the multiplex scheme from Table X. For each reaction, a quarter of the DNA obtained from a 4ml extraction was used in order to meet the important requirement that only a portion of the total sample is used.

### Total copy number quantification

The results from the total copy number quantification can be seen in Figures 14A and B. In Figure 14A, the copy number for each sample is shown. Two samples (nos. 25 and 26) have a significantly higher total copy number than all the other samples. In general, a mean of approximately 1300 amplifiable copies/ml plasma was obtained (range 766-2055). Figure 14B shows a box-and-whisker plot of the given values, summarizing the results.

### Correlation between results obtained from the methylation markers and the Y-chromosome marker

In Figures 15A and B, the numbers of fetal copies for each sample are plotted. As all samples were from male pregnancies. The copy numbers obtained can be calculated using either the methylation or the Y-chromosome-specific markers. As can be seen in Figure 15B, the box-and-whisker plot of the given values indicated minimal difference between the two different measurements.

The results showing the correlation between results obtained from the methylation markers and the Y-chromosome marker (SRY) is shown in Figure 16. Again, the methylation-based method performed equal to the Y-chromosome method (SRY markers), further validating the methylation-based method as a sex-independent and polymorphism-independent fetal quantifier. The multiplexed assays disclosed in Table X were used to determine the amount fetal nucleic.

Finally, the digestion efficiency was determined by using the ratio of digestion for the control versus the competitor and comparing this value to the mean total copy number assays. See Figure 17. Apart from sample 26 all reactions indicate the efficiency to be above 99%.

### Data Analysis

Mass spectra analysis was done using Typer 4 (a Sequenom software product). The peak height (signal over noise) for each individual DNA analyte and competitor assay was determined and exported for further analysis.

The total number of molecules present for each amplicon was calculated by dividing the DNA specific peak by the competitor specific peak to give a ratio. (The "DNA" Peak in Figures 18 and 19 can be thought of as the analyte peak for a given assay). Since the number of competitor molecules added into the reaction is known, the total number of DNA molecules can be determined by multiplying the ratio by the number of added competitor molecules.

The fetal DNA fraction (or concentration) in each sample was calculated using the Y-chromosome-specific markers for male pregnancies and the mean of the methylated fraction for all pregnancies. In brief, for chromosome Y, the ratio was obtained by dividing the analyte (DNA) peak by the competitor peak and multiplying this ratio by the number of competitor molecules added into the reaction. This value was divided by a similar ratio obtained from the total number of amplifiable genome equivalents determination (using the Assay(s) for Total Amount). See Figure 18. Since the total amount of nucleic acid present in a sample is a sum of maternal and fetal nucleic acid, the fetal contribution can be considered to be a fraction of the larger, background maternal contribution. Therefore, translating this into the equation shown in Figure 18, the fetal fraction (k) of the total nucleic acid present in the sample is equal to the equation: k=2xR/(1-2R), where R is the ratio between the Y-chromosome amount and the total amount. Since the Y-chromosome is haploid and Assays for the Total Amount are determined using diploid targets, this calculation is limited to a fetal fraction smaller than 50% of the maternal fraction.

In Figure 19, a similar calculation for the fetal concentration is shown by using the methylation specific markers (see Assays for Methylation Quantification). In contrast to Y-chromosome specific markers, these markers are from diploid targets, therefore, the limitations stated for the Y-Chromosome Specific Assay can be omitted. Thus, the fetal fraction (k) can be determined using the equation: k=R(1-R), where R is the ratio between the methylation assay and the total assay.

### Simulation

A first simple power calculation was performed that assumes a measurement system that uses 20 markers from chromosome 21, and 20 markers from one or more other autosomes. Starting with 100 copies of fetal DNA, a measurement standard deviation of 25 copies and the probability for a type I error to be lower than 0.001, it was found that the methods of the technology will be able to differentiate a diploid from a triploid chromosome set in 99.5% of all cases. The practical implementation of such an approach could for example be achieved using mass spectrometry, a system that uses a competitive PCR approach for absolute copy number measurements. The method can run 20 assays in a single reaction and has been shown to have a standard deviation in repeated measurements of around 3 to 5%. This method was used in combination with known methods for differentiating methylated and non-methylated nucleic acid, for example, using methyl-binding agents to separate nucleic acid or using methylation-sensitive enzymes to digest maternal nucleic acid. Figure 8 shows the effectiveness of MBD-FC protein (a methyl-binding agent) for capturing and thereby separating methylated DNA in the presence of an excess of unmethylated DNA (see Figure 8).

A second statistical power analysis was performed to assess the predictive power of an embodiment of the Methylation-Based Fetal Diagnostic Method described herein. The simulation was designed to demonstrate the likelihood of differentiating a group of trisomic chromosome 21 specific markers from a group of reference markers (for example, autosomes excluding chromosome 21). Many parameters influence the ability to discriminate the two populations of markers reliably. For the present simulation, values were chosen for each parameter that have been shown to be the most likely to occur based on experimentation. The following parameters and respective values were used:
Copy Numbers
   Maternal copy numbers = 2000
   Fetal copy numbers for chromosomes other than 21, X and Y = 200
   Fetal copy numbers for chromosome 21 in case of euploid fetus = 200
   Fetal copy numbers for chromosome 21 in case of aneuploid T21 fetus = 300
Percent fetal DNA (before methylation-based enrichment) = 10% (see above)
Methylation Frequency
   Average methylation percentage in a target region for maternal DNA = 10%
   Average methylation percentage in a target region for fetal DNA = 80%
Average percentage of non-methylated and non-digested maternal DNA (i.e., a function of restriction efficiency (among other things) = 5%
Number of assays targeting chromosome 21 = 10
Number of assays targeting chromosomes other than 21, X and Y = 10

The results are displayed in Figure 20. Shown is the relationship between the coefficient of variation (CV) on the x-axis and the power to discriminate the assay populations using a simple t-test (y-axis). The data indicates that in 99% of all cases, one can discriminate the two population (euploid vs. aneuploid) on a significance level of 0.001 provided a CV of 5% or less.. Based on this simulation, the method represents a powerful noninvasive diagnostic method for the prenatal detection of fetal aneuploidy that is sex-independent and will work in all ethnicities (i.e., no allelic bias).

**TABLE 1**

| **GENE NAME** | **CHROM** | **START** | **END** | **CpG ISLAND** | **MEAN LOG RATIO MICRO-ARRAY** | **MEAN MATERNAL METHYLATION EPITYPER** | **MEAN PLACENTA METHYLATION EPITYPER** | **METHYLATION DIFFERENCE PLACENTA-MATERNAL** | **RELATIVE METHYLATION PLACENTA TO MATERNAL** |
|---|---|---|---|---|---|---|---|---|---|
| chr13 group00016 | chr13 | 19773745 | 19774050 | chr13: 19773518-19774214 | 0.19 | 0.22 | 0.32 | 0.1 | HYPERMETHYLATION |
| chr13 group00005 | chr13 | 19290394 | 19290768 | :- | -0.89 | 0.94 | 0.35 | -0.59 | HYPOM ETHYLATION |
| CRYL1 | chr13 | 19887090 | 19887336 | chr13: 19887007-19887836 | -0.63 | 0.74 | 0.21 | -0.53 | HYPOM ETHYLATION |
| IL17D | chr13 | 20193675 | 20193897 | chr13:20193611-20194438 | -1.01 | 0.53 | 0.13 | -0.39 | HYPOM ETHYLATION |
| CENPJ | chr13 | 24404023 | 24404359 | :- | 0.57 | 0.17 | 0.49 | 0.32 | HYPERMETHYLATION |
| ATP8A2 | chr13 | 25484475 | 25484614 | chr13:25484287-25484761 | 0.81 | 0.16 | 0.43 | 0.27 | HYPERMETHYLATION |
| GSH1 | chr13 | 27265542 | 27265834 | chr13:27264549-27266505 | 0.57 | 0.13 | 0.19 | 0.05 | HYPERMETHYLATION |
| PDX1 | chr13 | 27393789 | 27393979 | chr13:27392001-27394099 | 0.55 | 0.06 | 0.2 | 0.14 | HYPERMETHYLATION |
| PDX1 | chr13 | 27400459 | 27401165 | chr13:27400362-27400744; chr13:27401057-27401374 | 0.73 | 0.12 | 0.26 | 0.14 | HYPERMETHYLATION |
| MAB21 L1 | chr13 | 34947737 | 34948062 | chr13:34947570-34948159 | 0.66 | 0.11 | 0.17 | 0.06 | HYPERMETHYLATION |
| RB1 | chr13 | 47790983 | 47791646 | chr13:47790636-47791858 | 0.18 | 0.45 | 0.48 | 0.03 | HYPERMETHYLATION |
| PCDH17 | chr13 | 57104856 | 57106841 | chr13:57104527-57106931 | 0.46 | 0.15 | 0.21 | 0.06 | HYPERMETHYLATION |
| KLHL1 | chr13 | 69579933 | 69580146 | chr13:69579733-69580220 | 0.79 | 0.09 | 0.28 | 0.2 | HYPERMETHYLATION |
| POU4F1 | chr13 | 78079515 | 78081073 | chr13:78079328-78079615; chr13:78080860-78081881 | 0.66 | 0.12 | 0.23 | 0.11 | HYPERMETHYLATION |
| GPC6 | chr13 | 92677402 | 92678666 | chr13:92677246-92678878 | 0.66 | 0.06 | 0.19 | 0.13 | HYPERMETHYLATION |
| SOX21 | chr13 | 94152286 | 94153047 | chr13: 94152190-94153185 | 0.94 | 0.16 | 0.4 | 0.25 | HYPERMETHYLATION |
| ZIC2 | chr13 | 99439660 | 99440858 | chr13:99439335-99440189; chr13:99440775-99441095 | 0.89 | 0.13 | 0.35 | 0.22 | HYPERMETHYLATION |
| IRS2 | chr13 | 109232856 | 109235065 | chr13: 109232467-109238181 | -0.17 | 0.73 | 0.38 | -0.35 | HYPOM ETHYLATION |
| chr13 group00350 | chr13 | 109716455 | 109716604 | chr13: 109716325-109716726 | -0.37 | 0.77 | 0.41 | -0.36 | HYPOM ETHYLATION |
| chr13 group00385 | chr13 | 111595578 | 111595955 | chr13: 111595459-111596131 | 0.87 | 0.06 | 0.2 | 0.14 | HYPERMETHYLATION |
| chr13 group00390 | chr13 | 111756337 | 111756593 | chr13: 111755805-111756697 | 0.71 | 0.12 | 0.34 | 0.22 | HYPERMETHYLATION |
| chr13 group00391 | chr13 | 111759856 | 111760045 | chr13: 111757885-111760666 | 0.86 | 0.11 | 0.36 | 0.25 | HYPERMETHYLATION |
| chr13 group00395 | chr13 | 111808255 | 111808962 | chr13: 111806599-111808492; chr13: 111808866-111809114 | 0.96 | 0.13 | 0.35 | 0.22 | HYPERMETHYLATION |
| chr13 group00399 | chr13 | 112033503 | 112033685 | chr13: 112032967-112033734 | 0.38 | 0.26 | 0.43 | 0.18 | HYPERMETHYLATION |
| MCF2L | chr13 | 112724910 | 112725742 | chr13: 112724782-112725121 ; chr13: 112725628-112725837 | -0.47 | 0.91 | 0.33 | -0.58 | HYPOM ETHYLATION |
| F7 | chr13 | 112799123 | 112799379 | chr13: 112798487-112799566 | -0.05 | 0.97 | 0.55 | -0.41 | HYPOM ETHYLATION |
| PROZ | chr13 | 112855566 | 112855745 | chr13: 112855289-112855866 | 0.29 | 0.15 | 0.3 | 0.16 | HYPERMETHYLATION |
| chr18 group00039 | chr18 | 6919797 | 6919981 | chr18:6919450-6920088 | -0.38 | 0.88 | 0.39 | -0.49 | HYPOM ETHYLATION |
| CIDEA | chr18 | 12244327 | 12244696 | chr18:12244147-12245089 | 0.23 | 0.14 | 0.23 | 0.1 | HYPERMETHYLATION |
| chr18 group00091 | chr18 | 12901467 | 12901643 | chr18:12901024-12902704 | 0.16 | 0.15 | 0.43 | 0.29 | HYPERMETHYLATION |
| chr18 group00094 | chr18 | 13126819 | 13126986 | chr18:13126596-13127564 | 0.41 | 0.07 | 0.34 | 0.27 | HYPERMETHYLATION |
| C18orf1 | chr18 | 13377536 | 13377654 | chr18:13377385-13377686 | -0.12 | 0.95 | 0.69 | -0.26 | HYPOM ETHYLATION |
| KLHL14 | chr18 | 28603978 | 28605183 | chr18:28603688-28606300 | 0.83 | 0.07 | 0.19 | 0.12 | HYPERMETHYLATION |
| CD33L3 | chr18 | 41671477 | 41673011 | chr18:41671386-41673101 | -0.34 | 0.49 | 0.44 | -0.05 | HYPOM ETHYLATION |
| ST8SIA3 | chr18 | 53171265 | 53171309 | chr18:53170705-53172603 | 1.02 | 0.09 | 0.25 | 0.16 | HYPERMETHYLATION |
| ONECUT2 | chr18 | 53254808 | 53259810 | chr18:53254152-53259851 | 0.74 | 0.09 | 0.23 | 0.14 | HYPERMETHYLATION |
| RAX | chr18 | 55086286 | 55086436 | chr18:55085813-55087807 | 0.88 | 0.11 | 0.26 | 0.16 | HYPERMETHYLATION |
| chr18 group00277 | chr18 | 57151972 | 57152311 | chr18:57151663-57152672 | 0.58 | 0.08 | 0.21 | 0.13 | HYPERMETHYLATION |
| TNFRSF11A | chr18 | 58203013 | 58203282 | chr18:58202849-58203367 | -0.33 | 0.88 | 0.28 | -0.6 | HYPOMETHYLA TION |
| NETO1 | chr18 | 68685099 | 68687060 | chr18:68684945-68687851 | 0.65 | 0.09 | 0.22 | 0.13 | HYPERMETHYLATION |
| chr18 group00304 | chr18 | 70133945 | 70134397 | chr18:70133732-70134724 | 0.12 | 0.93 | 0.92 | -0.01 | NOT CONFIRMED |
| TSHZ1 | chr18 | 71128742 | 71128974 | chr18:71128638-71129076 | 0.23 | 0.95 | 0.92 | -0.03 | NOT CONFIRMED |
| ZNF236 | chr18 | 72664454 | 72664736 | chr18:72662797-72664893 | -0.62 | 0.17 | 0.1 | -0.07 | HYPOM ETHYLATION |
| MBP | chr18 | 72953150 | 72953464 | chr18:72953137-72953402 | 0.6 | 0.44 | 0.72 | 0.28 | HYPERMETHYLATION |
| chr18 group00342 | chr18 | 74170347 | 74170489 | chr18:74170210-74170687 | -0.2 | 0.78 | 0.48 | -0.3 | HYPOMETHYLATION |
| NFATC1 | chr18 | 75385424 | 75386008 | chr18:75385279-75386532 | 0.23 | 0.14 | 0.84 | 0.7 | HYPERMETHYLATION |
| CTDP1 | chr18 | 75596358 | 75596579 | chr18:75596009-75596899 | 0.07 | 0.97 | 0.96 | -0.01 | NOT CONFIRMED |
| chr18 group00430 | chr18 | 75653272 | 75653621 | :- | 0.52 | 0.24 | 0.62 | 0.39 | HYPERMETHYLATION |
| KCNG2 | chr18 | 75760343 | 75760820 | chr18:75759900-75760988 | 0.01 | 0.84 | 0.75 | -0.09 | NOT CONFIRMED |
| OLIG2 | chr21 | 33317673 | 33321183 | chr21 :33316998-33322115 | 0.66 | 0.11 | 0.2 | 0.09 | HYPERMETHYLATION |
| OLIG2 | chr21 | 33327593 | 33328334 | chr21 :33327447-33328408 | -0.75 | 0.77 | 0.28 | -0.49 | HYPOM ETHYLATION |
| RUNX1 | chr21 | 35180938 | 35185436 | chr21 :35180822-35181342; chr21 :35182320-35185557 | -0.68 | 0.14 | 0.07 | -0.07 | HYPOM ETHYLATION |
| SIM2 | chr21 | 36994965 | 36995298 | chr21 :36990063-36995761 | 0.83 | 0.08 | 0.26 | 0.18 | HYPERMETHYLATION |
| SIM2 | chr21 | 36999025 | 36999410 | chr21 :36998632-36999555 | 0.87 | 0.06 | 0.24 | 0.18 | HYPERMETHYLATION |
| DSCR6 | chr21 | 37300407 | 37300512 | chr21 :37299807-37301307 | 0.22 | 0.04 | 0.14 | 0.11 | HYPERMETHYLATION |
| DSCAM | chr21 | 41135559 | 41135706 | chr21 :41135380-41135816 | 1.03 | 0.06 | 0.29 | 0.23 | HYPERMETHYLATION |
| chr21 group00165 | chr21 | 43643421 | 43643786 | chr21 :43643322-43643874 | 1.14 | 0.16 | 0.81 | 0.65 | HYPERMETHYLATION |
| AIRE | chr21 | 44529935 | 44530388 | chr21 :44529856-44530472 | -0.55 | 0.62 | 0.27 | -0.35 | HYPOM ETHYLATION |
| SUMO3 | chr21 | 45061293 | 45061853 | chr21 :45061154-45063386 | -0.41 | 0.55 | 0.46 | -0.09 | HYPOM ETHYLATION |
| C21orf70 | chr21 | 45202815 | 45202972 | chr21 :45202706-45203073 | -0.46 | 0.96 | 0.51 | -0.46 | HYPOMETHYLATION |
| C21orf123 | chr21 | 45671984 | 45672098 | chr21 :45671933-45672201 | -0.63 | 0.92 | 0.43 | -0.49 | HYPOM ETHYLATION |
| COL18A1 | chr21 | 45754383 | 45754487 | chr21 :45753653-45754639 | -0.18 | 0.97 | 0.72 | -0.25 | HYPOM ETHYLATION |
| PRMT2 | chr21 | 46911967 | 46912385 | chr21 :46911628-46912534 | 1.08 | 0.04 | 0.25 | 0.21 | HYPERMETHYLATION |
| SIX2 | chr2 | 45081223 | 45082129 | chr2:45081148-45082287 | 1.15 | 0.08 | 0.36 | 0.28 | HYPERMETHYLATION |
| SIX2 | chr2 | 45084851 | 45085711 | chr2:45084715-45084986; chr2:45085285-45086054 | 1.21 | 0.07 | 0.35 | 0.28 | HYPERMETHYLATION |
| SOX14 | chr3 | 138971870 | 138972322 | chr3:138971738-138972096; chr3:138972281-138973691 | 1.35 | 0.08 | 0.33 | 0.25 | HYPERMETHYLATION |
| TLX3 | chr5 | 170674439 | 170676431 | chr5:170674208-170675356; chr5:170675783-170676712 | 0.91 | 0.11 | 0.35 | 0.24 | HYPERMETHYLATION |
| FOXP4 | chr6 | 41623666 | 41624114 | chr6:41621630-41624167 | 1.1 | 0.07 | 0.27 | 0.2 | HYPERMETHYLATION |
| FOXP4 | chr6 | 41636384 | 41636779 | chr6:41636244-41636878 | 1.32 | 0.04 | 0.33 | 0.29 | HYPERMETHYLATION |
| chr7 group00267 | chr7 | 12576755 | 12577246 | chr7: 12576690-12577359 | 0.94 | 0.08 | 0.26 | 0.17 | HYPERMETHYLATION |
| NPY | chr7 | 24290224 | 24291508 | chr7:24290083-24291605 | 0.93 | 0.09 | 0.3 | 0.21 | HYPERMETHYLATION |
| SHH | chr7 | 155291537 | 155292091 | chr7:155288453-155292175 | 0.98 | 0.19 | 0.52 | 0.33 | HYPERMETHYLATION |
| OSR2 | chr8 | 100029764 | 100030536 | chr8:100029673-100030614 | 1.21 | 0.08 | 0.43 | 0.35 | HYPERMETHYLATION |
| GLIS3 | chr9 | 4288283 | 4289645 | chr9:4287817 - 4290182 | 1.24 | 0.06 | 0.24 | 0.18 | HYPERMETHYLATION |
| PRMT8 | chr12 | 3472714 | 3473190 | chr12:3470227-3473269 | 0.86 | 0.07 | 0.23 | 0.16 | HYPERMETHYLATION |
| TBX3 | chr12 | 113609153 | 113609453 | chr12:113609112-113609535 | 1.45 | 0.09 | 0.56 | 0.48 | HYPERMETHYLATION |
| chr12 group00801 | chr12 | 118516189 | 118517435 | chr12:118515877-118517595 | 1.1 | 0.06 | 0.25 | 0.19 | HYPERMETHYLATION |
| PAX9 | chr14 | 36201402 | 36202386 | chr14:36200932-36202536 | 0.89 | 0.11 | 0.32 | 0.21 | HYPERMETHYLATION |
| SIX1 | chr14 | 60178801 | 60179346 | chr14:60178707-60179539 | 0.95 | 0.1 | 0.33 | 0.22 | HYPERMETHYLATION |
| ISL2 | chr15 | 74420013 | 74421546 | chr15:74419317-74422570 | 1.08 | 0.08 | 0.27 | 0.19 | HYPERMETHYLATION |
| DLX4 | chr17 | 45397228 | 45397930 | chr17:45396281-45398063 | 1.25 | 0.1 | 0.32 | 0.22 | HYPERMETHYLATION |
| CBX4 | chr17 | 75428613 | 75431793 | chr17:75427586-75433676 | 1 | 0.07 | 0.27 | 0.21 | HYPERMETHYLATION |
| EDG6 | chr19 | 3129836 | 3130874 | chr19:3129741-3130986 | 1.35 | 0.04 | 0.87 | 0.83 | HYPERMETHYLATION |
| PRRT3 | chr3 | 9963364 | 9964023 | chr3:9962895-9964619 | -0.85 | 0.9 | 0.09 | -0.81 | HYPOM ETHYLATION |
| MGC29506 | chr5 | 138757911 | 138758724 | chr5:138755609-138758810 | -0.63 | 0.93 | 0.17 | -0.76 | HYPOM ETHYLATION |
| TEAD3 | chr6 | 35561812 | 35562252 | chr6:35561754-35562413 | -1.17 | 0.92 | 0.13 | -0.8 | HYPOMETHYLATION |
| chr12 group00022 | chr12 | 1642456 | 1642708 | chr12:1642195-1642774 | -1.33 | 0.66 | 0.09 | -0.57 | HYPOM ETHYLATION |
| CENTG1 | chr12 | 56406249 | 56407788 | chr12:56406176-56407818 | -1.07 | 0.95 | 0.19 | -0.77 | HYPOM ETHYLATION |
| CENTG1 | chr12 | 56416146 | 56418794 | chr12:56416095-56416628; chr12:56418745-56419001 | -0.94 | 0.85 | 0.16 | -0.69 | HYPOM ETHYLATION |

| | | | | | | | | | |
|---|---|---|---|---|---|---|---|---|---|
| Information based on the March 2006 human reference sequence (NCBI Build 36.1), which was produced by the International Human Genome Sequencing Consortium. | | | | | | | | | |

**TABLE 2**

| **GENE NAME** | **CHROM** | **START** | **END** | **SNPs** |
|---|---|---|---|---|
| chr13 group00016 | chr13 | 19773745 | 19774050 | rs7996310; rs12870878 |
| chr13 group00005 | chr13 | 19290394 | 19290768 | rs11304938 |
| CENPJ | chr13 | 24404023 | 24404359 | rs7326661 |
| ATP8A2 | chr13 | 25484475 | 25484614 | rs61947088 |
| PDX1 | chr13 | 27400459 | 27401165 | rs58173592; rs55836809; rs61944011 |
| RB1 | chr13 | 47790983 | 47791646 | rs2804094; rs4151432; rs4151433; rs4151434; rs4151435 |
| PCDH17 | chr13 | 57104856 | 57106841 | rs35287822; rs34642962; rs41292834; rs45500496; rs45571031; rs41292836; rs28374395; rs41292838 |
| KLHL1 | chr13 | 69579933 | 69580146 | rs3751429 |
| POU4F1 | chr13 | 78079515 | 78081073 | rs11620410; rs35794447; rs2765065 |
| GPC6 | chr13 | 92677402 | 92678666 | rs35689696; rs11839555; rs55695812; rs35259892 |
| SOX21 | chr13 | 94152286 | 94153047 | rs41277652; rs41277654; rs35276096; rs5805873; rs35109406 |
| ZIC2 | chr13 | 99439660 | 99440858 | rs9585309; rs35501321; rs9585310; rs7991728; rs1368511 |
| IRS2 | chr13 | 109232856 | 109235065 | rs61747993; rs1805097; rs9583424; rs35927012; rs1056077; rs1056078; rs34889228; rs1056080; rs1056081; rs12853546; rs4773092; rs35223808; rs35894564; rs3742210; rs34412495; rs61962699; rs45545638; rs61743905 |
| chr13 group00395 | chr13 | 111808255 | 111808962 | rs930346 |
| MCF2L | chr13 | 112724910 | 112725742 | rs35661110; rs2993304; rs1320519; rs7320418; rs58416100 |
| F7 | chr13 | 112799123 | 112799379 | rs2480951; rs2476320 |
| CIDEA | chr18 | 12244327 | 12244696 | rs60132277 |
| chr18 group00091 | chr18 | 12901467 | 12901643 | rs34568924; rs8094284; rs8094285 |
| C18orf1 | chr18 | 13377536 | 13377654 | rs9957861 |
| KLHL14 | chr18 | 28603978 | 28605183 | rs61737323; rs61737324; rs12960414 |
| CD33L3 | chr18 | 41671477 | 41673011 | rs62095363; rs2919643 |
| ONECUT2 | chr18 | 53254808 | 53259810 | rs35685953; rs61735644; rs8084084; rs35937482; rs35427632; rs7232930; rs3786486; rs34286480; rs3786485; rs28655657; rs4940717; rs4940719; rs3786484; rs34040569; |
| | | | | rs35542747; rs33946478; rs35848049; rs7231349; rs7231354; rs34481218; rs12962172; rs3911641 |
| RAX | chr18 | 55086286 | 55086436 | rs58797899; rs45501496 |
| chr18 group00277 | chr18 | 57151972 | 57152311 | rs17062547 |
| TNFRSF11A | chr18 | 58203013 | 58203282 | rs35114461 |
| NETO1 | chr18 | 68685099 | 68687060 | rs4433898; rs34497518; rs35135773; rs6566677; rs57425572; rs36026929; rs34666288; rs10627137; rs35943684; rs9964226; rs4892054; rs9964397; rs4606820; rs12966677; rs8095606 |
| chr18 group00304 | chr18 | 70133945 | 70134397 | rs8086706; rs8086587; rs8090367; rs999332; rs17806420; rs58811193 |
| TSHZ1 | chr18 | 71128742 | 71128974 | rs61732783; rs3744910; rs1802180 |
| chr18 group00342 | chr18 | 74170347 | 74170489 | rs7226678 |
| NFATC1 | chr18 | 75385424 | 75386008 | rs28446281; rs56384153; rs4531815; rs3894049 |
| chr18 group00430 | chr18 | 75653272 | 75653621 | rs34967079; rs35465647 |
| KCNG2 | chr18 | 75760343 | 75760820 | rs3744887; rs3744886 |
| OLIG2 | chr21 | 33317673 | 33321183 | rs2236618; rs11908971; rs9975039; rs6517135; rs2009130; rs1005573; rs1122807; rs10653491; rs10653077; rs35086972; rs28588289; rs7509766; rs62216114; rs35561747; rs7509885; rs11547332 |
| OLIG2 | chr21 | 33327593 | 33328334 | rs7276788; rs7275842; rs7275962; rs7276232; rs16990069; rs13051692; rs56231743; rs35931056 |
| RUNX1 | chr21 | 35180938 | 35185436 | rs2843956; rs55941652; rs56020428; rs56251824; rs13051109; rs13051111; rs3833348; rs7510136; rs743289; rs5843690; rs33915227; rs11402829; rs2843723; rs8128138; rs8131386; rs2843957; rs57537540; rs13048584; rs7281361; rs2843965; rs2843958 |
| SIM2 | chr21 | 36994965 | 36995298 | rs2252821 |
| SIM2 | chr21 | 36999025 | 36999410 | rs58347144; rs737380 |
| DSCAM | chr21 | 41135559 | 41135706 | rs35298822 |
| AIRE | chr21 | 44529935 | 44530388 | rs35110251; rs751032; rs9978641 |
| SUMO3 | chr21 | 45061293 | 45061853 | rs9979741; rs235337; rs7282882 |
| C21orf70 | chr21 | 45202815 | 45202972 | rs61103857; rs9979028; rs881318; rs881317 |
| COL18A1 | chr21 | 45754383 | 45754487 | rs35102708; rs9980939 |
| PRMT2 | chr21 | 46911967 | 46912385 | rs35481242; rs61743122; rs8131044; rs2839379 |
| SIX2 | chr2 | 45081223 | 45082129 | rs62130902 |
| SIX2 | chr2 | 45084851 | 45085711 | rs35417092; rs57340219 |
| SOX14 | chr3 | 138971870 | 138972322 | rs57343003 |
| TLX3 | chr5 | 170674439 | 170676431 | rs11134682; rs35704956; rs2964533; rs35601828 |
| FOXP4 | chr6 | 41623666 | 41624114 | rs12203107; rs1325690 |
| FOXP4 | chr6 | 41636384 | 41636779 | rs56835416 |
| chr7 group00267 | chr7 | 12576755 | 12577246 | rs56752985; rs17149965; rs6948573; rs2240572 |
| NPY | chr7 | 24290224 | 24291508 | rs2390965; rs2390966; rs2390967; rs2390968; rs3025123; rs16146; rs16145; rs16144; rs13235842; rs13235935; rs13235938; rs13235940; rs13235944; rs36083509; rs3025122; rs16143; rs16478; rs16142; rs16141; rs16140; rs16139; rs2229966; rs1042552; rs5571; rs5572 |
| SHH | chr7 | 155291537 | 155292091 | rs9333622; rs1233554; rs9333620; rs1233555 |
| GLIS3 | chr9 | 4288283 | 4289645 | rs56728573; rs12340657; rs12350099; rs35338539; rs10974444; rs7852293 |
| PRMT8 | chr12 | 3472714 | 3473190 | rs12172776 |
| TBX3 | chr12 | 113609153 | 113609453 | rs60114979 |
| chr12 group00801 | chr12 | 118516189 | 118517435 | rs966246; rs17407022; rs970095; rs2711748 |
| PAX9 | chr14 | 36201402 | 36202386 | rs17104893; rs12883298; rs17104895; rs35510737; rs12882923; rs12883049; rs28933970; rs28933972; rs28933971; rs28933373; rs61734510 |
| SIX1 | chr14 | 60178801 | 60179346 | rs761555 |
| ISL2 | chr15 | 74420013 | 74421546 | rs34173230; rs 11854453 |
| DLX4 | chr17 | 45397228 | 45397930 | rs62059964; rs57481357; rs56888011; rs17638215; rs59056690; rs34601685; rs17551082 |
| CBX4 | chr17 | 75428613 | 75431793 | rs1285243; rs35035500; rs12949177; rs3764374; rs62075212; rs62075213; rs3764373; rs3764372; rs55973291 |
| EDG6 | chr19 | 3129836 | 3130874 | rs34728133; rs34573539; rs3826936; rs34914134; rs61731111; rs34205484 |
| MGC29506 | chr5 | 138757911 | 138758724 | rs11748963; rs7447765; rs35262202 |
| CENTG1 | chr12 | 56406249 | 56407788 | rs61935742; rs12318065; rs238519; rs238520; rs238521; rs808930; rs2640595; rs2640596; rs2640597; rs2640598; rs34772922 |
| CENTG1 | chr12 | 56416146 | 56418794 | rs11830475; rs34482618; rs2650057; rs2518686; rs12829991 |

**TABLE 3**

| **GENE NAME** | **RELATIVE METHYLATION PLACENTA TO MATERNAL** | **PRC2 TARGET** |
|---|---|---|
| CRYL1 | HYPOMETHYLATION | TRUE |
| IL17D | HYPOMETHYLATION | TRUE |
| GSH1 | HYPERMETHYLATION | TRUE |
| MAB21L1 | HYPERMETHYLATION | TRUE |
| PCDH17 | HYPERMETHYLATION | TRUE |
| KLHL1 | HYPERMETHYLATION | TRUE |
| POU4F1 | HYPERMETHYLATION | TRUE |
| SOX21 | HYPERMETHYLATION | TRUE |
| ZIC2 | HYPERMETHYLATION | TRUE |
| CIDEA | HYPERMETHYLATION | TRUE |
| KLHL14 | HYPERMETHYLATION | TRUE |
| ONECUT2 | HYPERMETHYLATION | TRUE |
| RAX | HYPERMETHYLATION | TRUE |
| TNFRSF11A | HYPOMETHYLATION | TRUE |
| OLIG2 | HYPERMETHYLATION | TRUE |
| OLIG2 | HYPOMETHYLATION | TRUE |
| SIM2 | HYPERMETHYLATION | TRUE |
| SIM2 | HYPERMETHYLATION | TRUE |
| SIX2 | HYPERMETHYLATION | TRUE |
| SIX2 | HYPERMETHYLATION | TRUE |
| SOX14 | HYPERMETHYLATION | TRUE |
| TLX3 | HYPERMETHYLATION | TRUE |
| SHH | HYPERMETHYLATION | TRUE |
| OSR2 | HYPERMETHYLATION | TRUE |
| TBX3 | HYPERMETHYLATION | TRUE |
| PAX9 | HYPERMETHYLATION | TRUE |
| SIX1 | HYPERMETHYLATION | TRUE |
| ISL2 | HYPERMETHYLATION | TRUE |
| DLX4 | HYPERMETHYLATION | TRUE |
| CBX4 | HYPERMETHYLATION | TRUE |
| CENTG1 | HYPOMETHYLATION | TRUE |
| CENTG1 | HYPOMETHYLATION | TRUE |

**TABLE 4**

| **SEQ ID NO** | **GENE NAME** | **SEQUENCE** |
|---|---|---|
| 1 | chr13 group-00016 | CAGCAGGCGCGCTCCCGGCGAATCTGCCTGAATCGCCGTGAATGCGGTGGGGTGCAGGGCAGGGGCTGGTTTTCTCAGCCGGTCTTGGCTTTTCTCTT TCTCTCCTGCTCCACCAGCAGCCCCTCCGCGGGTCCCATGGGCTCCGCGCTCAGAACAGCCCGGAACCAGGCGCCGCTCGCCGCTCGCTGGGGGCCAC CCGCCTCTCCCCGGAACAGCCTCCCGCGGGCCTCTTGGCCTCGCACTGGCGCCCTCACCCACACATCGTCCCTTTATCCGCTCAGACGCTGCAAAGGG CCTTCTGTCTC |
| 2 | CENPJ | GCTTTGGATTTATCCTCATTGGCTAAATCCCTCCTGAAACATGAAACTGAAACAAAGCCCTGAACCCCCTCAGGCTGAAAAGACAAACCCCGCCTGAG |
| | | GCCGGGTCCCGCTCCCCACCTGGAGGGACCCAATTCTGGCGCCTTCTGGCGACGGTCCCTGCTAGGGACGCTGCGCTCTCCGAGTGCGAGTTTTCGC CAAACTGATAAAGCACGCAGAACCGCAATCCCCAAACTAACACTGAACCCGGACCCGCGATCCCCAAACTGACAAGGGACCCGGAACAGCGACCCCCA AACCGACACGGGACTCGGGAACCGCTATCTCCAAAGGGCAGC |
| 3 | ATP8A 2 | TTTCCACAACAGCGAGCCACCATTGAGCCGCCCAGATGCCATCTGCTGGAAATCACGGCCCCCTGGTCAACCACCACGCCTTACCCGACCTCGCGAGC TGATCCCCCACCTCATCCCACCCCCTTCTGTCTGTCTCCTT |
| 4 | GSH1 | GCTGGACAAGGAGCGCTCACTGTAGCTCTGCTGTGGATTGTGTTGGGGCGAAGAGATGGGTAAGAGGTCAAAGTCGTAGGATTCTGGCGACCGCCTAC CAAGGGATTGGGTCCACAGCACAGAGGTCTGATCGCTTCCTTCTCTGCTCTGCCACCTCCAGACAGCAGCTCTAACCAGCTGCCCAGCAGCAAGAGGA TGCGCACGGCTTTCACCAGCACGCAGCTGCTAGAGCTGGAGCGCGAGTTCGCTTCTAATATGTACCTGTCCCGCCTACGTCGCATCGAGATCGCGA |
| 5 | PDX1 | TGCCTGACACTGACCCCAGGCGCAGCCAGGAGGGGCTTTGTGCGGGAGAGGGAGGGGGACCCCAGCTTGCCTGGGGTCCACGGGACTCTCTTCTTCCT AGTTCACTTTCTTGCTAAGGCGAAGGTCCTGAGGCAGGACGAGGGCTGAACTGCGCTGCAATCGTCCCCACCTCCAGCGAAACCCAGTTGAC |
| 6 | PDX1 | TCGGCGGAGAGACCTCGAGGAGAGTATGGGGAAAGGAATGAATGCTGCGGAGCGCCCCTCTGGGCTCCACCCAAGCCTCGGAGGCGGACGGTGGGCT CCGTCCCGACCCCTTAGGCAGCTGGACCGATACCTCCTGGATCAGACCCCACAGGAAGACTCGCGTGGGGCCCGATATGTGTACTTCAAACTCTGAGC GGCCACCCTCAGCCAACTGGCCAGTGGATGCGAATCGTGGGCCCTGAGGGGCGAGGGCGCTCGGAACTGCATGCCTGTGCACGGTGCCGGGCTCTCCA GAGTGAGGGGGCCGTAAGGAGATCTCCAAGGAAGCCGAAAAAAGCAGCCAGTTGGGCTTCGGGAAAGACTTTTCTGCAAAGGAAGTGATCTGGTCCCA GAACTCCAGGGTTGACCCCAGTACCTGACTTCTCCGGGAGCTGTCAGCTCTCCTCTGTTCTTCGGGCTTGGCGCGCTCCTTTCATAATGGACAGACAC CAGTGGCCTTCAAAAGGTCTGGGGTGGGGGAACGGAGGAAGTGGCCTTGGGTGCAGAGGAAGAGCAGAGCTCCTGCCAAAGCTGAACGCAGTTAGCCC TACCCAAGTGCGCGCTGGCTCGGCATATGCGCTCCAGAGCCGGCAGGACAGCCCGGCCCTGCTCACCCCGAGGAGAAATCCAACAGCGCAGCCTCCTG CACCTCCTTGCCCCAGAGAC |
| 7 | MAB21 L1 | AGATCCCGGTGCATTTAAAGGCCGGCGTGATCTGCACCACGTACCTATCTCGGATTCTCAGTTTCACTTCGCTGGTGTCTGCCACCATCTTTACCACA TCCCGGTAGCTACATTTGTCTACCGCTTGAGCCACCAGCGTCTGAAACCTGGACCGGATTTTGCGCGCCGAGAGGTAGCCGGAGGCGGTAATGAATTC CACCCAGAGGGACATGCTCCTCTTGCGCCCGTCGCTCAACTTCAGCACCGCGCAGCCGGGCAGTGAGCCATCGTCCACGAAGTTGAACACCCCCATTT GGTTGAGATAAAGCACCACTTCAAATTCGGT |
| 8 | RB1 | ACTATGCCTTGAGGGTCAAAACGTCTGGATTTCCTGATCGATGCTGTCGTCGCTGTCCACGGAGCTACTGTCGCCGTCAGAGCGGGAAGGCACGTTCA GGGAGTAGAAGCGTGGGCTTGCAGAAAGGGACCTGTTGCTGCCTTACATGGGGGCCGGCAGGGTAGTCTTGGAAATGCCCAAGATTGCTTCCGCGCGC GTCAGTTCAGCGGACGTGTCTGCCTGGCACGAGGACCGTTCTACAAACTCGTTCCTGGAAGCCGGGCTCGCTGGAGGCGGAGCTTTGGTTTCCTTCGG GAGCTTGTGGGGAATGGTCAGCGTCTAGGCACCCCGGGCAAGGGTCTGTGGCCTTGGTGGCCACTGGCTTCCTCTAGCTGGGTGTTTTCCTGTGGGTC TCGCGCAAGGCACTTTTTTGTGGCGCTGCTTGTGCTGTGTGCGGGGTCAGGCGTCCTCTCTCCTCCCGGCGCTGGCCCTCTGGGGCAGGTCCCCGTT GGCCTCCTTGCGTGTTTGCCGCAGCTAGTACACCTGGATGGCCTCCTCAGTGCCGTCGTTGCTGCTGGAGTCTGACGCCTCGGGCGCCTGCGCCGCAC TTGTGACTTGCTTTCCCCTTCTCAGGGCGCCAGCGCTCCTCTTGACCCCGCTTTTATTCTGTGGTGCTTCTGAAG |
| 9 | PCDH1 7 | GCAAGTCGGGTAGCTACCGGGTGCTGGAGAACTCCGCACCGCACCTGCTGGACGTGGACGCAGACAGCGGGCTCCTCTACACCAAGCAGCGCATCGAC CGCGAGTCCCTGTGCCGCCACAATGCCAAGTGCCAGCTGTCCCTCGAGGTGTTCGCCAACGACAAGGAGATCTGCATGATCAAGGTAGAGATCCAGGA CATCAACGACAACGCGCCCTCCTTCTCCTCGGACCAGATCGAAATGGACATCTCGGAGAACGCTGCTCCGGGCACCCGCTTCCCCCTCACCAGCGCAC |
| | | ATGACCCCGACGCCGGCGAGAATGGGCTCCGCACCTACCTGCTCACGCGCGACGATCACGGCCTCTTTGGACTGGACGTTAAGTCCCGCGGCGACGGC ACCAAGTTCCCAGAACTGGTCATCCAGAAGGCTCTGGACCGCGAGCAACAGAATCACCATACGCTCGTGCTGACTGCCCTGGACGGTGGCGAGCCTCC ACGTTCCGCCACCGTACAGATCAACGTGAAGGTGATTGACTCCAACGACAACAGCCCGGTCTTCGAGGCGCCATCCTACTTGGTGGAACTGCCCGAGA ACGCTCCGCTGGGTACAGTGGTCATCGATCTGAACGCCACCGACGCCGATGAAGGTCCCAATGGTGAAGTGCTCTACTCTTTCAGCAGCTACGTGCCT GACCGCGTGCGGGAGCTCTTCTCCATCGACCCCAAGACCGGCCTAATCCGTGTGAAGGGCAATCTGGACTATGAGGAAAACGGGATGCTGGAGATTGA CGTGCAGGCCCGAGACCTGGGGCCTAACCCTATCCCAGCCCACTGCAAAGTCACGGTCAAGCTCATCGACCGCAACGACAATGCGCCGTCCATCGGTT TCGTCTCCGTGCGCCAGGGGGCGCTGAGCGAGGCCGCCCCTCCCGGCACCGTCATCGCCCTGGTGCGGGTCACTGACCGGGACTCTGGCAAGAACGGA CAGCTGCAGTGTCGGGTCCTAGGCGGAGGAGGGACGGGCGGCGGCGGGGGCCTGGGCGGGCCCGGGGGTTCCGTCCCCTTCAAGCTTGAGGAGAACTA CGACAACTTCTACACGGTGGTGACTGACCGCCCGCTGGACCGCGAGACACAAGACGAGTACAACGTGACCATCGTGGCGCGGGACGGGGGCTCTCCTC CCCTCAACTCCACCAAGTCGTTCGCGATCAAGATTCTAGACGAGAACGACAACCCGCCTCGGTTCACCAAAGGGCTCTACGTGCTTCAGGTGCACGAG AACAACATCCCGGGAGAGTACCTGGGCTCTGTGCTCGCCCAGGATCCCGACCTGGGCCAGAACGGCACCGTATCCTACTCTATCCTGCCCTCGCACAT CGGCGACGTGTCTATCTACACCTATGTGTCTGTGAATCCCACGAACGGGGCCATCTACGCCCTGCGCTCCTTTAACTTCGAGCAGACCAAGGCTTTTG AGTTCAAGGTGCTTGCTAAGGACTCGGGGGCGCCCGCGCACTTGGAGAGCAACGCCACGGTGAGGGTGACAGTGCTAGACGTGAATGACAACGCGCCA GTGATCGTGCTCCCCACGCTGCAGAACGACACCGCGGAGCTGCAGGTGCCGCGCAACGCTGGCCTGGGCTATCTGGTGAGCACTGTGCGCGCCCTAGA CAGCGACTTCGGCGAGAGCGGGCGTCTCACCTACGAGATCGTGGACGGCAACGACGACCACCTGTTTGAGATCGACCCGTCCAGCGGCGAGATCCGCA CGCTGCACCCTTTCTGGGAGGACGTGACGCCCGTGGTGGAGCTGGTGGTGAAGGTGACCGACCACGGCAAGCCTACCCTGTCCGCAGTGGCCAAGCTC ATCATCCGCTCGGTGAGCGGATCCCTTCCCGAGGGGGTACCACGGGTGAATGGCGAGCAGCACCACTGGGACATGTCGCTGCCGCTCATCGTGACTCT GAGCACTATCTCCATCATCCTCCTA |
| 10 | KLHL1 | ATGCGCCCTCTGCACCCCTAGAGCCAGAAGACGCTAGGTGGGCTGCGCGCTCTGCCAGGCGAAGGCTGGAGCGCACACGGCAAAGCCGCGCGTTTCAG CCGTGGTCGGGTCCGCAGGACCTGGGCGTGGGGACACCACCAGGCAGGAGCAGAGGCAGGACTGGGACGCCAAAAGCTGAGAATCCTCGATGCCCGCG CGAGAGCCCCGTGTTAT |
| 11 | POU4F 1 | TTCTGGAAACCGGGCCCCACTTGCAGGCCCGGCCACCTTGGGTTCTGGTGGCCGAAGCCGGAGCTGTGTTTCTCGCAGACTCGGGGAGCTACATTGTG CGTAGGCAATTGTTTAGTTTGAAAGGAGGCACATTTCACCACGCAGCCAGCGCCCTGCATGCAGGAGAAGCCCCCAGGGCCCAGGGTCGGCTGGCTTT AGAGGCCACTTAGGTTGTTTTAAGCACATGTGAAAGGGCAGACAGCAGGGGAGCAGGATATGGGTAAGATCTTCGGGTCTCAGAACAGGGGCTGCCCT TGGGCTGTCCCGGCGCCCTGGGCTCTGACACTGAAGGGTGGAATGGAGGAAGGAATGGAGAAAGGACGGTGGAACTTTCGCTTCCCCTCTGGGCCGCC TTCCCAGGGTCATGCCTGAGCTGCTTTGATCCCAGTGTCGCGCATCTTGGTCCGCTACCTCCCAGGCGATAGCTACTGGGCTCCTCGCTGGCCTCACT GGGGGCCATCCCGGGCAGTGGCCTGCCCTCCGAGGCCCGCGGGACCCAGCCCAGAGCTGAGGTTGGAGTTCTCCGGGCCACGTTCCGGGTCGCTTAGG CTCGGAGATTTCCCGGAGACCGTCGTCCTCCCTTTCTGCTTGGCACTGCGGAGCTCCCTCGGCCTCTCTCCTCCTCTGGTCCCTAAGGCCCGGAGTGG TTGGCGGTACTGGGGCCCGTCGTCATCTCTGCTTCTAAGGCATTCAGACTGGGCTCCAGCTGGGACCGGCAGAGGAGGTTCTCAAGGAAACTGGTGGG AAATATAGTTTTCTTTCGTCTGGTCGTTTAATTTAAATGCAACTTCCCTTGGGGACATTTTCCTGGACGTTAACCAGACCACCTTGAGATGTCGTTGA TGACCTAGAGACCCAGATGATGCGTCCCAGGAAAGTTCACTGCTGACTATTGTCACTCTTGGCGTTATATCTATAGATATAGACCTATGTACATATCT CCACCCTGATCTCTCCGTGGACATGAAACCCACCTACCTTGTGAAAGCCCTACGGGTGACACATGACTACTACGTCTCTGTCCCAACAGGGGCTGGGC CTCCCCTGCCTAATAGTTGCCAGGAGTTTCGCAGCCCAAGTGAATAATGTCTTATGGCTGAACGTGGCCAAGGACTCCTGTGATTTAGGTCCCAGGAG |
| | | GAGCAGAGACGTCCCCGCCCCGCCTGGGCCCTGCCGCATTCAAAGCTGGAAGAAGGCGCTGATCAGACAAGGGGCTTCCAGGTCCTGGGTTAGAACAA CAACAAACAAACGAAACTCCACAACAGACACGCCTGCCCATGACCCCACGCAAGGACATAGGAAGTTCTGTCGCCTTCCTGCTCCGCGGATAGCCGCC TGCCGTCTGCTGCCACCAGAACGCACGGACGCTCGGGGTGGAGGTAGTCAATGGGCAGCAGGGGACCCCCAGCCCCCACAAGCGCGGCTCCGAGGACC TGGAAGCGGGTGCCTGTCGCTCTCCGCAGGCTCCGCTCTGCCTCCAGGAGCAAGATCCCCAAAAGGGTCTGGAAGCTGTGGAGAAAAC |
| 12 | GPC6 | TTTTTTAAACACTTCTTTTCCTTCTCTTCCTCGTTTTGATTGCACCGTTTCCATCTGGGGGCTAGAGGAGCAAGGCAGCAGCCTTCCCAGCCAGCCCT TGTTGGCTTGCCATCGTCCATCTGGCTTATAAAAGTTTGCTGAGCGCAGTCCAGAGGGCTGCGCTGCTCGTCCCCTCGGCTGGCAGAAGGGGGTGACG CTGGGCAGCGGCGAGGAGCGCGCCGCTGCCTCTGGCGGGCTTTCGGCTTGAGGGGCAAGGTGAAGAGCGCACCGGCCGTGGGGTTTACCGAGCTGGAT TTGTATGTTGCACCATGCCTTCTTGGATCGGGGCTGTGATTCTTCCCCTCTTGGGGCTGCTGCTCTCCCTCCCCGCCGGGGCGGATGTGAAGGCTCGG AGCTGCGGAGAGGTCCGCCAGGCGTACGGTGCCAAGGGATTCAGCCTGGCGGACATCCCCTACCAGGAGATCGCAGGTAAGCGCGGGCGCGCTGCAGG GGCAGGCTGCAGCCCTCGGCTGCCGCACGTCCCACTGGCCGCCCGGCGTCCCCTTCCTTCCCCCTGTTGCTGAGTTGGTGCTCACTTTCTGCCACCGC TATGGGACTCCGCGTCTCCGTGTTGGGCGGCGGATGCTCCTGCGGCTTCTTCGGCGGGGAAGGTGTGCGTCTCCGCCGCCTCATTGTGTGCACACGC GGGAGCACCCTGGCTCCCGCCTCCCGCTGCTCTCGCGCCCTTCTACCCCTTAGTTGATGGCTCAGGCCCGGCTGGCCAGGGAGCCCGGGTCACTCCGG GGCGGCTGCAAGGCGCAGACGGAGAGCCGAGCCGGGCGCTCACTCCGCGTTCTGGTTCGGGCAAACTTGGAAGAACTGCGACCGCAGTTTGCCCAGCG CCACAGTCTGAGTGGCGCCTTCTCCACTCCCGCCCTTGCGCCGGCAGGGGCGGTGGAGAGACGCGGAGGGCTCCCCCAGCCCCTCTCTCCCCTATCCG TCCTTCGGGCGACAGAGCGCCCGGCGCTCGGGCCGGGGGCGGGCAAGGCTGGGAGGGACCCTCGCCGGGGACCTGGCCTCTGGACGCCGGCGTTTCAA GGCTGGTTTGGGGACTTCACGGGCTGCCTGTTTCAGATGTGGGGCGGGCTTTCCCGTTAGGGTTCCTCAGTGCTTCCCCAGTTGCTGTTGGCCACTCA GGGCCCGGGGACACCCTGCCACCCGGTCTGGAGCCGGCCTCGTCTGCCAGCGAACAGCCAACTTTAGCGGGTGGCTCAGCTGGGGATT |
| 13 | SOX21 | CACTCAGTGTGTGCATATGAGAGCGGAGAGACAGCGACCTGGAGGCCATGGGTGGGGGCGGGTGGTGAAGCTGCCGAAGCCTACACATACACTTAGCT TTGACACTTCTCGTAGGTTCCAAAGACGAAGACACGGTGGCTTCAGGGAGACAAGTCGCAAGGGCGACTTTTCCAAGCGGGAGATGGTGAAGTCTTTG GACGTGTAGTGGGTAGGTGATGATCCCCGCAGCCGCCTGTAGGCCCGCAGACTTCAGAAAACAAGGGCCTTCTGTGAGCGCTGTGTCCTCCCCGGAAT CCGCGGCTTAACACATTCTTTCCAGCTGCGGGGCCAGGATCTCCACCCCGCGCATCCGTGGACACACTTAGGGTCGCCTTTGTTTTGCGCAGTGATTC AAGTTGGGTAACCCTTGCTCAACACTTGGGAAATGGGGAGAATCTCCCCCACCCGCAACCTCCCGCACCCCAGGTTCCCAAAATCTGAATCTGTATCC TAGAGTGGAGGCAGCGTCTAGAAAGCAAAGAAACGGTGTCCAAAGACCCCGGAGAGTTGAGTGAGCGCAGATCCGTGACGCCTGCGGTACGCTAGGGC ATCCAGGCTAGGGTGTGTGTGTGCGGGTCGGGGGGCGCACAGAGACCGCGCTGGTTTAGGTGGACCCGCAGTCCCGCCCCGCATCTGGAACGAGCTGCT TCGCAGTTCCGGCTCCCGGCGCCCCAGAGAAGTTCGGGGAGCGGTGAGCCTAGCCGCCGCGCGCTCATGTTTATT |
| 14 | ZIC2 | AGTCACTCCAGGATCAGAGGCCGCGTCGGTTCTGCTTGGGGCATGGCAGAGGGAGGCTGCTGGGGCCAAGCCCCGGCTGGACGCGAGGGAAGAAACT CGTCCCAGGACCCGCACGCCCATACCTGGCTGTCCCAGAGCTCTTCCCTAGGCCGGCACCTTCGCTCTTCCTCTTCCCCACCCCCTAGCCCTTTTGTC TCTTTTTCAGACGGATGTTTTCAGTCTCAAGTGGTTTTATTTCCGCACAAAACCCTGAGATCAAGGGCAGATCACAGACTGTACCGGAGGCTCGGGT TTCCCTGGACTCTGTGCTGTTCTGCGTCCCAGGGTTGGCTAGGAAGGAAGGCCTGGGCCGGCGAGGTGACGGGTCTCCCGCCCAGGTCGGCAGGACGG GGGGAGGTGTGTCCCGGTAGGTCCCTGGTGAGCTCACCCGTGGCATCGGGGACCCGCGGGAACCCACCGGGCGCCCACTAGAGACTCGGGTCCTACCC TCCCCCACACTACTCCACCCAAATCATCCGAACGCCCCCCTACGCCTGCTTCCAAGGGCTCACTGATAAAGCCCTCAAAATCACACTCCATCAAGACT TGGTTGAAGCTTTGGGTAGGTTTGTTGTTGTTGTTGTTGTTGTTTGTTTGTTTGTTTTAGCAGACACGTCCTGGAAAGAGGTCCTCAGAACCCAAAGG TTCAATAATGATTTGTGGATGGATTGATTATAGTCTGATATCGCTCTGGTTCCACAGAAACCCGGAGCTCCTTGGCCCACTGTTACCCCAGCAGACCT |
| | | AAATGGACGGTTTCTGTTTTTCACTGGCAGCTCAGAACTGGACCGGAAGAAGTTCCCCTCCACTTCCCCCCTCCCGACACCAGATCATTGCTGGGTTT TTATTTTCGGGGGAAAAACAACAACAACAACAACAAAAAAAAACACTAGGTCCTTCCAGACTGGATCAGGTGATCGGCAAAAACCCTCAGGCTAGTCC GGCTGGGTGCCCGAGCATGAAAAGGCCTCCGTGGCCGTTTGAACAGGGTGTTGCAAATGAGAACTTTTGTAAGCCATAACCAGGGCATCCTGAGGGTC TGAGTTCACGGTCAAGGCTGTGGGCTACTAGGTCCAGCGAGTCCAGGCCTCGCCCCGCCCCCGAGCTGCCACAGCCAAGATCTTCGGCAGGGAATTCG AGACCAGGGTCCTCCCACTCCT |
| 15 | chr13 group-00385 | TTTCGTGCCGCTGTTTCAATGCGCTAACGAGGCACGTTATTCTTAGCCGCGTCCGGGAGGGGATCACATTCCTGCGCAGTTGCGCTGCTGGCGGAAG TGACTTGTTTTCTAACGACCCTCGTGACAGCCAGAGAATGTCCGTTTCTCGGAGCGCAGCACAGCCTGTCCCATCGAGAAGCCTCGGGTGAGGGGCCC GGTGGGCGCCCGGAGGCCGCTGGAGGGCTGTGGAGGGACGGTGGCTCCCCACTCCCGTGGCGAAGGGCAGGCAAACCAGAAGCCTCTTTTGAGAGCC GTTTGGGATTGAGACGAGTAAGCCACAGCGAGTGGTTAGAAGTAGGTTAGGAAGAAGGGGAGGTAAGAAAGCCGAGTAGGGTT |
| 16 | chr13 group-00390 | GTTCGGTGGACAAGGGGGCAGCGCCCACAGCAAGCCGGAAAGAGGAGGCGCGGGGCCGCGCTTGGGGCCTGCCGCTGCACGCCAGCCTGGGCAAAGA GCTGCCACCTTCTGCGGGCGAAGCGGGTCGGGACGCAGGACGGCAGCGGGGCTGGAGGCAGCTACGTGGGTCCACACCCCCATGCCCTGCAAGGCTCC TTGGCCCTGCTTCTCCTCTGTCTCGGCGGGAGAGGAGCAGCCTCGGTTTTACAGAATTTC |
| 17 | chr13 group-00391 | TGTGCCATTTAGTGAGAGGTGTTTTGGGCAAAGAATCAATTTAACTGTGACTGACCGACGGGCTTGACTGTATTAATTCTGCTACCGAAAAAAAAAAA AAAAAAAAAGCAATGAGCCGCAAGCCTTGGACTCGCAGAGCTGCCGGTGCCCGTCCGAGAGCCCCACCAGCGCGGCTCACGCCTCAGTCTC |
| 18 | chr13 group-00395 | AGAGTCCCAGTTCTGCAGGCCGCTCCAGGGCTAGGGGTAGAGATGGTGGCAGGTGGTGCGTCAACTCTCTAGGGAAGAGGAACTTGCATTACAAAGAC TTGTCTTTCTGAGCTGAAGTCAAAACGGGGGCGTCAAGCGCGCTCCGTTTGGCGGCGGTGGAGGGGCCGCGCGCCCGCGCTGTCCCAGCCGGAGCTGC CCTGGCTGGTGATTGGAGGTTTAACGTCCGGAATTCAGGCGCTTCTGCAGCTCAGATTTGCCGGCCAAGGGGCCTCAGTTGCAACTTTTCAAAATGGT GTTTCTGGAAAATAACAAATTCAGACTCAACTGGTGACAGCTTTTGGCTATAGAGAATGAAACTGCTTCCCTTTGGCGGTGGAACTCTTAAACTTCGA AGAGTGAAAGAATACAATGAAATAAAATGCCATAAGATCACTGGATTTTTCAGAAAAAGGAAGACCCCAAATTACTCCCAAAATGAGGCTTTGTAAAT TCTTGTTAAAAATCTTTAAATCTCGAATTTCCCCCTACAACATCTGATGAGTGCTTTAAGAGCAAACGAGCAAATCCCACCTCGAGAATCAACAAACC CAAGCTCTGGCCAAGGCTCTCCCCGCGTTTTCTTCTCGTGACCTGGGGAATGTCCCGCCCCATCGCTCACCTGGCTCTTGTCATCTCGCTCATCTTGA AGTGACCCGTGGACAATGCTG |
| 19 | chr13 group-00399 | AGCTGCCCTCTGTGGCCATGAGCGGGTGTCCAGCCCCTTCCAAGGCTGCACCGGGGAGACGCTGGTTTTCTGCTCGCTGTGACCGAACAAAGCCCCTA AGAGTCAGTGCGCGGAACAGAAGAGCCGGACCCCGACGGGCCGAGTCCCAACGTGAGGCACCCGGCAGAGAAAACACGTTCACG |
| 20 | PROZ | CCTCGGCAGCACCGGCATGGCTGGAGGCCAGTACGGCCAGGTGTGGCGGAGGGAGCGCCGTCTGGCTGGGTCGTCCATCCTGACAGGACGCTGCAA GGGCAGGAGCCCCGCGCCCCGTGTCCTGCGCCCCCGCTCGAGGACAAGCCCCAGCCGCCGGTCTCCGCTGGGTTCCGACAG |
| 21 | CIDEA | CTTTAAGAGGCTGTGCAGGCAGACAGACCTCCAGGCCCGCTAGGGGATCCGCGCCATGGAGGCCGCCCGGGACTATGCAGGAGCCCTCATCAGGCGAG TGCCCCGCGTCCCCCTGATTGCCGTGCGCTTCCAATCGCCTTGCGTTCGGTGGCCTCATATTCCCCTGTGCGCCTCTAGTACCGTACCCCGCTCCCTT CAGCCCCCTGCTCCCCGCATTCTCTTGCGCTCCGCGACCCCGCGCACACACCCATCCGCCCCACTGGTGCCCAAGCCGTCCAGCCGCGCCCGCGGGCA |
| | | GAGCCCAATCCCGTCCCGCGCCTCCTCACCCTCTTGCAGCTGGGCACAGGTACCAGGTGTGGCTCTTGCGAGGTG |
| 22 | chr18 group-00091 | AGACTTGCAGAACTCGGGCCCCCTGGAGGAGACCTAACCGCCACGGTCTTGGGGAGGTTCCGGAGGGCCTCGGTTGTCTGCACTCCCAACACCAAGAA ACCCCTGAGACGCGAAGCTGCCAGCGTGCTGCCCTCAGAGCAGGGCGACGCAAAGCCAGCGGACCCCGGGGTGGCGGG |
| 23 | chr18 group-00094 | TGCTCGGCTGGGGGGCTCGCTCCGCACTTTCGGTGCCAGAAAATGCCCAGAGGAGCGGGGCGGCCCCAGAGCCTCCTTTCGGGGCGCGAGGCCCGGCG CGTGTGTACGGAGTCCAGTCCCCCCAGGGAGTGGGGTGCCCGCACCTTCCCCTCCGCGCTCGGAGCCAC |
| 24 | KLHL14 | TCTTGCACACCTGCTTGTAGTTCTGCACCGAGATCTGGTCGTTGAGGAACTGCACGCAGAGCTTGGTGACCTGGGGGATGTGCAGGATCTTGCTGACC GACAGCACCTCCTCCACCGTGTCCAGGGACAGGGTCACGTTGGCCGTGTAGAGGTACTCGAGCACCAGGCGCAGCCCGATGGACGAGCAGCCCTGCAG CACCAGGTTGTTGATGGCCCGGGGGCTGGTCAGCAGCTTGTCGTCGGGGGAGGAAGAAGGAGTCCCGGGCTCCTCCTGCGGCGGCGGCTGCTGCTGCT GTGACGGCTGCTGCTGCGGCGGCTGCTGCTGGTCCTTGGGGGCCCCCAGGCCGTCCTGGCCGCCGACCCCTCCCCCGAGAGGGGGGTGGCTGGAGAAG AGCGATCGGAAGTACTGCGAGCAGGAGGCCAGCACGGCCTTGTGGCAATGGAACTGCTGGCCCTGGGCCGTCAGGGTCACGTCGCAAAACAGCTGCTT CCTCCACAGCAGGTTGAGGCCGTGCAGCAGGTTGTCGCTGTGGCTGGGGTCGAAGGTGGAGGTCCTGTCCCCGGATCTGGACATGGCGAGCTGACTCG GTGCACCTGGCTTTAAACCCTCCTCCAACCTGGCAGACAGGGGTGGGGGATGGGAGGGAGGGGAGCAGGGTGGTGGAGCGGGTGGGGTGTGGTCGGGG TGGGGAAGGGTGTGGAGGGGAGGGGAGGGCGAAGAACAAGAATCAAGGCTCAGCTTGACTCCCTCCTGGCGCGCTCCGGACCCCGACCCTAGGAGGAA AGTCCGAAGACGCTGGATCCGTGAGCGCCACCAGAAGGGCCCTGTCTGGGGTCCCGGCGCCGGTTCTGCGCCCTGCGGCTCCTCTCGCCACCTCCCAC ACACTTCGTCCCTCACTTTCCTAAAACCAACCACCTCAGCTCGGCTGTTGGCAGCAACAGCAGTGGCAGCAGCGACGGCAAAGTGGCGGCTGAGGCCG AGGCACCTCGTGGGCTCGTGTCCATGCCGGGCCAGATGAAGGGAAAGGCCGGGAAGTGGGGAGCCGGGGGTGCCCTGAAAGCTCAGAGGCGACCGACG GCGAAGGTTCCAGGTCAACTTGTGCCCGAAGCTTTGCTTTTCGCAGTTGGCCCAGTTTGGGGGAGGGGGTAGGAACAGGGGCCCGACCAGCGTGCGGG GTGTGCGAATCTTAGCTCTCCAAAAGCTG |
| 25 | ST8SIA 3 | CCTCTGTGTTAGTGCCCTCGGGAATTTGGTTGATGGGGTGTTTG |
| 26 | ONECU T2 | TGATGTCGCACCTGAACGGCCTGCACCACCCGGGCCACACTCAGTCTCACGGGCCGGTGCTGGCACCCAGTCGCGAGCGGCCACCCTCGTCCTCATCG GGCTCGCAGGTGGCCACGTCGGGCCAGCTGGAAGAAATCAACACCAAAGAGGTGGCCCAGCGCATCACAGCGGAGCTGAAGCGCTACAGTATCCCCCA GGCGATCTTTGCGCAGAGGGTGCTGTGCCGGTCTCAGGGGACTCTCTCCGACCTGCTCCGGAATCCAAAACCGTGGAGTAAACTCAAATCTGGCAGGG AGACCTTCCGCAGGATGTGGAAGTGGCTTCAGGAGCCCGAGTTCCAGCGCATGTCCGCCTTACGCCTGGCAGGTAAGGCCGGGGCTAGCCAGGGGCCA GGCTGCTGGGAAGAGGGCTCCGGGTCCGGTGCTTGTGGCCCAAGTCTGCGCGCCGAGTCACTTCTCTTGATTCTTTCCTTCTCTTTCCTATACACGTC CTCTTTCTTCTCGTTTTTATTTCTTCTTCCATTTTCTCTTTCTCTTCCGCTCTTCCCCTACTTTCCCTTCTCCCTTTTCTTTTTCTTTCTTACTCTCT CCTTGTCCCTGAGCTTTCATTGACCGACCCCCCCCCATTTCATTCGCCCTCCCCTCAATGTGCCAACCTTTGCCCTATTTCCGATCTTCCCAGGTACT GGGAGGCGGGATGGGGGTGTGCGTTTTCCTCTAGGAGCCCTGTCTTTCCAAGACCCACAGAAACCAGGACCTGCCCTTATTCAAAACCCCATGCACTT CAAGTCTCTTTTAGACAACACATTTCAATTTTCCGGGCTGACTAGTCTCCCTGTGCAGAGGCAGTTGAGAGGCTTTGCTCTGCAGAGGGAAAAGAGCT |
| | | CTCTACTCTCCCACCCACCATATAGGCAAACTTATTTGGTCATTGGCTGAAGGCACAGCCTTGCCCCCGCGGGAACCGGCGGCCAGGATACAACAGC GCTCCTGCAGCCCATCTCTGGCCTTGGCGTTCGCGCAGGCACTTTCTCACCGGCTTGACGGCTCGGCCCACCTCCAATGTCACTACCTACAGCGAG GGCAGGGTGTAAGGTTGAGAAGGTCACATTCACCGCTTTGGGAGGACGTGGGAGAAGAGACTGAGGTGGAAAGCGCTTTGCCTTGCTCACCGGCCGTC CTTGCCCCGGTCCCAGCGTTTGCTGGGATTTGCCAGGATTTGCCGGGGCTCCGGGAGACCCTGAGCACTCGCAGGAAGAGGTGCTGAGAAATTAAAAA TTCAGGTTAGTTAATGCATCCCTGCCGCCGGCTGCAGGCTCCGCCTTTGCATTAAGCGGGCGCTGATTGTGCGCGCCTGGCGACCGCGGGGAGGACTG GCGGCCCGCGGGAGGGGACGGGTAGAGGCGCGGGTTACATTGTTCTGGAGCCGGCTCGGCTCTTTGTGCCTCCTCTAGCGGCCAAGCTGCGAGGTACA GCCCTCTATTGTTCTAGGAGCACAGAAACCTCCTGTGTGGGCGGCGGGTGCGCGAGCTAGAGGGAAAGATGCAGTAGTTACTGCGACTGGCACGCAGT TGCGCGCTTTTGTGCGCACGGACCCCGCGCGGTGTGCGTGGCGACTGCGCTGCCCCTAGGAGCAAGCCACGGGCCCAGAGGGGCAAAATGTCCAGGTC CCCCGCTGGGAAGGACACACTATACCCTATGGCAAGCCAGGGTGGGCGACTTCCCATGGATCGGGTGGAGGGGGTATCTTTCAGGATCGGCGGGCGG TCTAGGGGAACAATTCGTGGTGGCGATGATTTGCATAGCGCGGGTCTTGGGATGCGCGCGGTTCCGAGCCAGCCTCGCACAGCTCGCTTCCGGAGCTG CGAGCTCAGGTTTCCACCCCCGATCCCCCGGGCTTTCCTCGCACCGCTGAGCCCAGCTTGTGGGGTGCACTCGACCAACGCCCGACAGGGCTGGGGAA TGTGACAGGCAGCAGGTTCACCCGGGCTTGGGGAGGGGGAGTTTCCGCTTTGACAGCATTTTCCTTTGCCGTCTGCTGGTGGATTCCTATTCCCAGTC GGTAATCGCCCCGCAGTGTTGATCTAAGAAGGTAAAGAAAACTAGGTTTCCCTGCAAAGAGCCTCCCCCAAATCGGCGGACTCCGGATACTTTGAGTG GATTTAGAAATTTATGTAATCTTTCTCCTTTAGTTTATTTTTCATCCTCTCCTACAGTTTTCTCTGATTTGCTGTTGGTTCGGGGCAAGATAAAGCAG CCAGTAGAGAGCGATAATAATAGCGGCGGGAAATGAACTGGAGACTGGCTGACAGTTCTTAACATTTTGTCATAGATCCCCCCGAATGTCCCAGGCTG TCTCTGGTGGGTTTTAGTACCCGCCGGCTTCTTGGGCACCGGGGACCAGAAGGAACTTGGCAGCTGGTCTTAGGGGTACAGTTAAAGGCAGGATGACA GCTATTCTCCTGCTCATCTCAGAGCGCTGCCGCCCCCTCATGCCGGTCGCGCAAAGAACACAGCTTTTAAAAAACACGTGCCTTCTGCCCATATAGGT CTGAAAGTGATGAGGAAAGTAATGCTTCGCCTATTAGCGAGTTTCAGCTTTTAAAATGATCCCAAGCGTTGCTGAGATGAGAAAGCGTGGCATCCCGG GGGTCCTCAGCCCCACCCGCGCCCATGGTGCAAGTCTGCAGGGACAGGCCCGGGACAGCACTGCCCACGCTGCTAGATTTTCCGCAGAGGATCGCTGA AGCTGCCTTCGTGGGAGACAGAATGCCTCCTCCAGCGAGTGGAAAAGGCCTGCTGAGGACCCCGCTTTGCTCGAGCATTCAAATGTGTGTCTGTTTTA TTACCCTGGGTTGAAAAGGGACAAGAGCTTTAGCCTTTTTATCTGGCCATTTTATCAGCAACTACAAGTGTGTTGAGTGGTTATTATTACATAGGAGG CTTTTCAGTTTGGGGTCAGTAGATCAGTCTCTTCAGACACTGATGCAGAAGCTGGGACTGGTAAGTAGGTATTATGTGCTCGGAGCGCTAGGGGACAG GAGCAAATGGAGAAGAAAAGCGGAGGCTTTCTCCGCCCGGAGTATCGATCGGAATCCCCGCCGGTACGCCGCAGAGGGCCCTCGCCGTTGGGCCCCGG GGGTTTAACAAGCCCAGCCGCTCCGCAGGCGGCTCGGCCGGACTCTCAGACCGGTGCCTGGAAGACACCGTCCCTGCCCCCCTCCCGCCAAACCTGCC TCTTCTCTTTCTCTCATAGGTTATAGGTTCCCTTTCTCTCTCATTTGGCCCCGCCCCCGGGTCCTGCCAAACAGCCAAGCAGGCCGGGGTTTAGGGG GCTCAGAATGAAGAGGTCTGATTTGGCCAGCGCCGGCAAAGCTCACCCTTAGGCGAGGTCACAACAGAGGCAGGTCCTTCCTGCCCAGCCTGCCGGTG TAGTCACAGCCAAGGGTGGCACTTGAAAGGAAAAGGGAGAAAACTTCGGAGAAATTTAGATTGCCCCAACGTTAGATTTCAGAGAAATTGACTCCAAA TGCACGGATTCGTTCGGAAAGGGCGGCTAAGTGGCAGGTGGTTGCAACCCCGCCCGGTCGGGCCTTCGCAGAGGTTCCCCAAGACCAGCCCTTGCAGG GCGGTTTTCAGCAACCTGACAAGAGGCGGCCAAGACAAATTTCTGCGGGTTCGAGCACACACTCTCGGGCGTTGGGCCCCAGAGACCTCTAAACCAAG CACAAACAAGAAGGGAGTGAGAGAACCCAGGCTAGAACTTGCACGGGCATCCCACTGAGGAAAAGCGAGGCCTCGGTGGCAGGCATGTTTTCTTCCGA CGCCCGAAAATCGAGCCGAGCGCCCGACTACATTTACTGCAGAGGTTTCCGCCTCCAGTGAGCCCGGATCCCCCAGCGGCCTGCCCGGAGCTGGTCTC CAGTCCCCGCCGTAGTCCGACGCACGGCCCTCTCCTGGCAGCAAGCTCCCAGCGGCCAGTCTGAAGCCAATTCTGTTCAGGCGGCCGAGGGCCCTTAG CCAACCCACCATGATGTCGCCTGGGCCACCTGATGCCCGCAGCGGCGGGACACGGCCCGGGCAGTGCGCAGTGGCTCCTGCTAGGGGCACCGCGTGCG |
| | | TGCTTGTCTCCCGCTGCGCCGGGGACGTCCTTGGGTGACACGGGCCGCTGGGCACCTCCCAAGCCGAGGAAACGGACCCCCTTCGCAGAGTCTCGCGC CCACCCCCCAACCTCCCACCTCGTTTCTCGCCTGCTAGCGCTCCCGACTCAGCCCACCTCTCCTGCCGCTTTACTTACGCATCAGAGCTCGAGAGCCTC AACGCAACCCGTGCCAGTACGGAACAGACGATATGTTTGCCTGCTAGCTGCTTGGATGAATAATTGAAAAGTTCGCTGCAGTCTGTGCTTCGTCAAGT CCCGGGTGCCGGGAGAACACCTTCCCAACACGCATCAGGGTGGGCGGGAGCGGGCAGAGGAGGCGGGACCCGAGGGAGGAGAGTGAACCCGAGCAGGA GAAGCAGCCCAGGCAGCCAGGCGCCCTCGATGCGAGAGGCTGGGCATTTATTTTTATTCCAGGCTTTCCACTGTGTGGTTATGTCACTTTCTCAAACA AATGTGTATATGGAGGGAGATCGATGCTGATAATGTTTAGAAGATTAAAAGAGCATTAATGCTGGCAACAATAACGTAAACGTGTGGACCCAGATTTC ATTGATCTGGAACTTGATCCGGCGCGTTTCCAGTAAGCCCGACGGCGCGCTCTTCCCAGCAGAGCGCTCACCAGCGCCACGGCCCCGCGGTTTTCCAG CGGTGCCGCTTCGCCAGCTCTGCGCGGGTTCTCCCGTCTGACCGCAGCTCCTCCCCCGCGAGGCCCCAGCCCGCCTTACTTCCCCGAGGTTTTCTCCT CCTCTCGCCGCGCCTCTCTGCCCTCTCCACCCCCTCCCCCCACCTCTGCACCACCCCGCCCCTGTGCGCACACACCGCTACTTGCGCTTCCGCCCATCCC CCTG |
| 27 | RAX | AACCGGAGATCTGCTTCGTGAACTGAGAGGAGTCCTTAGGAGAGCGGGGACGCCAGGGGCCGGGGGACACTTCGCTCTCGCCCTAGGGAAGGTGGTCT TGACGCTTTCTATTGAAGTCAAACTTGAAAATATCAGCTGCCGCTGGACTAT |
| 28 | chr18 group-00277 | CGTGAGCAGAACGCCCGCCCTGGAGCAGTTAGGACCGAAGGTCTCCGGAGAGTCGCCGGCGGTGCCAGGTAACGCAGAGGGCTCGGGTCGGCCCCGC TTCTGGGGCTTGGGACTCCGGGCGCGCGGAGCCAGCCCTCTGGGGCGAAATCCCCGGGCGGCGTGCGCGGTCCCTCTCCGCGCTGTGCTCTCCCAGCA ACTCCCTGCCACCTCGACGAGCCTACCGGCCGCTCCGAGTTCGACTTCCTCGGACTTAGTGGGAGAAGGGGTTGGAAATGGGCTGCCGGGACTGGGGG AGCTGCTCTCTGGAAGCAGGGAAGCTGGGGCGCACCGGGGCAGGT |
| 29 | NETO1 | TAGAAGAGGAAGACTCCTCTGGCCCCACTAGGTATCATCCGCGCTCTCCCGCTTTCCACCTGCGCCCTCGCTTGGCCAATCTCTGCCGCACGTGTCC ATCCCTGAACTGCACGCTATCCTCCACCCCCGGGGGGTTCCTGCGCACTGAAAGACCGTTCTCCGGCAGGTTTTGGGATCCGGCGACGGCTGACCGCG CGCCGCCCCCACGCCCGGTTCCACGATGCTGCAATACAGAAAGTTTACGTCGGCCCCGACCCGCGCGGACTGCAGGGTCCGCCGGAGCGCGGCGCAG AGGCTTTTCCTGCGCGTTCGGCCCCGGGAAAGGGGCGGGAGGGCTGGCTCCGGGAGCGCACGGGCGCGGCGGGGAGGGTACTCACTGTGAAGCACGCT GCGCCCATGGATCATGTCTGTGCGTTACACCAGAGGCTCCGGGCTCCACTAATTCCATTTAGAGACGGAAGACTTCCAGTGGCGGGGGGAGGACAGG GTCGAGAGGTGTTAAAGACGCAAAGCAAGAAGGAAATAAAGGGGGGCCGAGAGGGAGACCGAGAGGAAGGGGGAGCTCCGAGCCCACGCTGCAGCCAG ATCCGGATGAGTCCGTCCTCCGCCCCGGGCGGGCTCTCGCTCTCGCTGGCCCTCAGCGCCGCGCAGCCAGCAGCATCCCCACCGTGACGCTCGCATCA CACCCGGGCGCCGGCCGCCACCATCCGCGCCGCCGCCGTCAGGACCCTCCTCCCGGGCATCGTCGCCGCCGCGGGGTCGGGAGGACGCGGCGCGCGGG AGGCGGCGGTCGCAGGGCGAGCCCCGGGACGCCCCGAGCCGGGGCCGGGGCCGGGGAGAGGGCGCAGCGAGGTGGGGGCCAGTCCAGACCGACGGCAG CGACGGAGCGGGCGGCGGCGGCGGCGCCGGCGGCGGCGGGGTGGCTCAGTCCCCAGTCTCAGACGCGCCGCGCAGCAGGTCGGAGCAGCCTCCCCGGG AGGATGTCCAGCGGCAGCGCTCCTCGCTCCAGCCCTTGGGGATCTTCCGCTGAGGCATTGAAGGCAGGAAGAAGGGGTCCGTCATCGGCTCGCCGGGC TGCGCGCCACCTCTGCTATCTTGCGGAAAGAGGAGCGGGTGGGTGGGCGTCTGGGAGGCGGGCTGGAGGGCGGTGCAGGGGAGCGGGGCGGCCGGGGG GGGGGCCGGGGGGCGGGGAAGGGAGGGAGGAGAAAGGAGCCGGAAGAGGGCAGAGTTACCAAATGGGCTCCTTAGTCATGGCTTGGGGCTCCACGACC CTCCTGGAAGCCCGGAGCCTGGGTGGGATAGCGAGGCTGCGCGCGGCCGGCGCCCCGGGGCTGGTGCGCGGCAGAATGGGGCCGCGGCGGCGGCAGCA AGGACATCCCAGCCGCGCGGATCTGGGGGAGGGGCGGGGAGGGGGTGAGGACCCGGCTGGGATCCGCGGCTCGGCCCGCCAGGGCGCAGAGAGAGGAT GCAGCCGCAAATCCCGAGCCGGATCCTCGTGCCGGACGGAAGGCGTGGAAGCGGGAGGGGCCTTCGTGTGAAAATCCCTTGTGGGGTTTGGTGTTTCA CTTTTTAAAGGTTAGACCTTGCGGGCTCTCTGCCTCCCACCCCTTCTTTTCCATCCGCGTAAAGGAACTGGGCGCCCCCTCTCCCTCCCTCCCTGGGG |
| | | CGCAGGTTTCGCCGCGGACTCCGCGCTCAGCTTGGGAGACACGGCAGGGGCGCGCCCCAGGGAAAGGCGGCCGTAAAAGTTTCGCGGTTGAGCACTGG GCCTGATGTCCAGTCCCCCCACCAAATTACTCCTGCAAAGACGCGGGCTTCTTGCAATTGAGCCCCCCACCTCGAGGTATTTAAAACCACCCCAAGGC ACACACGGACCCCCGTTCCCCCGCGCCACTTCCTCCTACAGGCTCGCGCGGCGCGTTAAAGTCTGGGAGACACGAGTTGCGGGGAAACAGCACCGGAA G |
| 30 | MBP | AAGAAACAGCTCATTTCGGAGCTGAGGACAAGGCGTGGAAGAAGACGCGTTTGGTTTCACCCAGGCGGGTGGCGGCAAAGCTGTGGGATGCGCGCTG CACACTCCTTCCGTCATCCCGTTCCCACCTTCCACACACACCTGCGGGAGGTCGGACATGTCCTGATTGCGTGTTCATCACGATGGCAAACCGAACAT GAGGAGAACGCCACTGACGCTGGGTGCGCCGGCTTTCCCAGCCCTCGTGCATAACGGGGAGGGAGATGCAGAAGTTTTTTCCAACATCGGTGCAAAGG GGAAGCTGAGGTTTTCCTAT |
| 31 | NFATC 1 | TCTGTCAGCTGCTGCCATGGGGCAGCGGGAAGGCCCTGGAGGGTGCCTGGGCTGTGTCTGGTCCCGGCCACGCGTCCCTGCAGCGTCTGAGACCTTGT GGAACACACTTGACCCGGCGCTGGGACGGGGTCGGCCCACACGCACCGCCAGCCCGCAGGAGTGAGGTGCAGGCTGCCGCTGGCTCCTTAGGCCTCGA CAGCTCTCTTGAGGTCGGCCCTCCTCCCCTCCCGAGAGCTCAGCAGCCGCAGACCCAGGCAGAGAGAGCAAAGGAGGCTGTGGTGGCCCCCGACGGGA ACCTGGGTGGCCGGGGGACACACCGAGGAACTTTCCGCCCCCCGACGGGCTCTCCCACCGAGGCTCAGGTGCTCGTGGGCAGCAAGGGGAAGCCCCAT GGCCATGCCGCTTCCCTTTCACCCTCAGCGACGCGCCCTCCTGTGCCCGCGGGGAACAAGACGGCTCTCGGCGGCCATGCAGGCGGCCTGTCCCACGA ACACGATGGAGACCTCAGACGCCGTCCCCACCCTGTCACTGTCACCATCACCCATCCTGTCCCCTCACGCCTCCCCACATCCCATCATTACTAC |
| 32 | chr18 group-00430 | GAAGTAGAATCACAGTAAATGAGGAGTTAGGGAATTTAGGGTAGAGATTAAAGTAATGAACAGAGGAGGAGGCCTGAGACAGCTGCAGAGAGACCCTG TGTTCCCTGTGAGGTGAAGCGTCTGCTGTCAAAGCCGGTTGGCGCTGAGAAGAGGTACCGGGGGCAGCACCCGCCTCCTGGGAGAGGGATGGGCCTGC GGGCACCTGGGGGAACCGCACGGACACAGACGACACTATAAACGCGGGCGAGACATCAGGGACCGGGAAACAGAAGGACGCGCGTTTCGAGCAGCTGC CCAGTGGGCCACAAGCCCCGCCACGCCACAGCCTCTTCCCCTCAGCACGCAGAGA |
| 33 | OLIG2 | TACTCCGGCGACGGGAGGATGTTGAGGGAAGCCTGCCAGGTGAAGAAGGGGCCAGCAGCAGCACAGAGCTTCCGACTTTGCCTTCCAGGCTCTAGACT CGCGCCATGCCAAGACGGGCCCCTCGACTTTCACCCCTGACTCCCAACTCCAGCCACTGGACCGAGCGCGCAAAGAACCTGAGACCGCTTGCTCTCAC CGCCGCAAGTCGGTCGCAGGACAGACACCAGTGGGCAGCAACAAAAAAAGAAACCGGGTTCCGGGACACGTGCCGGCGGCTGGACTAACCTCAGCGGC TGCAACCAAGGAGCGCGCACGTTGCGCCTGCTGGTGTTTATTAGCTACACTGGCAGGCGCACAACTCCGCGCCCCGACTGGTGGCCCCACAGCGCGCA CCACACATGGCCTCGCTGCTGTTGGCGGGGTAGGCCCGAAGGAGGCATCTACAAATGCCCGAGCCCTTTCTGATCCCCACCCCCCCGCTCCCTGCGTC GTCCGAGTGACAGATTCTACTAATTGAACGGTTATGGGTCATCCTTGTAACCGTTGGACGACATAACACCACGCTTCAGTTCTTCATGTTTTAAATAC ATATTTAACGGATGGCTGCAGAGCCAGCTGGGAAACACGCGGATTGAAAAATAATGCTCCAGAAGGCACGAGACTGGGGCGAAGGCGAGAGCGGGCTG GGCTTCTAGCGGAGACCGCAGAGGGAGACATATCTCAGAACTAGGGGCAATAACGTGGGTTTCTCTTTGTATTTGTTTATTTTGTAACTTTGCTACTT GAAGACCAATTATTTACTATGCTAATTTGTTTGCTTGTTTTTAAAACCGTACTTGCACAGTAAAAGTTCCCCAACAACGGAAGTAACCCGACGTTCCT CACACTCCCTAGGAGACTGTGTGCGTGTGTGCCCGCGCGTGCGCTCACAGTGTCAAGTGCTAGCATCCGAGATCTGCAGAAACAAATGTCTGAATTCG AAATGTATGGGTGTGAGAAATTCAGCTCGGGGAAGAGATTAGGGACTGGGGGAGACAGGTGGCTGCCTGTACTATAAGGAACCGCCAACGCCAGCATC TGTAGTCCAAGCAGGGCTGCTCTGTAAAGGCTTAGCAATTTTTTCTGTAGGCTTGCTGCACACGGTCTCTGGCTTTTCCCATCTGTAAAATGGGTGAA TGCATCCGTACCTCAGCTACCTCCGTGAGGTGCTTCTCCAGTTCGGGCTTAATTCCTCATCGTCAAGAGTTTTCAGGTTTCAGAGCCAGCCTGCAATC GGTAAAACATGTCCCAACGCGGTCGCGAGTGGTTCCATCTCGCTGTCTGGCCCACAGCGTGGAGAAGCCTTGCCCAGGCCTGAAACTTCTCTTTGCAG TTCCAGAAAGCAGGCGACTGGGACGGAAGGCTCTTTGCTAACCTTTTACAGCGGAGCCCTGCTTGGACTACAGATGCCAGCGTTGCCCCTGCCCCAAG |
| | | GCGTGTGGTGATCACAAAGACGACACTGAAAATACTTACTATCATCCGGCTCCCCTGCTAATAAATGGAGGGGTGTTTAACTACAGGCACGACCCTGC CCTTGTGCTAGCGCGGTTACCGTGCGGAAATAACTCGTCCCTGTACCCACACCATCCTCAACCTAAAGGAGAGTTGTGAATTCTTTCAAAACACTCTT CTGGAGTCCGTCCCCTCCCTCCTTGCCCGCCCTCTACCCCTCAAGTCCCTGCCCCCAGCTGGGGGCGCTACCGGCTGCCGTCGGAGCTGCAGCCACGG CCATCTCCTAGACGCGCGAGTAGAGCACCAAGATAGTGGGGACTTTGTGCCTGGGCATCGTTTACATTTGGGGCGCCAAATGCCCACGTGTTGATGAA ACCAGTGAGATGGGAACAGGCGGCGGGAAACCAGACAGAGGAAGAGCTAGGGAGGAGACCCCAGCCCCGGATCCTGGGTCGCCAGGGTTTTCCGCGCG CATCCCAAAAGGTGCGGCTGCGTGGGGCATCAGGTTAGTTTGTTAGACTCTGCAGAGTCTCCAAACCATCCCATCCCCCAACCTGACTCTGTGGTGGC CGTATTTTTTACAGAAATTTGACCACGTTCCCTTTCTCCCTTGGTCCCAAGCGCGCTCAGCCCTCCCTCCATCCCCCTTGAGCCGCCCTTCTCCTCCC CCTCGCCTCCTCGGGTCCCTCCTCCAGTCCCTCCCCAAGAATCTCCCGGCCACGGGCGCCCATTGGTTGTGCGCAGGGAGGAGGCGTGTGCCCGGCCT GGCCACTTTCATTCACCGGAATTAGCCCGGATGACATCACCTTCCCACCCCCCCGCCGGCCCCACCTCATTCCCGAGGCACCCCCTCCAGCACACCGCA CATTGTTCCCCGCCCCCGCCCCCGCCACCGCTGCCGCCGTCGCCGCTGCCACCGGGCTATAAAAACCGGCCGAGCCCCTAAAGGTGCGGATGCTTATT ATAGATCGACGCGACACCAGCGCCCGGTGCCAGGTTCTCCCCTGAGGCTTTTCGGAGCGAGCTCCTCAAATCGCATCCAGAGTAAGTGTCCCCGCCCC ACAGCAGCCGCAGCCTAGATCCCAGGGACAGACTCTCCTCAACTCGGCTGTGACCCAGAATGCTCCGATACAGGGGGTCTGGATCCCTACTCTGCGGG CCATTTCTCCAGAGCGACTTTGCTCTTCTGTCCTCCCCACACTCACCGCTGCATCTCCCTCACCAAAAGCGAGAAGTCGGAGCGACAACAGCTCTTTC TGCCCAAGCCCCAGTCAGCTGGTGAGCTCCCCGTGGTCTCCAGATGCAGCACATGGACTCTGGGCCCCGCGCCGGCTCTGGGTGCATGTGCGTGTGCG TGTGTTTGCTGCGTGGTGTCGATGGAGATAAGGTGGATCCGTTTGAGGAACCAAATCATTAGTTCTCTATCTAGATCTCCATTCTCCCCAAAGAAAGG CCCTCACTTCCCACTCGTTTATTCCAGCCCGGGGGCTCAGTTTTCCCACACCTAACTGAAAGCCCGAAGCCTCTAGAATGCCACCCGCACCCCGAGGG TCACCAACGCTCCCTGAAATAACCTGTTGCATGAGAGCAGAGGGGAGATAGAGAGAGCTTAATTATAGGTACCCGCGTGCAGCTAAAAGGAGGGCCAG AGATAGTAGCGAGGGGGACGAGGAGCCACGGGCCACCTGTGCCGGGACCCCGCGCTGTGGTACTGCGGTGCAGGCGGGAGCAGCTTTTCTGTCTCTCA CTGACTCACTCTCTCTCTCTCTCCCTCTCTCTCTCTCTCATTCTCTCTCTTTTCTCCTCCTCTCCTGGAAGTTTTCGGGTCCGAGGGAAGGAGGACCC TGCGAAAGCTGCGACGACTATCTTCCCCTGGGGCCATGGACTCGGACGCCAGCCTGGTGTCCAGCCGCCCGTCGTCGCCAGAGCCCGATGACCTTTTT CTGCCGGCCCGGAGTAAGGGCAGCAGCGGCAGCGCCTTCACTGGGGGCACCGTGTCCTCGTCCACCCCGAGTGACTGCCC |
| 34 | SIM2 | TTAATTCGAAAATGGCAGACAGAGCTGAGCGCTGCCGTTCTTTTCAGGATTGAAAATGTGCCAGTGGGCCAGGGGCGCTGGACCCGCGGTGCGGAAG ACTCGGAACAGGAAGAAATAGTGGCGCGCTGGGTGGGCTGCCCCGCCGCCCACGCCGGTTGCCGCTGGTGACAGTGGCTGCCCGGCCAGGCACCTCCG AGCAGCAGGTCTGAGCGTTTTTGGCGTCCCAAGCGTTCCGGGCCGCGTCTTCCAGAGCCTCTGCTCCCAGCGGGGTCGCTGCGGCCTGGCCCGAAGGA TTTGACTCTTTGCTGGGAGGCGCGCTGCTCAGGGTTCTG |
| 35 | SIM2 | CCGGTCCCCAGTTTGGAAAAAGGCGCAAGAAGCGGGCTTTTCAGGGACCCCGGGGAGAACACGAGGGCTCCGACGCGGGAGAAGGATTGAAGCGTGCA GAGGCGCCCCAAATTGCGACAATTTACTGGGATCCTTTTGTGGGGAAAGGAGGCTTAGAGGCTCAAGCTATAGGCTGTCCTAGAGCAACTAGGCGAGA ACCTGGCCCCAAACTCCCTCCTTACGCCCTGGCACAGGTTCCCGGCGACTGGTGTTCCCAAGGGAGCCCCCTGAGCCTACCGCCCTTGCAGGGGGTCG TGCTGCGGCTTCTGGGTCATAAACGCCGAGGTCGGGGGTGGCGGAGCTGTAGAGGCTGCCCGCGCAGAAAGCTCCAGGATCCCAATATGTG |
| 36 | DSCR6 | GCGCAGGTCCCCCCAGTCCCCGAGGGAGTGCGCCCGACGGAAACGCCCCTAGCCCGCGGCCTCGCTTTCCTCTCCCGGGTTCCTGGGTCACTTCCCG CTGTCTC |
| 37 | DSCAM | TTCCCTCGCGGCTTTGGAAAGGGGGTGCAAATGCACCCTTCTGCGGGCCCGCTACCCGCTGCAACACCTGTGTTTCCTTTCTGGGCACCTTCTAGGTT TCTAGATATTGCTGTGAATACGGTCCTCCGCTGTACAGTTGAAAACAAA |
| 38 | chr21 group-00165 | TGGGAATTTAGGTCGGGCACTGCCGATATGTCGCCTTCCACAAGGCGGGCCCGGGCCTCTGCTGACCGTGCACCGGTCCTGGGGCTGGGTAATTCTGC AGCAGCAGCGCAGCCCATGCCGGGGAATTTGCGGGCAGAGGAGACAGTGAGGCCCGCGTTCTGTGCGGGAACTCCCGAGCTCACAGAGCCCAAGACCA CACGGCTGCATCTGCTTGGCTGACTGGGCCAGGCCCACGCGTAGTAACCCGGACGTCTCTCTCTCACAGTCCCCTTGCGTCTGGCCAGGGAGCTGCCA GGCTGCACCCCGCGGTGGGGATCGGGAGAGGGGCAGTGTCGCCCATCCCCGGAAGGCTGAGCCTGGTGCAG |
| 39 | PRMT2 | CGGTTTTCTCCTGGAGGACTGTGTTCAGACAGATACTGGTTTCCTTATCCGCAGGTGTGCGCGGCGCTCGCAAGTGGTCAGCATAACGCCGGGCGAAT TCGCAAACCCCCTGCCTCCCTGCACCACCCACTTCCAACGAGTTGGCACAAGTCCTTCTTCCCACCCCCCGCACCCTTCCCTCCGTGTGTCCTTCCACC CACAAAAAGCCCAGACCCTAACCCGCTCCTTTCTCCCGCCGCGTCCATGCAGAACTCCGCCGTTCCTGGAGGGGAAGCCCGCGAGGCGTCGGGAGAG GCACGTCCTCCGTGAGCAAAGAGCTCCTCCGAGCGCGCGGCGGGACGCTGGCCGACAGGGGACCGCGGGGCAGGGCGGAGAGGACCCGCCCTCGA GTCGGCCCAGCCCTAACACTCAGGAC |
| 40 | SIX2 | AGGGAATCGGGCTGACCAGTCCTAAGGTCCCACGCTCCCCTGACCTCAGGGCCCAGAGCCTCGCATTACCCCGAGCAGTGCGTTGGTTACTCTCCCTG GAAAGCCGCCCCCGCCGGGGCAAGTGGGAGTTGCTGCACTGCGGTCTTTGGAGGCCTAGGTCGCCCAGAGTAGGCGGAGCCCTGTATCCCTCCTGGAG CCGGCCTGCGGTGAGGTCGGTACCCAGTACTTAGGGAGGGAGGACGCGCTTGGTGCTCAGGGTAGGCTGGGCCGCTGCTAGCTCTTGATTTAGTCTCA TGTCCGCCTTTGTGCCGGCCTCTCCGATTTGTGGGTCCTTCCAAGAAAGAGTCCTCTAGGGCAGCTAGGGTCGTCTCTTGGGTCTGGCGAGGCGGCAG GCCTTCTTCGGACCTATCCCCAGAGGTGTAACGGAGACTTTCTCCACTGCAGGGCGGCCTGGGGCGGGCATCTGCCAGGCGAGGGAGCTGCCCTGCCG CCGAGATTGTGGGGAAACGGCGTGGAAGACACCCCATCGGAGGGCACCCAATCTGCCTCTGCACTCGATTCCATCCTGCAACCCAGGAGAAACCATTT CCGAGTTCCAGCCGCAGAGGCACCCGCGGAGTTGCCAAAAGAGACTCCCGCGAGGTCGCTCGGAACCTTGACCCTGACACCTGGACGCGAGGTCTTTC AGGACCAGTCTCGGCTCGGTAGCCTGGTCCCCGACCACCGCGACCAGGAGTTCCTTCTTCCCTTCCTGCTCACCAGCCGGCCGCCGGCAGCGGCTCCA GGAAGGAGCACCAACCCGCGCTGGGGGCGGAGGTTCAGGCGGCAGGAATGGAGAGGCTGATCCTCCTCTAGCCCCGGCGCATTCACTTAGGTGCGGGA GCCCTGAGGTTCAGCCTGACTTTC |
| 41 | SIX2 | CACTACGGATCTGCCTGGACTGGTTCAGATGCGTCGTTTAAAGGGGGGGGCTGGCACTCCAGAGAGGAGGGGGCGCTGCAGGTTAATTGATAGCCACG GAAGCACCTAGGCGCCCCATGCGCGGAGCCGGAGCCGCCAGCTCAGTCTGACCCCTGTCTTTTCTCTCCTCTTCCCTCTCCCACCCCTCACTCCGGGA AAGCGAGGGCCGAGGTAGGGGCAGATAGATCACCAGACAGGCGGAGAAGGACAGGAGTACAGATGGAGGGACCAGGACACAGAATGCAAAAGACTGGC AGGTGAGAAGAAGGGAGAAACAGAGGGAGAGAGAAAGGGAGAAACAGAGCAGAGGCGGCCGCCGGCCCGGCCGCCCTGAGTCCGATTTCCCTCCTTCC CTGACCCTTCAGTTTCACTGCAAATCCACAGAAGCAGGTTTGCGAGCTCGAATACCTTTGCTCCACTGCCACACGCAGCACCGGGACTGGGCGTCTGG AGCTTAAGTCTGGGGGTCTGAGCCTGGGACCGGCAAATCCGCGCAGCGCATCGCGCCCAGTCTCGGAGACTGCAACCACCGCCAAGGAGTACGCGCGG CAGGAAACTTCTGCGGCCCAATTTCTTCCCCAGCTTTGGCATCTCCGAAGGCACGTACCCGCCCTCGGCACAAGCTCTCTCGTCTTCCACTTCGACCT CGAGGTGGAGAAAGAGGCTGGCAAGGGCTGTGCGCGTCGCTGGTGTGGGGAGGGCAGCAGGCTGCCCCTCCCCGCTTCTGCAGCGAGTTTCCCAGCC AGGAAAAGGGAGGGAGCTGTTTCAGGAATTTCAGTGCCTTCACCTAGCGACTGACACAAGTCGTGTGTATAGGAAG |
| 42 | SOX14 | GGAGCCTGAAGTCAGAAAAGATGGGGCCTCGTTACTCACTTTCTAGCCCAGCCCCTGGCCCTGGGTCCCGCAGAGCCGTCATCGCAGGCTCCTGCCCA GCCTCTGGGGTCGGGTGAGCAAGGTGTTCTCTTCGGAAGCGGGAAGGGCTGCGGGTCGGGGACGTCCCTTGGCTGCCACCCCTGATTCTGCATCCTTT TCGCTCGAATCCCTGCGCTAGGCATCCTCCCCGATCCCCCAAAAGCCCAAGCACTGGGTCTGGGTTGAGGAAGGGAACGGGTGCCCAGGCCGGACAGA GGCTGAAAGGAGGCCTCAAGGTTCCTCTTTGCTACAAAGTGGAGAAGTTGCTCTACTCTGGAGGGCAGTGGCCTTTTCCAAACTTTTCCACTTAGGTC CGTAAGAAAAGCAATTCATACACGATCAGCGCTTTCGGTGCGAGGATGGAAAGAAACTTC |
| 43 | TLX3 | TTTTCCTGTTACAGAGCTGAGCCCACTCATGTGGTGCCAAGTAGCGACTATCTCTCGGCCACCTCCACCCAGAGCAATGTGGGCGCCCCCAGCGGGTG GGAGCGATTGCCGAGCGGCGCAAGGGCGTTTAACGCCTAACCCCCTCCTCCTGGGTTGCCAAGCCGCTAGGTCGCCGTTTCCAACGTGGCTGCGCGGG ACTGAAGTCCGACGACTCCTCGTCCTCAGTAGGAGACACACCTCCCACTGCCCCCAGCCACGCGAGCTATGGCAGAATCGGGGCAACGGTAATATCT GGATGGGGCAGGCTCCCCTGAGGCTGTGCTTAAGAAAAAAGGAATCTGGAGTAGCCTGAGGGGCCCCACGAGGGGGCCTCCTTTGCGATCGTCTCCCA GCCTTAGGCCAAGGCTACGGAGGCAGGCGGCCGAGTGTTGGCGCCCAGCCCGGCCGAGGACTGGATGGAGGACGAGAAGCAGCCTGCCTCTGGGCGAC AGCTGCGGACGCAGCCTCGCCGCCTCGCCGCCTCAGCCTCGGTCCCAGCGTCTCTAAAGCCGCGCCCATTTTACAGATGCAGGGCAGGGAGACAAGAG GCATCTCCGGGGGCCGAGTAGAATGATGGCGCGGGTTCTCCCGGCGCCCTGATTTCGAGGCTGCGCCCGGGGCCCTACATGCAGGCGGGGAGGCCTGG GCCGAAGGCGTCTGCAAGGAGGGGCGAGTCTGCCCGGTCCGGGCAGGGAGTGAGGCCACAGTCAGTTCTCCCTAGGAGGCCGCGCAGCGGGTAGGGTA TGCCACTCCCGCACGCAACGGCGACCTGGCCCAATCACACCCCTCAGCAGACAACCCCGAAAACTCTCGGCCCGCCCGCTCGCTTCCCCCTACTCCCA ATGGTTTCCGGTCATCCGTTCCCAGTCCAGCCCCGGGTAGGGAGCTCTGATTTGCAATGCACAGCACTTGCGAGGTTCGAATGCCCCCGCAATTTGCA GATGGAAATACTAAGCCTAGGCCGGGCGTGGTGGCTCAAGCCTATCATCTCAGCCCTTTGGGAGGCCAAGCCGGGAGGATTGTTTGAGCCCAAGAATT CAAAACCAGCCTGAGCAACATAGCGACCCCGTCTCTACAAAATAAAATAAAATAAATTATCCGGGCGTGGTGGCACGCGCCTGTGGTTCCAGCTACTC CGGAGGCTGAGGTGGGAGGATCGCTTGAGTCCGGGAGGTCGAGGCTACAGTGAGCCGTGATCGCACCACTGCACTCCAGCCTGGGCGACAGAGTGAGA CCTTGTCTCAAAAAAGGAAAAAAAGAAAAAGAAAGTAAGCTTCAAAGAAGCTCTGATAATAGTTCTGGGTCGTGCAGCGGTGGCGGCCCCGCGCTCTC GCCCCTAAAGCAAGCGCTCTTTGTACTGGGTGGAGGAGCTTTGAGTAGTGAGGGTGGAGATGCAGCTTCGGGGTGGCGCAGCCACCCTGACACTAGGC CCGGGGTCGCAGTGGGACAGAAGAGTCTGCCGCTCTGACTTGGGCTCTGAGTTCCAAGGGCGCCCGGCACTTCTAGCCTCCCAGGCTTGCGCGCTGGC GCCTTTGCCATCCGTGCCGAAGTGGGGAGACCTAGCCGCGACCACCACGAGCGCAGCGGTGACACCCAGAGGTCCCACCGGGCCCCTGGGCAGGGTAA CCTTAGCCTGTCCGCTTCGGCAGCTTTGCGAAGAGTGGCGCGCAGCTAGGGCTGAGGCTCTTGCGGACCTGCGGTCGAAGCAGGCGGCTGAGCCAGTT CGATCGCCAAGGCCTGGGCTGCCGACAGTGGTGCGCGCTCTGTTCCGCCGCGGCCGGGCCAGGCGCTCTGGAATAGCGATGGGGGGACACGGCCTCCA ACTTTCTGCAGAGACCATCGGGCAGCTCCGGGCCTAAGCAGCGACCTCACCGAAGGTTCCTGGGAACCTTTGCCAAAATCCCAGCCTCTGCCTCGGTC CAGCTAAACCGTGTGTAAACAAGTGCACCAAG |
| 44 | FOXP4 | ATAAAGGACCGGGTAATTTCGCGGAATGCGGATTTTGAGACAGGCCCAGACGGCGGCGGATTCCCTGTGTCCCCCAACTGGGGCGATCTCGTGAACAC ACCTGCGTCCCACCCCGATCCTAGGTTGGGGGGAAAGGGTATGGGAACCCTGAGCCCAGAGCGCGCCCCGCTCTTTCCTTTGCTCCCCGGCTTCCCTG GCCAGCCCCCTCCCGGCTGGTTTCCTCGCTCACTCGGCGCCTGGCGTTTCGGGCGTCTGGAGATCACCGCGTGTCTGGCACCCCAACGTCTAGTCTCC CCGCAGGTTGACCGCGGCGCCTGGAGCCGGGAATAGGGGTGGGGAGTCCGGAGAACCAAACCCGAGCCTGAAGTTGCCATTCGGGTGACTCCCGAGAA AGCCCGGGAGCATTTTGGCCAATGCGGGTTTTTACCTGAACTTCAGCATCTTCACC |
| 45 | FOXP4 | AATTGGAAAACCCTGGTATTGTGCCTGTTTGGGGGAAGAAAACGTCAATAAAAATTAATTGATGAGTTGGCAGGGCGGCGGTGCGGGTTCGCGGCGA GGCGCAGGGTGTCATGGCAAATGTTACGGCTCAGATTAAGCGATTGTTAATTAAAAAGCGACGGTAATTAATACTCGCTACGCCATATGGGCCCGTGA AAAGGCACAAAAGGTTTCTCCGCATGTGGGGTTCCCCTTCTCTTTTCTCCTTCCACAAAAGCACCCCAGCCCGTGGGTCCCCCCTTTGGCCCCAAGGT AGGTGGAACTCGTCACTTCCGGCCAGGGAGGGGATGGGGCGGTCTCCGGCGAGTTCCAAGGGCGTCCCTCGTTGCGCACTCGCCCGCCCAGGTTCTTT GAA |
| 46 | chr7 | GGGAAGCGATCGTCTCCTCTGTCAACTCGCGCCTGGGCACTTAGCCCCTCCCGTTTCAGGGCGCCGCCTCCCCGGATGGCAAACACTATAAAGTGGCG GCGAATAAGGTTCCTCCTGCTGCTCTCGGTTTAGTCCAAGATCAGCGATATCACGCGTCCCCCGGAGCATCGCGTGCAGGAGCCATGGCGCGGGAGCT |
| | group-00267 | ATACCACGAAGAGTTCGCCCGGGCGGGCAAGCAGGCGGGGCTGCAGGTCTGGAGGATTGAGAAGCTGGAGCTGGTGCCCGTGCCCCAGAGCGCTCACG GCGACTTCTACGTCGGGGATGCCTACCTGGTGCTGCACACGGCCAAGACGAGCCGAGGCTTCACCTACCACCTGCACTTCTGGCTCGGTAAGGGACGG CGGGCGGCGGGACCCCGACGCACCAAGGCCGGCGAGGGGAGGGCGTAGGGGTCTGAGATTTGCAGGCGTGGGAGTAAAGGGGACCGCAAACTGAGCTA G |
| 47 | NPY | CTCAGGGGCGGGAAGTGGCGGGTGGGAGTCACCCAAGCGTGACTGCCCGAGGCCCCTCCTGCCGCGGCGAGGAAGCTCCATAAAAGCCCTGTCGCGAC CCGCTCTCTGCACCCCATCCGCTGGCTCTCACCCCTCGGAGACGCTCGCCCGACAGCATAGTACTTGCCGCCCAGCCACGCCCGCGCGCCAGCCACCG TGAGTGCTACGACCCGTCTGTCTAGGGGTGGGAGCGAACGGGGCGCCCGCGAACTTGCTAGAGACGCAGCCTCCCGCTCTGTGGAGCCCTGGGGCCCT GGGATGATCGCGCTCCACTCCCCAGCGGACTATGCCGGCTCCGCGCCCCGACGCGGACCAGCCCTCTTGGCGGCTAAATTCCACTTGTTCCTCTGCTC CCCTCTGATTGTCCACGGCCCTTCTCCCGGGCCCTTCCCGCTGGGCGGTTCTTCTGAGTTACCTTTTAGCAGATATGGAGGGAGAACCCGGGACCGCT ATCCCAAGGCAGCTGGCGGTCTCCCTGCGGGTCGCCGCCTTGAGGCCCAGGAAGCGGTGCGCGGTAGGAAGGTTTCCCCGGCAGCGCCATCGAGTGAG GAATCCCTGGAGCTCTAGAGCCCCGCGCCCTGCCACCTCCCTGGATTCTTGGGCTCCAAATCTCTTTGGAGCAATTCTGGCCCAGGGAGCAATTCTCT TTCCCCTTCCCCACCGCAGTCGTCACCCCGAGGTGATCTCTGCTGTCAGCGTTGATCCCCTGAAGCTAGGCAGACCAGAAGTAACAGAGAAGAAACTT TTCTTCCCACACAAGACTTTGCGCAAGAACGCACAAAAGTGACCCAGCAGGAAGAACTTCCAATTCGCTTTTCAATCCTAAACTCGCGCGCCCCCCAC CTTGCACTCTCGCCGCGCGCTTCTTGGTCCCTGAGACTTCGAACGAAGTTGCGCGAAGTTTTCAGGTGGAGCAGAGGGGCAGGTCCCGACCGGACGGC GCCCGGAGCCCGCAAGGTGGTGCTAGCCACTCCTGGGTTCTCTCTGCGGGACTGGACGAGAGCGGATTGGGGGTCGCGTGTGGTAGCAGGAGGAGGA GCGCGGGGGGCAGAGGAGGGAGGTGCTGCGCGTGGGTGCTCTGAATCCCCAAGCCCGTCCGTTGAGCCTTCTGTGCCTGCAGATGCTAGGTAACAAGC GACTGGGGCTGTCCGGACTGACCCTCGCCCTGTCCCTGCTCGTGTGCCTGGGTGCGCTGGCCGAGGCGTACCCCTCCAAGCCGGACAACCCGGGCGAG GACGCACCAG |
| 48 | SHH | TGGAGAACCTTGGGCTCTGTGGCCTCAAAGGTAGGGGTGATTTCGAGGGGCCGGCACCTCACAGGGCAGGTTCCACCGCGGAAACGCAGTCATCGCCC AGCGACCCTGCTCCTGGCCCTCAGCCTCCCCCCAGGTTTCTTTTTCTCTTGAATCAAGCCGAGGTGCGCCAATGGCCTTCCTTGGGTCGGATCCGGGG GGCCAGGGCCAGCTTACCTGCTTTCACCGAGCAGTGGATATGTGCCTTGGACTCGTAGTACACCCAGTCGAAGCCGGCCTCCACCGCCAGGCGGGCCA GCATGCCGTACTTGCTGCGGTCGCGGTCAGACGTGGTGATGTCCACTGCGCGGCCCTCGTAGTGCAGAGACTCCTCTGAGTGGTGGCCATCTTCGTCC CAGCCCTCGGTCACCCGCAGTTTCACTCCTGGCCACTGGTTCATCACCGAGATGGCCAAAGCGTTCAACTTGTCCTTACACCTCTGCGAAGACAAGGG GACCCCCACCGACGGACACGTTAGCCTGGGCAACCGCCACCCCTCCCGGCCCCTCCATCAGCCT |
| 49 | OSR2 | TCTCACGACCCATCCGTTAACCCACCGTTCCCAGGAGCTCCGAGGCGCAGCGGCGACAGAGGTTCGCCCCGGCCTGCTAGCATTGGCATTGCGGTTGA CTGAGCTTCGCCTAACAGGCTTGGGGAGGGTGGGCTGGGCTGGGCTGGGCTGGGCTGGGTGCTGCCCGGCTGTCCGCCTTTCGTTTTCCTGGGACCGA GGAGTCTTCCGCTCCGTATCTGCCTAGAGTCTGAATCCGACTTTCTTTCCTTTGGGCACGCGCTCGCCAGTGGAGCACTTCTTGTTCTGGCCCCGGGC TGATCTGCACGCGGACTTGAGCAGGTGCCAAGGTGCCACGCAGTCCCCTCACGGCTTTCGGGGGGTCTTGGAGTCGGGTGGGGAGGGAGACTTAGGTG TGGTAACCTGCGCAGGTGCCAAAGGGCAGAAGGAGCAGCCTTGGATTATAGTCACGGTCTCTCCCTCTCTTCCCTGCCATTTTTAGGGCTTTCTCTAC GTGCTGTTGTCTCACTGGGTTTTTGTCGGAGCCCCACGCCCTCCGGCCTCTGATTCCTGGAAGAAAGGGTTGGTCCCCTCAGCACCCCCAGCATCCCG GAAAATGGGGAGCAAGGCTCTGCCAGCGCCCATCCCGCTCCACCCGTCGCTGCAGCTCACCAATTACTCCTTCCTGCAGGCCGTGAACACCTTCCCGG CCACGGTGGACCACCTGCAGGGCCTGTACGGTCTCAGCGCGGTACAGACCATGCACATGAACCACTGGACGCTGGGGTATCCCAAT |
| 50 | GLIS3 | TGGTTTCCTTTCGCTTCTCGCCTCCCAAACACCTCCAGCAAGTCGGAGGGCGCGAACGCGGAGCCAGAAACCCTTCCCCAAAGTTTCTCCCGCCAGGT ACCTAATTGAATCATCCATAGGATGACAAATCAGCCAGGGCCAAGATTTCCAGACACTTGAGTGACTTCCCGGTCCCCGAGGTGACTTGTCAGCTCCA GTGAGTAACTTGGAACTGTCGCTCGGGGCAAGGTGTGTGTCTAGGAGAGAGCCGGCGGCTCACTCACGCTTTCCAGAGAGCGACCCGGGCCGACTTCA AAATACACACAGGGTCATTTATAGGGACTGGAGCCGCGCGCAGGACAACGTCTCCGAGACTGAGACATTTTCCAAACAGTGCTGACATTTTGTCGGGC CCCATAAAAAATGTAAACGCGAGGTGACGAACCCGGCGGGGAGGGTTCGTGTCTGGCTGTGTCTGCGTCCTGGCGGCGTGGGAGGTTATAGTTCCAGA CCTGGCGGCTGCGGATCGCCGGGCCGGTACCCGCGAGGAGTGTAGGTACCCTCAGCCCGACCACCTCCCGCAATCATGGGGACACCGGCTTGGATGAG ACACAGGCGTGGAAAACAGCCTTCGTGAAACTCCACAAACACGTGGAACTTGAAAAGACAACTACAGCCCCGCGTGTGCGCGAGAGACCTCACGTCAC CCCATCAGTTCCCACTTCGCCAAAGTTTCCCTTCAGTGGGGACTCCAGAGTGGTGCGCCCCATGCCCGTGCGTCCTGTAACGTGCCCTGATTGTGTAC CCCTCTGCCCGCTCTACTTGAAATGAAAACACAAAAACTGTTCCGAATTAGCGCAACTTTAAAGCCCCGTTATCTGTCTTCTACACTGGGCGCTCTTA GGCCACTGACAGAAACATGGTTTGAACCCTAATTGTTGCTATCAGTCTCAGTCAGCGCAGGTCTCTCAGTGACCTGTGACGCCGGGAGTTGAGGTGCG CGTATCCTTAAACCCGCGCGAACGCCACCGGCTCAGCGTAGAAAACTATTTGTAATCCCTAGTTTGCGTCTCTGAGCTTTAACTCCCCCACACTCTCA AGCGCCCGGTTTCTCCTCGTCTCTCGCCTGCGAGCAAAGTTCCTATGGCATCCACTTACCAGGTAACCGGGATTTCCACAACAAAGCCCGGCGTGCGG GTCCCTTCCCCCGGCCGGCCAGCGCGAGTGACAGCGGGCGGCCGGCGCTGGCGAGGAGTAACTTGGGGCTCCAGCCCTTCAGAGCGCTCCGCGGGCTG TGCCTCCTTCGGAAATGAAAACCCCCATCCAAACGGGGGGACGGAGCGCGGAAACCCGGCCCAAGTGCCGTGTGTGCGCGCGCGTCTG |
| 51 | PRMT8 | GAAAGCCATCCTTACCATTCCCCTCACCCTCCGCCCTCTGATCGCCCACCCGCCGAAAGGGTTTCTAAAAATAGCCCAGGGCTTCAAGGCCGCGCTTC TGTGAAGTGTGGAGCGAGCGGGCACGTAGCGGTCTCTGCCAGGTGGCTGGAGCCCTGGAAGCGAGAAGGCGCTTCCTCCCTGCATTTCCACCTCACCC CACCCCCGGCTCATTTTTCTAAGAAAAAGTTTTTGCGGTTCCCTTTGCCTCCTACCCCCGCTGCCGCGCGGGGTCTGGGTGCAGACCCCTGCCAGGTT CCGCAGTGTGCAGCGGCGGCTGCTGCGCTCTCCCAGCCTCGGCGAGGGTTAAAGGCGTCCGGAGCAGGCAGAGCGCCGCGCGCCAGTCTATTTTTACT TGCTTCCCCCGCCGCTCCGCGCTCCCCCTTCTCAGCAGTTGCACATGCCAGCTCTGCTGAAGGCATCAATGAAAACAGCAGTAG |
| 52 | TBX3 | ATCGAAAATGTCGACATCTTGCTAATGGTCTGCAAACTTCCGCCAATTATGACTGACCTCCCAGACTCGGCCCCAGGAGGCTCGTATTAGGCAGGGAG GCCGCCGTAATTCTGGGATCAAAAGCGGGAAGGTGCGAACTCCTCTTTGTCTCTGCGTGCCCGGCGCGCCCCCCTCCCGGTGGGTGATAAACCCACTC TGGCGCCGGCCATGCGCTGGGTGATTAATTTGCGAACAAACAAAAGCGGCCTGGTGGCCACTGCATTCGGGTTAAACATTGGCCAGCGTGTTCCGAAG GCTTGT |
| 53 | chr12 group-00801 | ATCAACATCGTGGCTTTGGTCTTTTCCATCATGGTGAGTGAATCACGGCCAGAGGCAGCCTGGGAGGAGAGACCCGGGCGGCTTTGAGCCCCTGCAGG GGAGTCCGCGCGCTCTCTGCGGCTCCCTTCCTCACGGCCCGGCCCGCGCTAGGTGTTCTTTGTCCTCGCACCTCCTCCTCACCTTTCTCGGGCTCTCA GAGCTCTCCCCGCAATCATCAGCACCTCCTCTGCACTCCTCGTGGTACTCAGAGCCCTGATCAAGCTTCCCCCAGGCTAGCTTTCCTCTTCTTTCCAG CTCCCAGGGTGCGTTTCCTCTCCAACCCGGGGAAGTTCTTCCGTGGACTTTGCTGACTCCTCTGACCTTCCTAGGCACTTGCCCGGGGCTTCTCAACC CTCTTTTCTAGAGCCCCAGTGCGCGCCACCCTAGCGAGCGCAGTAAGCTCATACCCCGAGCATGCAGGCTCTACGTTCCTTTCCCTGCCGCTCCGGGG GCTCCTGCTCTCCAGCGCCCAGGACTGTCTCTATCTCAGCCTGTGCTCCCTTCTCTCTTTGCTGCGCCCAAGGGCACCGCTTCCGCCACTCTCCGGGG GGTCCCCAGGCGATTCCTGATGCCCCCTCCTTGATCCCGTTTCCGCGCTTTGGCACGGCACGCTCTGTCCAGGCAACAGTTTCCTCTCGCTTCTTCCT ACACCCAACTTCCTCTCCTTGCCTCCCTCCGGCGCCCCCTTTTTAACGCGCCCGAGGCTGGCTCACACCCACTACCTCTTTAGGCCTTTCTTAGGCTC CCCGTGTGCCCCCCTCACCAGCAAAGTGGGTGCGCCTCTCTTACTCTTTCTACCCAGCGCGTCGTAGTTCCTCCCCGTTTGCTGCGCACTGGCCCTAA CCTCTCTTCTCTTGGTGTCCCCCAGAGCTCCCAGGCGCCCCTCCACCGCTCTGTCCTGCGCCCGGGGCTCTCCCGGGAATGAACTAGGGGATTCCACG |
| | | CAACGTGCGGCTCCGCCCGCCCTCTGCGCTCAGACCTCCCGAGCTGCCCGCCTCTCTAGGAGTGGCCGCTGGGGCCTCTAGTCCGCCCTTCCGGAGCT CAGCTCCCTAGCCCTCTTCAACCCTGGTAGGAACACCCGAGCGAACCCCACCAGGAGGGCGACGAGCGCCTGCTAGGCCCTCGCCTTATTGACTGCAG CAGCTGGCCCGGGGGTGCGGCGGGGTGAGGTTCGTACCGGCACTGTCCCGGGACAACCCTTGCAGTTGC |
| 54 | PAX9 | ACAAATAAAACACCCTCTAGCTTCCCCTAGACTTTGTTTAACTGGCCGGGTCTCCAGAAGGAACGCTGGGGATGGATGGGTGGAGAGAGGGAGCGGC TCAAGGACTTTAGTGAGGAGCAGGCGAGAAGGAGCACGTTCAGGCGTCAAGACCGATTTCTCCCCCTGCTTCGGGAGACTTTTGAACGCTCGGAGAGG CCCCGCATCTCACCACTTTACTTCGCCCTAGCGCCCTCCCGCACGGCAGGAATGACGGACCGGGTCCCATTCCACAGTCACGCTTTCGGCCCCTTCCG CTATGTTCAGGGACCATATGGTTTGGGGACAGCCCCAGTAGTTAGTAGGGGACGGGTGCGTTCGCCCAGTCCCCGGATGCGTAGGGAGGCCCAGTGGC AGGCAGCTGTCCCAAGCAGCGGGTGCGCGTCCCTGCGCGCTGTGTGTTCATTTTGCAGAGCCAGCCTTCGGGGAGGTGAACCAGCTGGGAGGAGTGTT CGTGAACGGGAGGCCGCTGCCCAACGCCATCCGGCTTCGCATCGTGGAACTGGCCCAACTGGGCATCCGACCGTGTGACATCAGCCGCCAGCTACGGG TCTCGCACGGCTGCGTCAGCAAGATCCTGGCGCGATACAACGAGACGGGCTCGATCTTGCCAGGAGCCATCGGGGGCAGCAAGCCCCGGGTCACTACC CCCACCGTGGTGAAACACATCCGGACCTACAAGCAGAGAGACCCCGGCATCTTCGCCTGGGAGATCCGGGACCGCCTGCTGGCGGACGGCGTGTGCGA CAAGTACAATGTGCCCTCCGTGAGCTCCATCAGCCGCATTCTGCGCAACAAGATCGGCAACTTGGCCCAGCAGGGTCATTACGACTCATACAAGCAGC ACCAGCCGACGCCGCAGCCAGCGCTGCCCTACAACCACATCTACTCGTACCCCAGCCCTATCACGGCGGCGGCCGCCAAGGTGCCCACGCCACCCGGG GTGC |
| 55 | SIX1 | AGGAGGCGCAACGCGCTGCCAGGGCGGCTTTATCCTGCCGCCACAGGGCGGGGACCAGCCCGGCAGCCGGGTGTCCAGCGCCGCTCACGTGCCTCGCC TGGAGCTTAGCTCTCAGACTCCGAAGAGGGCGACTGAGACTTGGGCCTGGGAGTTGGCTTCGGGGTACCCAAGGCGACGACAGCTGAGTTGTACCACG AAGCTCAGGCCGAGGCCTCCTCCCTTGTCTGGCCTTCGAATCCATACTGGCAGCCTCTCCTCTCAGGCACTCCGCGGGCCGGGCCACTAGGCCCCCTG CTCCTGGAGCTGCGCTATGATCCGGGTCTTGAGATGCGCGCGATTCTCTCTGAACCGGTGGAGAGGAGGCTCTGCCCCGCGCGGAGCGAGGACAGCGG CGCCCGAGCTTCCCGCGCCTCTCCAGGGCCCAATGGCAAGAACAGCCTCCGAAGTGCGCGGATGACAGGAAAAGATCTTCAGTTCTTCTGCCGCTAGA GAAGTGCGGGATACAAGCCTCTATTGGATCCACAACCTGGAGTCCTGCCTTCGGA |
| 56 | ISL2 | ATCTGCGTGCCCTTTTCTGGGCGAGCCCTGGGAGATCCAGGGAGAACTGGGCGCTCCAGATGGTGTATGTCTGTACCTTCACAGCAAGGCTTCCCTTG GATTTGAGGCTTCCTATTTTGTCTGGGATCGGGGTTTCTCCTTGTCCCAGTGGCAGCCCCGCGTTGCGGGTTCCGGGCGCTGCGGAGCCCAAGGCT GCATGGCAGTGTGCAGCGCCCGCCAGTCGGGCTGGTGGGTTGTGCACTCCGTCGGCAGCTGCAGAAAGGTGGGAGTGCAGGTCTTGCCTTTCCTCACC GGGCGGTTGGCTTCCAGCACCGAGGCTGACCTATCGTGGCAAGTTTGCGGCCCCCGCAGATCCCCAGTGGAGAAAGAGGGCTCTTCCGATGCGATCGA GTGTGCGCCTCCCCGCAAAGCAATGCAGACCCTAAATCACTCAAGGCCTGGAGCTCCAGTCTCAAAGGTGGCAGAAAAGGCCAGACCTAACTCGAGCA CCTACTGCCTTCTGCTTGCCCCGCAGAGCCTTCAGGGACTGACTGGGACGCCCCTGGTGGCGGGCAGTCCCATCCGCCATGAGAACGCCGTGCAGGGC AGCGCAGTGGAGGTGCAGACGTACCAGCCGCCGTGGAAGGCGCTCAGCGAGTTTGCCCTCCAGAGCGACCTGGACCAACCCGCCTTCCAACAGCTGGT GAGGCCCTGCCCTACCCGCCCCGACCTCGGGACTCTGCGGGTTGGGGATTTAGCCACTTAGCCTGGCAGAGAGGGGAGGGGGTGGCCTTGGGCTGAGG GGCTGGGTACAGCCCTAGGCGGTGGGGGAGGGGGAACAGTGGCGGGCTCTGAAACCTCACCTCGGCCCATTACGCGCCCTAAACCAGGTCTCCCTGGA TTAAAGTGCTCACAAGAGAGGTCGCAGGATTAACCAACCCGCTCCCCCGCCCTAATCCCCCCCTCGTGCGCCTGGGGACCTGGCCTCCTTCTCCGCAG GGCTTGCTCTCAGCTGGCGGCCGGTCCCCAAGGACACTTTCCGACTCGGAGCACGCGGCCCTGGAGCACCAGCTCGCGTGCCTCTTCACCTGCCTCT TCCCGGTGTTTCCGCCGCCCCAGGTCTCCTTCTCCGAGTCCGGCTCCCTAGGCAACTCCTCCGGCAGCGACGTGACCTCCCTGTCCTCGCAGCTCCCG GACACCCCCAACAGTATGGTGCCGAGTCCCGTGGAGACGTGAGGGGGACCCCTCCCTGCCAGCCCGCGGACCTCGCATGCTCCCTGCATGAGACTCAC |
| | | CCATGCTCAGGCCATTCCAGTTCCGAAAGCTCTCTCGCCTTCGTAATTATTCTATTGTTATTTATGACAGAGTACCGAGAGACACGGTCTGGACAGCC CAAGGCGCCAGGATGCAACCTGCTTTCACCAGACTGCAGACCCCTGCTCCGAGGACTCTTAGTTTTTCAAAACCAGAATCTGGGACTTACCAGGGTTA GCTCTGCCCTCTCCTCTCCTCTCTACGTGGCCGCCGCTCTGTCTCTCCACGCCCCACCTGTGT |
| 57 | DLX4 | AGGTCTCTTCAGACTGCCCATTCTCCGGGCCTCGCTGAATGCGGGGGCTCTATCCACAGCGCGCGGGCCGAGCTCAGGCAGGCTGGGGCGAAGATCT GATTCTTTCCTTCCCGCCGCCAAACCGAATTAATCAGTTTCTTCAACCTGAGTTACTAAGAAAGAAAGGTCCTTCCAAATAAAACTGAAAATCACTGC GAATGACAATACTATACTACAAGTTCGTTTTGGGGCCGGTGGGTGGATGGAGGAGAAAGGGCACGGATAATCCCGGAGGGCCGCGGAGTGAGGAGGA CTATGGTCGCGGTGGAATCTCTGTTCCGCTGGCACATCCGCGCAGGTGCGGCTCTGAGTGCTGGCTCGGGGTTACAGACCTCGGCATCCGGCTGCAGG GGCAGACAGAGACCTCCTCTGCTAGGGCGTGCGGTAGGCATCGTATGGAGCCCAGAGACTGCCGAGAGCACTGCGCACTCACCAAGTGTTAGGGGTGC CCGTGATAGACCGCCAGGGAAGGGGCTGGTTCGGAGGGAATTCCCGCTACCGGGAAGGTCGGAACTCGGGGTGATCAAACAAGGAATGCATCTCACCT CCGTGGGTGCTTGTGCTGCGCAAGGAATTATTACCGGAGCGGTTGCGATGGCCTTTGCCCGGCGACCCAAGAAGAGTAAGCAAACTACCGTCCACCCA GCGGATCAGGTCCAAT |
| 58 | CBX4 | GATGTCCTGTTTCTAGCAGCCTCCAGAGCCAAGCTAGGCGAGAGGCGTAGGAGGCAGAGAGAGCGGGCGCGGGAGGCCAGGGTCCGCCTGGGGGCCTG AGGGGACTTCGTGGGGTCCCGGGAGTGGCCTAGAAACAGGGAGCTGGGAGGGCCGGGAAGAGCTTGAGGCTGAGCGGGGGACGAACGGGCAGCGCAAA GGGGAGATGAACGGAATGGCCGAGGAGCCACGCATTCGCCTTGTGTCCGCGGACCCTTGTTCCCGACAGGCGACCAAGCCAAGGCCCTCCGGACTGAC GCGGCCTGAGCAGCAGCGAGTGTGAAGTTTGGCACCTCCGGCGGCGAGACGGCGCGTTCTGGCGCGCGGCTCCTGCGTCCGGCTGGTGGAGCTGCTGC GCCCTATGCGGCCTGCCGAGGGCGCCGCCGAGGGCCCGCGAGCTCCGTGGGGTCGGGGTGGGGGGACCCGGGAGCGGACAGCGCGGCCCGAGGGGCAG GGGCAGGGGCGCGCCTGGCCTGGGGTGTGTCTGGGCCCCGGCTCCGGGCTCTTGAAGGACCGCGAGCAGGAGGCTTGCGCAATCCCTTGGCTGAGCGT CCACGGAGAAAGAAAAAGAGCAAAAGCAGAGCGAGAGTGGAGCGAGGGATGGGGGCGGGCAAAGAGCCATCCGGGTCTCCACCACCGCCCTGACACGC GACCCGGCTGTCTGTTGGGGACCGCACGGGGGCTCGGGCGAGCAGGGGAGGGAGGAGCCTGCGCGGGGCTCGTGTTCGCCCAGGAATCCCGGAGAAGC TCGAAGACGGTCTGGTGTTGAACGCACACGTGGACTCCATTTCATTACCACCTTGCAGCTCTTGCGCCACGGAGGCTGCTGCTGCCCGGCGGCTGCTA CCCACCGAGACCCACGTGGCCCCTCCCCAGGGGTGTAGGGGTGACGGTTGTCTTCTGGTGACAGCAGAGGTGTTGGGTTTGCGACTGATCTCTAACGA GCTTGAGGCGCAAACCTAGGATTCCCTGAGTGTTGGGGTGCGGCGGGGGGGCAAGCAAGGTGGGACGACGCCTGCCTGGTTTCCCTGACTAGTTGCGG GGGGTGGGGGCCGGCTCTCAGGGGCCACCAGAAGCTGGGTGGGTGTACAGGAAAATATTTTTCTCCTGCCGTGTTTGGCTTTTTCCTGGCATTTTTGC CCAGGGCGAAGAACTGTCGCGCGGGGCAGCTCCACCGCGGAGGGAGAGGGGTCGCGAGGCTGGCGCGGGAAGCGCTGTAGGTGGCAGTCATCCGTCCA CGCCGCACAGGCCGTCTGCGCCGTCGGACCATCGGGAGGTCTGCAGCAACTTTGTCCCGGCCAGTCCCCTTGTCCGGGAAGGGGCTGAGCTTCCCGAC ACTCTACCCTCCCCCTCTTGAAAATCCCCTGGAAAATCTGTTTGCAATGGGTGTTTCCGCGGCGTCCAGGTCTGGGCTGCCGGGGGAGGCCGAGCGGC TGCTGCAGCCTCCCTGCTGCCAGGGGCGTCGGACTCCGCTTCGCTCACTACGCCCAGGCCCCTCAGGGGCCCACGCTCAGGACTTCGGGGCCACACAG CAGGACCCGGTGCCCCGACGACGAGTTTGCGCAGGACCCGGGCTGGGCCAGCCGCGGAGCTGGGGAGGAAGGGGCGGGGGTCGGTGCAGCGGATCTTT TCTGTTGCTGCCTGTGCGGCGGCAGGAAGCGTCTTGAGGCTCCCCAAGACTACCTGAGGGGCCGCCCAAGCACTTCAGAAGCCCAAGGAGCCCCCGGC CACCCCCGCTCCTGGCCTTTTTGCCAACGACTTTGAAAGTGAAATGCACAAGCACCAGCAATTGACTTCCCTTCCGTGGTTATTTATTTTGTCTTTGT GGATGGTGGGCAGATGGGGAGAGAGGCCCCTACCTAACCTCGGTGGCTGGTCCCTAGACCACCCCTGCCAGCCGGTGTGGGGAGGAGCTCAGGTCCGC GGGAGAGCGAATGGGCGCCAGGAGGTGGGACAGAATCCTGGGAAGGTACAGCGGACGCCCTGGAAGCTCCCCTGATGCCCCAGAGGGCCCTTCCTGGG AAACCTCCCGGGGGGGTGCCCCATACCATCCCACCCGGCTGTCTTGGCCCCTCCCAGGGAGCCGCAGGAGAAACTAGCCCTACACCTGGGATTCCCAG |
| | | AGCCTTCTGCTGGGGCTCCTGCCCCCGACTTCGGATAACCAGCTCCGCACAGGTCCCCGAGAAGGGCCGCTGGCCTGCTTATTTGATACTGCCCCCTC CCAGACAGGGGCTGGTCGAGCCCCTGGTTCTGCTGCCAGACTGAAGCCTTCCAGACGCCACCTCGGTTTGGGCCCCCAGGGCCCTCAGGGGCCCCAGG AGAGGAGAGCTGCTATCTAGCTCAGCCACAGGCTCGCTCCTGGTGGGGGCCAGGCTGAAGGAGTGGACCCTGGAGAGGTCGGGAACCTTTTAACAGCC GTGGGCTGGAGGGTGGCTACTAAGTGTTCGGTCTGGGAAGAGGCATGACCCGCACCATCCCGGGGAAATAAACGACTTCTTAAGGGAATCTTCTCGCT GAGCGGGTGCTCTGGGCCAGGAGATTGCCACCGCCAGCCCACGGAACCCAGATTTGGGCTCTGCCTTGAGCGGGCCGCCTGTGGCTTCCCGGGTCGCT CCCCCGACTCAGAAAGCTCTCAAGTTGGTATCGTTTTCCCGGCCCTCGGAGGTGGATTGCAGATCACCGAGAGGGGATTTACCAGTAACCACTACAGA ATCTACCCGGGCTTTAACAAGCGCTCATTTCTCTCCCTTGTCCTTAGAAAAACTTCGCGCTGGCGTTGATCATATCGTACTTGTAGCGGCAGCTTAGG GGCAGCGGAACTGGTGGGGTTGTGCGTGCAGGGGGAGGCTGTGAGGGAGCCCTGCACTCCGCCCCTCCACCCTTCTGGAGGAGTGGCTTTGTTTCTAA GGGTGCCCCCCCAACCCCCGGGTCCCCACTTCAATGTTTCTGCTCTTTGTCCCACCGCCCGTGAAAGCTCGGCTTTCATTTGGTCGGCGAAGCCTCCG ACGCCCCCGAGTCCCACCCTAGCGGGCCGCGCGGCACTGCAGCCGGGGGTTCCTGCGGACTGGCCCGACAGGGTGCGCGGACGGGGACGCGGGCCCCG AGCACCGCGACGCCAGGGTCCTTTGGCAGGGCCCAAGCACCCCT |
| 59 | EDG6 | TGGCGGCCGGCGGGCACAGCCGGCTCATTGTTCTGCACTACAACCACTCGGGCCGGCTGGCCGGGCGCGGGGGCCGGAGGATGGCGGCCTGGGGGCC CTGCGGGGGCTGTCGGTGGCCGCCAGCTGCCTGGTGGTGCTGGAGAACTTGCTGGTGCTGGCGGCCATCACCAGCCACATGCGGTCGCGACGCTGGGT CTACTATTGCCTGGTGAACATCACGCTGAGTGACCTGCTCACGGGCGCGGCCTACCTGGCCAACGTGCTGCTGTCGGGGGCCCGCACCTTCCGTCTGG CGCCCGCCCAGTGGTTCCTACGGGAGGGCCTGCTCTTCACCGCCCTGGCCGCCTCCACCTTCAGCCTGCTCTTCACTGCAGGGGAGCGCTTTGCCACC ATGGTGCGGCCGGTGGCCGAGAGCGGGGCCACCAAGACCAGCCGCGTCTACGGCTTCATCGGCCTCTGCTGGCTGCTGGCCGCGCTGCTGGGGATGCT GCCTTTGCTGGGCTGGAACTGCCTGTGCGCCTTTGACCGCTGCTCCAGCCTTCTGCCCCTCTACTCCAAGCGCTACATCCTCTTCTGCCTGGTGATCT TCGCCGGCGTCCTGGCCACCATCATGGGCCTCTATGGGGCCATCTTCCGCCTGGTGCAGGCCAGCGGGCAGAAGGCCCCACGCCCAGCGGCCCGCCGC AAGGCCCGCCGCCTGCTGAAGACGGTGCTGATGATCCTGCTGGCCTTCCTGGTGTGCTGGGGCCCACTCTTCGGGCTGCTGCTGGCCGACGTCTTTGG CTCCAACCTCTGGGCCCAGGAGTACCTGCGGGGCATGGACTGGATCCTGGCCCTGGCCGTCCTCAACTCGGCGGTCAACCCCATCATCTACTCCTTCC GCAGCAGGGAGGTGTGCAGAGCCGTGCTCAGCTTCCTCTGCTGCGGGTGTCTCCGGCTGGGCATGCGAGGGCCCGGGGACTGCCTGGCCCGGGCCGTC GAGGCTCACTCCGGAGCTTCCACCACCGACAGCTCTCTGAGGCCAAGGGACAGCTTTC |
| 60 | chr13 group-00005 | TAGTAAGGCACCGAGGGGTGGCTCCTCTCCCTGCAGCGGCTGTCGCTTACCATCCTGTAGACCGTGACCTCCTCACACAGCGCCAGGACGAGGATCGC GGTGAGCCAGCAGGTGACTGCGATCCTGGAGCTGGTCGCAGCAGGCCATCCTGCACGCGGTGGAGGCGCCCCCTGCAGGCCGCAGCGCATCCCCAGCT TCTGGACGCACTGTGAGCGGTTATGCAGCAGCACGCTCATATGAGATGCCCCGCAGGGTGCTATGCAGGCCCACGTCCCCACAAAGCCCATGGCAGGC GCCCGGGTGCCGGAGCACGCACTTGGCCCCATGGATCTCTGTGCCCAGGGCTCAGCCAGGCATCTGGCCGCTAAAGGTTT |
| 61 | CRYL1 | TCTCATCTGAGCGCTGTCTTTCACCAGAGCTCTGTAGGACTGAGGCAGTAGCGCTGGCCCGCCTGCGAGAGCCCGACCGTGGACGATGCGTCGCGCCC TTCCCATCGCGGCCTGGGCGGGCCCGCCTGCCCTCGGCTGAGCCCGGTTTCCCTACCCCGGGGCACCTCCCCTCGCCCGCACCCGGCCCCAGTCCCTC CCAGGCTTGCGGGTAGAGCCTGTCTTTGCCCAGAAGGCCGTCTCCAAGCT |
| 62 | IL17D | CAGTCCCCGAGGCCCTCCCCGGTGACTCTAACCAGGGATTTCAGCGCGCGGCGCGGGGCTGCCCCCAGCGTGACCTCACCCGTGCTCTCTCCCTGCA GAATCTCCTACGACCCGGCGAGGTACCCCAGGTACCTGCCTGAAGCCTACTGCCTGTGCCGGGGCTGCCTGACCGGGCTGTTCGGCGAGGAGGACGTG CGCTTCCGCAGCGCCCCTGTCTACAT |
| 63 | IRS2 | AGAGAGACATTTTCCACGGAGGCCGAGTTGTGGCGCTTGGGGTTGTGGGCGAAGGACGGGGACACGGGGGTGACCGTCGTGGTGGAGGAGAAGGTCTC GGAACTGTGGCGGCGGCGGCCCCCCTGCGGGTCTGCGCGGATGACCTTGGCGCCGCGGTGGGGGTCCGGGGGCTGGCTGGCCTGCAGGAAGGCCTCGA CTCCCGACACCTGCTCCATGAGGCTCAGCCTCTTCACGCCCGACGTCGGGCTGGCCACGCGGGCAGCTTCTGGCTTCGGGGGGGCCGCGATAGGTTGC GGCGGGGTGGCGGCCACACCAAAAGCCATCTCGGTGTAGTCACCATTGTCCCCGGTGTCCGAGGACAACGATGAGGCGGCGCCCGGGCCCTGGGCGGT GGCAACGGCCGAGGCGGGGGGCAGGCGGTACAGCTCCCCCGGGGCCGGCGGCGGTGGCGGCGGCTGCAGAGACGACGACGGGGACGCGGACGGACGCG GGGGCAACGGCGGATACGGGGAGGAGGCCTCGGGGGACAGGAGGCCGTCCAAGGAGCCCACGGGGTGGCCGCTCGGGGCGCCCGGCTTAGGAGACTTG GGGGAGCTGAAGTCGAGGTTCATGTAGTCGGAGAGCGGAGACCGCTGCCGGCTGTCGCTGCTGGTGCCCGGGGTGCCTGAGCCCAGCGACGAGGCCGG GCTGCTGGCGGACAAGAGCGAGGAGGACGAGGCCGCCGACGCCAGCAGGGGAGGCGCGGGCGGCGACAGGCGGGCCCCGGGCTCGCCAAAGTCGATGT TCATGTACTCGCCCGGCCCTCTTGCGCTCCCGTGCCAGTGCGTACTCGTGCATGCTCGGCACGCTCGGCAGCCCCTCCACGCACACGCCCGTGCGCCTC ACCGCCCGGCCGCGCTGGCCCAAGAAGCCCTCCGGGCGGCCGCCGCTAGGCCGCACGGGCGAAGGCACTACAGGGTGAGGGGGCTGCGTGGGGCCGGC CCCGAAGGCGCTGGCCGCCTGGCTGGGCCCTGGCGTGGCCTGAGGCTCCAGACGCTCCTCCTCCAGGATGCGCCCCACGGGGGAGCTCATGAGCACGT ACTGGTCGCTGTCCCCGCCACAGGTGTAGGGGGCCTTGTAGGAGCGGGGCAAGGAGCTGTAGCAGCAGCCGGGAACGCCCCTGAGCGGCTCCCCGCCG GGGTGCAGGGCTGCGGAGAAGAAGTCGGGCGGGGTGCCCGTGGTGACCGCGTCGCTGGGGGACACGTTGAGGTAGTCCCCGTTGGGCAGCAGCTTGCC ATCTGCATGCTCCATGGACAGCTTGGAACCGCACCACATGCGCATGTACCCACTGTCCTCGGGGGAGCTCTCGGCGGGCGAGCTGGCCTTGTAGCCGC CCCCGCTCGCCGGGAATGTCCTGCCCGCCGCAGAGGTGGGTGCTGGCCCCGCAGGCCCCGCAGAAGGCACGGCGGCGGCGGCGGCGGCGGCCCTG GGCTGCAAGATCTGCTTGGGGGCGGACACGCTGGCGGGGCTCATGGGCATGTAGTCGTCGCTCCTGCAGCTGCCGCTCCCACTGCCCGCGAGGCCGC GCCGGGCGTCATGGGCATGTAGCCGTCGTCTGCCCCCAGGTTGCTGCTGGAGCTCCTGTGGGAGCCGATCTCGATGTCTCCGTAGTCCTCTGGGTAGG GGTGGTAGGCCACCTTGGGAGAGGACGCGGGGCAGGACGGGCAGAGGCGGCCCGCGCTGCCCGAGAAGGTGGCCCGCATCAGGGTGTATTCATCCAGC GAGGCAGAGGAGGGCTGGGGCACCGGCCGCTGCCGGGCTGGCGTGGTCAGGGAGTAGGTCCTCTTGCGCAGCCCTCGGTCCAGGTCCTGGGCCGCGTC CCCCGAGACCCGGCGGTAGGAGCGGCCACAGTGGCTCAGGGGCCTGTCCATGGTCATGTACCCGTAGAACTCACCGCCGCCGCCGCCGTCTCGGGCCG GGGGCGTCTCCGCGATGGACTCGGGCGTGTTGCTTCGGTGGCTGCAGAAGGCGCGCAGGTCGCCTGGGCTGGAGCCGTACTCGTCCAGGGACATGAAG CCGGGGTCGCTGGGGGAGCCCGAGGCGGAGGCGCTGCCGCTGGAGGGCCGCTGGCCGGGGCCGTGGTGCAGCGGATGCGGCAGAGGCGGGTGCGGGCC GGGCGGCGGCGGGTAGGAGCCCGAGCCGTGGCCGCTGCTGGACGACAGGGAGC |
| 64 | chr13 group-00350 | TAACCTAAAGAATGAAGTCATGCCCCGGCCTGCACCCGGGAAACTGCACACAGCGAAAGATCGCCACTGAGATAAAGAGCTGAAAGCTATTCCCCAAT TCAGCTGTTTCAGCCGTGCGGTCTCACAATGGGCTCACAGACGGCAGCATC |
| 65 | MCF2L | GTTTCCACAATCCACCTCGTAGCTGGGGCGTGCCGCTTGCCTCGGCTTGTCCCGGCAGAACACTCTTACCTTTAATGGCGACTGAAAAGTTGCCACGA GTTCCTGATCATTGTGGTAGGTGCTGCGTGAAGCTGAGACGTGCGTGAGCCACATCCCAGGGGGCTTTGAGCCCCCACCGCGGCGGCGGCTGAGGGGA GCCTTCTCCTACTCGCACACGAGCACACAGGCCTGCACTCTTCACTCCAGGCCCTCTTATCATTCGGATCTCAGGAATTTTCCGAGAGCAAGTGCCGAA TTAACAATGATGAAAGGTTTGTGAGTGAGTGACAGGCACGTTCTATTGAGCACTGCATGGGGCATTATGTGCCACCAGAGACGGGGGCAGAGGTCAAG AGCCCTCGAGGGCTGGGAGAGTTCGGAGGATAGAAGTCATCAGAGCACAATGAAGCCAGACCCTGCAGCCGCCTTCCCCTTCGGGGGCTTCCTTAGAA TGCAGCATTGCGGGGACTGAGCTGTCCCAGGTGAAGGGGGGCCGTCACGGTGTGTGGACGCCCCTCGGCTCAGCCCTCTAAGAGACTCGGCAGCCAGG |
| | | ATGGGCTCAAGGCATGAGCCCTCAAAGGAGGTTAGGAAGGAGCGAGGGAGAAAAGATATGCTTGTGTGACGTCCTCGCCGAAGTGAGAACAATTGTAT CAGATAATGAGTCATGTCCCATTGAGGGGTGCCGACAAGGACTCGGGAGGAGGCCACGGAGCCCTGTACTGAGGACACGCCCACAGGGAGCCTCGGGG GCCCAGCGTCCCGGGATCACTGGATGGTAAAGCCGCCCTGCCTGGCGT |
| 66 | F7 | TCCAGCTGCAGCGAGGCGGCCAGGCCCCCTTCTCCGACCTGCAGGGGTAGCGCGGCCTCGGCGCCGGAGACCCGCGCGCTGTCTGGGGCTGCGGTGG CGTGGGGAGGGCGCGGCCCCCGGACGCCCCGAGGAAGGGGCACCTCACCGCCCCCACCCAGAGCGCCTGGCCGTGCGGGCTGCAGAGGACCCCTCCGG GGCAGAGGCAGGTTCCACGGAAGACCCCGGCCCGCTGGGGCTTCCCCGGAGACTCCAGAG |
| 67 | chr18 group-00039 | ACTTACTGCTTCCAAAAGCGCTGGGCACAGCCTTAIATGACTGACCCCGCCCCCGAGTCCCAGGCCGCCCCATGCAACCGCCCAACCGCCCAACCGCC ACTCCAAAGGTCACCAACCACTGCTCCAGGCCACGGGCTGCCTCTCCCCACGGCTCTAGGGCCCTTCCCCTCCACCGCAGGCTGAC |
| 68 | C18orf 1 | TGCCACACCCAGGTACCGCCCGCCCGCGCGAGAGCCGGGCAGGTGGGCCGCGGATGCTCCCAGAGGCCGGCCCAGCAGAGCGATGGACTTGGACAGGC TAAGATGGAAGTGACCTGAG |
| 69 | CD33L3 | TCGCCAGCGCAGCGCTGGTCCATGCAGGTGCCACCCGAGGTGAGCGCGGAGGCAGGCGACGCGGCAGTGCTGCCCTGCACCTTCACGCACCCGCACCG CCACTACGACGGGCCGCTGACGGCCATCTGGCGCGCGGGCGAGCCCTATGCGGGCCCGCAGGTGTTCCGCTGCGCTGCGGCGCGGGGCAGCGAGCTCT GCCAGACGGCGCTGAGCCTGCACGGCCGCTTCCGGCTGCTGGGCAACCCGCGCCGCAACGACCTCTCGCTGCGCGTCGAGCGCCTCGCCCTGGCTGAC GACCGCCGCTACTTCTGCCGCGTCGAGTTCGCCGGCGACGTCCATGACCGCTACGAGAGCCGCCACGGCGTCCGGCTGCACGTGACAGGCGAGGCGGC GTGGGAGCGGGTCCCCGGCCTCCCTTCCCGCCCTCCCGCCTGCCCCGCCCCAAGGGCTACGTGGGTGCCAGGCGCTGTGCTGAGCCAGGAAGGGCAAC GAGACCCAGCCCTCTCCTCTACCCCAGGGATCTCACACCTGGGGGTAGTTTAGGACCACCTGGGAGCTTGACACAAATGCAGAATCCAGGTCCCAGGA AGGGCTGAGGTGGGCCCGGGAATAGGCATTGCCGTGACTCTCGTAGAGTGACTGTCCCCAGTGGCTCTCAGACGAAGAGGCGAGAAAGACAAGTGAAT GGCAATCCTAAATATGCCAAGAGGTGCAATGTGGTGTGTGCTACCAGCCCGGAAAGACACTCGCAGCCCCTCTACCCAGGGGTGCACAGACAGCCCAC CAAGTAGTGCCTAGCACTTTGCCAGACCCTGATATACAAAGATGCCTGAACCAGGGTCCCGTCCCTAGAGCAGTGGCTCTCCACTCTAGCCCCCACCC TGCTCTGCGACAATAATGGCCACTTAGCATTTGCTAGGGAGCCGGGACCTAGTCCAAGCACCCACAAGCATGAATTTGCCAAATCTTTTCAGCAACCT CTTAAGGCAACTGCTATCATGATCCTCACTTTACACATGGAGAAGCAGAAGCAGAGATGATAGAATCTTTCGCCCAAGGCCACATCTGTATTGGGACG GGGGCAGCCTGGCACCCAAGTGCCCATTCCTCCCTTCTGACCAGCCCCCACCCCTCCGGCTCTGGCGTCCAAAGGGCTAAGGGGAGGGGTGCCCTTGT GACAGTCACCCGCCTTCTCCCCTGCAGCCGCGCCGCGGATCGTCAACATCTCGGTGCTGCCCAGTCCGGCTCACGCCTTCCGCGCGCTCTGCACTGCC GAAGGGGAGCCGCCGCCCGCCCTCGCCTGGTCCGGCCCGGCCCTGGGCAACAGCTTGGCAGCCGTGCGGAGCCCGCGTGAGGGTCACGGCCACCTAGT GACCGCCGAACTGCCCGCACTGACCCATGACGGCCGCTACACGTGTACGGCCGCCAACAGCCTGGGCCGCTCCGAGGCCAGCGTCTACCTGTTCCGCT TCCATGGCGCCAGCGGGGCCTCGACGGTCGCCCTCCTGCTCGGCGCTCTCGGCTTCAAGGCGCT |
| 70 | TNFRSF 11A | ATGAACTTCAAGGGCGACATCATCGTGGTCTACGTCAGCCAGACCTCGCAGGAGGGCGCGGCGGCGGCTGCGGAGCCCATGGGCCGCCCGGTGCAGGA GGAGACCCTGGCGCGCCGAGACTCCTTCGCGGGGAACGGCCCGCGCTTCCCGGACCCGTGCGGCGGCCCCGAGGGGCTGCGGGAGCCGGAGAAGGCCT CGAGGCCGGTGCAGGAGCAAGGCGGGGCCAAGGCTTGAGCGCCCCCCATGGCTGGGAGCCCGAAGCTCGGAGC |
| 71 | ZNF236 | TCAGTGTTATGTGGGGAGCGCTAGATCGTGCACACAGTAGGCGTCAGGAAGTGTTTTCCCCAGTAATTTATCTCCATGGTACTTTGCTAAAGTCATG AAATAACTCAGATTTTGTTTTCCAAGGAAGGAGAAAGGCCCAGAATTTAAGAGCAGGCAGACACACAACCGGGCACCCCCAGACCCTGGCCCTTCCAG |
| | | CAGTCAGGAATTGACTTGCCTTCCAAAGCCCCAGCCCGGAGCTTGAGGAACGGACTTTCCTGCGCAGGGGGATCGGGCGCACTCG |
| 72 | chr18 group-00342 | GTGGAAACACAACCTGCCTTCCATTGTCTGCGCCTCCAAAACACACCCCCCGCGCATCCGTGAAGCTGTGTGTTTCTGTGTTACTACAGGGGCCGGCT GTGGAAATCCCACGCTCCAGACCGCGTGCCGGGCAGGCCCAGCC |
| 73 | OLIG2 | TCCACACCTCGGGCAGTCACTAGGAAAAGGGTCGCCAACTGAAAGGCCTGCAGGAACCAGGATGATACCTGCGTCAGTCCCGCGGCTGCTGCGAGTGC GCGCTCTCCTGCCAGGGGGACCTCAGACCCTCCTTTACAGCACACCGAGGGCCCTGCAGACACGCGAGCGGGCCTTCAGTTTGCAAACCCTGAAAGCG GGCGCGGTCCACCAGGACGATCTGGCAGGGCTCTGGGTGAGGAGGCCGCGTCTTTATTTGGGGTCCTCGGGCAGCCACGTTGCAGCTCTGGGGGAAGA CTGCTTAAGGAACCCGCTCTGAACTGCGCGCTGGTGTCCTCTCCGGCCCTCGCTTCCCCGACCCCGCACAGGCTAACGGGAGACGCGCAGGCCCACCC CACCGGCTGGAGACCCCGGCACGGCCCGCATCCGCCAGGATTGAAGCAGCTGGCTTGGACGCGCGCAGTTTTCCTTTGGCGACATTGCAGCGTCGGTG CGGCCACAATCCGTCCACTGGTTGTGGGAACGGTTGGAGGTCCCCCAAGAAGGAGACACGCAGAGCTCTCCAGAACCGCCTACATGCGCATGGGGCCC AAACAGCCTCCCAAGGAGCACCCAGGTCCATGCACCCGAGCCCAAAATCACAGACCCGCTACGGGCTTTTGCACATCAGCTCCAAACACCTGAGTCCA CGTGCACAGGCTCTCGCACAGGGGACTCACGCACCTGAGTTCGCGCTCACAGATC |
| 74 | RUNX1 | CTGCCCTCGCGGATCTCCCCCGGCCTCGCCGGCCTCCGCCTGTCCTCCCACCACCCTCTCCGGGCCAGTACCTTGAAAGCGATGGCAGGGTCTTGTT GCAGCGCCAGTGCGTAGGCAGCACGGAGCAGAGGAAGTTGGGGCTGTCGGTGCGCACCAGCTCGCCCGGGTGGTCGGCCAGCACCTCCACCATGCTGC GGTCGCCGCTCCTCAGCTTGCCGGCCAGGGCAGCGCCGGCGTCCGGGGCGCCCAGCGGCAACGCCTCGCTCATCTTGCCTGGGCTCAGCGCGGTGGAA GGCGGCGTGAAGCGGCGGCTCGTGCTGGCATCTACGGGGATACGCATCACAACAAGCCGATTGAGTTAGGACCCTGCAAACAGCTCCTACCAGACGGC CACAGGCCCCCCATCTTCAGCAAGCACCTCCCCGCACACCAACATACACGTTCACGGCCCTTTATTACTGCCGGCCCGTGCCGGCGCGCCGCGTCCT TAGGGGAGGAGGGAGACTAAGTTACTAACAGTCCAGGAGGGGAAAACGTTCTGGTTCTGCGGATCGGCCTCTGACCCAGGATGGGCTCCTAGCAACCG ATTGCTTAGTGCATTAAAAAGTGGAGACTATCTTCCACGAATCTTGCTTGCAGAGGTTAAGTTCTGTCTTTGGCTGTTAGAAAAGTTCCTGAAGGCAA AATTCTCATACACTTCCTAAAATATTTATGCGAAGAGTAAAACGATCAGCAAACACATTATTTGGAAGTTCCAGTAGTTAATGCCTGTCAGTTTTTTG CAGGTGAGTTTTGTCTAAAGTCCCAACAGAACACAATTATCTCCCGTAACAAGGCCACTTTTATCATGCAAAACTGGCTTCAGTCCCGAAAAGCAAGA GCTGAGACTTCCAAAGGTAGTGCTACTAATGTATGTGCACGTATATATAAATATATACATATGCTCTACTTCATAAAATATTTACAATACAATCTGTG GAGAATTTAAACACAACAGAAATCCATTAATGTACGCTGCAGATTTTTTAAGTAGCCTTGAAAATCAGCTTCAGTAGTTGGAGCAGTGCTGAGCTAG AAGTACTTCTCATCTTCTCTGTTCTCTCAATCAATTCTGTCAAAACGCTCACTGCAGAAAATTCAGCCTTTCAGACATCTTCAGCTAATCTTAAAACA ACAATCATAAGAAGGCCCAGTCGATGACACTCAGGGTTCTACAGCTCTCCCACATCTGTGAACTCGGGTTTGGGGATGTTGGTTAAGTTTGTGGCTGG TCCTCTGGTTTGTTGGGAGTTGAGCAGCCGCAGAGTCACACACATGCAAACACGCACTCTTCGGAAGGCAGCCACTGTCTACATCAGCTGGGTGACTC AGCCCTGACTCGGGCAGCAGCGAGACGATACTCCTCCACCGTCGCCCAGCACCCGCCGGTTAGCTGCTCCGAGGCACGAACACCCACGAGCGCCGCGT AACCGCAGCAGGTGGAGCGGGCCTTGAGGGAGGGCTCCGCGGCGCAGATCGAAACAGATCGGGCGGCTCGGGTTACACACGCACGCACATCCTGCCAC GCACACTGCCACGCACACGCAACTTCACGGCTCGCCTCGGACCACAGAGCACTTTCTCCCCCTGTTGTAAAAGGAAAACAATTGGGGAAAAGTTCGCA GCCAGGAAAGAAGTTGAAAACATCCAGCCAAGAAGCCAGTTAATTCAAAAGGAAGAAAGGGGAAAAACAAAAAAAAACAACAAAAAAAGGAAGGTCCA ACCCACGCCAACCAGAACCAGCACACGTTCACTTCCTTCTGCCCTCCCTAGCAGCCCCCGCAAAGAAGTCCCTCGCCCCCCAGCGCCCCGCCAGCGTCCT GCCCTGGCTGGGTCCGGCCGCGGGGCGCCCGTCCCGCCCGCGCCCGCTGGCTCTATGAATGAGAGTGCCTGGAAATGAACGTGCTTTTACTGTAAGCC |
| | | CGGCCGGAGGAATTCCATTCCCTCAGCTCGTTTGCATAGGGGCGGCCGGCGGCCAATCACAGGCCTTTCCGGTATCAGCCAGGGCGCGGCTCGCCGCC GCCGGCTCCTGGAATTGGCCCGCGCGCCCCCGCCGCCGCGCCGCGCGCTACTGTACGCAGCCCGGGCGGGGAGTCGGAGGCCACCCCCGCGCCCCGCA TCCAAGCCTGCATGCTGGCCCGGGGCCCCGCCCGCGTGCGGACCCCTTTCCGCAGCCACACGCAGGCTTGTGCGGCTCCGCGAGTGGCCACGGTCCGG AGACCTGGAAAAAGAAAGCAGGCCCCGCCGGCCCGAGGAGGACCCGGCCGGCGCGCCGCACCCGGAGAGGCCCGGCCCCGCGAGCCGCTGCAGGCAGG CGCAGTGGCCGCCACGAGGCTCCCGAACCGGGCTGCAGCCCGCGGACGGCCCCAGATCCTGCGCGGCCGCCCAGGGCCAGGCCTCCGCTTCCAGGGCG GGGGTGCGATTTGGCCGCGGGGCCCGGGGGAGCCACTCCGCGCTCCTGCACCGTCCGGCTGGCAGCTGCGGCGAAGCGGCGCTGATTCCTTGCATGAG GCCGGACGGCGTCCGCGCGTGCCGTTTGCTCTCAGCGTCTTCCCTTGGGTCGGTTTCTGTAATGGGTGTTTTTTACCGCTGCGCCCGGGCCGCGGCTC GATCCCTCCGCGCGTCTCACTTGCTGCGTGCGTCAGCGGCCAGCGAAGAGTTTCCTAGTCAGGAAAGACCCCAAGAACGCGCGGCTGGAAGGAAAGTT GAAAGCAGCCACGCGGCTTGCTCCCGGGCCTTGTAGCGCCGGCACCCGCAGCAGCCGGACAGCCTGCCCGGGCCCCGCGTCTCCCCTCCGGCTCCCCG GAAGCGGCCCCCGCTCCTCTCCCCGCCCCCGTGCGCTCGAGCGGCCCCAGGTGCGGAACCCACCCCGGCTTCGCGTGCGGGCGGCCGCTTCCCCCTGC GCCGGTCCCCGCGGTGCTGCGGGCATTTTCGCGGAGCTCGGAGGGCCCCGCCCCCGGTCCGGCGTGCGCTGCCAACTCCGACCCCGCCCGGCGGGGCT CCCTCCCAGCGGAGGCTGCTCCCGTCACCATGAGTCCCTCCACGCCCTCCCTGCCGGGCCCTGCACCTCCCGGGGCCTCTCATCCACCCCGGGGCTGC AACCCAGTCCCCGGATCCCGGCCCCGTTCCACCGCGGGCTGCTTTGTGGTCCCCGCGGAGCCCCTCAATTAAGCTCCCCGGCGCGGGGGTCCCTCGCC GACCTCACGGGGCCCCTGACGCCCGCTCCTCCCTCCCCCAGGGCTAGGGTGCTGTGGCCGCTGCCGCGCAGGGACTGTCCCCGGGCGTTGCCGCGGGC CCGGACGCAGGAGGGGGCCGGGGTTGACTGGCGTGGAGGCCTTTCCCGGGCGGGCCCGGACTGCGCGGAGCTGTCGGGACGCGCCGCGGGCTCTGGCG CACCCCAGCCGCCAGCACCCGCCCTCCCTGGACCCCGCGCGCAGTCCCCGGAGCTCCCGCAACGCCCCCCGACCGCCGCGCCTGTGCGCCCCGCCCTCG TGGCCTTCGGGTCGCCCGGGAAGAACTAGCGTTCGAGGATAAAAGACAGGAAGCCGCCCCAGAGCCCACTTGAGCTGGAACGGCCAAGGCGCGTTTCC GAGGTTCCAATATAGAGTCGCAGCCGGCCAGGTGGGGACTCTCGGACCAGGCCTCCCCGCTGTGCGGCCCGGTCGGGGTCTCTTCCCGAAGCCCCTGT TCCTGGGGCTTGACTCGGGCCGCTCTTGGCTATCTGTGCTTCAGGAGCCCGGGCTTCCGGGGGGCTAAGGCGGGCGGCCCGCGGCCTCAACCCTCTCC GCCTCCGCTCCCCCTGGGCACTGCCAGCACCCGAGTTCAGTTTTGTTTTAATGGACCTGGGGTCTCGGAAAGAAAACTTACTACATTTTTCTTTTAAA ATGATTTTTTTAAGCCTAATTCCAGTTGTAAATCCCCCCCTCCCCCCGCCCAAACGTCCACTTTCTAACTCTGTCCCTGAGAAGAGTGCATCGCGCGC GCCCGCCCGCCCGCAGGGGCCGCAGCGCCTTTGCCTGCGGGTTCGGACGCGGCCCGCTCTAGAGGCAAGTTCTGGGCAAGGGAAACCTTTTCGCCTGG TCTCCAATGCATTTCCCCGAGATCCCACCCAGGGCTCCTGGGGCCACCCCCACGTGCATCCCCCGGAACCCCCGAGATGCGGGAGGGAGCACGAGGGT GTGGCGGCTCCAAAAGTAGGCTTTTGACTCCAGGGGAAATAGCAGACTCGGGTGATTTGCCCCTCGGAAAGGTCCAGGGAGGCTCCTCTGGGTCTCGG GCCGCTTGCCTAAAACCCTAAACCCCGCGACGGGGGCTGCGAGTCGGACTCGGGCTGCGGTCTCCCAGGAGGGAGTCAAGTTCCTTTATCGAGTAAGG AAAGTTGGTCCCAGCCTTGCATGCACCGAGTTTAGCCGTCAGAGGCAGCGTCGTGGGAGCTGCTCAGCTAGGAGTTTCAACCGATAAA |
| 75 | AIRE | TTCCGAACTCACACTTCTCTGAGTCCCGCACAGACCCAGTCTCTGTCCCCAGCCCCCAAGGCAGCTGCCCTCGTCCGTGAGTCAGGCCAGGCCCGGAG ACTTCCCGAGAGCGAGGGAGGGACAGCAGCGCCTCCATCACAGGGAAGTGTCCCTGCGGGAGGCCCTGGCCCTGATTGGGCGCCGGGGCGGAGCGGCC TTTGCTCTTTGCGTGGTCGCGGGGGTATAACAGCGGCGCGCGTGGCTCGCAGACCGGGGAGACGGGCGGGCGCACAGCCGGCGCGGAGGCCCCACAGC CCCGCCGGGACCCGAGGCCAAGCGAGGGGCTGCCAGTGTCCCGGGACCCACCGCGTCCGCCCCAGCCCCGGCTCCCCGCGCCCACCCCATGGCGACGG ACGCGGCGCTACGCCGGCTTCTGAGGCTGCACCGCACGGAGATCGCGGTGGCCGTGGACAG |
| 76 | SUMO | ACGCACACTGGGGGTGTGATGGAAAGGGGGACGCGATGGATAGGGGTGGCGCACACTGGGGGACGCGACGGGGACGGGTGAGCACACACTGGGGGTG TGATGGAGAGGGCGACGCAATAGGGAGGGGTGGCGCACACCAGGGACGCGATGATGGGGACGGTGGCGCACACCAGGTGGCATGATGGGGAGGAG |
| | 3 | TGGGTACACACCATGGGGGGCGTGATGGGGAGGCGTGGGCGTACACCGGGGGGCGCGATGGGGAGGGGTGGCGCACACCGGGGGACGCGATGGAGGC GGTGGGTGCACACGGGGCGCGATGGGTGGGAGTAGGTGCACACTGAGGGCACGATTGGGGAGACACGAAGGAGAGCGGTGGGCGCACACTGGGGGACG CGATGGCCGGGACACGATGCGGAGAAGTGGGTGAATACCGGGGTCGCGATGGGCGCCCTGGAAGGACGGCACTGCTGCTCACAGGGGCCAGGCCCCTC AGAGCGCGCCCCTTGGGGGTAACCCCAGACGCTTGTTCCCGAGCCGACTCCGTGCACTCGACACAGGATC |
| 77 | C21orf 70 | CCACAGGGTGGGGTGCGCCCACCTGCCCTGTCCATGTGGCCTTGGGCCTGCGGGGGAGAGGGAATCAGGACCCACAGGGCGAGCCCCCTCCGTAGCCC GCGGCACCGACTGGATCTCAGTGAACACCCGTCAGCCCATCCAGAGGCTAGAAGGGGGA |
| 78 | C21orf 123 | TTGAGGTCTCTGTGCATGCTTGTGCGTACCCTGGACTTTGCCGTGAGGGGTGGCCAGTGCTCTGGGTGCCTTTGCCAGACAACTGGTCTGCCGGGCCG AGCATTCATGCTGGTC |
| 79 | COL18 A1 | TGACGCGCCCCTCTCCCCGCAGCTCCACCTGGTTGCGCTCAACAGCCCCCTGTCAGGCGGCATGCGGCGCATCCGCGGGGCCGACTTCCAGTGCTTCC AGCAGG |
| 80 | PRRT3 | AACACACTGTCTCGCACTAGGTGCTCGCGGAAGAGCGCGGCGTCGATGCTGCGGCTCAGGTTGATGGGCGATGGCGGCCGCAGATCCAGCTCGCTCAG CGATGGCGCCGGTCCCACACCGTTGCGGGACAGTCCCGGGCCACCCTGGGGTCCGCGACCCAACGACGCAGCCGAGCCCCAGGCGCCTGAACTGGGCG TGGCCAGCTGCCCACTCTCCGCCGGGTTGCGGATGAGGCTCTTGCTGATGTCCAAGCTGCCTGCACCAACGTTGCTGGGCCCTGCATAGCAGTTATTG GGTCGCTCCGGCACCTCGCTCTTTCCTGACGGCGCCGGGCACGCCAGACGCATCAGCTTAGCCCAGCAAGCGTGCTCCGTGGGCGGCCTGGGTCTCGC GGCAGCCACCGCGGCCAACGCCAGGGCGAGCGCCCATGTCAGCTCCAGGAGGCGCAGCCAGAAGTGGACACCCCACCAGGCCCACGAGAAGCGGCCCA CGCGGCCTGGGCCCGGGTACAGCCAGAGCGCAGCCGCCAGCTGCAAGCCGCTAGCCAGCAGCCCCAGCGCGCCCGCCACAGCCAACAGCCGAGGGCCC GGGCTGGCATCCCAGCCCCGTGGGCCGTCCAGCAGGCGGCGACGGCACAGGCAGAGCGTGCCCAGAGCCAC |
| 81 | MGC29 506 | GTCTGCACGAAGCCCGCGGCGGCCTGCAGGGGGCCCAGCGACTCGTCCAGGGAACCGGTGCGCAGGAGCAGCCGGGGGCGCGGCGCGCCGGCCGCCCT TGGGGGACTCTGGGGCCGGGGGCGCAGCTCGATCTGACGCTTGGGCACTGTCCGGGGCCTGGCGGGCGCGGCGCCCTCCTCCAGAGCCACCTCCACAC ACTCGAACTGCGCTGGGGCGGCAGGACTTGGCCCACGGGGCCGCAGCTCTAGGTAGGTGGCCCAGCGGGAGCCACCATCGGGGACCTGGGACTGGCGT GGGACCGCGGCGGGAGACGCTGGCCCCGGCGGCAAGGGGCTGATGAAGGCCGGCTCCGTGAACTGTTGTTGCGCCTCGCGATCGTCTGCGCCGGAGCA GCCGAACAGGGGTCCGACGCCGAAGATGACTTCCATCTCCCCCGACGGCAGCGTGCGCAGCTGGGGCTGGGGTGGCCGTGGCCGGAACCTGGGCCTC GCGGGAAACCCGAGCCGGGCCCGTGCCGCTGGCGGCTATTCTGGGCGCTGACGGACAGGCGAGGCTGCGCGCCCGCCCCCCGCCCAGGAGCCACCCAG GGCCAATTCGCTGGGCCTTTCGCGTCCGGCCCAACGTCCGGGGGCTCCGGAGAACCTGGAGCCGTGTAGTAGGAGCCTGACGAACCGGAGGAGTCCTG GCGCCGCGCGGGGGCCGTGGGCAGCTGCCTCGGGATCCCAGGCAGGGCTGGCGGGGCGAGCGCGGTCAGCATGGTGGGGCCGGACGCCGTGCACTATC TCCCTCGCATTCGCCTCCGCTGGTGGCGC |
| 82 | TEAD3 | CTGGAGAGAACTATACGGGCTGTGGGAGTCACCGGGCGACTATCACCGGGCCTCCTTTCCACATCCTCCTCCGGGAAGGGACCCCGTTCCGGGCCTCG ACCGGCGCAGACTGGGCTGACCCACTTTCTTGGGCCCACTGAGTCACCTCGAAACCTCCAGGCCGGTAGCGCGGACGAGAGGAGGAGCAGGCGGGGGT GCCAAGGTGTGGGCTGCGCCCTGGTTAGGGGGCGAGCCCGGCTTGTTTATGAGGAGGAGCGCGGAGGAGGATCCAGACACACAGGCTTGCGCGCCCAG ACTCGCCCGGCCAGCGGCTGGCGGCCTCCGACGTCACCAAACCGGTTGGGTGAGAGGGCAGAGAGCAGGGGGAAGGGCCGCAGTCCCGCCCGCGCCCC CCGGCACGCACCGTACATCTTGCCCTCGTCTGACAGGATGATCTTCCG |
| 83 | chr12 group-00022 | GAGTGCGGAGTGAAGGGGTGCACTGGGCACTCAGCGCGGCCCTTGGGAGGCAGGGCCGCCCCAGCCTGCCCTCCTGTCTGGAAGGCCGTCCAGAAGC AGGAGCCCCGGGGAAAACAACTGGCTGGACGGGGCGGCCTTCAGTGTCTCTCCCAGCCTGAGAGTCGCTTCCCACCACCTGGGCACGAACCTGCTCTG CGATCTCCGGCAAGTTCCTGCGCCTCCTGTCGGTAAAATGCAGATCGTGGCGTCTT |
| 84 | CENTG 1 | TCTTCTTTCCGCCCCTAGGGGGCACAAGCGGGCATGTCCAAGCGCCTAGGAGCCCGTACCGCTGGGGACCTCCCCTTCCGCGAACCCCGAGCGGGTAG ACCCAGAGCAATCCGAGTGTGGAAACAATGGAGAGGGGGCGTGTTGAGCTGGGGTCTCCATGCCTCGTTGGGGAGAGGGAGGTGAGTTTGTGTCTTCT GGAAGGCGTGGGGGCTGTGCCCTCGTGGGGGTAGGAAGTGCTCCCGTGGGGCGGGGTGCGGATCGGAGAGGTGAGTGGGTGCGTCTGTCCAGCGGTCC GCCCGGTGTGGTCGTGCCCGGCCCGCGTGGGGATGGGGGTGTCTCTCCCGCTGGGCAACTATACCAGCGCAACCGGGGCGTCGGCGCGGCCCACGCTA GCGGCGCTGCTCCGGCGGCGGGGGCTGGGCGTGGCGGTGATGCTGGGCGTGGTGGCCGCGCTGGGCGTGGTGGCCGCGCTGCCGCCCTCACCCGGGCA GCCGTGCTGGAGAAGGATGTCGGCGCACAGCTGGCTTCCAGCCTGGCGGGCGTAGAACAGCGCCGTGCGGCCCTGGGCGTCACGGGCCGCCACGTCCG CGCCGTACTAGAGGGCGGAAACGGCCGCGTGACCGCGCGTCCCCAGGGCGCCCACACCCGGCGCCGCCTCCCCCACATGGCCAAGCCTACTTCCGGGG TCCCTCTGGGAATTTCGGGCTTTCCCGCGCCAGGCGTTTTCCGAGATGAAGCCTCAAAGACCCCCTTTCCTCCCCCCAGCTCACGTACCCACAGCAGC AGTTGCGTGATGACGACGTGGGCGAGCTCGGCCGCCAGGTGGAGTGGGGAGCGCAGCTGTGGGTCCTCTACGCTGGTGTCGAGCGGCCCGTGTCGCGC ATGGGCCAAAAGCAGGAGAACGGTAGCCACGTCCTGGGCCTGCACGGCGGCCCACAGCTGGCGGCCCAGCGGCTCCTCCGAGGTGCTCAGCGGCGCCA GGAACAGTAGCTGCTCGTACTTGGCGCGAATCCACGACTCGCGCTCCTCCCTGCAAGACCAGGGATCAACGGAAAAGGCTCTAGGGACCCCCAGCCAG GACTTCTGCCCCTACCCACGGGACCGTCTCAGGTTCGCACACCCTCAGCAACCCTCCCCCCGCTCTGTTCCCTCACGCTTACCGCGAAGAGTCCCGCG AGGGCTTGGCACGGCCTCGCGTGTCGCTTTCCCACACGCGGTTGGCCGTGTCGTTGCCAATAGCCGTCAGCACCAGGGTCAGCTCCCGTGGCCAGTCG TCCAAGTCCAGCGAGCGAACGCGGGACAGGTGTGTGCCCAGGTTGCGGTGGATGCCAGAACACTCGATGCAGATGAGGGCGCCCAGGTTCAAGCTGGC CCACGTGGGGTCTGCGGAAGGAGCGTAGAGGTCGGCTCCCAGCCGGGCAGCACAGGCACCCCGGCATTCACTACACTCCCTAGCCCCTCCGCTGCCTC CTGGCACTCACTGGGGGCCCCGCAGTCCACGCAGATTGAATTCCCCTTGGCGTTCCGGATCGCCTGGAT |
| 85 | CENTG 1 | AGCCAGGTCCAGCCCCCGCGCCTGACACCGGCCGGACGTTCCCGGGGCGCCGCAGCTGCGGCGGGAACTCTGGGATCCGGAGCCATCTGCTCCCACCC GCTCCGGAGCCAAACCCCGGGGGCCGCCTCCGCTCCCGGACCCGCCTCCTCTCCCGGGAGTGTGAGCCGAACCAAGAGTCTCCTGCCTATCTCCTCCA GTAGGAAAATAGTAATAATAATAGACACCCTGCCCCCGTAAAAACACTACCTTCCCCGTACCGCCTCCCAAGTCTCCCGGGGTACGGATTGCCTTTG CAGCAGTTCCGCCCCACCTGACTCACTCCAGGGTCAGCCCCGGGTGGGTTTCAATGCGGCTCTGGGGAGGGGGTGGGCAGTGGGGGAAGTGAGGCTTC CTATCCGCCCCCTCTCACTTCACATTTAAATATTCTGCACGTTCCAGCCCCCGCGGACTCGCGTACCGCCCAATCCGCCTTCACCGCACGAAAAACAT CACTAGCCTGCTCTCAGCCCAGGGGACGACTAGTCCCTGGCGAGAAGCTGCCTGCAAGGTCACTGTCATGCCACCTGCCCCAAGTGCTCAGGGGAAAC TGAGGCTTCCTCATCCCCTTCACCTTCAACGTCGCTCTAAACACGGCAAAGCCCCGTTTCCATGCTCCCAGAGTTCAGCTGAGGCTGGAAGTGGGGTC CTGGGCTTCTCTGGGAGCAATTTTCTAGTCACTCTGATCAAGGACGTTACTTTCCCAGAAAGCTCTGAGGCTGAGTCCCTCTGAAATCAAGTCCTTTC TCCTGTCGCACAATGTAGCTACTCGCCCCGCTTCAGGACTCCTATTCTTTGCCCCAATCCTTGACAGAGGGGTGAGCTTGGTTCATCCGCCCACCCCA GAGAAAAGCTTCCCTAGTTTCCTGGACCTCGCTCCTCCACCCCAAGCTGAGCATTCCAGGTACCCTTCCCTCCCTGTTCTCAAGCCCTGACTCAACTC ACTAGGGGAAGCGCGGAGCTCGGCGCCCAGCAGCTCCCTGGACCCGCTGCCAGAAGACAGGCTGGGGGGTCCGGGAAGGGGCCCGGAGCCAGGAGGCC CTCCTGTGCTCTTGGTGAAGATGCCGCTGATAAACTTGAGCATCTTGCGGTCACGAGTGGATGCTCGGCCCCCCTCCCGGCCCCGTTTCAGCCCCGGA GCTGGAGGCTCCAGAGTGATTGGAGGTGCAGGCCCGGGGGGCTGCGCGGAAGCAGCGGTGACAGCAGTGGCTGGACTCGGAGTTGGTGGGAGGGTTAG |
| | | CGGAGGAGGAGAGCCGGCAGGCGGTCCCGGATGCAAGTCACTGTTGTCCAAGGTCTTACTCTTGCCTTTCCGAGGGGACAACTTCCCTCGGGCTCCAG CCCCAGCCCCGACCCCACCAGAGGTCGAAGCTGTAGAGCCCCCTCCCCCGGCGGCGGCGGCGGTGGCGGCGGCAGAGACCGAAGCTCCAGTCCCGGCG CTGCTCTTTGACCCCTTGACCCTGGGCTTGCCCTCGCTTTCGGGCCATGACAGGCGGCTACCCGCGCCCTTGCCCCCGCCGGCTTTGGCTCCACTCGT GGTCACGGTCTTGCAAGGCTTGGGAGCCGGCGGAGGAGGCGCCACCTTGAGCCTCCGGCTGCCGGTGCCAGGGTGCGGAGAGGATGAGCCAGGGATGC CGCCGCCCGCCCGGCCTTCGGGCTCCGGGCCGCCCCAGCTCGGGCTGCTGAGCAGGGGGCGCCGGGAGGAGGTGGGGGCGCCCCCAGGCTTGGGGTCG GGGCTCAGTCCCCCGGAGAGCGGGGGTCCCGGAGGGACGGCCCAGAGGGAGAGGCGGCGGCCGGGAGCGGGGGAGACTGGGCGGGCCGGACTGGCCGG AGCCGGGGACAGGGCTGGGGGCTCCGCGCCCCCGGTGCCCGCGCTGCTCGTGCTGATCCACAGCGCATCCTGCCGGTGGAAGAGACGTTCGTGCCGCT TCTTGCCCGGCTCCTCCGCGCCTCGGGGGCTGCCAGGATCCCCAGTCTCGGAGCCTCTGGCACCGGCGGCGCCGGCCGCGGCCGCAGACGGAGAAGGC GGCGGCGGAGGCACCGACTCGAGCTTAACCAGGGTCAGCGAGATGAGGTAGGTCGTTGTCCGGCGCTGAAGCGCGCCCGCGCCCCGGCTCATGGGGCC CGGAGACCCCCGAGCTGGGGAGGGGAGGGGACTCCCCCGGACTGCCTCAGGGGGGCCCGGCCATGGGGCCGCCCTGCTCGCTGCCCCCAGCCCCCGGA CCCCGCTGAGCCCCCGGCCCGGCTCCGCTGTCGCCGCCGCCTCCGCCGCCTCCGCTTGCGCCCCCTCCCATCACATGGGGCGCCCCCTCCCCATGCT CCCCGCCCTGCGCCCCCACCCTCTTGGAGCCCCGGGACCTTGGTGCTGCTCCAGGGAGGCGCGCCGGACCGTCCACCCCGGCCTGGGTGGGGGCGCTG AGATGGGTGGGGGAGGCGGGGAGGACAGTAGTGGGGGCAAATGGGGGAGAGAGAGGAAAAGGGAGCAGAAAAGGGGACCGGAGGCTAGGGGAAACGA ACCTGTGCGGGGGAGGCAGGGGCGGGGAATTGGGACTCAAGGGACAGGGGCCGCGGATGCGGTCGGAAAGAGGGTCTAGAGGAGGGTGGGAAGCTAGT GG |
| 86 | chr18 group-00304 | AGGAGCGCAAGGCTTGCAGGGCATGCTGGGAGAGCGCAGGGAACGCTGGGAGAGCGCGGGAAATACTCGGATTGGCTCCCGAGGGCTGTGAGGAGGGC ACGAGGGGACACTCCGATGAAGGCAGGGCACGCGGGGCGAGCCGGGAGCGTCTCCTGAGGGCAGCGACGAGGGAGCTGAGGCACGCGGGCTCTCAATC GACGCCCCACAGAGACCAAGAGGCCTGGCCTTGGGGGGCAGCTGCTTGAAGGAGGCAGAGCGGAAGCGAGGGAGACTGCTGGAGGCCCTGCCGCCCAC CCGCCCTTTCCTCCCCCTGAGGAGACGCCTGACGCATCTGCAGTGCAGGAGGCCGTGGGCGTTAGAAGTGTTGCTTTTCCAGTTTGTAAGACCATTTT CCTGATTCTCTTCCCCACGGTTGCGGAGGAGCAGGTCAGGGCCGCCATGAGGGCAGGATC |
| 87 | TSHZ1 | TCGACCGCTACTATTATGAAAACAGCGACCAGCCCATTGACTTAACCAAGTCCAAGAACAAGCCGCTGGTGTCCAGCGTGGCTGATTCGGTGGCATCA CCTCTGCGGGAGAGCGCACTCATGGACATCTCCGACATGGTGAAAAACCTCACAGGCCGCCTGACGCCCAAGTCCTCCACGCCCTCCACAGTTTCAGA GAAGTCCGATGCTGATGGCAGCAGCTTTGAGGAGGC |
| 88 | CTDP1 | TGTGCCGTCGCACACAGACGCCCTCAACGTCGGAGAGCTGTGAGCGGGGCCGTGCTCTTGGGATGGGAGCCCCCGCGAGAGCTGCCCGCCAACACCAC TCCGACGTGATCCATGCTGGACATAAAGTGCTCTTCCCTCCGCTAGTCATCGGCCGAGCGGGCCCCTCGCTCCTGGGTGTAAGTTCTTTCTGTGCGTC CTTCTCCCATCTCCGTGCAGTTCAG |
| 89 | KCNG2 | CCATGCGCCGCTGCGCGCGCGAGTTCGGGCTGCTGCTGCTGTTCCTCTGCGTGGCCATGGCGCTCTTCGCGCCACTGGTGCACCTGGCCGAGCGCGAG CTGCGCGCGCGCCGCCCACTTCTCCACCCTGCCCCGCCAGCTATTGCTCGCCCCGTCATCTCCATCACCACCCTCGCCTACCGCCCACATCGTCCCGCCCAC CCTGCCCGGGCAGGTGGTGGCGCTCAGCAGCATCCTCAGCGGCATCCTGCTCATGGCCTTCCCGGTCACCTCCATCTTCCACACCTTTTCGCGCTCCT ACTCCGAGCTCAAGGAGCAGCAGCAGCGCGCGGCCAGCCCCGAGCCGGCCCTGCAGGAGGACAGCACGCACTCGGCCACAGCCACCGAGGACAGCTCG CAGGGCCCCGACAGCGCGGGCCTGGCCGACGACTCCGCGGATGCGCTGTGGGTGCGGGCAGGGCGCTGACGCCTGCGCCGCCCAC |

The entirety of each patent, patent application, publication and document referenced herein hereby is incorporated by reference. Citation of the above patents, patent applications, publications and documents is not an admission that any of the foregoing is pertinent prior art, nor does it constitute any admission as to the contents or date of these publications or documents.

Modifications may be made to the foregoing without departing from the basic aspects of the invention. Although the invention has been described in substantial detail with reference to one or more specific embodiments, those of ordinary skill in the art will recognize that changes may be made to the embodiments specifically disclosed in this application, yet these modifications and improvements are within the scope and spirit of the invention.

The invention illustratively described herein suitably may be practiced in the absence of any element(s) not specifically disclosed herein. Thus, for example, in each instance herein any of the terms "comprising," "consisting essentially of," and "consisting of" may be replaced with either of the other two terms. The terms and expressions which have been employed are used as terms of description and not of limitation, and use of such terms and expressions do not exclude any equivalents of the features shown and described or portions thereof, and various modifications are possible within the scope of the invention claimed. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%). Thus, it should be understood that although the present invention has been specifically disclosed by representative embodiments and optional features, modification and variation of the concepts herein disclosed may be resorted to by those skilled in the art, and such modifications and variations are considered within the scope of this invention.

Certain embodiments of the invention are set forth in the claims that follow.

The invention furthermore comprises the following items:
1. A method for preparing fetal nucleic acid, which comprises:
   a) providing a sample from a pregnant female;
   b) separating fetal nucleic acid from maternal nucleic acid from the sample of the pregnant female according to a different methylation state between the fetal nucleic acid and the maternal nucleic acid counterpart, wherein the fetal nucleic acid comprises one or more CpG sites from one or more of the polynucleotide sequences of SEQ ID NOs: 1-89; and
   c) preparing nucleic acid comprising fetal nucleic acid by a process in which fetal nucleic acid separated in part (b) is utilized as a template.
2. The method of item 1, wherein the fetal nucleic acid is separated from the maternal nucleic acid by an agent that specifically binds to methylated nucleotides.
3. The method of item 2, wherein the agent that binds to methylated nucleotides is a methyl-CpG binding protein (MBD) or fragment thereof.
4. The method of item 2, wherein the agent that binds to methylated nucleotides binds to methylated fetal nucleic acid.
5. The method of item 2, wherein the agent that binds to methylated nucleotides binds to methylated maternal nucleic acid.
6. The method of item 2, wherein the fetal nucleic acid is separated from the maternal nucleic acid by an agent that specifically binds to non-methylated nucleotides.
7. The method of item 1, wherein the fetal nucleic acid is separated from the maternal nucleic acid by an agent that specifically digests non-methylated maternal nucleic acid.
8. The method of item 7, wherein the agent that specifically digests non-methylated maternal nucleic acid is a methyaltion-sensitive restriction enzyme.
9. The method of item 8, wherein two or more methyaltion-sensitive restriction enzymes are used in the same reaction.
10. The method of item 1, wherein the process of step c) is an amplification reaction.
11. The method of item 1 wherein the process of step c) is a method for determining the absolute amount of fetal nucleic acid.
12. The method of item 1, wherein three or more of the polynucleotide sequences of SEQ ID NOs: 1-89 are prepared.
13. A method for determining the absolute amount of fetal nucleic acid in a maternal sample, wherein the maternal sample comprises differentially methylated maternal and fetal nucleic acid, comprising:
   a) digesting the maternal nucleic acid in a maternal sample using one or more methylation sensitive restriction enzymes, thereby enriching the fetal nucleic acid; and
   b) determining the absolute amount of fetal nucleic acid from step a) using a non-polymorphic-based and non-bisulfite-based quantitative method.
14. The method of item 13, wherein the absolute amount or concentration of fetal nucleic acid is used in conjunction with a diagnostic method to determine a fetal trait, wherein the diagnostic method requires a given absolute amount or concentration of fetal nucleic acid to meet certain clinical sensitivity or specificity requirements.
15. A method for determining the concentration of fetal nucleic acid in a maternal sample, wherein the maternal sample comprises differentially methylated maternal and fetal nucleic acid, comprising:
   a) determining the total amount of nucleic acid present in the maternal sample;
   b) digesting the maternal nucleic acid in a maternal sample using a methylation sensitive restriction enzyme thereby enriching the fetal nucleic acid;
   c) determining the amount of fetal nucleic acid from step b) using a non-polymorphic-based and non-bisulfite-based quantitative method; and
   d) comparing the amount of fetal nucleic acid from step c) to the total amount of nucleic acid from step a), thereby determining the concentration of fetal nucleic acid in the maternal sample.
16. The method of item 15, wherein the absolute amount or concentration of fetal nucleic acid is used in conjunction with a diagnostic method to determine a fetal trait, wherein the diagnostic method requires a given absolute amount or concentration of fetal nucleic acid to meet certain clinical sensitivity or specificity requirements.
17. A method for determining the presence or absence of a fetal aneuploidy using fetal nucleic acid from a maternal sample, wherein the maternal sample comprises differentially methylated maternal and fetal nucleic acid, comprising:
   a) digesting the maternal nucleic acid in a maternal sample using a methylation sensitive restriction enzyme thereby enriching the fetal nucleic acid;
   b) determining the amount of fetal nucleic acid from a target chromosome using a non-polymorphic-based and non-bisulfite-based quantitative method;
   c) determining the amount of fetal nucleic acid from a reference chromosome using a non-polymorphic-based and non-bisulfite-based quantitative method;
   d) comparing the amount of fetal nucleic acid from step b) to step c), wherein a statistically significant difference between the amount of target and reference fetal nucleic acid is indicative of the presence of a fetal aneuploidy.
18. The method of item 17, wherein the amount of fetal nucleic acid at between 3 and 15 loci on each of the target chromosome and reference chromosome is determined.
19. The method of item 13, 15 or 17, wherein the digestion efficiency of the methylation sensitive restriction enzyme is determined.
20. The method of item 13, 15 or 17, wherein a non-polymorphic-based and non-bisulfite-based method for performing said quantification uses a competitor-based method to determine the amount of fetal nucleic acid.
21. The method of item 13, 15 or 17, wherein the method further comprises determining the presence or absence of Y-chromosome nucleic acid present in a maternal sample.
22. The method of item 21, wherein the amount of Y-chromosome nucleic acid present in a maternal sample is determined for a male fetus.
23. The method of item 22, wherein the amount of fetal nucleic acid is compared to the amount of Y-chromosome nucleic acid.
24. The method of item 13 or 15 , wherein the amount of fetal nucleic acid at 2, 3, 4, 5, 6, 7, 8, 9, 10, 15, 20, 30, 40, 50 or more loci is determined.
25. The method of item 13 or 17, wherein the total amount of nucleic acid present in a maternal sample is determined.
26. The method of item 13, 15 or 17, wherein the total amount of nucleic acid and the amount of Y-chromosome nucleic acid for a male fetus are determined.
27. The method of item 13, 15 or 17, wherein the total amount of nucleic acid, the amount of Y-chromosome nucleic acid for a male fetus, and the digestion efficiency of the methylation sensitive restriction enzyme are all determined.
28. The method of item 27, wherein two or more assays are used to determine the total amount of nucleic acid, one or more assays are used to determine the amount of Y-chromosome nucleic acid for a male fetus, and one or more assays are used to determine the digestion efficiency of the methylation sensitive restriction enzyme.
29. The method of item 28, wherein the amount of fetal nucleic acid at 3 or more loci is determined.
30. The method of item 13, 15 or 17, wherein the amount of fetal nucleic acid is determined by an amplification reaction that generates amplicons larger than the average length of the digested maternal nucleic acid, thereby further enriching the fetal nucleic acid.

## Claims

1. A method for determining the presence or absence of a fetal aneuploidy using fetal DNA from a maternal sample, wherein the maternal sample comprises differentially methylated maternal and fetal DNA, comprising
a) selectively digesting the maternal DNA in a maternal sample using one or more methylation sensitive restriction enzymes thereby enriching the fetal DNA;
b) determining the amount of fetal DNA from a target chromosome;
c) determining the amount of fetal DNA from a reference chromosome; and
d) comparing the amount of fetal DNA from step b) to step c),
wherein a biologically or statistically significant difference between the amount of target and reference fetal DNA is indicative of the presence of a fetal aneuploidy.

2. The method of claim 1, wherein determining the amount of fetal DNA in steps b) and c) is done by using a non-polymorphic-based and non-bisulfite-based quantitative method.

3. The method of claim 1 or 2, wherein determining the amount of fetal DNA in steps b) and c) is done by introducing one or more competitors at known concentrations.

4. The method of claim 1, wherein determining the amount of fetal DNA in steps b) and c) is done by RT-PCR, sequencing or counting.

5. The method of any one of claims 1 to 4, wherein the amount of fetal DNA from a target chromosome determined in step b) is compared to a standard control.

6. The method of claim 5, wherein the standard control is the amount of fetal DNA from a target chromosome from euploid pregnancies.

7. The method of any one of claims 1 to 6, wherein the restriction efficiency is determined and used to further determine the amount of fetal DNA from a target chromosome and from a reference chromosome.

8. The method of any one of claims 1 to 7, wherein the differentially methylated maternal and fetal DNA is selected from the sequences of SEQ ID NOs: 1-89.

9. The method of any one of claims 1 to 7, wherein the differentially methylated maternal and fetal DNA is selected from the sequences of SEQ ID NOs: 1-59.

10. The method of any one of claims 1 to 7, wherein the differentially methylated maternal and fetal DNA is selected from the sequences of SEQ ID NOs: 60-85.
